# EUROPEAN PATENT APPLICATION

(11) **EP 4 215 526 A1**
(43) Date of publication of application: **26.07.2023**
(21) Application number: 21868750.7
(22) Date of filing: 18.09.2021
(51) Int. Cl.: C07D 403/14, C07D 471/10, A61K 31/395, A61P 3/00, A61P 39/00

(54) **CARBONYL HETEROCYCLIC COMPOUND AND APPLICATION THEREOF**

(30) Priority: 18.09.2020 CN 202010989302; 10.02.2021 CN 202110184789
(71) Applicant: Shanghai Pharmaceuticals Holding Co., Ltd., Shanghai 201203 (CN)
(72) Inventor: ZHANG, Chaoxin, Shanghai 201203 (CN); XIA, Guangxin, Shanghai 201203 (CN); XIANG, Zhijun, Shanghai 201203 (CN); KE, Ying, Shanghai 201203 (CN); LOU, Jiangsong, Shanghai 201203 (CN); ZHAO, Menghao, Shanghai 201203 (CN); HAO, Lijun, Shanghai 201203 (CN)
(74) Representative: Dehns
(86) International application number: PCT/CN2021/119377
(87) International publication number: WO 2022/057928

(57) **Abstract**

Provided is the carbonyl heterocyclic compound shown in formula (I) or a pharmaceutically acceptable salt thereof, capable of being used as a compound having a targeted lanthionine synthetase C-like protein 2 pathway and being used for the treatment of various conditions, including infectious diseases, autoimmune diseases, diabetes, and chronic inflammatory diseases.

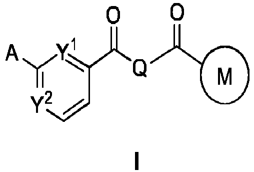

## Description

The present application claims priorities of Chinese Patent Application 2020109893021 filed on September 18, 2020 and Chinese Patent Application 2021101847890 filed on February 10, 2021. The contents of the Chinese Patent Applications are incorporated herein by reference in their entireties.

### TECHNICAL FIELD

The present disclosure relates to a carbonyl heterocyclic compound and application thereof.

### BACKGROUND

Lanthionine C-like protein 2 (LANCL2) (also known as "lanthionine synthetase C-like protein 2" or "lanthionine synthetase component C-like protein 2") is a signaling pathway protein expressed in immune cells, gastrointestinal tracts, neurons, testes, and pancreas. Activation of the LANCL2 pathway increases insulin sensitivity and reduces inflammation associated with various autoimmune, inflammatory and metabolic conditions. The results of *in vivo* and *in vitro* tests in mice demonstrate that the use of compounds targeting this pathway results in a 2-fold reduction in glucose levels in glucose tolerance tests compared to control group and provides an effective treatment in prescription AVANDIA^{®} (GlaxoSmithKline plc, Brentford, England), but with significant side effects. Targeting the LANCL2 pathway also results in a 90% reduction in intestinal inflammation and a corresponding 4-fold reduction in the number of lesions. The results of this test and other validations of the pathway have been mentioned in several articles.

Within the category of autoimmune-related inflammation, there is currently a global pandemic of autoimmune disorders such as inflammatory bowel disease (IBD), systemic lupus, rheumatoid arthritis, type 1 diabetes, psoriasis, and multiple sclerosis. There is also a pandemic of chronic metabolic inflammatory diseases, including metabolic syndrome, obesity, prediabetes, cardiovascular disease, and type 2 diabetes. Current treatments are moderately effective, but expensive and have serious side effects. For the most effective treatment of autoimmune diseases (e.g., anti-TNF antibodies), the route of administration is by IV or subcutaneous injections, thus requiring clinic visits/surgery and frequent monitoring. The unique mode of action of LANCL2 provides an orally administered therapeutic agent that is as effective as anti-TNF antibodies but without the side effects and high cost. Given the overall prevalence of inflammatory and autoimmune diseases, the LANCL2 pathway has the potential to significantly impact millions of patients.

Abscisic acid ("ABA") is a natural compound bound to LANCL2 discovered during the original screening process.

A large number of compounds are described in the field of synthetic organic chemistry. Various compounds are provided by the following references: WO1997/036866 by Diana et al., WO2006/053109 by Sun et al., WO2006/080821 by Kim et al., WO2007/019417 by Nunes et al., WO2009/067600 and WO2009/067621 by Singh et al., WO2008/079277 by Adams et al., JP2008/056615 by Urasoe et al., WO2011/066898 by Stoessel et al., US2013/0142825 by Bassaganya-Riera et al., and US Patent 7,741,367 by Bassaganya-Riera et al. International patent application WO2016064445 discloses a compound that targets the lanthionine synthetase C-like protein 2 pathway, and the compound can be used for the treatment of various conditions, including infectious diseases, autoimmune diseases, diabetes, and chronic inflammatory diseases.

Some of the compounds described in these references are known to activate the LANCL2 pathway, while others do not. Novel ligands for the LANCL2 pathway need to be developed to allow treatments of individual diseases specifically and potentially maximize their efficacy.

### CONTENT OF THE PRESENT INVENTION

The technical problem to be solved by the present disclosure is to overcome the lack of therapeutic agents based on lanthionine synthetase C-like protein 2 in the prior art; and provide a carbonyl heterocyclic compound and application thereof. The carbonyl heterocyclic compound provided by the present disclosure is a compound targeting lanthionine synthetase C-like protein 2 pathway; the compound can bind to LANCL2 protein and achieve beneficial reactions in various disease conditions, and it can be used for the treatment of various conditions, including metabolic and infectious diseases, autoimmune diseases, diabetes, and chronic inflammatory diseases.

The present disclosure solves the above technical problem by the following technical solutions.

The present disclosure provides a carbonyl heterocyclic compound represented by formula I or a pharmaceutically acceptable salt thereof; 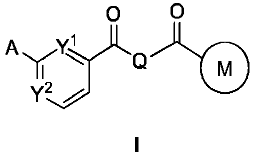
wherein, A is 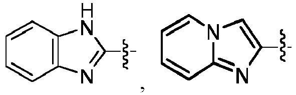 or -NR¹R²;
R¹ and R² are independently H or C₆₋₁₈ aryl;
Y¹ and Y² are independently CH or N;
Qis 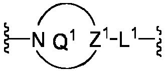 (including 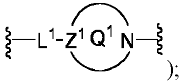
Z¹-L¹- is 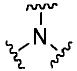 (i.e., L¹ is a linking bond) or 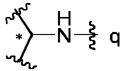 (q-terminus indicates a connection to carbonyl);
the carbon atom with "^{∗}" indicates that when the carbon atom is a chiral carbon atom, it is S-configuration, R-configuration or a mixture of both;
ring Q' ( 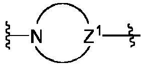 ) is 6- to 10-membered fused heterocycloalkyl, 6- to 12-membered spiro heterocycloalkyl or 6- to 10-membered bridged heterocycloalkyl; Q' contains 1 to 3 N atoms;
M is 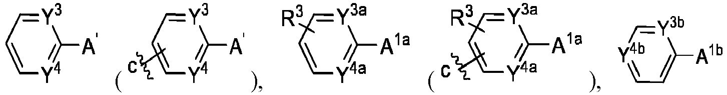 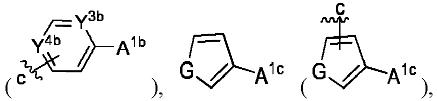 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 5- to 10-membered cycloalkyl substituted by R⁵; the heteroatoms in the 6- to 10-membered fused heterocycloaryl, the heteroatoms in the 6- to 10-membered fused heterocycloaryl substituted by R⁴, the heteroatoms in the 5- to 10-membered heterocycloalkenyl and the heteroatoms in the 5- to 10-membered heterocycloalkenyl substituted by oxo are independently one or more than one of N, S and O, and the number of heteroatoms is independently 1, 2 or 3; the number of R³ is 1, 2 or 3; (c-terminus indicates a connection to the C=O as shown)
G is S or O;
A', A^{1a}, A^{1b} and A^{1c} are independently 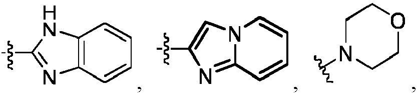 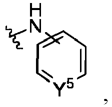 NO₂, 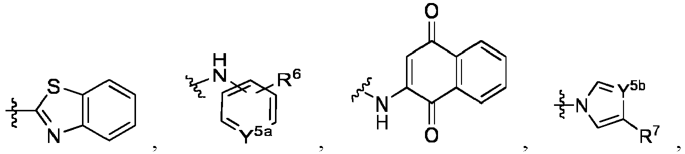 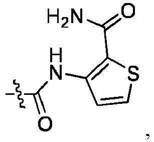 -OR⁸, C₆₋₁₄ aryl or H;
Y³, Y^{3a}, Y^{3b}, Y⁴, Y^{4a}, Y^{4b}, Y⁵, Y^{5a}, Y^{5b} and Y^{5c} are independently CH or N;
R⁶ is halogen;
R⁷ is H or C₁₋₆ alkyl;
R⁸ is C₆₋₁₄ aryl;
R³ is C₁₋₆ alkyl or halogen;
R⁴ is C₁₋₆ alkyl;
R⁵ is C₆₋₁₄ aryl or C₆₋₁₄ aryl substituted by halogen.

In certain preferred embodiments of the present disclosure, some groups of the carbonyl heterocyclic compound represented by formula I, the pharmaceutically acceptable salt thereof, the solvate thereof or the salt of the solvate thereof are defined as follows (unmentioned groups are as described in any one of the embodiments of the present disclosure), hereinafter referred to as in certain preferred embodiments of the present disclosure.

In certain preferred embodiments of the present disclosure, 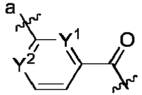 is 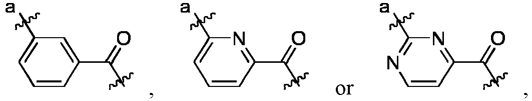 for example, 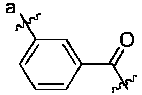 or 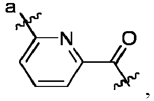 for another example, 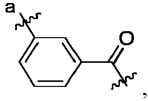 (a-terminus indicates the position connected to A).

In certain preferred embodiments of the present disclosure, when R¹ and R² are independently C₆₋₁₈ aryl, the C₆₋₁₄ aryl is phenyl, naphthyl, phenanthryl or anthracenyl, for example, phenyl.

In certain preferred embodiments of the present disclosure, when Q' is 6- to 10-membered fused heterocycloalkyl, the 6- to 10-membered fused heterocycloalkyl is 6- to 8-membered fused heterocycloalkyl, containing 1 or 2 N atoms (e.g., the ring atoms other than the N atom connected to carbonyl and Z' are all carbon), and can also be heterocycloalkyl of 5-membered N-heterocycloalkyl fused with 3-membered cycloalkyl or heterocycloalkyl of 5-membered N-heterocycloalkyl fused with 5-membered N-heterocycloalkyl, for example,

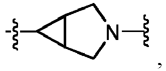

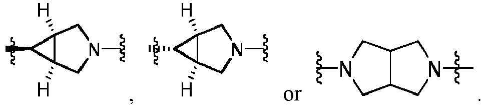

In certain preferred embodiments of the present disclosure, when Q' is 6- to 12-membered spiro heterocycloalkyl, the 6- to 12-membered spiro heterocycloalkyl is 7- to 11-membered spiro heterocycloalkyl, containing 1 or 2 N atoms (e.g., spiro[5,5]undecane heterocycloalkyl, spiro[5,4]decane heterocycloalkyl, spiro[4,4]nonane heterocycloalkyl, or spiro[3,3]heptane cycloalkyl), and can also be 9- to 11-membered spiro heterocycloalkyl, containing 1 or 2 N atoms (e.g., the ring atoms other than the N atom connected to carbonyl and Z^{1a} are all carbon), and can also be azaspiro[5,5]undecane heterocycloalkyl, azaspiro[5,4]decane heterocycloalkyl, or azaspiro[4,4]nonane heterocycloalkyl, for example, 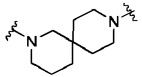 (for another example, 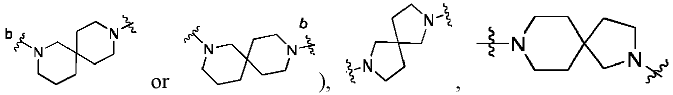 (for another example, 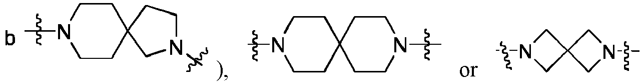 (b-terminus indicates a connection to

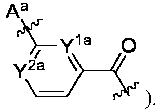

In certain preferred embodiments of the present disclosure, when Q' is 6- to 10-membered bridged heterocycloalkyl, the 6- to 10-membered bridged heterocycloalkyl is 7-membered bridged heterocycloalkyl, containing 1 or 2 N atoms (e.g., the ring atoms other than the N atom connected to carbonyl and Z' are all carbon), for example,

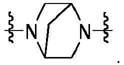

In certain preferred embodiments of the present disclosure, Y³, Y^{3a}, Y^{3b}, Y⁴, Y^{4a}, Y^{4b} and Y^{5b} are independently CH or N.

In certain preferred embodiments of the present disclosure, the number of R³ is 1, 2 or 3; for example, 1 or 2.

In certain preferred embodiments of the present disclosure, when M is

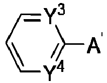

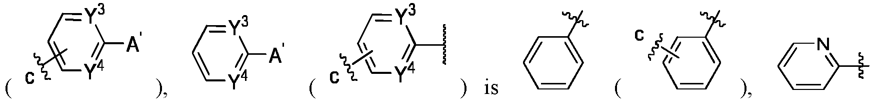

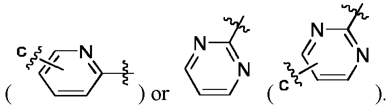

In certain preferred embodiments of the present disclosure, when M is

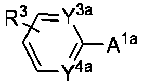

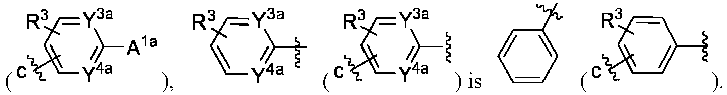

In certain preferred embodiments of the present disclosure, when M is

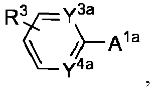

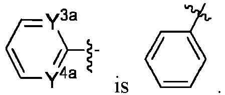

In certain preferred embodiments of the present disclosure, when R³ is C₁₋₆ alkyl, the C₁₋₆ alkyl is methyl, ethyl, n-propyl, isopropyl, n-butyl, isobutyl, sec-butyl or tert-butyl; for example, methyl.

In certain preferred embodiments of the present disclosure, when R³ is halogen, the halogen is F, Cl, Br or I, for example, F or Cl.

In certain preferred embodiments of the present disclosure, when M is

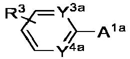

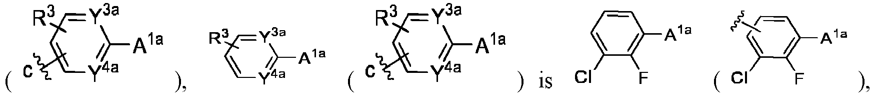

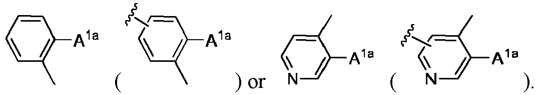

In certain preferred embodiments of the present disclosure, when M is

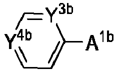

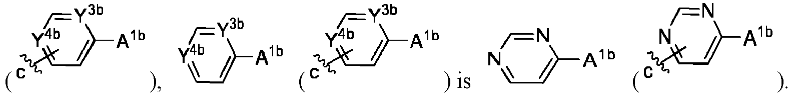

In certain preferred embodiments of the present disclosure, when M is

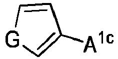

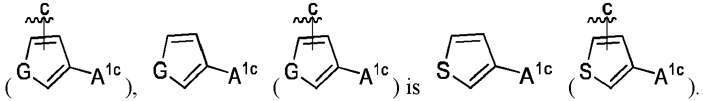

In certain preferred embodiments of the present disclosure, when M is 6- to 10-membered fused heterocycloaryl or 6- to 10-membered fused heterocycloaryl substituted by R⁴, the 6- to 10-membered fused heterocycloaryl is independently 9- to 10-membered fused heterocycloaryl, the heteroatom is N and/or S, and the number is 1 or 2 (e.g., heterocycloaryl of 5-membered N-heteroaryl fused with phenyl, heterocycloaryl of 6-membered N-heteroaryl fused with phenyl, heterocycloaryl of 5-membered N-heteroaryl fused with 6-membered N-heteroaryl), for example, indolyl 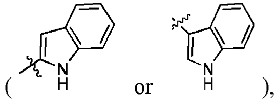 quinolinyl 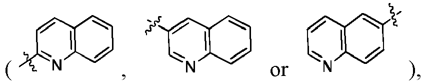 isoindolyl 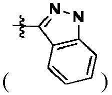 or imidazopyridyl

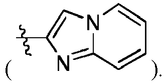

In certain preferred embodiments of the present disclosure, when R⁴ is C₁₋₆ alkyl, the C₁₋₆ alkyl is methyl, ethyl, *n*-propyl, isopropyl, *n*-butyl, isobutyl, *sec*-butyl or *tert*-butyl; for example, methyl.

In certain preferred embodiments of the present disclosure, when M is 6- to 10-membered fused heterocycloaryl substituted by R⁴, the 6- to 10-membered fused heterocycloaryl substituted by R⁴ is

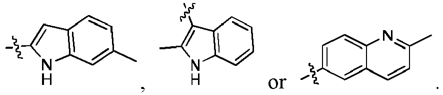

In certain preferred embodiments of the present disclosure, when M is 5- to 10-membered heterocycloalkenyl or 5- to 10-membered heterocycloalkenyl substituted by oxo, the 5- to 10-membered heterocycloalkenyl is 6-membered heterocycloalkenyl, the heteroatom is N, the number is 2, for example,

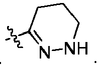

In certain preferred embodiments of the present disclosure, when M is 5- to 10-membered heterocycloalkenyl substituted by oxo, the 5- to 10-membered heterocycloalkenyl substituted by oxo is

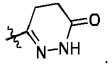

In certain preferred embodiments of the present disclosure, when M is 4- to 10-membered cycloalkyl substituted by R⁵, the 4- to 10-membered cycloalkyl is cyclopentyl, cyclohexyl or cycloheptyl, for example, cyclohexyl.

In certain preferred embodiments of the present disclosure, when R⁵ is C₆₋₁₄ aryl or C₆₋₁₄ aryl substituted by halogen, the C₆₋₁₄ aryl is independently phenyl, naphthyl, anthracenyl or phenanthryl, for example, phenyl.

In certain preferred embodiments of the present disclosure, when R⁵ is C₆₋₁₄ aryl substituted by halogen, the halogen is F, Cl, Br or I, for example, Cl.

In certain preferred embodiments of the present disclosure, when R⁵ is C₆₋₁₄ aryl substituted by halogen, the C₆₋₁₄ aryl substituted by halogen is chlorophenyl, for example,

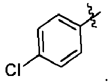

In certain preferred embodiments of the present disclosure, when M is 4- to 10-membered cycloalkyl substituted by R⁵, the 4- to 10-membered cycloalkyl substituted by R⁵ is

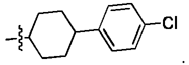

In certain preferred embodiments of the present disclosure, when A', A^{1a}, A^{1b} and A^{1c} are independently

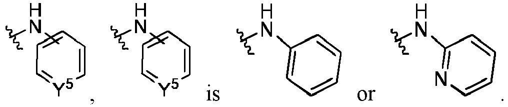

In certain preferred embodiments of the present disclosure, when A', A^{1a}, A^{1b} and A^{1c} are independently

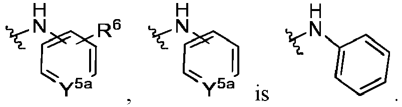

In certain preferred embodiments of the present disclosure, when R⁶ is halogen, the halogen is F, Cl, Br or I, for example, F.

In certain preferred embodiments of the present disclosure, when A', A^{1a}, A^{1b} and A^{1c} are independently

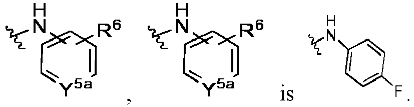

In certain preferred embodiments of the present disclosure, when A', A^{1a}, A^{1b} and A^{1c} are independently

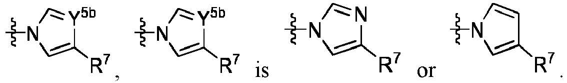

In certain preferred embodiments of the present disclosure, when R⁷ is C₁₋₆ alkyl, the C₁₋₆ alkyl is methyl, ethyl, *n*-propyl, isopropyl, *n*-butyl, isobutyl, *sec*-butyl or *tert*-butyl; for example, methyl.

In certain preferred embodiments of the present disclosure, when A', A^{1a}, A^{1b} and A^{1c} are independently

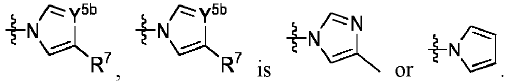

In certain preferred embodiments of the present disclosure, when A', A^{1a}, A^{1b} and A^{1c} are independently C₆₋₁₄ aryl, the C₆₋₁₄ aryl is independently phenyl, naphthyl, anthracenyl or phenanthryl, for example, phenyl.

In certain preferred embodiments of the present disclosure, when R⁸ is C₆₋₁₄ aryl, the C₆₋₁₄ aryl is independently phenyl, naphthyl, anthracenyl or phenanthryl, for example, phenyl.

In certain preferred embodiments of the present disclosure, A', A^{1a}, A^{1b} and A^{1c} are independently selected from the following structures:
NO₂,

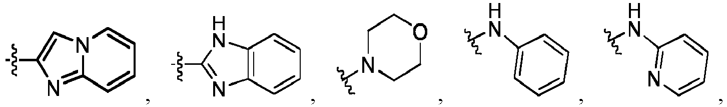

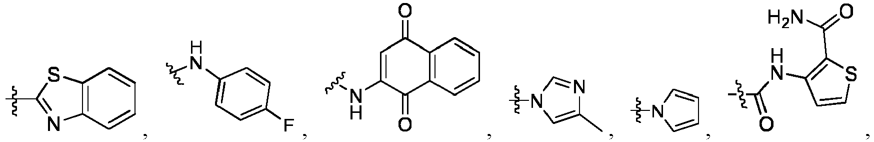

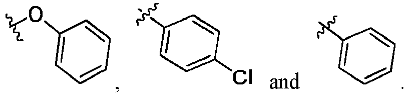

In certain preferred embodiments of the present disclosure, 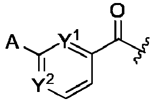 is

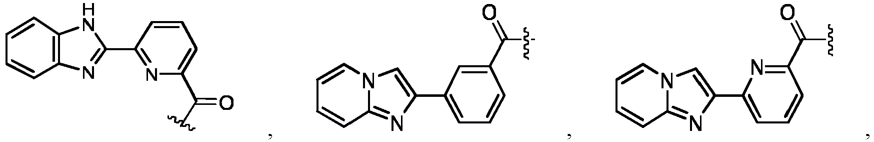

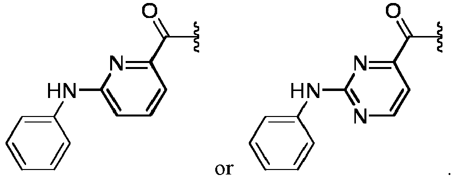

In certain preferred embodiments of the present disclosure, one of R¹ and R² is H, and the other is C₆₋₁₄ aryl.

In certain preferred embodiments of the present disclosure, Y² is CH.

In certain preferred embodiments of the present disclosure, Q' is 6- to 10-membered fused heterocycloalkyl or 6- to 12-membered spiro heterocycloalkyl.

In certain preferred embodiments of the present disclosure, Q' is 6- to 10-membered fused heterocycloalkyl, and Z¹-L¹- is 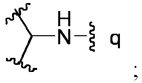 for example, Q is 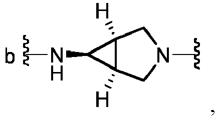 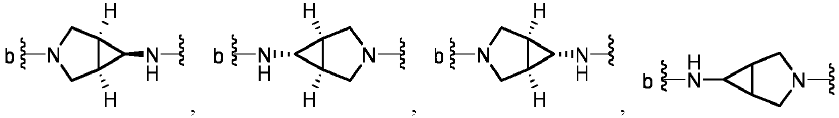 or

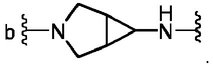

In certain preferred embodiments of the present disclosure, Q' is 6- to 10-membered fused heterocycloalkyl, and Z¹-L¹- is 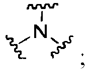 for example, Q is

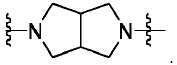

In certain preferred embodiments of the present disclosure, Q' is 6- to 12-membered spiro heterocycloalkyl, and Z¹-L¹- is

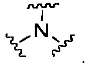

In certain preferred embodiments of the present disclosure, Q' is 6- to 10-membered bridged heterocycloalkyl, and Z¹-L¹- is

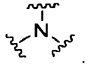

In certain preferred embodiments of the present disclosure, 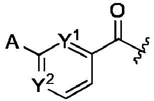 is 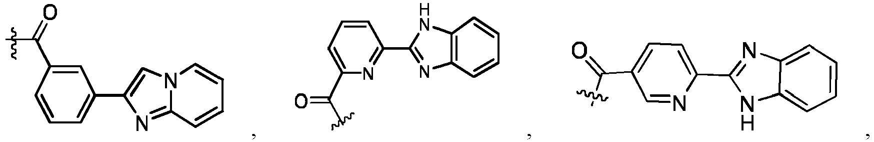 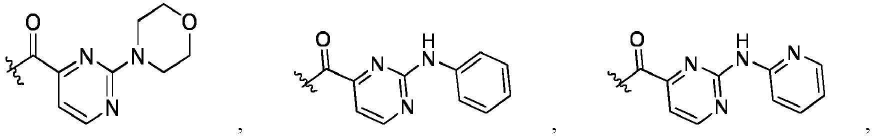 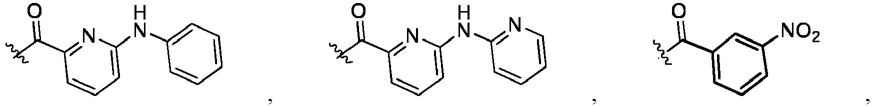 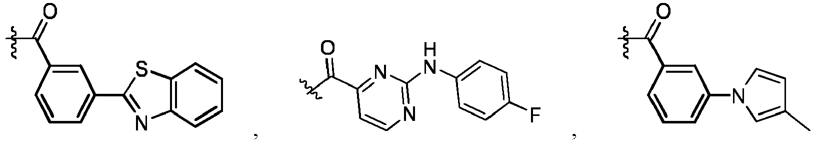 or

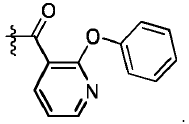

In certain preferred embodiments of the present disclosure, Q is

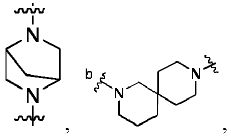

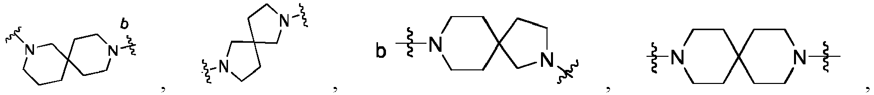

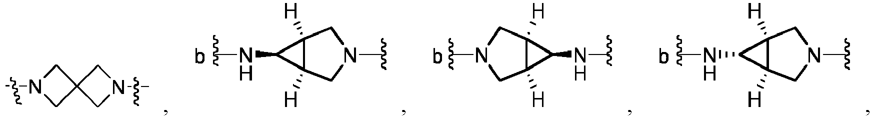

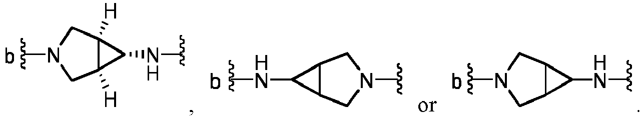

In certain preferred embodiments of the present disclosure, 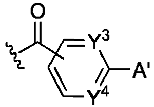 is

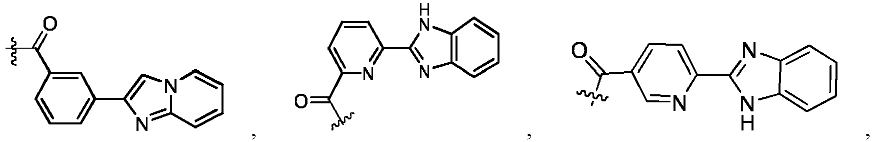

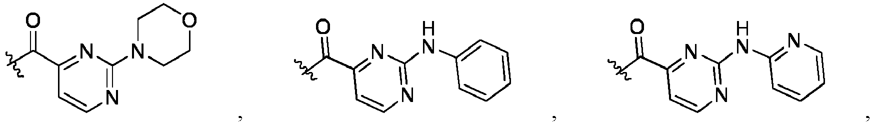

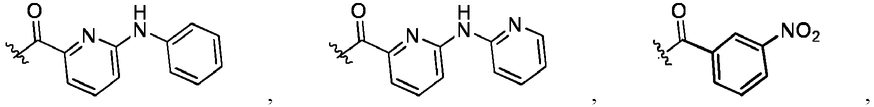

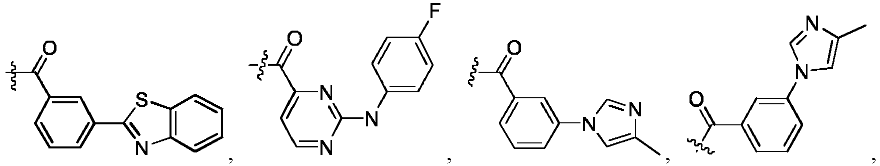

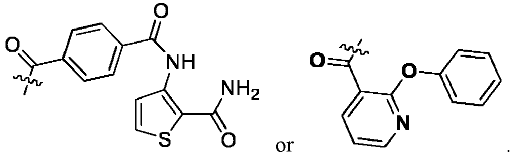

In certain preferred embodiments of the present disclosure, 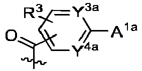 is

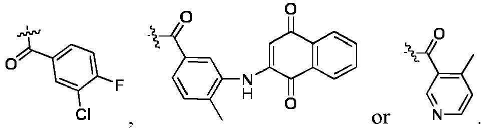

In certain preferred embodiments of the present disclosure, 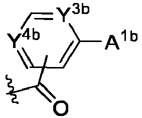 is

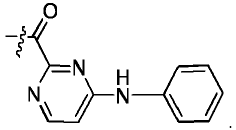

In certain preferred embodiments of the present disclosure, 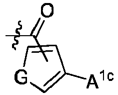 is

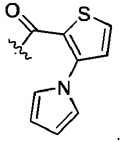

In certain preferred embodiments of the present disclosure, A is

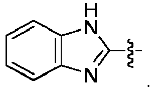

In certain preferred embodiments of the present disclosure, A is

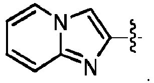

In certain preferred embodiments of the present disclosure,
wherein, A is 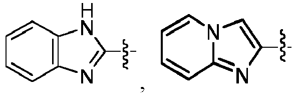 or -NR¹R²;
R¹ and R² are independently H or C₆₋₁₈ aryl;
Y¹ and Y² are independently CH or N;
Q is 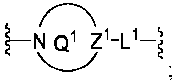
Z¹-L¹- is 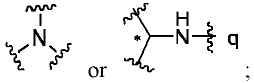
the carbon atoms with "^{∗}" indicates that when the carbon atom is a chiral carbon atom, it is S-configuration, R-configuration or a mixture of both;
ring Q' is 6- to 10-membered fused heterocycloalkyl, 6- to 12-membered spiro heterocycloalkyl or 6- to 10-membered bridged heterocycloalkyl; Q' contains 1 to 3 N atoms;
M is 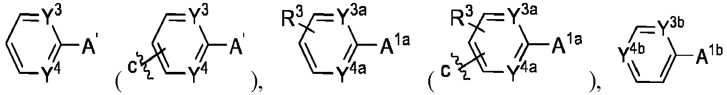 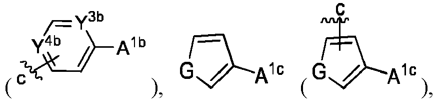 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 5- to 10-membered cycloalkyl substituted by R⁵; the heteroatoms in the 6- to 10-membered fused heterocycloaryl, the heteroatoms in the 6- to 10-membered fused heterocycloaryl substituted by R⁴, the heteroatoms in the 5- to 10-membered heterocycloalkenyl and the heteroatoms in the 5- to 10-membered heterocycloalkenyl substituted by oxo are independently one or more than one of N, S and O, and the number of heteroatoms is independently 1, 2 or 3;
G is S and O;
A', A^{1a}, A^{1b} and A^{1c} are independently 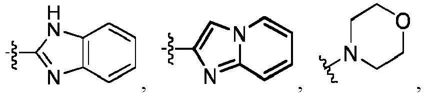 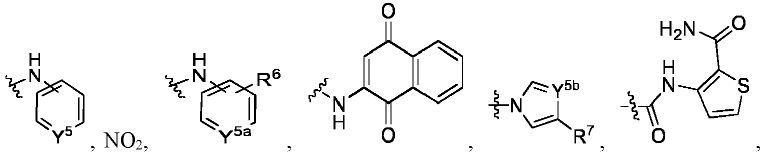 -OR⁸, C₆₋₁₄ aryl or H;
Y⁵, Y^{5a} and Y^{5c} are independently CH or N;
R⁶ is halogen;
R⁷ is C₁₋₆ alkyl;
R⁸ is C₆₋₁₄ aryl;
R³ is C₁₋₆ alkyl or halogen;
R⁴ is C₁₋₆ alkyl;
R⁵ is C₆₋₁₄ aryl or C₆₋₁₄ aryl substituted by halogen.

In certain preferred embodiments of the present disclosure, A is 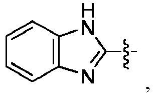 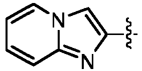 or -NR¹R²;
R¹ and R² are independently H or C₆₋₁₈ aryl;
Y¹ and Y² are independently CH or N;
Q is 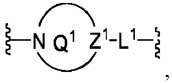 and Z¹-L¹- is 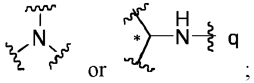
the carbon atom with "^{∗}" indicates that when the carbon atom is a chiral carbon atom, it is S-configuration, R-configuration or a mixture of both;
ring Q' 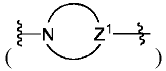 is 6- to 10-membered fused heterocycloalkyl or 6- to 12-membered spiro heterocycloalkyl; Q' contains 1 to 3 N atoms;
M is 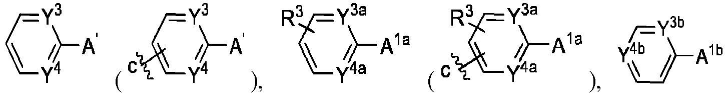 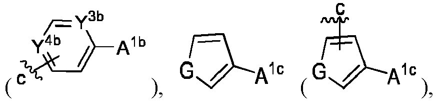 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 5- to 10-membered cycloalkyl substituted by R⁵;
G is S and O;
A', A^{1a}, A^{1b} and A^{1c} are independently 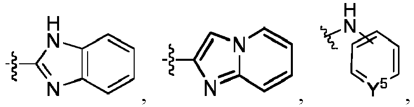 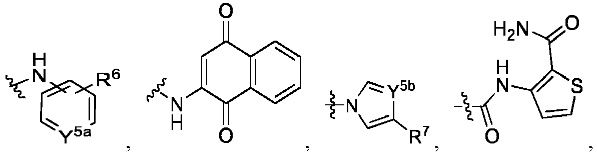 -OR⁸, C₆₋₁₄ aryl or H;
Y⁵, Y^{5a} and Y^{5b} are independently CH or N;
R⁶ is halogen;
R⁷ is C₁₋₆ alkyl;
R⁸ is C₆₋₁₄ aryl;
R³ is C₁₋₆ alkyl or halogen;
R⁴ is C₁₋₆ alkyl;
R⁵ is C₆₋₁₄ aryl or C₆₋₁₄ aryl substituted by halogen.

In certain preferred embodiments of the present disclosure,
wherein, A is 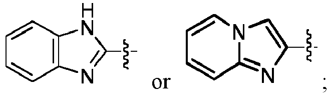
Y² is CH;
Qis 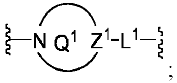
Z¹-L¹- is 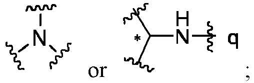
ring Q' is heterocycloalkyl of 5-membered N-heterocycloalkyl fused with 3-membered cycloalkyl (e.g., 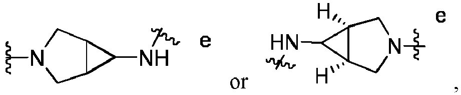 e-terminus is connected to 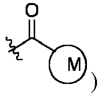 or spiro[5,5]undecane heterocycloalkyl (e.g., 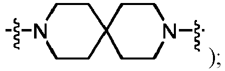
M is 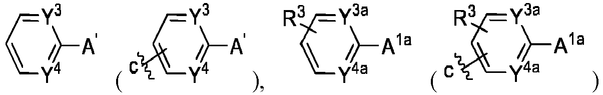 or 6- to 10-membered fused heterocycloaryl;
A' and A^{1b} are independently 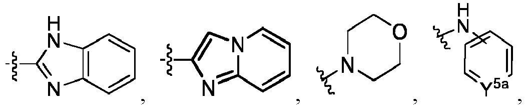 NO₂,

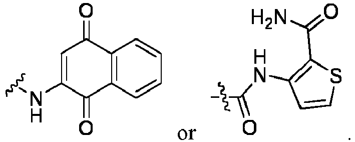

In certain preferred embodiments of the present disclosure,
A is 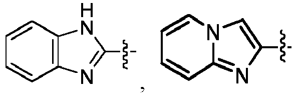 or -NR¹R²;
R¹ and R² are independently H or C₆₋₁₄ aryl;
Y¹ and Y² are independently CH or N;
Q is 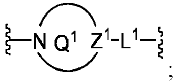
Z¹-L¹- is 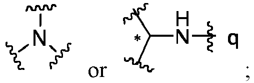
ring Q' is 6- to 10-membered fused heterocycloalkyl; Q' contains 1 or 2 N atoms;
M is 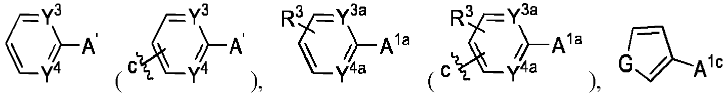 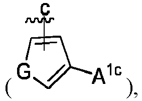 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 4- to 10-membered cycloalkyl substituted by R⁵;
GisS;
A' and A^{1c} are independently 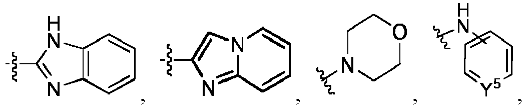 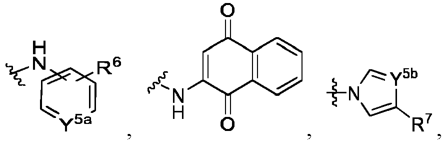 -OR⁸, C₆₋₁₄ aryl or H;
Y⁵, Y^{5a} and Y^{5b} are independently CH or N;
R⁶ is halogen;
R⁷ is C₁₋₆ alkyl;
R⁸ is C₆₋₁₄ aryl;
R³ is C₁₋₆ alkyl or halogen;
R⁴ is C₁₋₆ alkyl;
R⁵ is C₆₋₁₄ aryl or C₆₋₁₄ aryl substituted by halogen.

In certain preferred embodiments of the present disclosure,
A is 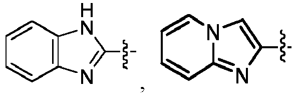 or -NR¹R²;
R¹ and R² are independently H or C₆₋₁₄ aryl;
Y¹ and Y² are independently CH or N;
Q is 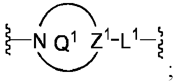
Z¹-L¹- is 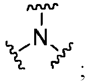
ring Q' is 6- to 12-membered spiro heterocycloalkyl; Q' contains 2 N atoms;
M is 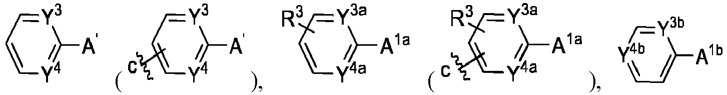 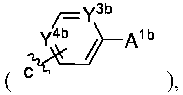 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 4- to 10-membered cycloalkyl substituted by R⁵;
A', A^{1a}, A^{1b} and A^{1c} are independently 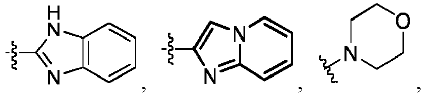 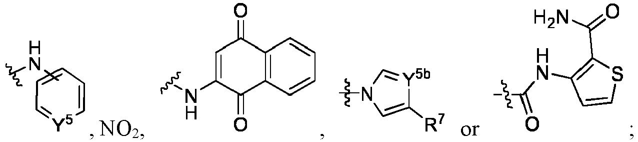
Y⁵ and Y ^{5b} are independently CH or N.

In some preferred embodiments of the present disclosure, the carbonyl heterocyclic compound represented by formula I is as shown in formula I-1: 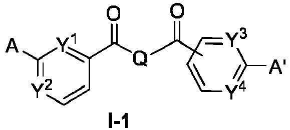
wherein, A is 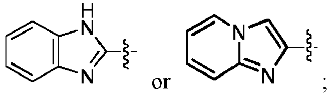
Y¹ and Y² are independently CH or N;
Q is 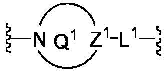 (including 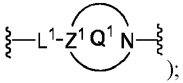
Z¹-L¹- is 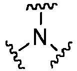 (i.e., L¹ is a linking bond) or 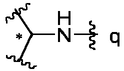 (q-terminus indicates a connection to carbonyl);
ring Q¹ 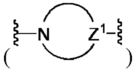 is 6- to 10-membered fused heterocycloalkyl, 6- to 12-membered spiro heterocycloalkyl or 6- to 10-membered bridged heterocycloalkyl; Q' contains 1 to 3 N atoms;
Y³ and Y⁴ are independently CH or N;
A'is 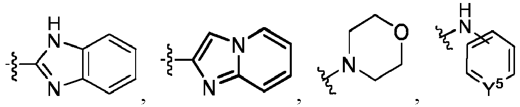 or NO₂;
Y⁵ is CH or N;
the carbon atom with "^{∗}" indicates that when the carbon atom is a chiral carbon atom, it is S-configuration, R-configuration or a mixture of both.

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure), 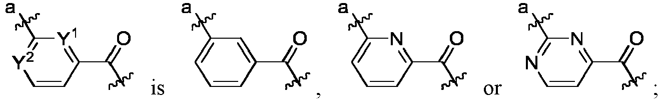 for example, 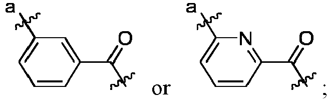 for another example, 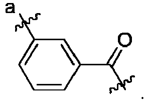 (a-terminus indicates the position connected to A)

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
when Q' is 6- to 10-membered fused heterocycloalkyl, the 6- to 10-membered fused heterocycloalkyl is 6- to 8-membered fused heterocycloalkyl, containing 1 to 2 N atoms (e.g., the ring atoms other than the N atom connected to carbonyl and Z' are all carbon); for example,

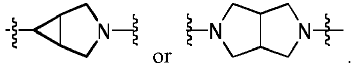

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
when Q' is 6- to 12-membered spiro heterocycloalkyl, the 6- to 12-membered spiro heterocycloalkyl is 9- to 11-membered spiro heterocycloalkyl, containing 1 to 2 N atoms (e.g., the ring atoms other than the N atom connected to carbonyl and Z' are all carbon); for example, 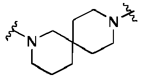 (for another example, 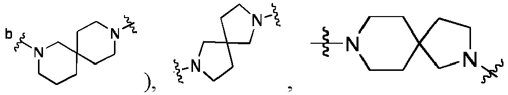 (for another example, 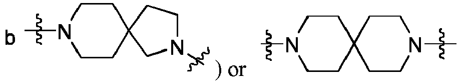 (b-terminus indicates a connection to the left carbonyl).

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
when Q' is 6- to 10-membered bridged heterocycloalkyl, the 6- to 10-membered bridged heterocycloalkyl is 7-membered bridged heterocycloalkyl, containing 1 to 2 N atoms (e.g., the ring atoms other than the N atom connected to carbonyl and Z' are all carbon); for example,

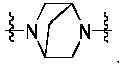

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure), 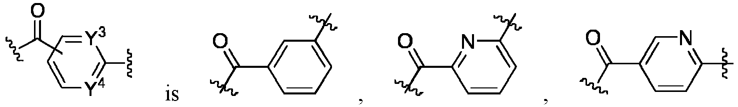 or

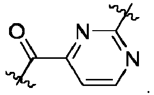

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
when A' is

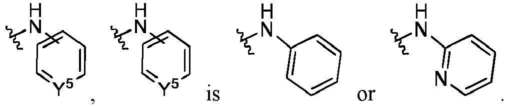

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
A' is selected from the following structures:
NO₂,

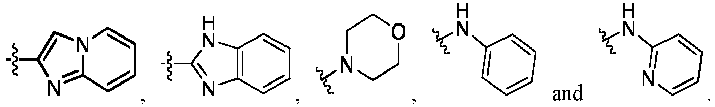

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
A is the same as A';
and/or, 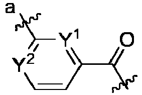 is the same as 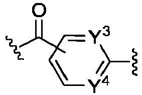 (when M is

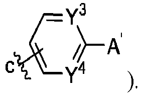

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure), 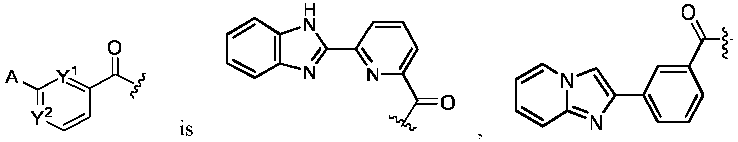 or

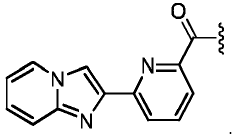

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
Q is selected from the following structures: 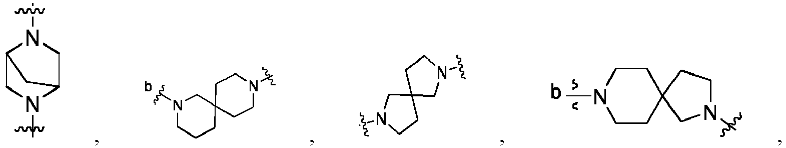 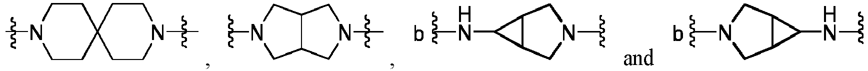 (b-terminus indicates a connection to B).

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure), 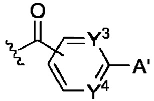 is selected from the following structures:

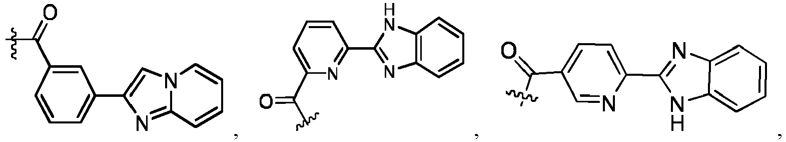

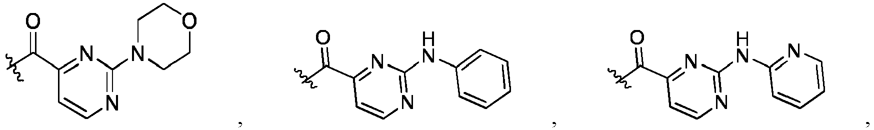

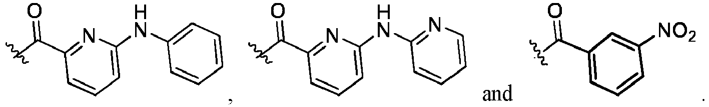

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
A'is 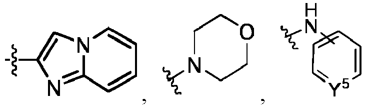 or NO₂; for example,

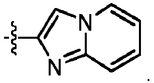

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
wherein, A is 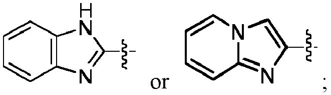
Y¹ and Y² are independently CH or N; for example, 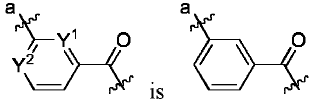 or 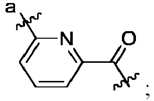
Q is 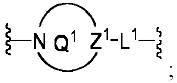 Q¹ is 6- to 10-membered fused heterocycloalkyl, 6- to 12-membered spiro heterocycloalkyl or 6- to 10-membered bridged heterocycloalkyl, and Z¹-L¹- is 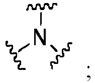 alternatively, Q' is 6- to 10-membered fused heterocycloalkyl, and Z¹-L¹- is 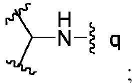
Y³ and Y⁴ are independently CH or N; for example, 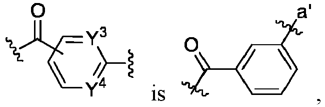 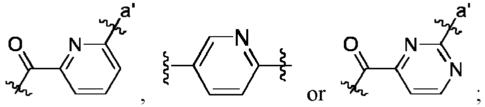
A'is 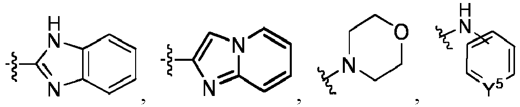 or NO₂;
Y⁵ is CH or N.

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
wherein, A is 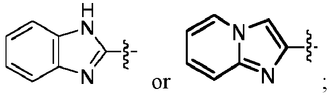
Y¹ and Y² are independently CH or N;
Q is 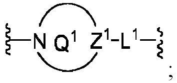 Q' is independently 6- to 10-membered fused heterocycloalkyl or 6- to 12-membered spiro heterocycloalkyl, and Z¹-L¹- is 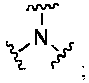 alternatively, Q' is 6- to 10-membered fused heterocycloalkyl, and Z¹-L¹- is 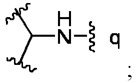
Y³ and Y⁴ are independently CH or N;
A'is 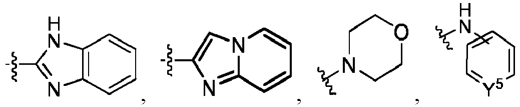 or NO₂;
Y⁵ is CH or N.

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
wherein, A is 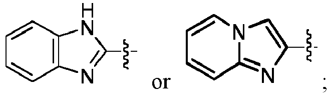 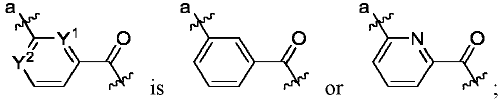
Q is 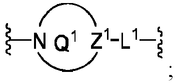 Q' is independently 6- to 10-membered fused heterocycloalkyl; Z¹-L¹- is 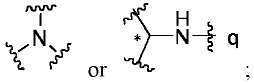 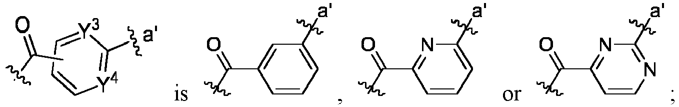
A'is 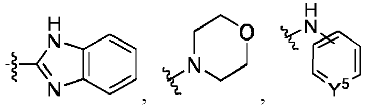 or NO₂;
Y⁵ is CH or N.

In certain preferred embodiments of the present disclosure, some groups of the carbonyl heterocyclic compound represented by formula I or the pharmaceutically acceptable salt thereof are defined as follows (unmentioned groups are as described in any one of the embodiments of the present disclosure),
wherein, A is 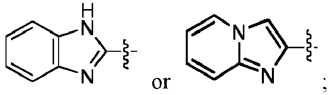 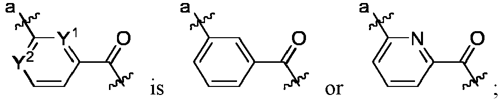
Q is 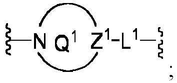 Q' is independently 6- to 12-membered spiro heterocycloalkyl; Z¹-L¹- is 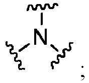 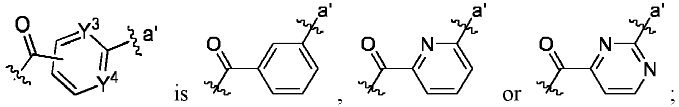
A'is 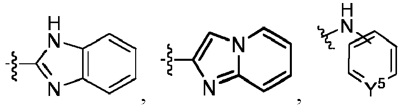 or NO₂.

In some preferred embodiments of the present disclosure, the carbonyl heterocyclic compound represented by formula I is selected from the following group consisting of:

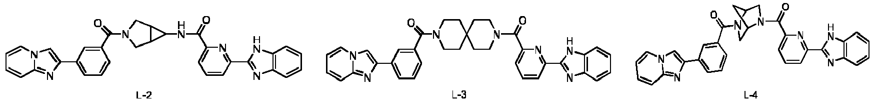

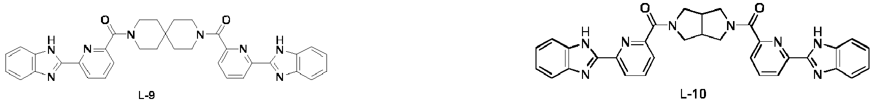

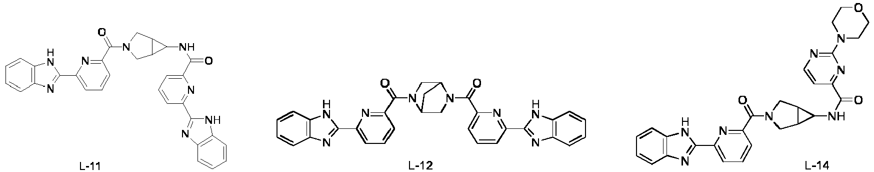

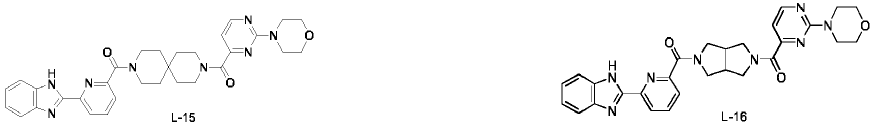

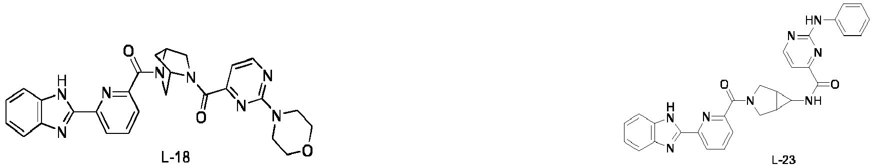

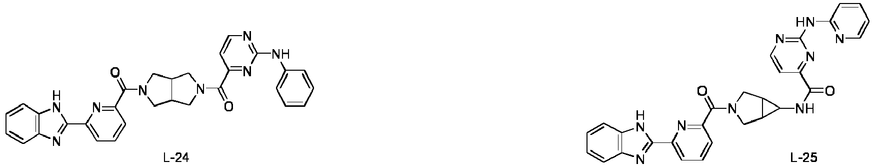

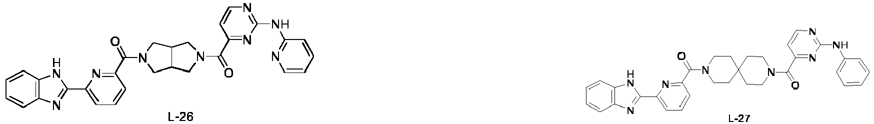

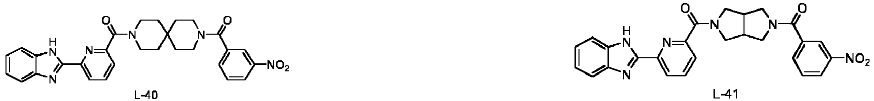

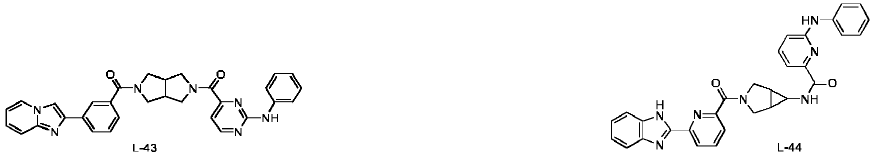

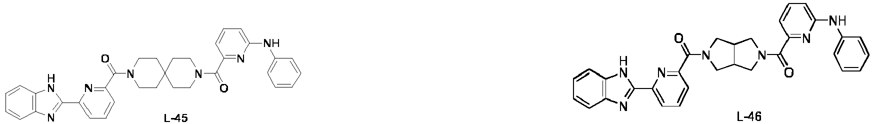

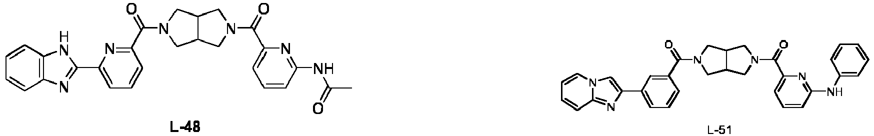

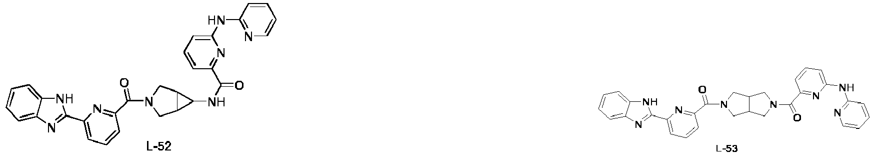

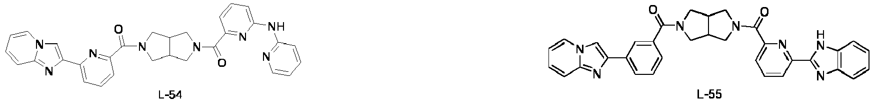

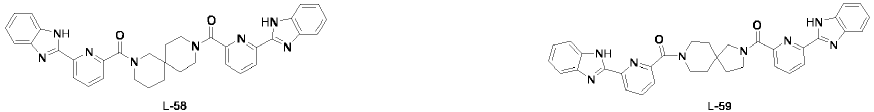

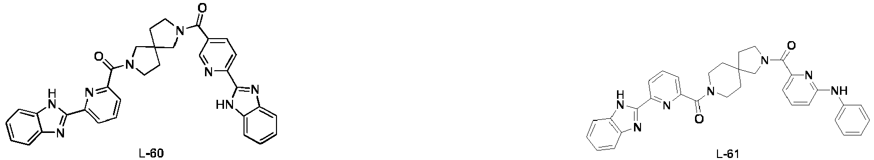

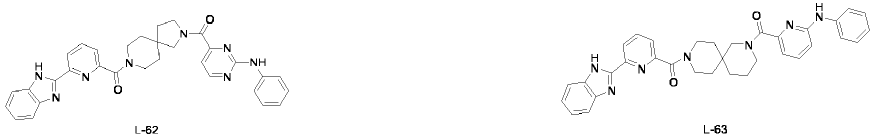

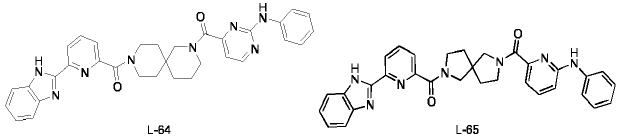

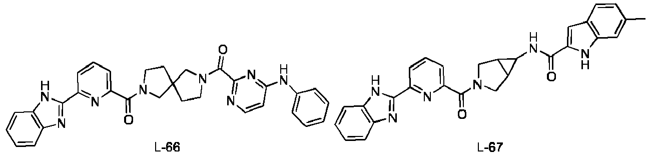

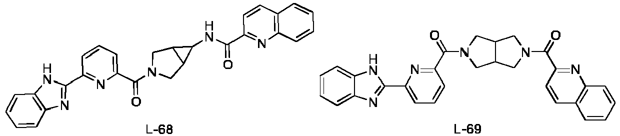

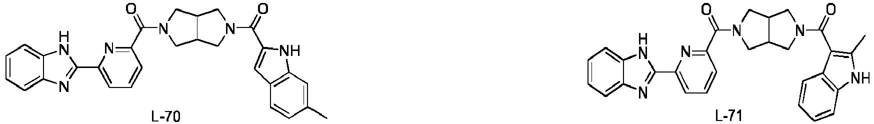

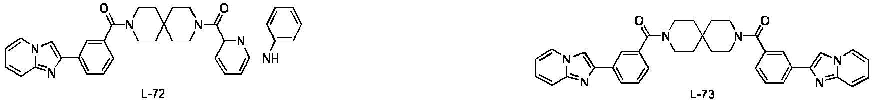

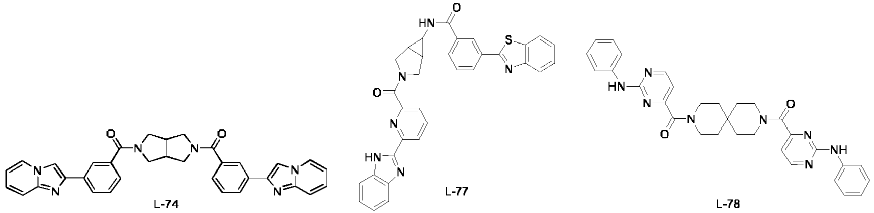

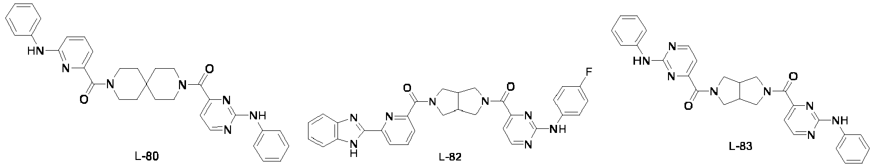

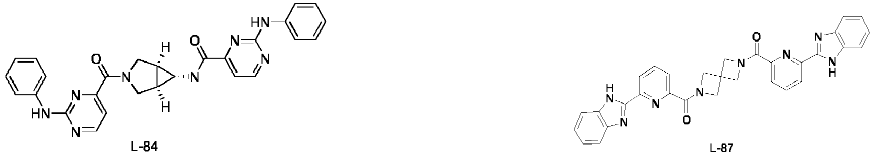

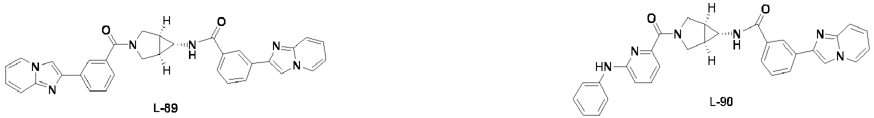

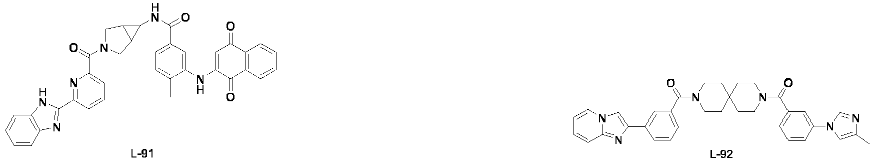

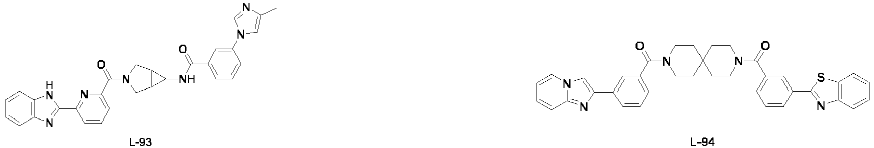

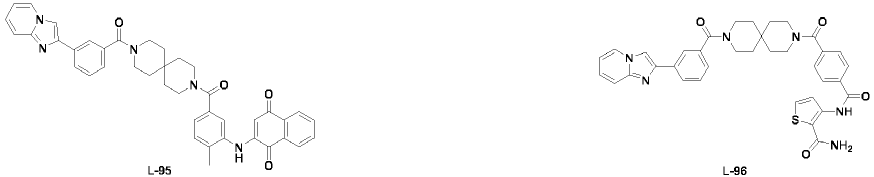

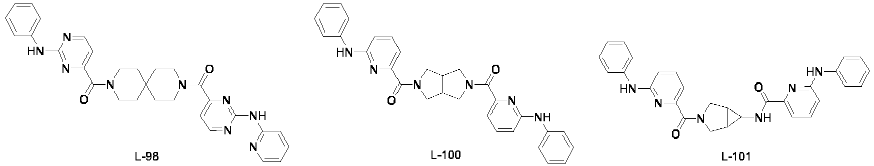

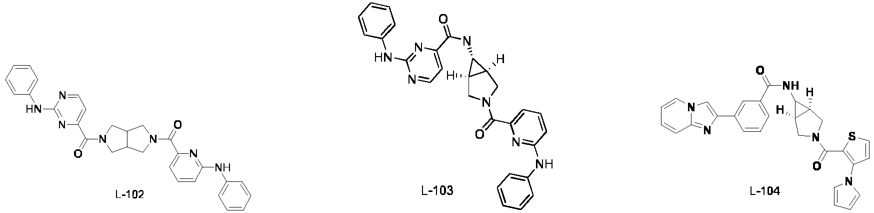

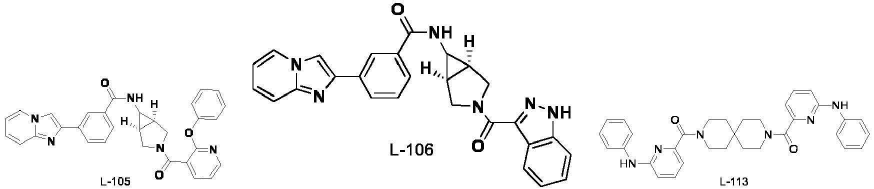

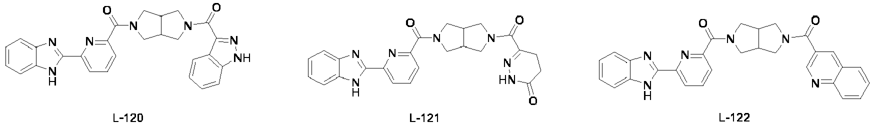

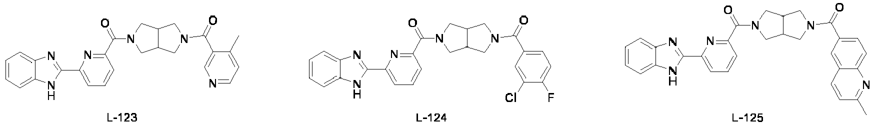

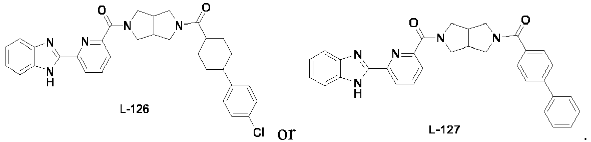

In the present disclosure, the carbonyl heterocyclic compound represented by formula I or the pharmaceutically acceptable salt thereof has one or more than one of chiral carbon atoms, and thus can be separated to obtain optically pure isomers, such as pure enantiomers, or racemates, or mixed isomers. Pure single isomers can be obtained by separation methods in the art, such as salt formation by chiral crystallization, or chiral preparative column separation.

In the present disclosure, if a stereoisomer of the carbonyl heterocyclic compound represented by formula I or the pharmaceutically acceptable salt thereof exists, the stereoisomer may exist as a single stereoisomer or a mixture thereof (such as racemate). The term "stereoisomer" refers to *cis-trans* isomers or optical isomers. Such stereoisomers can be separated, purified and enriched by asymmetric synthetic methods or chiral separation methods (including but not limited to thin-layer chromatography, rotary chromatography, column chromatography, gas chromatography, high-pressure liquid chromatography, etc.), and can also be obtained by chiral resolution through bond formation (chemical bonding, etc.) or salt formation (physical bonding, etc.) with other chiral compounds. The term "single stereoisomer" means that the mass content of one stereoisomer of the compound of the present disclosure relative to all stereoisomers of the compound is not less than 95%.

The carbonyl heterocyclic compound represented by formula I or the pharmaceutically acceptable salt thereof of the present disclosure may be synthesized by methods including the methods similar to those well known in the field of chemistry, whose steps and conditions can refer to the steps and conditions of similar reactions in the art, in particular according to the description herein. Starting materials are generally available from commercial sources such as Aldrich or can be readily prepared using methods well known to those skilled in the art (available via SciFinder and Reaxys online databases).

The raw materials or reagents necessary for the preparation of the carbonyl heterocyclic compound represented by formula I or the pharmaceutically acceptable salt thereof are commercially available or can be prepared by synthetic methods known in the art. The compounds of the present disclosure can be prepared as a free alkali or as a salt thereof with the addition of an acid, as described in the experimental section below. The term pharmaceutically acceptable salt refers to a pharmaceutically acceptable salt as defined herein and has all the effects of the parent compound. A pharmaceutically acceptable salt can be prepared by adding the corresponding acid to a suitable organic solvent of an organic alkali according to conventional methods.

Examples of salt formation include: for alkali addition salts, it is possible to prepare salts of alkali metals (for example, sodium, potassium or lithium) or alkaline earth metals (for example, aluminum, magnesium, calcium, zinc or bismuth) by treating compounds of the present disclosure having appropriate acidic protons in an aqueous medium using hydroxides or alkoxides (for example, ethoxides or methoxides) of alkali metal or alkaline earth, or appropriate alkaline organic amines (for example, diethanolamine, choline or methylglucamine).

Alternatively, for acid addition salts, salts formed with inorganic acids, such as hydrochloric acid, hydrobromic acid, sulfuric acid, nitric acid, phosphoric acid; and salt formed with organic acids, such as acetic acid, benzenesulfonic acid, benzoic acid, camphorsulfonic acid, citric acid, ethanesulfonic acid, fumaric acid, glucoheptonic acid, glutamic acid, glycolic acid, hydroxynaphthoic acid, 2-hydroxyethanesulfonic acid, lactic acid, maleic acid, malic acid, oxalic acid, pyruvic acid, malonic acid, mandelic acid, methanesulfonic acid, 2-naphthalenesulfonic acid, propionic acid, salicylic acid, succinic acid, tartaric acid, citric acid, cinnamic acid, *p-*toluenesulfonic acid or pivalic acid.

In the present disclosure, the carbonyl heterocyclic compounds represented by formula I or the pharmaceutically acceptable salts thereof may also be obtained through peripheral modification of the prepared carbonyl heterocyclic compounds represented by formula I or the pharmaceutically acceptable salts thereof, using conventional methods in the art, so as to obtain the other carbonyl heterocyclic compounds represented by formula I or the pharmaceutically acceptable salts thereof.

Generally, the compounds of the present disclosure can be prepared by the methods described herein, unless further specified, the definitions of substituents are as shown in formula I.

The present disclosure also provides a pharmaceutical composition, comprising the carbonyl heterocyclic compound represented by formula I or the pharmaceutically acceptable salt thereof as described above, and one or more than one of pharmaceutically acceptable carriers. In the pharmaceutical composition, the amount of the carbonyl heterocyclic compound represented by formula I or the pharmaceutically acceptable salt thereof may be therapeutically effective amount.

The pharmaceutically acceptable carriers (pharmaceutical excipients) may be those widely used in the field of pharmaceutical production. Excipients are mainly used to provide a safe, stable and functional pharmaceutical composition, and may also provide a method to allow the active ingredients to dissolve at a desired rate after the subject is administered, or to facilitate effective absorption of the active ingredients after the subject is administered of the composition. The pharmaceutical excipients may be inert fillers, or provide a certain function, such as stabilizing the overall pH value of the composition or preventing the degradation of the active ingredients of the composition. The pharmaceutical excipients may comprise one or more than one of the following excipients: binders, suspending agents, emulsifiers, diluents, fillers, granulating agents, adhesives, disintegrants, lubricants, anti-adhesive agents, glidants, wetting agents, gelling agents, absorption retarders, dissolution inhibitors, enhancers, adsorbents, buffers, chelators, preservatives, coloring agents, corrigents and sweetening agents.

The pharmaceutical composition of the present disclosure may be prepared according to the disclosure using any method known to those skilled in the art. For example, conventional mixing, dissolving, granulating, emulsifying, grinding, encapsulating, embedding or freeze-drying processes.

The pharmaceutical composition of the present disclosure can be administered in any form, including injection (intravenous), mucosal, oral (solid and liquid preparations), inhalation, ophthalmic, rectal, topical or parenteral (infusion, injection, implantation, subcutaneous, intravenous, intra-arterial, intramuscular) administration. The pharmaceutical composition of the present disclosure can also be a controlled release or delayed release dosage form (for example, liposome or microsphere). Examples of solid preparations for oral administration include, but are not limited to, powders, capsules, caplets, soft capsules and tablets. Examples of liquid preparations for oral or mucosal administration include, but are not limited to, suspensions, emulsions, elixirs and solutions. Examples of topical preparations include, but are not limited to, emulsions, gels, ointments, creams, patches, pastes, foams, lotions, drops or serum preparations. Examples of preparations for parenteral administration include, but are not limited to, solutions for injection, dry preparations that can be dissolved or suspended in pharmaceutically acceptable carriers, suspensions for injection and emulsions for injection. Examples of other suitable preparations of the pharmaceutical composition include, but are not limited to, eye drops and other ophthalmic preparations; aerosol: such as nasal spray or inhalant; liquid dosage forms suitable for parenteral administration; suppositories and lozenges.

The present disclosure also provides a use of the carbonyl heterocyclic compound represented by formula I, the pharmaceutically acceptable salt thereof, or the pharmaceutical composition as described above in the manufacture of an agonist of lanthionine C-like protein 2 (LANCL2). In the use, the agonist of lanthionine C-like protein 2 (LANCL2) can be used *in vivo* in mammals; it can also be used *in vitro,* mainly for experimental purposes, for example, it can be used as a standard sample or control sample to provide a comparison, or made into a kit according to conventional methods in the art, to provide a rapid detection for the activation effect of lanthionine C-like protein 2 (LANCL2).

The present disclosure also provides a use of the carbonyl heterocyclic compound represented by formula I, the pharmaceutically acceptable salt thereof, or the pharmaceutical composition as described above in the manufacture of a medicament; the medicament can be the medicament used for the prevention and/or treatment of diseases related to lanthionine C-like protein 2 (LANCL2). The diseases related to lanthionine C-like protein 2 (LANCL2) may be one or more than one of autoimmune, chronic inflammatory, chronic metabolic and infectious diseases.

The present disclosure also provides a use of the carbonyl heterocyclic compound represented by formula I or the pharmaceutically acceptable salt thereof in the manufacture of a medicament; the medicament can be the medicament used for the prevention and/or treatment of autoimmune, chronic inflammatory, chronic metabolic or infectious diseases.

Another aspect of the present disclosure relates to a method for the prevention and/or treatment of diseases related to lanthionine C-like protein 2 (LANCL2), comprising administering to a patient a therapeutically effective amount of the carbonyl heterocyclic compound represented by formula I, or the pharmaceutically acceptable salt thereof, or the pharmaceutical composition comprising the same as described above.

Another aspect of the present disclosure relates to a method for the prevention and/or treatment of autoimmune, chronic inflammatory or infectious diseases, comprising administering to a patient a therapeutically effective amount of the carbonyl heterocyclic compound represented by formula I, the pharmaceutically acceptable salt thereof, or the pharmaceutical composition as described above.

Another aspect of the present disclosure relates to a medicament for lanthionine C-like protein 2 (LANCL2), comprising the carbonyl heterocyclic compound represented by formula I, the pharmaceutically acceptable salt thereof, or the pharmaceutical composition as described above.

The autoimmune diseases as described above may be inflammatory bowel disease (IBD) (including ulcerative colitis and/or Crohn's disease), systemic lupus, rheumatoid arthritis, type 1 diabetes, psoriasis, multiple sclerosis.

The chronic metabolic diseases as described above may be metabolic syndrome, obesity, prediabetes, cardiovascular disease, and type 2 diabetes.

The infectious diseases as described above may be viral diseases, such as influenza infection.

In a second aspect, the present disclosure also provides a carbonyl heterocyclic compound or a pharmaceutically acceptable salt thereof, a pharmaceutical composition comprising the same, and a use thereof.

Specifically, the present disclosure provides a carbonyl heterocyclic compound or a pharmaceutically acceptable salt thereof, the carbonyl heterocyclic compound is selected from the following group consisting of: 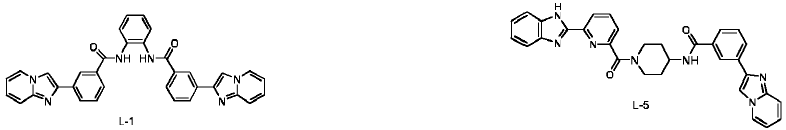 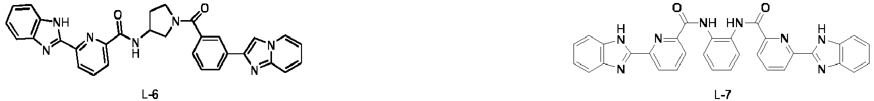 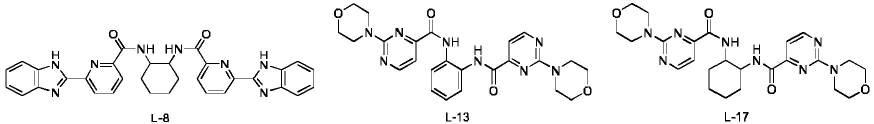 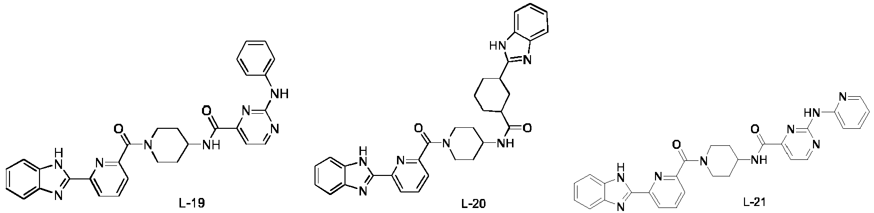 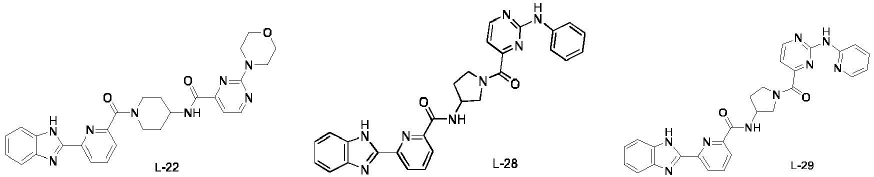 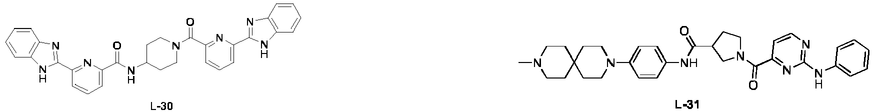 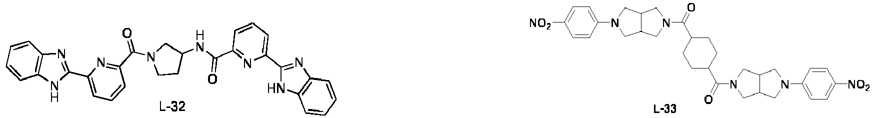 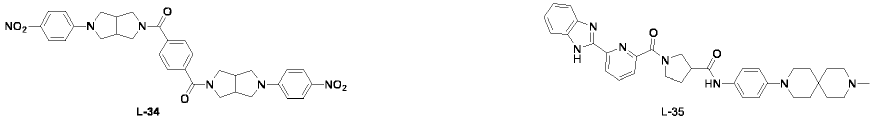 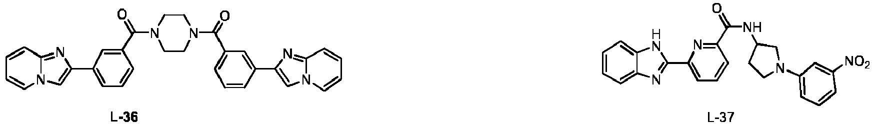 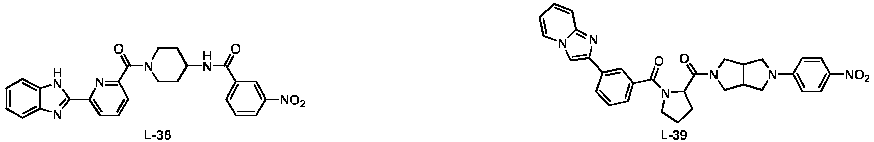 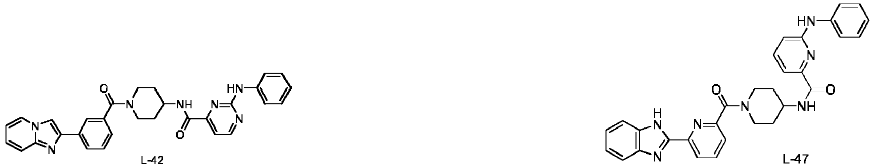 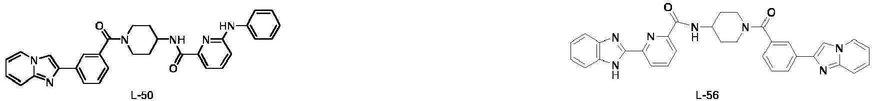 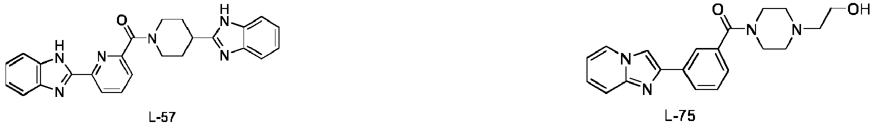 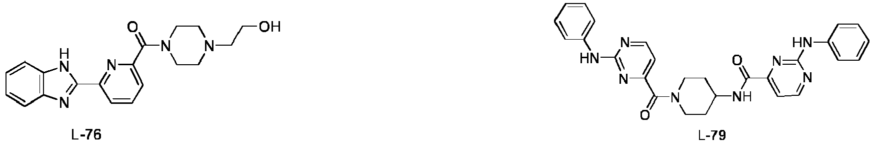 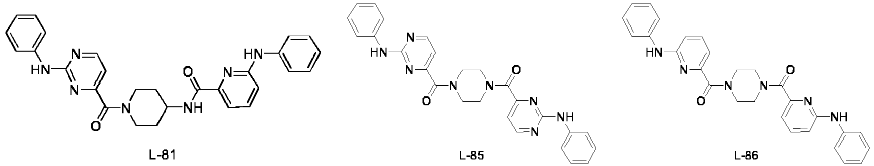 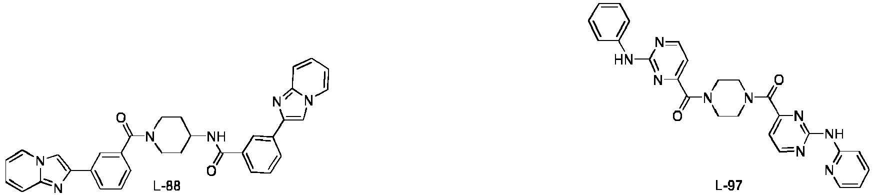 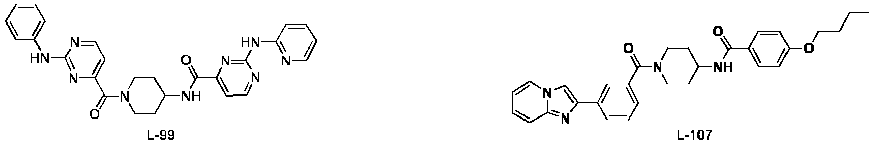 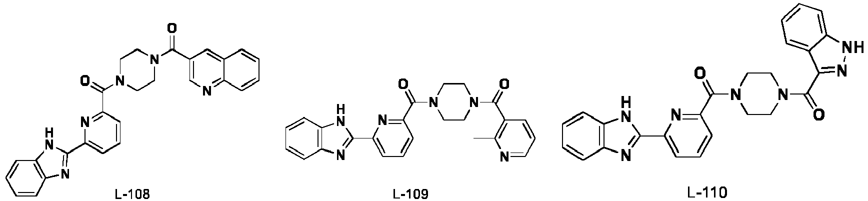 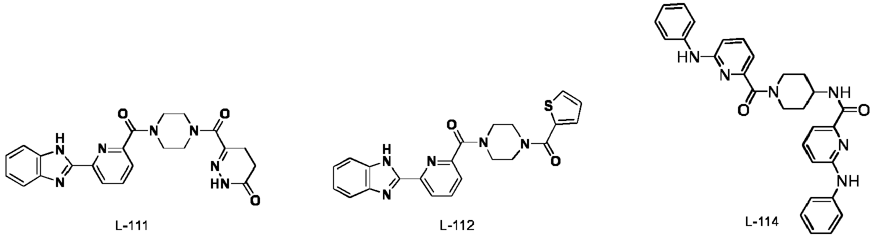 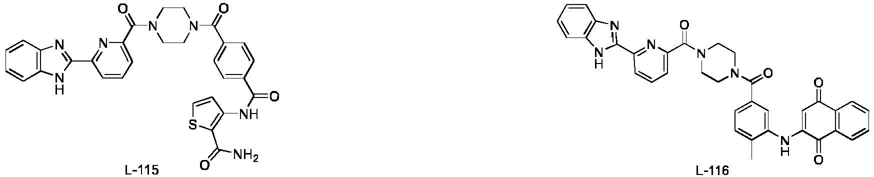 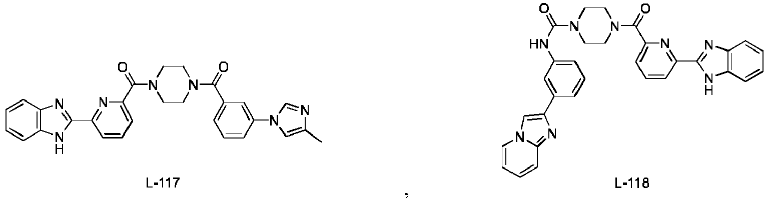 or

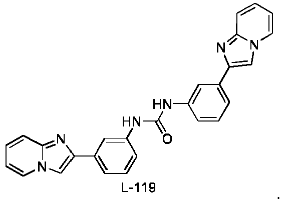

The present disclosure provides a pharmaceutical composition, comprising the carbonyl heterocyclic compound as described above (second aspect) or the pharmaceutically acceptable salt thereof, and one or more than one of pharmaceutically acceptable carriers. In the pharmaceutical composition, the amount of the carbonyl heterocyclic compound or the pharmaceutically acceptable salt thereof may be therapeutically effective amount.

The present disclosure also provides a use of the carbonyl heterocyclic compound as described above (second aspect), the pharmaceutically acceptable salt thereof, or the pharmaceutical composition as described above in the manufacture of an agonist of lanthionine C-like protein 2 (LANCL2). In the use, the agonist of lanthionine C-like protein 2 (LANCL2) can be used *in vivo* in mammals; it can also be used *in vitro,* mainly for experimental purposes, for example, it can be used as a standard sample or control sample to provide a comparison, or made into a kit according to conventional methods in the art, to provide a rapid detection for the activation effect of lanthionine C-like protein 2 (LANCL2).

The present disclosure also provides a use of the carbonyl heterocyclic compound as described above (second aspect), the pharmaceutically acceptable salt thereof, or the pharmaceutical composition as described above in the manufacture of a medicament; the medicament can be the medicament used for the prevention and/or treatment of diseases related to lanthionine C-like protein 2 (LANCL2). The diseases related to lanthionine C-like protein 2 (LANCL2) may be one or more than one of autoimmune, chronic inflammatory, chronic metabolic and infectious diseases.

The present disclosure also provides a use of the carbonyl heterocyclic compound as described above (second aspect) or the pharmaceutically acceptable salt thereof in the manufacture of a medicament; the medicament can be the medicament used for the prevention and/or treatment of autoimmune, chronic inflammatory, chronic metabolic or infectious diseases.

The present disclosure also provides a method for the prevention and/or treatment of diseases related to lanthionine C-like protein 2 (LANCL2), comprising administering to a patient a therapeutically effective amount of the carbonyl heterocyclic compound as described above (second aspect), or the pharmaceutically acceptable salt thereof, or the pharmaceutical composition comprising the same as described above.

The present disclosure also provides a method for the prevention and/or treatment of autoimmune, chronic inflammatory or infectious diseases, comprising administering to a patient a therapeutically effective amount of the carbonyl heterocyclic compound as described above (second aspect), the pharmaceutically acceptable salt thereof, or the pharmaceutical composition as described above.

The present disclosure also provides a medicament for lanthionine C-like protein 2 (LANCL2), comprising the carbonyl heterocyclic compound as described above (second aspect), the pharmaceutically acceptable salt thereof, or the pharmaceutical composition as described above.

The autoimmune disease as described above may be inflammatory bowel disease (IBD) (including ulcerative colitis and/or Crohn's disease), systemic lupus, rheumatoid arthritis, type 1 diabetes, psoriasis, and multiple sclerosis. The chronic metabolic diseases as described above may be metabolic syndrome, obesity, prediabetes, cardiovascular disease, and type 2 diabetes. The infectious disease as described above may be a viral disease, such as influenza infection.

The present disclosure also provides a method for the treatment of a diseasein an animal with any one or more than one of the compounds described herein. The method comprises administering to an animal an effective amount of one or more than one of the compounds described herein. The diseasemay be selected from the group consisting of: infectious diseases, autoimmune diseases, diabetes and chronic inflammatory diseases. In some methods, the infectious disease comprises a viral disease, such as influenza infection. In some methods, the autoimmune disease comprises an autoimmune inflammatory disease, such as inflammatory bowel disease, including ulcerative colitis and/or Crohn's disease. In some methods, diabetes is selected from the group consisting of type 1 diabetes and type 2 diabetes. In some methods, the chronic inflammatory disease comprises metabolic syndrome. In some methods, the method comprises administering an amount of a compound that is effective in increasing LANCL2 activity, reducing inflammation and/or increasing anti-inflammatory effects.

The present disclosure also provides the compound for use in the treatment of a disease in an animal with any one or more than one of the compounds described herein. The compound for such use include any one of the compounds described herein. The use may comprise administering to an animal an effective amount of one or more than one of the compounds described herein, wherein the disease is selected from the group consisting of: infectious diseases, autoimmune diseases, diabetes and chronic inflammatory diseases. In some patterns, the infectious disease comprises a viral disease, such as influenza infection. In some patterns, the autoimmune disease comprises an autoimmune inflammatory disease, such as inflammatory bowel disease, including ulcerative colitis and/or Crohn's disease. In some patterns, diabetes is selected from the group consisting of type 1 diabetes and type 2 diabetes. In some patterns, the chronic inflammatory disease comprises metabolic syndrome. In some patterns, the compound is effective in increasing LANCL2 activity, reducing inflammation and/or increasing anti-inflammatory effects.

The term "pharmaceutically acceptable" refers to salts, solvents, excipients and the like that are generally non-toxic, safe, and suitable for patient use. The "patient" is preferably a mammal, more preferably a human being.

The term "pharmaceutically acceptable salt" refers to a salt prepared from the compound of the present disclosure and a relatively non-toxic and pharmaceutically acceptable acid.

"Treatment" means any treatment of diseases in mammals, including: (1) preventing diseases, that is, causing clinical disease symptoms not to develop; (2) inhibiting diseases, that is, preventing the development of clinical symptoms; (3) alleviating diseases, that is, causing clinical symptoms to subside.

An "effective amount" means an amount of a compound, when administered to a patient in need of treatment, that is sufficient to (i) treat relevant disease, (ii) attenuate, ameliorate or eliminate one or more than one of symptoms of a particular disease or condition, or (iii) delay the onset of one or more than one of symptoms of a particular disease or condition as described herein. The amount of the carbonyl heterocyclic compound represented by formula I or the pharmaceutically acceptable salt thereof or the pharmaceutical composition, corresponding to such amount will vary depending on, for example, the particular compound, the disease condition and its severity, the characteristics of the patient to be treated (e.g., body weight) and the like, but nevertheless can be routinely determined by those skilled in the art.

The "prevention" of the present disclosure refers to a reduction in the risk of acquiring or developing a disease or disorder.

The "pharmaceutical composition" of the present disclosure refers to a preparation of one or more than one of the compounds of the present disclosure or salts thereof and a carrier generally accepted in the art for delivering a biologically active compound to an organism (e.g., a human being). The purpose of a pharmaceutical composition is to facilitate the delivery of a medicament to an organism.

The term "pharmaceutically acceptable carrier" refers to a substance that is co-administered with the active ingredient and facilitates the administration of the active ingredient, including but not limited to any acceptable glidants, sweeteners, diluents, preservatives, dyes/coloring agents, flavor enhancers, surfactants, wetting agents, dispersants, disintegrants, suspending agents, stabilizers, isotonic agents, solvents or emulsifiers for humans or animals (e.g., livestocks) as licensed by the State Food and Drug Administration. Examples include, but are not limited to, calcium carbonate, calcium phosphate, various sugars and various starches, cellulose derivatives, gelatin, vegetable oils, and polyethylene glycols.

The pharmaceutical compositions of the present disclosure may be formulated into solid, semi-solid, liquid or gaseous preparations, such as tablets, pills, capsules, powders, granules, ointments, emulsions, suspensions, solutions, suppositories, injections, inhalants, gels, microspheres and aerosols, etc.

The pharmaceutical compositions of the present disclosure may be prepared by methods well known in the art, such as conventional mixing, dissolving, granulating, dragee-making, grinding, emulsifying, freeze-drying processes and the like.

The route of administration of the compound of the present disclosure or the pharmaceutically acceptable salt thereof or the pharmaceutical composition thereof, includes, but is not limited to, oral, rectal, transmucosal, enteral administration, or topical, transdermal, inhalation, parenteral, sublingual, intravaginal, intranasal, intraocular, intraperitoneal, intramuscular, subcutaneous, intravenous administration. The preferred route of administration is oral administration.

For oral administration, the pharmaceutical composition can be formulated by mixing an active compound with a pharmaceutically acceptable carrier well known in the art. Such carriers enable the compounds of the present disclosure to be formulated into tablets, pills, lozenges, dragees, capsules, liquids, gels, slurries, suspensions and the like, for oral administration to a patient. For example, pharmaceutical compositions for oral administration may be obtained as tablets by combining the active ingredient with one or more solid carriers, granulating the resulting mixture if desired, and processing it into a mixture or granules with a small amount of excipients if desired to form tablets or tablet cores. The tablet core can be combined with an optional coating material suitable for enteric dissolution, and processed into a coating preparation form that is more conducive to the absorption of an organism (e.g., a human being).

Unless otherwise specified, the following definitions as used herein shall apply. For the purposes of the present disclosure, the chemical elements correspond to the Periodic Table of the Elements, CAS Edition, and "Handbook of Chemistry and Physics", 75th Edition, 1994. In addition, general principles of organic chemistry can be found in the description of "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

In this specification, groups and substituents thereof may be selected by those skilled in the art to provide stable structural moieties and compounds. When a substituent is described by a conventional chemical formula written from left to right, the substituent also includes the chemically equivalent substituent obtained when the structural formula is written from right to left.

Certain chemical groups defined herein are preceded by abbreviated notations to indicate the total number of carbon atoms present in the group. For example, C₁-C₆ alkyl refers to an alkyl having a total of 1, 2, 3, 4, 5 or 6 carbon atoms as defined below. The total number of carbon atoms in the abbreviated notation does not include the carbon that may be present in the substituent of the group.

The numerical range defined in the substituents of the present disclosure, such as 0-4, 1-4, 1-3 and the like, indicates integers within the range, for example, 1-6 refers to 1, 2, 3, 4, 5, 6.

In addition to the foregoing, when used in the description and claims of the present disclosure, the following terms have the meanings shown below unless otherwise specified.

The term "include" is open-ended, that is, it includes what is specified in the present disclosure, but does not exclude other aspects.

The term "substituted" means that one or more than one of hydrogen atoms on a specific atom are substituted by the substituent, including deuterium and hydrogen variables, as long as the valence of the specific atom is normal and the substituted compound is stable.

In general, the term "substituted" means that one or more than one of hydrogen atoms in a given structure are substituted by specific substituents. Further, when the group is substituted by the one or more substituents, the substituents are independent of each other, that is, the one or more substituents may be different from each other or identical. Unless otherwise specified, a substituent may substitute at each substitutable position of the group being substituted. When more than one position in a given structural formula can be substituted by one or more than one of substituents selected from specific groups, then the substituents can substitute equally or differently at each position.

The term "one or more than one" or "one, two or more than two" means 1, 2, 3, 4, 5, 6, 7, 8, 9 or more; for example, 1, 2, 3, 4 or 5.

In the present disclosure, as a group or part of another group, unless otherwise specified, the term "cycloalkyl" refers to a saturated monocyclic, polycyclic or bridged carbocyclic substituent consisting only of carbon atoms and hydrogen atoms, and it may be connected to the rest of the molecule by a single bond via any suitable carbon atom; in the case of a polycyclic cycloalkyl, it may be a spiro ring or bridged ring system with a fused ring or spiro ring connection (that is, two germinal hydrogen on a carbon atom are substituted by an alkylene).

In the present disclosure, the term "heterocycloalkyl", as a group or part of another group, refers to a stable 3- to 16-membered saturated cyclic group consisting of 2 to 11 carbon atoms as well as 1 to 5 heteroatoms selected from nitrogen, oxygen and sulfur. Unless otherwise specified in this specification, heterocycloalkyl groups may be either monocyclic ("monocyclic heterocycloalkyl"), or bicyclic, tricyclic or more cyclic ring systems, which may include fused (fused ring), bridged (bridged ring) or spiro (spiro ring) ring systems (such as bicyclic systems ("bicyclic heterocycloalkyl")). The ring system of bicyclic heterocycloalkyl may include one or more than one of heteroatoms in one or both rings; and it is saturated.

The terms "moiety", "structural moiety", "chemical moiety", "group", "chemical group" as used herein refer to a specific fragment or functional group in a molecule. Chemical moieties are generally considered to be chemical entities embedded or attached to molecules.

When the enumerative substituent does not indicate by which atom it is linked to the compound included but not specifically mentioned in a general chemical formula, such substituent can be bonded by any atom thereof. A combination of the substituent and/or the variant thereof is allowed only when the combination results in a stable compound.

When it is not clearly specified that the enumerative group has a substituent, the group only refers to an unsubstituted group. For example, when "C₁-C₄ alkyl" is not defined by "substituted or unsubstituted", it only refers to "C₁-C₄ alkyl" itself or "unsubstituted C₁-C₄ alkyl".

In various sections of the present disclosure, linking groups are described. When the structure clearly requires a linking group, the enumerative Markush variables for the group should be understood as linking groups. For example, if the structure requires a linking group and the Markush group definition for the variable enumerates "alkyl" or "aryl", it should be understood that the "alkyl" or "aryl" represents a linked alkylene or arylidene group, respectively.

In some specific structures, when an alkyl group is clearly indicated as a linking group, the alkyl group represents a linked alkylene group, for example, C₁-C₆ alkyl in the group "halo-C₁-C₆ alkyl" should be understood as C₁-C₆ alkylene.

Unless otherwise specified, all technical and scientific terms as used herein have the standard meaning in the art to which the claimed subject matter pertains. If a term has more than one definition, the definition herein shall prevail.

Unless otherwise specified, it should be understood that the singular form used in the present disclosure, such as "a", includes plural referents. In addition, the term "include" is open-ended and not closed, that is, it includes what is specified in the present disclosure, but does not exclude other aspects.

Unless otherwise specified, the present disclosure uses the conventional methods of mass spectrometry and elemental analysis, and the steps and conditions can be referred to the conventional operating steps and conditions in the art.

Unless otherwise specified, the present disclosure uses standard nomenclature and standard laboratory procedures and techniques of analytical chemistry, organic synthetic chemistry and optics. In some cases, standard techniques are used for chemical synthesis, chemical analysis, and performance testing of luminescent devices.

In addition, it should be noted that, unless otherwise clearly specified, the description "...be independently" used in the present disclosure should be understood in a broad sense, which means that the described individuals are independent of each other and can be the same or different specific groups independently. In more detail, the description "...be independently" can mean either that the specific options expressed between the same symbols in different groups do not affect each other; or that the specific options expressed between the same symbols in the same group do not affect each other.

It can be understood by those skilled in the art that, according to the convention used in the art, " 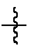 " used in the structural formula of the group described in the present disclosure means that the corresponding group is linked to other fragments and groups in the compound through this site.

The above preferred conditions can be combined arbitrarily to obtain preferred embodiments of the present disclosure without violating common knowledge in the art.

The reagents and raw materials used in the present disclosure are all commercially available.

The positive progressive effect of the present disclosure is that: the carbonyl heterocyclic compound provided by the present disclosure is a compound having a pathway targeting lanthionine synthetase C-like protein 2; the compound can be used for the treatment of various diseases, including infectious diseases, autoimmune diseases, diabetes, and chronic inflammatory diseases.

### BRIEF DESCRIPTION OF THE DRAWINGS

FIG. 1 shows the binding curve of compound L7-LANCL2.
FIG. 2 shows the binding curve of compound L8-LANCL2
FIG. 3 shows the binding curve of compound L12-LANCL2
FIG. 4 shows the binding curve of compound L17-LANCL2
FIG. 5 shows the binding curve of compound L22-LANCL2
FIG. 6 shows the binding curve of compound L28-LANCL2
FIG. 7 shows the binding curve of compound L29-LANCL2
FIG. 8 shows the binding curve of compound L30-LANCL2
FIG. 9 shows the binding curve of compound L32-LANCL2.
FIG. 10 shows the binding curve of compound L37-LANCL2
FIG. 11 shows the binding curve of compound L44-LANCL2
FIG. 12 shows the binding curve of compound L56-LANCL2.
FIG. 13 shows the changes in body weight and DAI scores of mice (compound L30, compound L56 and the control group), wherein, A) body weight change curve, B) DAI score data.
FIG. 14 shows the changes in the weight-to-length ratio of mouse colon (compound L30, compound L56 and the control group), wherein, A) weight-to-length ratio of colon, B) colon length, C) colon weight.
FIG. 15 shows the intestinal morphology (compound L30, compound L56 and the control group).
FIG. 16 shows the changes in body weight and DAI scores of mice (compound L11, compound L25, compound L84, compound L77, compound L101, compound L10, compound L23 and the control group), wherein, A) body weight change curve, B) DAI score data.
FIG. 17 shows the scores of diarrhea and blood in stool in mice (compound L11, compound L25, compound L84, compound L77, compound L101, compound L10, compound L23 and the control group), wherein, A) change in diarrhea in mice, B) change in blood in stool.
FIG. 18 shows the changes in the weight-to-length ratio of mouse colon (compound L11, compound L25, compound L10 and the control group), wherein, A) colon length, B) colon weight, C) weight-to-length ratio of colon.
FIG. 19 shows the intestinal morphology (compound L11, compound L10 and the control group).

### DETAILED DESCRIPTION OF THE PREFERRED EMBODIMENT

The present disclosure is further illustrated below by means of embodiments, but the present disclosure is not thereby limited to the scope of the embodiments. Experimental methods for which specific conditions are not specified in the following embodiments are selected according to conventional methods and conditions, or according to the trade description.

### Embodiment 1: 6-(1H-benzo[d]imidazol-2-yl)-N-(3-(3-(3-(imidazolylazo[1,2-a]pyridin-2-yl)benzoyl)-3-azabicyclo[3.1.0]hexane-6-yl)pyridineamide (L-1)

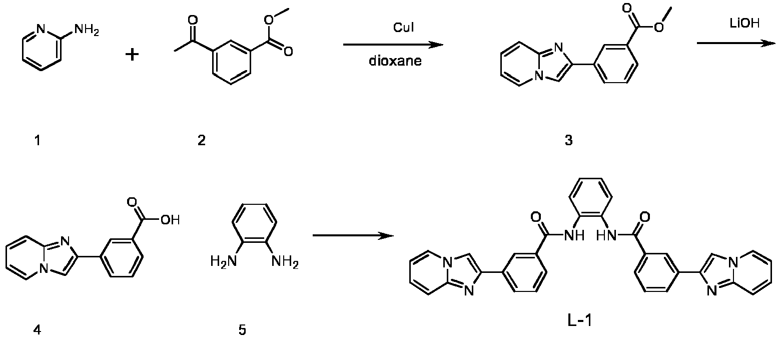

### I. Synthesis of methyl 3-(imidazo[1,2-a]pyridin-2-yl)benzoate

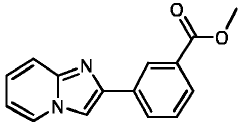

2-Aminopyridine (1.6 g, 17 mmol), methyl 3-acetylformate (2.3 g, 14 mmol) and cuprous iodide (0.5 g, 2.8 mmol) were dissolved in 50 mL of 1,4-dioxane at room temperature, the reaction mixture was heated to reflux overnight, and monitored by LC-MS until the end of the reaction. After concentration under reduced pressure, the mixture was added with 50 mL of water, and extracted with ethyl acetate (50 mL × 3). The organic phases were combined, dried over anhydrous sodium sulfate, and concentrated under reduced pressure to obtain a residue, which was purified by column chromatography (eluent: petroleum ether/ethyl acetate = 5/1) to obtain a white solid (1.8 g, 7.4 mmol) with a yield of 52%. LC-MS: [M+1]⁺: 253.09

### II. Synthesis of 3-(imidazo[1,2-a]pyridin-2-yl)benzoic acid

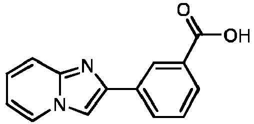

Methyl 3-(imidazo[1,2-a]pyridin-2-yl)benzoate (1.8 g, 7.4 mmol) and lithium hydroxide (0.88 g, 37 mmol) were dissolved in 20 mL of ethanol and water (v/v = 10:1) at room temperature, the mixture was heated to reflux, and the reaction was monitored by LC-MS until the end. The reaction mixture was concentrated under reduced pressure, and the concentrated solution was purified by silica gel column chromatography to obtain a white solid (1.6 g, 0.67 mmol) with a yield of 90%. LC-MS: 238.07.

### III. Synthesis of N,N'-(1,2-aniline)bis(3-(imidazo[1,2-a]pyridin-2-yl)benzamide

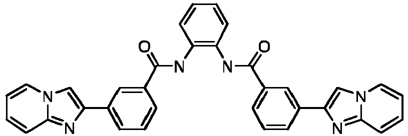

3-(Imidazo[1,2-a]pyridin-2-yl)benzoic acid (0.12 g, 0.5 mmol), 1-(3-dimethylaminopropyl)-3-ethylcarbodiimide hydrochloride (0.1 g, 0.52 mmol), 1-hydroxybenzotriazole (0.07 g, 0.52 mmol) and N,N-diisopropylethylamine (0.14 g, 1.13 mmol) were dissolved in 6 mL of N,N-dimethylformamide under ice bath, the mixture was stirred for 0.5 hours, then added with o-phenylenediamine (0.024 g, 0.228 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (15 mg, 2.7 µmol) with a yield of 5.4%. LC-MS: m/z: (M+H)⁺ = 549.

¹H NMR (400 MHz, Chloroform-*d*) δ 9.42 (s, 2H), 8.54 (t, *J* = 1.8 Hz, 2H), 8.27 (m, 2H), 8.04 (m, 4H), 7.93 - 7.87 (m, 2H), 7.66 - 7.56 (m, 6H), 7.20 - 7.08 (m, 4H), 6.73 (m, 2H).

### Embodiment 2: 6-(1H-benzo[d]imidazol-2-yl)-N-(3-(3-(3-(imidazolylazo[1,2-a]pyridin-2-yl)benzoyl)-3-azabicyclo[3.1.0]hexane-6-yl)pyridineamide (L-2)

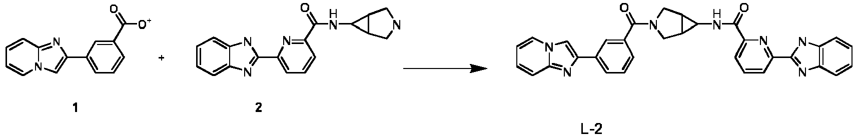

3-(Imidazo[1,2-a]pyridin-2-yl)benzoic acid (0.12 g, 0.5 mmol), 1-(3-dimethylaminopropyl)-3-ethylcarbodiimide hydrochloride (0.1 g, 0.52 mmol), 1-hydroxybenzotriazole (0.07 g, 0.52 mmol) and N,N-diisopropylethylamine (0.14 g, 1.13 mmol) were dissolved in 6 mL of N,N-dimethylformamide under ice bath, the mixture was stirred for 0.5 hours, then added with 6-(1H-benzo[d]imidazol-2-yl)-N-(3-azabicyclo[3.1.0]hexane-6-yl)pyridineamide (0.16 g, 0.5 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (20 mg, 37 µmol) with a yield of 7.4%. LC-MS: m/z: (M+H)⁺ = 540.

¹H NMR (400 MHz, Chloroform-*d*) δ 14.50 (s, 1H), 8.91 (s, 1H), 8.60 (dd, *J* = 7.9, 1.1 Hz, 1H), 8.25 (d, *J* = 6.8 Hz, 1H), 8.19 (dd, *J* = 7.7, 1.0 Hz, 1H), 8.09 - 7.98 (m, 3H), 7.91 (q, *J* = 8.0 Hz, 2H), 7.59 (d, *J* = 9.1 Hz, 1H), 7.50 (t, *J* = 7.7 Hz, 1H), 7.42 - 7.36 (m, 1H), 7.26 - 7.12 (m, 4H), 6.88 (m, 1H), 3.98 (d, *J* = 12.3 Hz, 1H), 3.39 (d, *J* = 11.6 Hz, 2H), 3.19 (d, *J* = 12.8 Hz, 1H), 1.79 (s, 1H), 1.33 (s, 1H), 1.06 (s, 1H).

### Embodiment 3: (9-(3-(1H-benzo[d]imidazol-2-yl)benzoyl)-3,9-diazaspiro[5.5]undecan-3-yl)(3-(imidazol[1,2-a]pyridin-2-yl)phenyl)methanone (L-3)

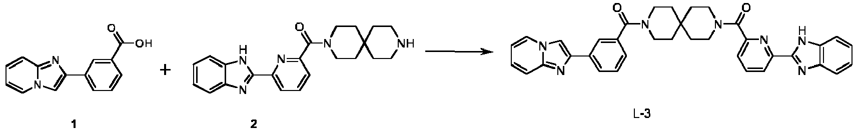

3-(Imidazo[1,2-a]pyridin-2-yl)benzoic acid (0.12 g, 0.5 mmol), 1-(3-dimethylaminopropyl)-3-ethylcarbodiimide hydrochloride (0.1 g, 0.52 mmol), 1-hydroxybenzotriazole (0.07 g, 0.52 mmol) and N,N-diisopropylethylamine (0.14 g, 1.13 mmol) were dissolved in 6 mL of N,N-dimethylformamide under ice bath, the mixture was stirred for 0.5 hours, then added with 6-(1H-benzo[d]imidazol-2-yl)-N-(3-azabicyclo[3.1.0]hexane-6-yl)pyridineamide (0.18 g, 0.5 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (30 mg, 50 µmol) with a yield of 10%. LC-MS: m/z: (M+H)⁺ = 596.

¹H NMR (400 MHz, Chloroform-*d*) δ 10.53 (s, 1H), 8.49 (dd, *J* = 7.9, 1.1 Hz, 1H), 8.15 (m, 1H), 8.06 - 7.94 (m, 3H), 7.89 (d, *J* = 14.0 Hz, 2H), 7.65 (d, *J* = 9.1 Hz, 1H), 7.60 (m, 1H), 7.51 (m, 2H), 7.35 (m, 3H), 7.21 (m, 1H), 6.82 (m, 1H), 3.84 (s, 4H), 3.52 (s, 4H), 1.74 (s, 4H), 1.58 (s, 4H).

### Embodiment 4: (1S, 4S)-5-(6-(1H-benzo[d]imidazol-2-yl)pyridyl)-2,5-diazabicyclo[2.2.1]heptan-2-yl)(3-(imidazolylazo[1,2-a]pyridin-2-yl)phenyl)methanone (L-4)

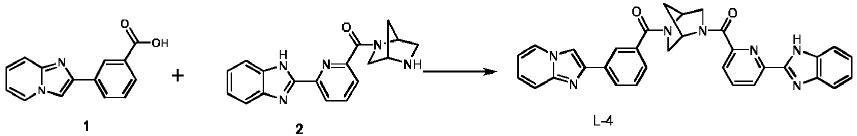

3-(Imidazo[1,2-a]pyridin-2-yl)benzoic acid (0.12 g, 0.5 mmol), 1-(3-dimethylaminopropyl)-3-ethylcarbodiimide hydrochloride (0.1 g, 0.52 mmol), 1-hydroxybenzotriazole (0.07 g, 0.52 mmol) and N,N-diisopropylethylamine (0.14 g, 1.13 mmol) were dissolved in 6 mL of N,N-dimethylformamide under ice bath, the mixture was stirred for 0.5 hours, then added with (6-(1H-benzo[d]imidazol-2-yl)pyridin-2-yl)(1S, 4S)-2,5-diazabicyclo[2.2.1]heptan-2-yl)methanone (0.18 g, 0.5 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (25 mg, 46 µmol) with a yield of 9%. LC-MS: m/z: (M+H)⁺ = 540.

¹H NMR (400 MHz, Chloroform-*d*) δ 10.80 (d, *J* = 23.5 Hz, 1H), 8.57 (d, *J* = 7.3 Hz, 1H), 8.21 - 8.09 (m, 2H), 8.02 (m, 3H), 7.91 (d, *J* = 10.4 Hz, 2H), 7.73 - 7.62 (m, 2H), 7.56 (m, 3H), 7.37 (s, 1H), 7.16 (s, 1H), 6.85 - 6.75 (m, 1H), 4.85 - 4.61 (m, 2H), 4.08 - 3.74 (m, 4H), 2.03 (s, 2H).

### Embodiment 5: N-(1-(6-(1H-benzo[d]imidazol-2-yl)pyridinyl)piperidin-4-yl)-3-(imidazol[1,2-a]pyridin-2-yl)benzamide (L-5)

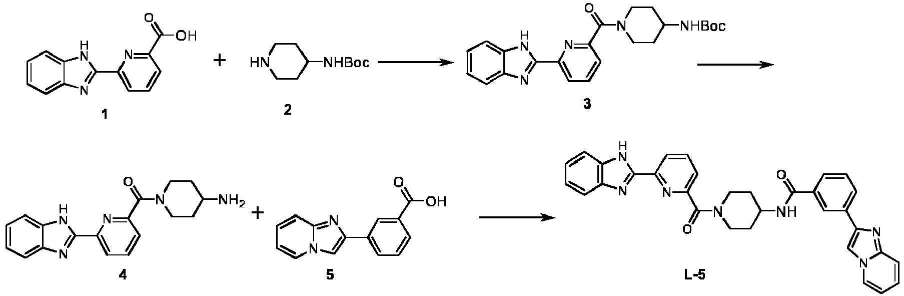

### I. Synthesis of tert-butyl (1-(6-(1H-benzo[d]imidazol-2-yl)picolinoyl)piperidin-4-yl)carbamate

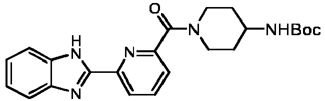

6-(1H-benzo[d]imidazol-2-yl)picolinic acid (1 g, 4.2 mmol), 1-(3-dimethylaminopropyl)-3-ethylcarbodiimide hydrochloride (0.96 g, 5 mmol), 1-hydroxybenzotriazole (0.66 g, 5 mmol) and N,N-diisopropylethylamine (1.19 g, 9.2 mmol) were dissolved in 20 mL of N,N-dimethylformamide under ice bath, the mixture was stirred for 0.5 hours, then added with *tert*-butyl piperidin-4-yl carbamate (0.92 g, 4.6 mmol), stirred continuously for 0.5 hours, returned to room temperature and reacted overnight. The reaction mixture was poured into 80 mL of water, extracted with ethyl acetate (80 mL × 3), and washed with saturated brine. The organic phases were combined, dried over anhydrous sodium sulfate, concentrated under reduced pressure, and purified by silica gel column chromatography (PE:EA = 5:1) to obtain an off-white solid (1.3 g, 3 mmol) with a yield of 73%. LC-MS: m/z: (M+H)⁺ = 421.

### II. Synthesis of 6-(1H-benzo[d]imidazol-2-yl)pyridin-2-yl)(4-aminopiperidin-1-yl)methanone

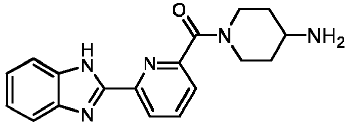

*tert*-Butyl (1-(6-(1H-benzo[d]imidazol-2-yl)picolinoyl)piperidin-4-yl)carbamate (1.3 g, 3 mmol) was dissolved in 20 mL of dichloromethane under ice bath, the mixture was added with a saturated solution of 1,4-dioxane in hydrochloric acid (2 mL, 8 mmol), stirred continuously for 0.5 hours, returned to room temperature and reacted overnight. The reaction mixture was concentrated under reduced pressure, and the resulting solid was directly used in the next reaction step. Yield > 90%. LC-MS: 321[M+1]⁺

### Synthesis of N-(1-(6-(1H-benzo[d]imidazol-2-yl)pyridinyl)piperidin-4-yl)-3-(imidazol[1,2-a]pyridin-2-yl)benzatnide

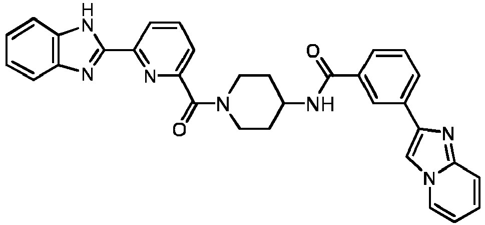

III. 3-(Imidazo[1,2-a]pyridin-2-yl)benzoic acid (0.12 g, 0.5 mmol), 1-(3-dimethylaminopropyl)-3-ethylcarbodiimide hydrochloride (0.1 g, 0.52 mmol), 1-hydroxybenzotriazole (0.07 g, 0.52 mmol) and N,N-diisopropylethylamine (0.14 g, 1.13 mmol) were dissolved in 6 mL of N,N-dimethylformamide under ice bath, the mixture was stirred for 0.5 hours, then added with (6-(1H-benzo[d]imidazol-2-yl)pyridin-2-yl)(4-aminopiperidin-1-yl)methanone (0.18 g, 0.5 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (15 mg, 27 µmol) with a yield of 5.5%. LC-MS: m/z: (M+H)⁺ = 542.

¹H NMR (400 MHz, Chloroform-*d*) δ 11.87 (s, 1H), 8.56 (dd, *J* = 7.9, 1.1 Hz, 1H), 8.40 (t, *J* = 1.7 Hz, 1H), 8.21 (m, 1H), 8.07 - 7.94 (m, 3H), 7.90 - 7.80 (m, 2H), 7.69 (d, *J* = 9.1 Hz, 1H), 7.61 (dd, *J* = 7.7, 1.1 Hz, 1H), 7.48 (m, 2H), 7.28 - 7.17 (m, 3H), 6.87 (m, 1H), 6.25 (d, *J* = 7.9 Hz, 1H), 4.68 (d, *J* = 13.7 Hz, 1H), 4.25 (dd, *J* = 7.7, 3.9 Hz, 1H), 3.85 (d, *J* = 13.9 Hz, 1H), 3.19 (t, *J* = 12.6 Hz, 1H), 3.02 - 2.91 (m, 1H), 2.12 (s, 1H), 1.92 (d, *J* = 12.8 Hz, 2H), 1.61 (m, 1H).

### Embodiment 6: 6-(1H-benzo[d]imidazol-2-yl)-N-(1-(3-(imidazol[1,2-a]pyridin-2-yl)benzoyl)pyrrolidin-3-yl)pyridineamide (L-6)

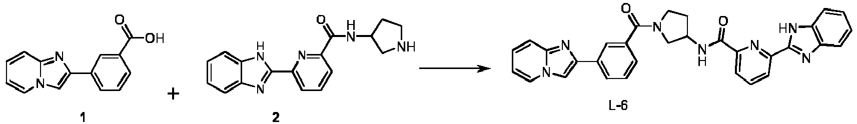

3-(Imidazo[1,2-a]pyridin-2-yl)benzoic acid (0.12g, 0.5 mmol), 1-(3-dimethylaminopropyl)-3-ethylcarbodiimide hydrochloride (0.1 g, 0.52 mmol), 1-hydroxybenzotriazole (0.07 g, 0.52 mmol) and N,N-diisopropylethylamine (0.14 g, 1.13 mmol) were dissolved in 6 mL of N,N-dimethylformamide under ice bath, the mixture was stirred for 0.5 hours, then added with 6-(1H-benzo[d]imidazol-2-yl)-N-(pyrrolidin-3-yl)pyridineamide (0.14 g, 0.5 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (18 mg, 34 µmol) with a yield of 5.5%.

LC-MS m/z: (M+H)⁺ = 528, ¹H NMR (400 MHz, Chloroform-*d*) δ 8.64 - 8.44 (m, 2H), 8.08 (d, *J* = 6.9 Hz, 1H), 8.02 - 7.67 (m, 7H), 7.62 - 7.44 (m, 3H), 7.28 - 7.11 (m, 4H), 6.83 (t, *J* = 6.6 Hz, 1H), 5.40 - 5.34 (m, 1H), 4.92 (d, *J* = 17.1 Hz, 1H), 4.69 - 4.61 (m, 1H), 4.09 (m, 1H), 4.02 - 3.72 (m, 3H).

### Embodiment 7: N,N'-(1,2-phenylene)bis(6-(1H-benzo[d]imidazol-2-yl)picolinamide) (L-7)

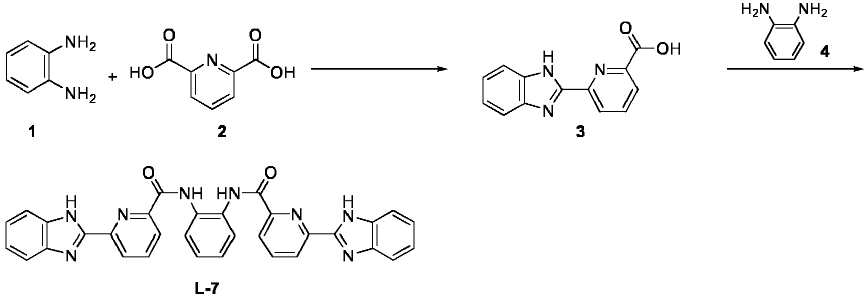

### I. Synthesis of 6-(1H-benzo[d]imidazol-2-yl)picolinic acid

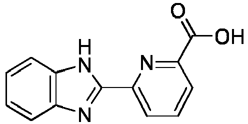

To a solution of o-phenylenediamine (3.5 g, 32 mmol) in propylene glycol (100 mL) was added pyridine 2,6-dicarboxylic acid (5 g, 21.919 mmol), the resulting mixture was heated to reflux for 24 hours, and then cooled to room temperature. The reaction mixture was added with ice water (50 mL), and stirred to precipitate a brown solid. The precipitate was collected and dissolved in hot methanol, and the solution was filtered with activated carbon. The resulting filtrate was slowly evaporated to remove the solvent to obtain 6-(1H-benzimidazole-2-)pyridinecarboxylic acid. Yield is 52%. LC-MS: [M+1]⁺: 240.1

### II. Synthesis of N,N'-(1,2-phenylene)bis(6-(1H-benzo[d]imidazol-2-yl)picolinamide)

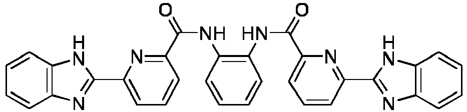

6-(1H-benzimidazole-2-)pyridinecarboxylic acid (0.12 g, 0.5 mmol), 1-(3-dimethylaminopropyl)-3-ethylcarbodiimide hydrochloride (0.1 g, 0.52 mmol), 1-hydroxybenzotriazole (0.07 g, 0.52 mmol) and N,N-diisopropylethylamine (0.14 g, 1.13 mmol) were dissolved in 6 mL of N,N-dimethylformamide under ice bath, the mixture was stirred for 0.5 hours, then added with o-phenylenediamine (0.024 g, 0.228 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (30 mg, 5.4 µmol) with ayield of 11%. LC-MS: m/z: (M+H)⁺ = 550.98, ¹H NMR (400 MHz, DMSO) δ 12.58 (s, 2H), 11.01 (s, 2H), 8.20 (d, *J* = 7.3 Hz, 2H), 8.07 - 7.80 (m, 6H), 7.67 - 7.53 (m, 2H), 7.43 (dd, *J* = 5.7, 3.6 Hz, 2H), 7.25 - 7.09 (m, 4H), 7.03 (d, *J* = 5.5 Hz, 2H).

### Embodiment 8: N,N'-(cyclohexane-1,2-diyl)bis(6-(1H-benzo[d]imidazol-2-yl)picolinamide) (L-8)

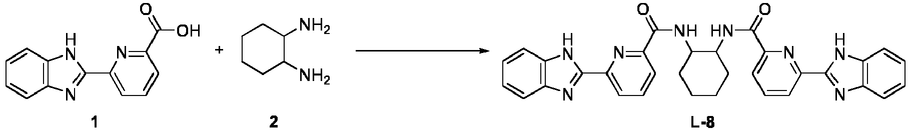

### N,N'-(cyclohexane-1,2-diyl)bis(6-(1H-benzo[d]imidazol-2-yl)picolinamide)

Same operation as L-7, cyclohexane-1,2-diamine was purchased from Sinopharm Reagent.

LC-MS: m/z: (M+H)⁺ = 557.26, ¹H NMR (400 MHz, CDCl₃) δ 8.83 (s, 2H), 8.42 (d, *J* = 7.8 Hz, 2H), 8.03 (d, *J* = 7.7 Hz, 2H), 7.79 (t, *J* = 7.8 Hz, 6H), 7.32 (dd, *J* = 5.8, 3.1 Hz, 2H), 4.13 (d, *J* = 16.6 Hz, 2H), 2.35 (d, *J* = 11.4 Hz, 2H), 1.94 - 1.37 (m, 6H).

### Embodiment 9: (3,9-diazaspiro[5.5]undecane-3,9-diyl)bis((6-(1H-benzo[d]imidazol-2-yl)pyridin-2-yl)methanone) (L-9)

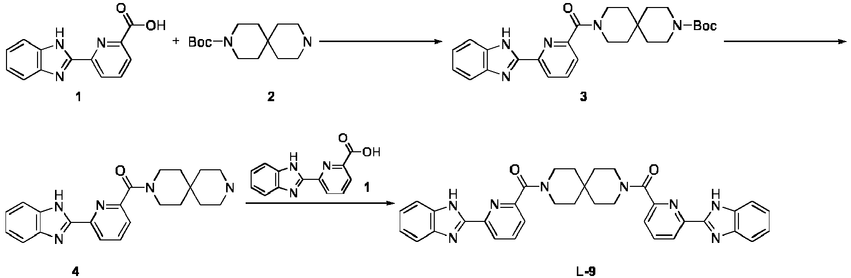

### I. Synthesis of tert-butyl (9-(6-(1H-benzo[d]imidazol-2-yl)picolinoyl)-3,9-diazaspiro [5.5]undecane-3 -carboxylate

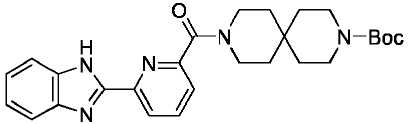

6-(1H-benzimidazole-2-)pyridinecarboxylic acid (1 g, 4.2 mmol), 1-(3-dimethylaminopropyl)-3-ethylcarbodiimide hydrochloride (0.96 g, 5 mmol), 1-hydroxybenzotriazole (0.66 g, 5 mmol) and N,N-diisopropylethylamine (1.19 g, 9.2 mmol) were dissolved in 20 mL ofN,N-dimethylformamide under ice bath, the mixture was stirred for 0.5 hours, then added with *tert*-butyl 3,9-diazaspiro[5.5]undecane-3-carboxylate (0.92 g, 4.6 mmol), stirred continuously for 0.5 hours, returned to room temperature and reacted overnight. The reaction mixture was poured into 80 mL of water, extracted with ethyl acetate (80 mL × 3), and washed with saturated brine. The organic phases were combined, dried over anhydrous sodium sulfate, concentrated under reduced pressure, and purified by silica gel column chromatography (PE:EA = 5:1) to obtain an off-white solid (1.3 g, 3 mmol) with a yield of 73%. LC-MS: m/z: (M+H)⁺ = 476.

### II. Synthesis of (6-(1H-benzo[d]imidazol-2-yl)pyridin-2-yl)(3,9-diazaspiro[5.5]undecan-3-yl)methanone

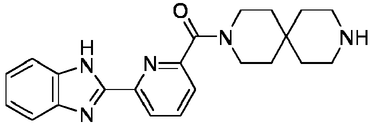

*tert*-Butyl 9-(6-(1H-benzo [d]imidazol-2-yl)picolinoyl)-3,9-diazaspiro [5.5]undecane-3 - carboxylate (1.3 g, 3 mmol) was dissolved in 20 mL of dichloromethane under ice bath, then the mixture was added with a saturated solution of 1,4-dioxane in hydrochloric acid (2 mL, 8 mmol), stirred continuously for 0.5 hours, returned to room temperature and reacted overnight. The reaction mixture was concentrated under reduced pressure, and the resulting solid was directly used in the next reaction step. Yield > 90%. LC-MS: 376[M+1]⁺.

### III. Synthesis of (3,9-diazaspiro[5.5]undecane-3,9-diyl)bis((6-(1H-benzo[d]imidazol-2-yl)pyridin-2-yl)methanone)

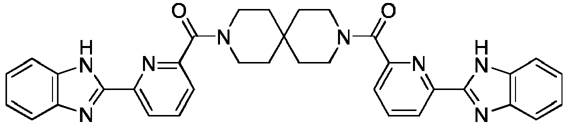

6-(1H-benzimidazole-2-)pyridinecarboxylic acid (0.14 g, 0.27 mmol) and N,N-diisopropylethylamine (0.12 g, 1.0 mmol) were dissolved in 6 mL of N,N-dimethylformamide under ice bath, the mixture was stirred for 0.5 hours, then added with (6-(1H-benzo[d]imidazol-2-yl)pyridin-2-yl)(3,9-diazaspiro[5.5]undecan-3-yl)methanone (0.089 g, 0.27 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (20 mg, 38 µmol) with a yield of 14.3%.

LC-MS: m/z: (M+H)⁺ = 598.1, ¹H NMR (400 MHz, CDCl₃) δ 10.70 (s, 2H), 8.49 (d, *J* = 7.9 Hz, 2H), 7.93 (dd, *J* = 25.5, 17.6 Hz, 4H), 7.56 (t, *J* = 8.1 Hz, 5H), 7.38 - 7.30 (m, 5H), 3.84 (s, 3H), 3.51 (s, 5H), 1.65 (d, *J* = 67.9 Hz, 8H).

### Embodiment 10: (tetrahydropyrrolo[3,4-c]pyrrole-2,5(1H,3H)-diyl)bis((6-(1H-benzo[d]imidazol-2-yl)pyridin-2-yl)methanone) (L-10)

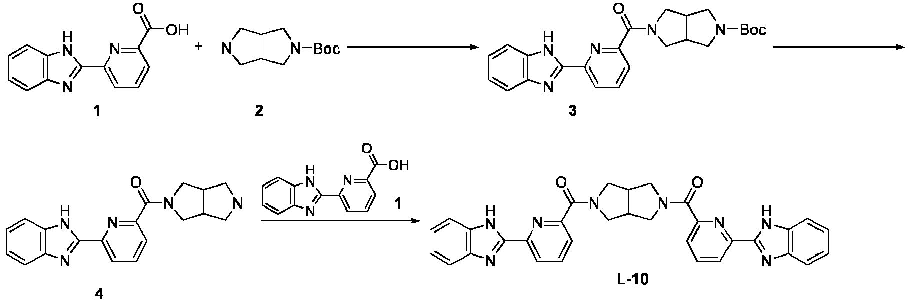

### I. Synthesis of tert-butyl 5-(6-(1H-benzo[d]imidazol-2-yl)picolinoyl)tert-hydropyrrolo[3,4-c]pyrrole-2(1H)-carboxylate

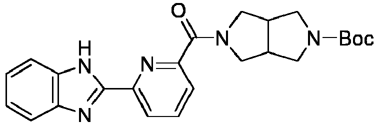

6-(1H-benzimidazole-2-)pyridinecarboxylic acid (1 g, 4.2 mmol), 1-(3-dimethylaminopropyl)-3-ethylcarbodiimide hydrochloride (0.96 g, 5 mmol), 1-hydroxybenzotriazole (0.66 g, 5 mmol) and N,N-diisopropylethylamine (1.19 g, 9.2 mmol) were dissolved in 20 mL of N,N-dimethylformamide under ice bath, the mixture was stirred for 0.5 hours, then added with *tert*-butyl hexahydropyrrolo[3,4-c]pyrrole-2(1H)-carboxylate (0.92 g, 4.6 mmol), stirred continuously for 0.5 hours, returned to room temperature and reacted overnight. The reaction mixture was poured into 80 mL of water, extracted with ethyl acetate (80 mL × 3), and washed with saturated brine. The organic phases were combined, dried over anhydrous sodium sulfate, concentrated under reduced pressure, and purified by silica gel column chromatography (PE:EA = 5:1) to obtain an off-white solid (1.3 g, 3 mmol) with a yield of 73%. LC-MS: m/z: (M+H)⁺ = 434.

### II. Synthesis of (6-(1H-benzo[d]imidazol-2-yl)pyridin-2-yl)(hexahydropyrrolo[3,4-c]pyrrol-2(1H)-yl)methanone

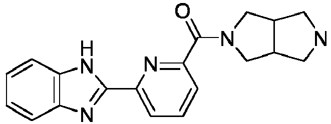

*tert*-Butyl 5-(6-(1H-benzo[d]imidazol-2-yl)picolinoyl)*tert*-hydropyrrolo[3,4-c]pyrrole-2(1H)-carboxylate (1.3 g, 3 mmol) was dissolved in 20 mL of dichloromethane under ice bath, the mixture was added with a saturated solution of 1,4-dioxane in hydrochloric acid (2 mL, 8 mmol), stirred continuously for 0.5 hours, returned to room temperature and reacted overnight. The reaction mixture was concentrated under reduced pressure, and the resulting solid was directly used in the next reaction step. Yield > 90%. LC-MS: m/z: (M+H)⁺ = 334.

### III. Synthesis of (tetrahydropyrrolo[3,4-c]pyrrole-2,5(1H,3H)-diyl)bis((6-(1H-benzo[d]imidazol-2-yl)pyridin-2-yl)methanone)

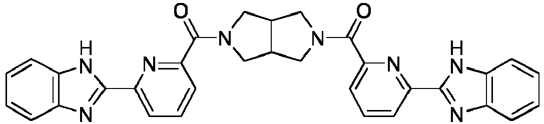

### Same operation as L-9

### (Tetrahydropyrrolo[3,4-c]pyrrole-2,5(1H,3H)-diyl)bis((6-(1H-benzo[d]imidazol-2-yl)pyridin-2-yl)methanone)

LC-MS: m/z: (M+H)⁺ = 555.61, ¹H NMR (400 MHz, MeOD) δ 8.49 - 8.29 (m, 3H), 8.23 - 8.04 (m, 3H), 8.03 - 7.93 (m, 1H), 7.86 (dd, *J* = 7.0, 5.6 Hz, 1H), 7.71 (dd, *J* = 6.0, 3.1 Hz, 1H), 7.64 - 7.54 (m, 2H), 7.50 (dd, *J* = 8.1, 5.7 Hz, 2H), 7.41 - 7.28 (m, 2H), 7.26 - 7.17 (m, 1H), 7.01 - 6.87 (m, 1H), 4.23 - 3.55 (m, 8H), 3.27 - 3.04 (m, 2H).

### Embodiment 11: N-(3-(6-(1H-benzo[d]imidazol-2-yl)picolinoyl)-3-azabicyclo[3.1.0]hexan-6-yl)-6-(1H-benzo[d]imidazol-2-yl)pyridinecarboxamide (L-11)

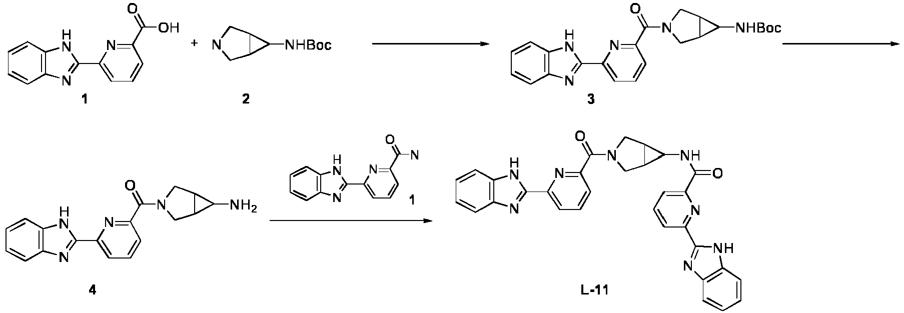

### I. Synthesis of tert-butyl (3-(6-(1H-benzo[d]imidazol-2-yl)picolinoyl)-3-azabicyclo [3.1.0]hexan-6-yl)carbamate

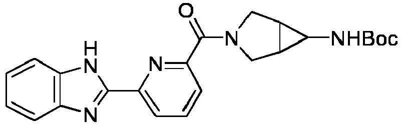

6-(1H-benzimidazole-2-)pyridinecarboxylic acid (1 g, 4.2 mmol), 1-(3-dimethylaminopropyl)-3-ethylcarbodiimide hydrochloride (0.96 g, 5 mmol), 1-hydroxybenzotriazole (0.66 g, 5 mmol) and N,N-diisopropylethylamine (1.19 g, 9.2 mmol) were dissolved in 20 mL of N,N-dimethylformamide under ice bath, the mixture was stirred for 0.5 hours, then added with tert-butyl (3-azabicyclo[3.1.0]hexan-6-yl)carbamate (0.92 g, 4.6 mmol), stirred continuously for 0.5 hours, returned to room temperature and reacted overnight. The reaction mixture was poured into 80 mL of water, extracted with ethyl acetate (80 mL × 3), and washed with saturated brine. The organic phases were combined, dried over anhydrous sodium sulfate, concentrated under reduced pressure, and purified by silica gel column chromatography (PE:EA = 5:1) to obtain an off-white solid (1.3 g, 3 mmol) with a yield of 73%. LC-MS: m/z: (M+H)⁺ = 421.

### II. Synthesis of (6-(1H-benzo[d]imidazol-2-yl)pyridin-2-yl)(6-amino-3-azabicyclo[3.1.0]hexan-3-yl)methanone

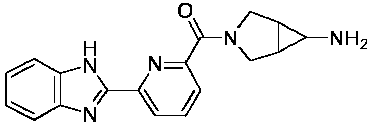

*tert*-Butyl (3-(6-(1H-benzo[d]imidazol-2-yl)picolinoyl)-3-azabicyclo[3.1.0]hexan-6-yl)carbamate (1.3 g, 3 mmol) was dissolved in 20 mL of dichloromethane under ice bath, the mixture was added with a saturated solution of 1,4-dioxane in hydrochloric acid (2 mL, 8 mmol), stirred continuously for 0.5 hours, returned to room temperature and reacted overnight. The reaction mixture was concentrated under reduced pressure, and the resulting solid was directly used in the next reaction step. Yield > 90%. LC-MS: m/z: (M+H)⁺ = 321.

### III. Synthesis of N-(3-(6-(1H-benzo[d]imidazol-2-yl)picolinoyl)-3-azabicyclo[3.1.0]hexan-6-yl)-6-(1H-benzo[d]imidazol-2-yl)pyridinecarboxamide

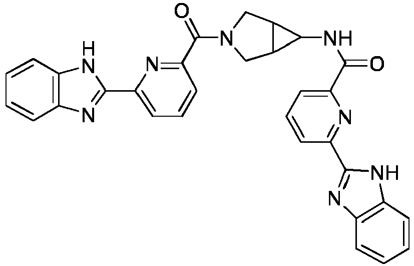

### Same operation as L-9

LC-MS: m/z: (M+H)⁺ = 541.20, ¹H NMR (400 MHz, CDCl₃) δ 13.25 (s, 1H), 12.09 (s, 1H), 8.68 (s, 1H), 8.48 (d, *J* = 7.6 Hz, 1H), 8.26 (d, *J* = 7.6 Hz, 1H), 8.01 (d, *J* = 7.5 Hz, 1H), 7.81 (t, *J* = 7.8 Hz, 4H), 7.50 (dt, *J* = 20.7, 5.8 Hz, 3H), 7.38 - 7.30 (m, 4H), 4.58 (d, *J* = 11.3 Hz, 1H), 4.45 - 4.22 (m, 2H), 3.64 - 3.43 (m, 3H), 2.63 (s, 1H), 2.00 (s, 9H), 1.60 (s, 2H).

### Embodiment 12: (2,5-diazabicyclo[2.2.1]heptane-2,5-diyl)bis((6-(1H-benzo[d]imidazol-2-yl)pyridin-2-yl)methanone) (L-12)

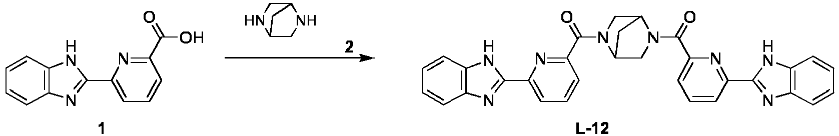

### (2,5-diazabicyclo[2.2.1]heptane-2,5-diyl)bis((6-(1H-benzo[d]imidazol-2-yl)pyridin-2-yl)methanone)

### Same operation as L-7

LC-MS: m/z: (M+H)⁺ = 541.59, ¹H NMR (400 MHz, CDCl₃) δ 12.67 (s, 1H), 8.13 - 7.82 (m, 4H), 7.54 - 7.33 (m, 4H), 7.26 (d, *J* = 7.6 Hz, 2H), 4.54 (d, *J* = 11.9 Hz, 3H), 4.04 (t, *J* = 15.9 Hz, 3H), 1.85 (s, 1H), 1.77 (s, 1H).

### Embodiment 13: N,N'-(1,2-phenylene)bis(2-morpholinopyrimidine-4-carboxamide) (L-13)

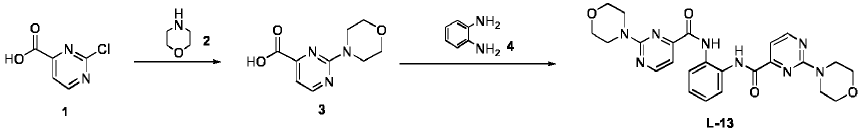

### I. Synthesis of 2-morpholinopyrimidine-4-carboxylic acid

2-Chloropyrimidine-4-carboxylic acid (500 mg, 3.15 mmol) was dissolved in 15 mL of tetrahydrofuran and 15 mL of dioxane, then 2 mL of morpholine was added, and the reaction mixture was stirred at 70°C for 18 hours. The reaction mixture was cooled to room temperature and filtered. The solid was dissolved in 20 mL of water, then acidified with 1 N hydrochloric acid to adjust the pH to 1, and the solution was extracted with dichloromethane/methanol = 10:1 (20 mL × 3). The combined organic phases were dried over anhydrous sodium sulfate, filtered, and concentrated to obtain a white solid of 580 mg with a yield of 87.9%. LC-MS: m/z: (M+H)⁺ = 210.0.

¹H NMR (400 MHz, CD₃OD) δ 8.57 (d, *J* = 4.8 Hz, 1H), 7.20 (d, *J* = 4.8 Hz, 1H), 3.93 - 3.83 (m, 4H) , 3.76 (m, 4H).

### II. Synthesis of N,N'-(1,2-phenylene)bis(2-morpholinopyrimidine-4-carboxamide)

2-Morpholinopyrimidine-4-carboxylic acid (100 mg, 0.48 mmol) (compound as shown in formula 3) was suspended in 2 mL of N,N-dimethylformamide, then added with 1-(3-dimethylaminopropyl)-3-ethylcarbodiimide hydrochloride (137 mg, 0.72 mmol), 1-hydroxybenzotriazole (97 mg, 0.72) and N,N-diisopropylethylamine (185 mg, 1.4340 mmol). The reaction mixture was stirred for 20 minutes, then added with o-phenylenediamine (25.8 mg, 0.24 mmol), and the reaction mixture was stirred at 15°C for 16 hours. The reaction mixture was added with 10 mL of water, and extracted with ethyl acetate (10 mL × 2). The combined organic layers were dried over anhydrous sodium sulfate, filtered, and concentrated to obtain a crude product. The resulting crude product was purified by thin-layer chromatography plate (dichloromethane: methanol = 10:1). The obtained product was slurried with a mixture of dimethyl sulfoxide (3 mL) and methanol (2 mL), and filtered. The solid was washed with methanol, and dried to obtain 23 mg of the desired product as a white solid with a yield of 9.81%.

LC-MS: m/z: (M+H)⁺ = 491.0, ¹H NMR (400 MHz, DMSO-*d₆*) δ 10.46 (s, 2H), 8.70 (d, *J* = 4.8 Hz, 2H), 7.78 (dd, *J* = 6.0, 3.6 Hz, 2H),7.36 (dd, *J* = 6.0, 3.6 Hz, 2H), 7.26 (d, *J* = 4.8 Hz, 2H), 3.90 - 3.69 (m, 4H), 3.60 (d, *J* = 4.4 Hz, 4H).

### Embodiment 14: N-(3-(6-(1H-benzo[d]imidazol-2-yl)picolinoyl)-3-azabicyclo[3.1.0]hexan-6-yl)-2-morpholinopyrimidine-4-carboxamide (L-14)

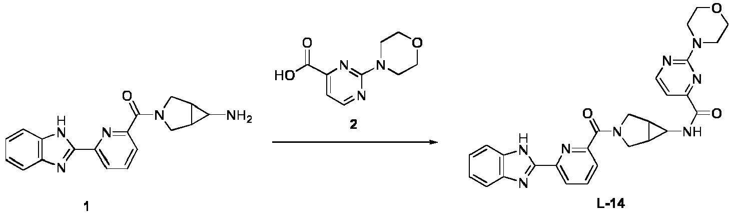

### N-(3-(6-(1H-benzo[d]imidazol-2-yl)picolinoyl)-3-azabicyclo[3.1.0]hexan-6-yl)-2-morpholinopyrimidine-4-carboxamide

2-Morpholinopyrimidine-4-carboxylic acid (100 mg, 0.48 mmol) (compound as shown in formula 3) was suspended in 2 mL of N,N-dimethylformamide, then added with 1-(3-dimethylaminopropyl)-3-ethylcarbodiimide hydrochloride (137 mg, 0.72 mmol), 1-hydroxybenzotriazole (97 mg, 0.72) and N,N-diisopropylethylamine (185 mg, 1.4340 mmol). The reaction mixture was stirred for 20 minutes, then added with (6-amino-3-azabicyclo[3.1.0]hexan-3-yl)-[6-(1H-benzimidazol-2-yl)-2-pyridyl]methanone (76 mg, 0.24 mmol), and the reaction mixture was stirred at 15°C for 16 hours. The reaction mixture was added with 10 mL of water, and extracted with ethyl acetate (10 mL × 2). The combined organic layers were dried over anhydrous sodium sulfate, filtered, and concentrated to obtain a crude product. The resulting crude product was purified by thin-layer chromatography plate (dichloromethane: methanol = 10:1) to obtain 38 mg of the desired product as a white solid with a yield of 31.14%.

LC-MS: m/z: (M+H)⁺ = 511.0, ¹H NMR (400 MHz, DMSO-*d₆*) δ 8.75 (s, 1H), 8.61 (d, *J* = 7.8 Hz, 1H), 8.35 (d, *J* = 4.8 Hz, 1H), 8.22 (t, *J* =13.6 Hz, 1H), 7.99 (t, *J* = 7.8 Hz, 1H), 7.63 (s, 2H), 7.31 (dd, *J* = 6.6, 3.5 Hz, 1H), 6.78 (d, *J* = 4.8 Hz,1H), 4.33 (d, *J* = 12.5 Hz, 1H), 4.20 - 4.10 (m, 2H), 3.98 (dd, *J* = 11.8, 4.4 Hz, 1H), 3.72 (dd, *J* = 12.9, 4.8Hz, 9H), 2.56 (d, *J* = 2.1 Hz, 1H), 2.12 (d, *J* = 2.5 Hz, 1H), 2.04 (d, *J* = 4.7 Hz, 1H).

### Embodiment 15: 9-(6-(1-H-benzo[d]imidazol-2-yl)picolinoyl)-3,9-diazaspiro[5.5]undecan-3-yl)(2-morpholinopyrimidin-4-yl)methanone (L-15)

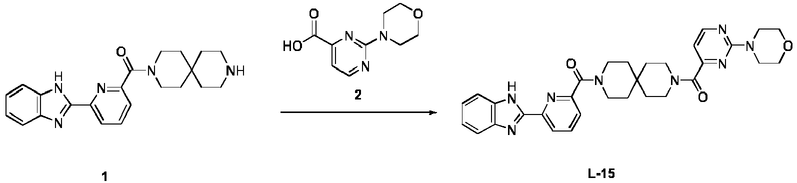

### (9-(6-(1-H-benzo[d]imidazol-2-yl)picolinoyl)-3,9-diazaspiro[5.5]undecan-3-yl)(2-morpholinopyrimidin-4-yl)methanone

### Same operation as L-14

LC-MS: m/z: (M+H)⁺ = 567.0, ¹H NMR (400 MHz, CD₃OD) δ 8.53 (d, *J* = 7.8 Hz, 1H), 8.44 (d, *J* = 4.8 Hz, 1H), 7.97 (t, *J* = 7.8 Hz, 1H),7.73 (s, 2H), 7.59 (dd, *J* = 7.7, 0.9 Hz, 1H), 7.39 - 7.31 (m, 2H), 6.70 (d, *J* = 4.8 Hz, 1H), 3.90-3.76 (m, 12H), 3.51 (d, *J* = 6.9 Hz, 4H), 1.75-1.69 (m, 4H), 1.59-1.57 (m, 4H).

### Embodiment 16: (5-(6-(1-(1-H-benzo[d]imidazol-2-yl)picolinoyl)hexahydropyrrolo[3,4-c]pyrrol-2(1H)-yl)(2-morpholinopyrimidin-4-yl)methanone (L-16)

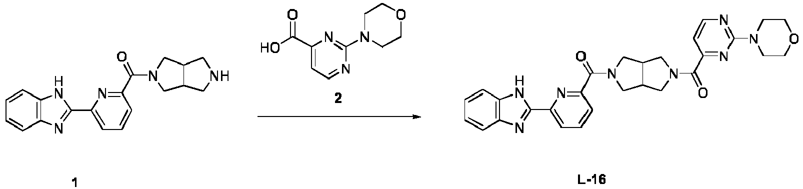

### (5-(6-(1-(1-H-benzo[d]imidazol-2-yl)picolinoyl)hexahydropyrrolo[3,4-c]pyrrol-2(1H)-yl)(2-morpholinopyrimidin-4-yl)methanone

### Same operation as L-14

LC-MS: m/z: (M+H)⁺ = 525.0, ¹H NMR (400 MHz, CDCl₃) δ 8.60 - 8.37 (m, 2H), 8.00 (dd, *J* = 17.8, 9.9 Hz, 1H), 7.81 (ddd, *J* = 14.2,8.7, 2.6 Hz, 2H), 7.65 (d, *J* = 4.6 Hz, 1H), 7.33 (dd, *J* = 6.0, 3.1 Hz, 2H), 6.97 (dd, *J* = 10.5, 4.8 Hz, 1H),4.25 - 3.56 (m, 16H), 3.21-2.81 (m, 2H).

### Embodiment 17: N,N'-(cyclohexane-1,2-diyl)bis(2-morpholinopyrimidine-4-carboxamide) (L-17)

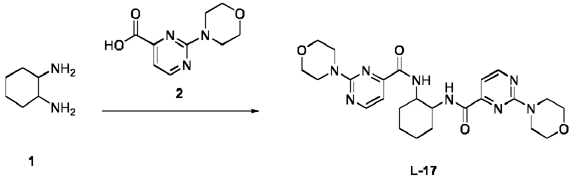

### N,N'-(cyclohexane-1,2-diyl)bis(2-morpholinopyrimidine-4-carboxamide)

### Same operation as L-7

LC-MS: m/z: (M+H)⁺ = 497.0, ¹H NMR (400 MHz, CD₃OD) δ 8.48 (d, *J* = 4.8 Hz, 2H), 8.14 -7.99 (m, 2H), 7.18 (d, *J* = 4.8 Hz, 2H), 3.96(s, 2H), 3.82-3.80 (m, 16H), 2.24 (d, *J* = 6.8 Hz, 2H), 1.85 (s, 2H), 1.45 (d, *J* = 5.2 Hz, 4H).

### Embodiment 18: (5-(6-(1H-benzo[d]imidazol-2-yl)picolinoyl)-2,5-diazabicyclo[2.2.1]heptan-2-yl)(2-morpholinopyrimidin-4-yl)methanone (L-18)

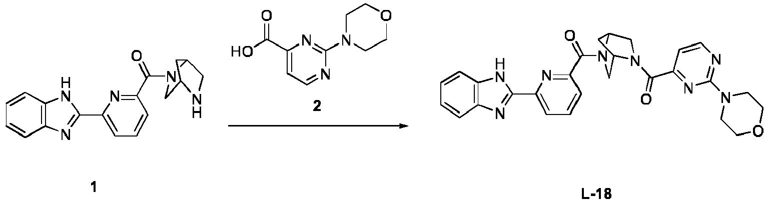

### (5-(6-(1H-benzo[d]imidazol-2-yl)picolinoyl)-2,5-diazabicyclo[2.2.1]heptan-2-yl)(2-morpholinopyrimidin-4-yl)methanone

### Same operation as L-14

LC-MS: m/z: (M+H)⁺ = 511.0, ¹H NMR(400 MHz, CDCl₃) δ 8.67 - 8.41 (m, 2H), 8.06 - 7.87 (m, 4H), 7.38 - 7.35 (m, 2H), 7.19 - 6.98 (m, 1H), 5.33 - 5.00 (m, 2H), 4.20 - 3.41 (m, 12H), 2.11-1.98 (m, 2H).

### Embodiment 19: (N-(1-(1-(6-(1H-benzo[d]imidazol-2-yl)picolinoyl)piperidin-4-yl)-2-(phenylamino)pyrimidine-4-carboxamide (L-19)

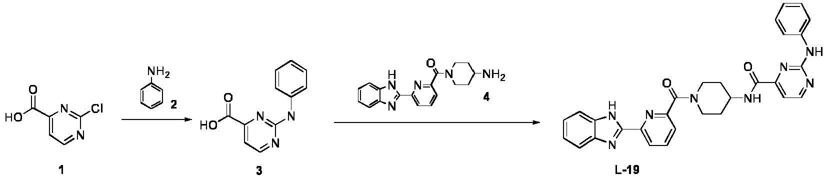

### I. 2-(phenylamino)pyrimidine-4-carboxylic acid

2-Chloropyrimidine-4-carboxylic acid (500 mg, 3.15 mmol) was dissolved in 15 mL of dioxane, then aniline (881 mg, 9.46 mmol) was added, and the reaction mixture was stirred at 70°C for 18 hours. The reaction mixture was cooled to room temperature, then 20 mL of water and 10 mL of 1 N sodium hydroxide were added, and the reaction mixture was extracted twice with ethyl acetate (20 mL × 2). The aqueous phase was acidified with 1 N hydrochloric acid to adjust the pH to 3, and the solid was filtered and dried to obtain 500 mg of a white solid with a yield of 73.67%. LC-MS: m/z: (M+H)⁺ = 216.0.

### II. Synthesis of tert-butyl (1-(6-(1H-benzo[d]imidazol-2-yl)picolinoyl)piperidin-4-yl)carbamate

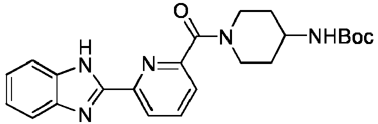

6-(1H-benzimidazole-2-)pyridinecarboxylic acid (1 g, 4.2 mmol), 1-(3-dimethylaminopropyl)-3-ethylcarbodiimide hydrochloride (0.96 g, 5 mmol), 1-hydroxybenzotriazole (0.66 g, 5 mmol) and N,N-diisopropylethylamine (1.19 g, 9.2 mmol) were dissolved in 20 mL of N,N-dimethylformamide under ice bath, the mixture was stirred for 0.5 hours, then added with *tert*-butyl piperidin-4-yl carbamate (0.92 g, 4.6 mmol), stirred continuously for 0.5 hours, returned to room temperature and reacted overnight. The reaction mixture was poured into 80 mL of water, extracted with ethyl acetate (80 mL × 3), and washed with saturated brine. The organic phases were combined, dried over anhydrous sodium sulfate, concentrated under reduced pressure, and purified by silica gel column chromatography (PE:EA = 5:1) to obtain an off-white solid (1.3 g, 3 mmol) with a yield of 73%. LC-MS: m/z: (M+H)⁺ = 421.

### III. Synthesis of (6-(1H-benzo[d]imidazol-2-yl)pyridin-2-yl)(4-aminopiperidin-1-yl)methanone

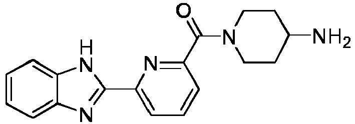

*Tert*-butyl (1-(6-(1H-benzo[d]imidazol-2-yl)picolinoyl)piperidin-4-yl)carbamate (1.3 g, 3 mmol) was dissolved in 20 mL of dichloromethane under ice bath, the mixture was added with a saturated solution of 1,4-dioxane in hydrochloric acid (2 mL, 8 mmol), stirred continuously for 0.5 hours, returned to room temperature and reacted overnight. The reaction mixture was concentrated under reduced pressure, and the resulting solid was directly used in the next reaction step. Yield > 90%. LC-MS: 321[M+1]⁺.

### IV (N-(1-(1-(6-(1H-benzo[d]imidazol-2-yl)picolinoyl)piperidin-4-yl)-2-(phenylamino)pyrimidine-4-carboxamide

### Same operation as L-14

LC-MS: m/z: (M+H)⁺ = 519.0, ¹H NMR (400 MHz, CD₃OD) δ 8.64 (d, *J* = 4.9 Hz, 1H), 8.40 (dd, *J* = 8.0, 1.0 Hz, 1H), 8.13 (t, *J* = 7.9 Hz,1H), 7.76 - 7.58 (m, 5H), 7.40 - 7.26 (m, 5H), 7.08 - 6.99 (m, 1H), 4.64 (d, *J* = 13.6 Hz, 1H), 4.27 - 4.15(m, 1H), 3.85 (d, *J* = 14.1 Hz, 1H), 3.43 (dd, *J* = 18.3, 7.0 Hz, 1H), 3.31 - 3.20 (m, 1H), 2.19 (d, *J* = 11.0Hz, 1H), 2.00 (s, 1H), 1.72-1.79 (m, 10.0 Hz, 2H).

### Embodiment 20: (N-(1-(1-(6-(1H-benzo[d]imidazol-2-yl]picolinoyl)piperidin-4-yl]-3-(1H-benzo[d]imidazol-2-yl)cyclohexane-1-carboxamide (L-20)

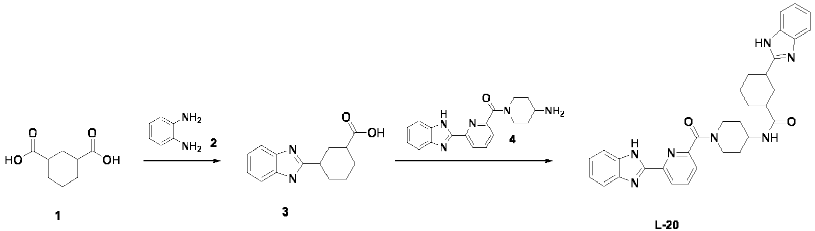

### I. 3-(1H-benzo[d]imidazol-2-yl)cyclohexane-1-carboxylic acid

1,3-Cyclohexanedicarboxylic acid (1 g, 5.8 mmol) and o-phenylenediamine (628 mg, 5.8 mmol) were dissolved in 10 mL of N,N-dimethylformamide, then added with 2-(7-azobenzotriazole)-N,N,N',N'-tetramethyluronium hexafluorophosphate (2.43 g, 6.34 mmol) and N,N-diisopropylethylamine (2.25 g, 17.4 0 mmol). The reaction mixture was stirred at 15°C for 16 hours. The reaction mixture was added with 50 mL of water, and extracted with ethyl acetate (50 mL × 3). The aqueous phase was concentrated to obtain 3 g of a black oil, and the oil was dissolved in 20 mL of acetic acid. The mixture was stirred at 55°C for 4 hours, concentrated, and purified by column chromatography to obtain 1.3 g of the desired product with a yield of 93%. LC-MS: m/z: (M+H)⁺ = 245.0.

### II. (N-(1-(1-(6-(1H-benzo[d]imidazol-2-yl]picolinoyl)piperidin-4-yl]-3-(1H-benzo[d]imidazol-2-yl)cyclohexane-1-carboxamide

### Same operation as L-14

LC-MS: m/z: (M+H)⁺ = 519.0, ¹H NMR (400 MHz, CD₃OD) δ 8.40 (d, *J* = 7.9 Hz, 1H), 8.13 (t, *J* = 7.8 Hz, 1H), 7.65 (dd, *J* = 7.7, 0.9 Hz,3H), 7.54 (d, *J* = 2.5 Hz, 2H), 7.32 (dd, *J* = 6.1, 3.1 Hz, 2H), 7.27 - 7.16 (m, 2H), 4.62 (d, *J* = 9.1 Hz, 1H),4.05 - 3.95 (m, 1H), 3.81 (d, *J* = 13.9 Hz, 1H), 3.50 - 3.42 (m, 1H), 3.39 (s, 1H), 3.18 (dd, *J* = 15.3, 9.6Hz, 1H), 2.64 (s, 1H), 2.43 - 2.29 (m, 1H), 2.24 - 2.04 (m, 3H), 1.94 (dd, *J* = 13.8, 10.6 Hz, 2H), 1.74 (dd, *J* = 11.1, 5.5 Hz, 3H), 1.66 - 1.45 (m, 3H).

### Embodiment 21: N-(1-(1-(6-(1H-benzo[d]imidazol-2-yl)picolinoyl)piperidin-4-yl]-2-(pyridin-2-ylamino)pyrimidine-4-carboxamide (L-21)

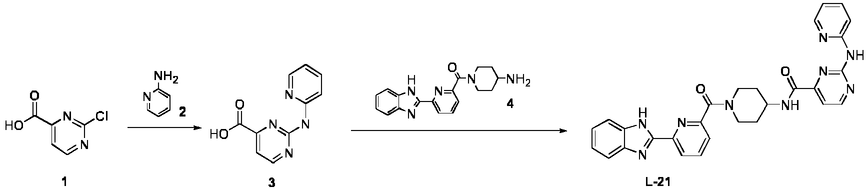

### I. 2-(pyridin-2-ylamino)pyrimidine-4-carboxylic acid

Pyridin-2-amine (534 mg, 5.67 mmol) was dissolved in 1 mL ofN,N-dimethylformamide, and 60% NaH (227 mg, 5.6768 mmol) was added at 20°C. The reaction mixture was stirred at 20°C for 1 hour. 2-Chloropyrimidine-4-carboxylic acid (300 mg, 1.89 mmol) was added, and the reaction mixture was stirred at 70°C for 18 hours. The reaction mixture was cooled to room temperature and 20 mL of water was added. The reaction mixture was extracted with dichloromethane (20 mL × 2). The aqueous phase was acidified with 1 N hydrochloric acid to adjust the pH to 5 and concentrated. The residue was dissolved in 5 mL of N,N-dimethylformamide, stirred for 10 minutes, filtered, and concentrated to obtain 80 mg of a yellow solid with a yield of 19.5%. LC-MS: m/z: (M+H)⁺ = 217.0.

### II. (N-(1-(1-(6-(1H-benzo[d]imidazol-2-yl)picolinoyl)piperidin-4-yl]-2-(pyridin-2-ylamino)pyrimidine-4-carboxamide

### Same operation as L-14

LC-MS: m/z: (M+H)⁺ = 520.0, ¹H NMR (400 MHz, CDCl₃) δ 8.74 (d, *J* = 4.9 Hz, 1H), 8.50 (t, *J* = 9.1 Hz, 2H), 8.36 (s, 1H), 8.27 (d, *J* =4.7 Hz, 1H), 8.04 (d, *J* = 8.2 Hz, 1H), 7.95 (t, *J* = 7.8 Hz, 1H), 7.84 (t, *J* = 7.4 Hz, 1H), 7.65 (ddd, *J* = 17.5,9.1, 5.3 Hz, 3H), 7.28 - 7.25 (m, 1H), 7.12 - 7.01 (m, 1H), 4.68 (d, *J* = 13.3 Hz, 1H), 4.37 - 4.13 (m, 1H),3.87 (d, *J* = 13.5 Hz, 1H), 3.28 (t, *J* = 11.8 Hz, 1H), 3.14 (t, *J* = 11.5 Hz, 1H), 2.15 (d, *J* = 11.2 Hz, 1H),2.01 (d, *J* = 10.6 Hz, 1H), 1.79 (dd, *J* = 21.2, 11.4 Hz, 2H).

### Embodiment 22: (N-(1-(1-(6-(1H-benzo[d]imidazol-2-yl)picolinoyl)piperidin-4-yl)-2-morpholinopyrimidine-4-carboxamide (L-22)

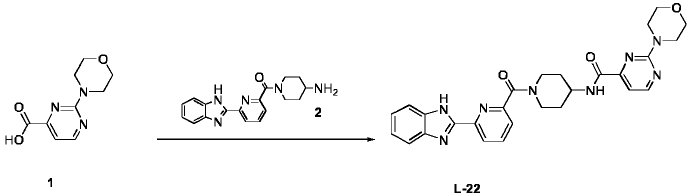

### (N-(1-(1-(6-(1H-benzo[d]imidazol-2-yl)picolinoyl)piperidin-4-yl)-2-morpholinopyrimidine-4-carboxamide

### Same operation as L-14

LC-MS: m/z: (M+H)⁺ = 513.0, ¹H NMR (400 MHz, CDCl₃) δ 8.57 (d, *J* = 4.8 Hz, 1H), 8.52 (d, *J* = 7.8 Hz, 1H), 7.95 (t, *J* = 7.8 Hz, 1H),7.75 (d, *J* = 8.2 Hz, 2H), 7.63 - 7.58 (m, 1H), 7.39 - 7.32 (m, 3H), 4.78 (d, *J* = 13.5 Hz, 1H), 4.26 (dt, *J* =10.9, 9.5 Hz, 1H), 3.92 (d, *J* = 13.9 Hz, 1H), 3.88 - 3.76 (m, 8H), 3.34 (t, *J* = 11.9 Hz, 1H), 3.12 (t, *J* = 11.6Hz, 1H), 2.21 (d, *J* = 9.7 Hz, 1H), 2.07 (d, *J* = 11.7 Hz, 1H), 1.81 - 1.63 (m, 2H).

### Embodiment 23: N-(3-(6-(1H-benzo[d]imidazol-2-yl)picolinoyl)-3-azabicyclo[3.1.0]hexan-6-yl)-2-(phenylamino)pyrimidine-4-carboxamide (L-23)

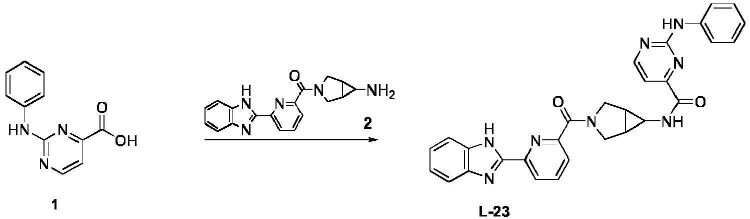

### N-(3-(6-(1H-benzo[d]imidazol-2-yl)picolinoyl)-3-azabicyclo [3.1.0]hexan-6-yl)-2-(phenylamino)pyrimidine-4-carboxamide

### Same operation as L-14

LC-MS: m/z: (M+H)⁺ = 517.0, ¹H NMR(400 MHz, CDCl₃) δ 8.61 - 8.52 (m, 2H), 8.48 (d, *J* = 4.9 Hz, 1H), 8.24 (d, *J* = 6.9 Hz, 1H), 7.98(t, *J* = 7.8 Hz, 1H), 7.63 (d, *J* = 7.7 Hz, 3H), 7.46 (s, 1H), 7.37 (t, *J* = 8.0 Hz, 2H), 7.30 (d, *J* = 3.2 Hz, 1H),7.09 (t, *J* = 7.4 Hz, 1H), 6.97 (d, *J* = 4.9 Hz, 1H), 4.24 (d, *J* = 12.6 Hz, 1H), 4.08 (d, *J* = 11.7 Hz, 1H), 3.87(dd, *J* = 11.7, 4.4 Hz, 1H), 3.69 (dd, *J* = 12.1, 4.5 Hz, 2H), 3.14 (dt, *J* = 11.7, 7.3 Hz, 1H), 2.56 (d, *J* = 2.2Hz, 1H), 1.97 (dd, *J* = 18.6,4.3 Hz, 2H).

### Embodiment 24: (5-(6-(1-(1-H-benzo[d]imidazol-2-yl)picolinoyl)hexahydropyrrolo[3,4-c]pyrrol-2(1H)-yl)(2-(phenylamino)pyrimidin-4-yl)methanone (L-24)

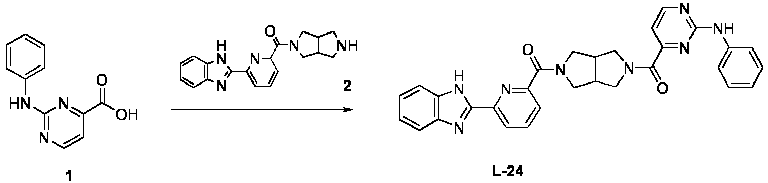

### (5-(6-(1-(1-H-benzo[d]imidazol-2-yl)picolinoyl)hexahydropyrrolo[3,4-c]pyrrol-2(1H)-yl)(2-(phenylamino)pyrimidin-4-yl)methanone

### Same operation as L-14

LC-MS: m/z: (M+H)⁺ = 531.0, ¹H NMR (400 MHz, CDCl₃) δ 8.60 - 8.44 (m, 2H), 8.02 - 7.92 (m, 1H), 7.79 (dd, *J* = 6.3, 3.1 Hz, 2H),7.75 - 7.62 (m, 2H), 7.51 (d, *J* = 7.7 Hz, 1H), 7.41 - 7.30 (m, 3H), 7.27 - 7.16 (m, 1H), 7.09 (dd, *J* = 9.6,6.2 Hz, 1H), 4.05-3.79 (m, 4H), 3.78-3.68 (m,4H), 3.07 (s, 2H).

### Embodiment 25: (N-(3-(6-(1H-benzo[d]imidazol-2-yl)picolinoyl)-3-azabicyclo[3.1.0]hexan-6-yl)-2-(pyridin-2-ylamino)pyrimidine-4-carboxamide (L-25)

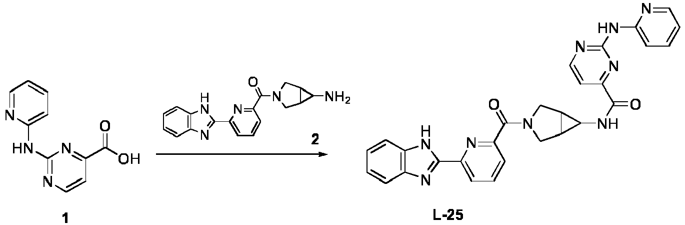

### (N-(3-(6-(1H-benzo[d]imidazol-2-yl)picolinoyl)-3-azabicyclo[3.1.0]hexan-6-yl)-2-(pyridin-2-ylamino)pyrimidine-4-carboxamide

### Same operation as L-14

LC-MS: m/z: (M+H)⁺ = 518.0, ¹H NMR (400 MHz, CD₃OD) δ 8.71 (d, *J* = 4.9 Hz, 1H), 8.41 (dd, *J* = 7.6, 1.3 Hz, 1H), 8.31 (dd, *J* = 9.5,4.8 Hz, 2H), 8.21 - 8.08 (m, 2H), 8.07 - 7.82 (m, 1H), 7.68 (s, 2H), 7.40 - 7.28 (m, 2H),7.17 (d, *J* = 4.9 Hz, 1H), 7.05 (ddd, *J* = 7.2, 5.0, 0.9 Hz, 1H), 4.24 (d, *J* = 12.5 Hz, 1H), 4.14 (d, *J* = 11.5Hz, 1H), 3.99 (dd, *J* = 11.5, 4.3 Hz, 1H), 3.75 (dt, *J* = 13.2, 5.1 Hz, 1H), 2.71 (t, *J* = 2.4 Hz, 1H), 2.17 - 2.05 (m, 2H).

### Embodiment 26: ((5-(6-(1-H-benzo[d]imidazol-2-yl)picolinoyl)hexahydropyrrolo[3,4-c]pyrrol-2(1H)-yl)(2-(pyridin-2-ylamino)pyrimidin-4-yl)methanone (L-26)

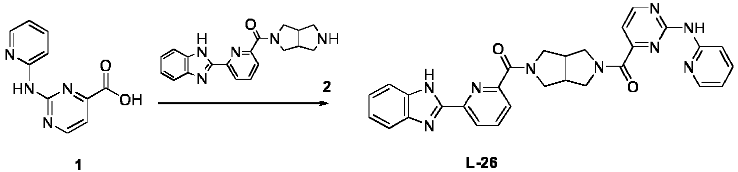

### ((5-(6-(1-H-benzo[d]imidazol-2-yl)picolinoyl)hexahydropyrrolo[3,4-c]pyrrol-2(1H)-yl)(2-(pyridin-2-ylamino)pyrimidin-4-yl)methanone

2-(2-pyridinylamino)pyrimidine-4-carboxylic acid (40 mg, 0.18 mmol) was dissolved in 2 mL ofN,N-dimethylformamide, then added with 1-(3-dimethylaminopropyl)-3-ethylcarbodiimide hydrochloride (145 mg, 0.28 mmol) and N,N-diisopropylethylamine (72 mg, 0.56 mmol). The reaction mixture was stirred for 20 minutes, then added with 2,3,3a,4,6,6a-hexahydro-1H-pyrrolo[3,4-c]pyrrol-5-yl-[6-(1H-benzimidazol-2-yl)-2-pyridyl]methanone (62 mg, 0.18 mmol), and the reaction mixture was stirred at 15°C for 16 hours. The reaction mixture was added with 10 mL of water, filtered, and the obtained solid was purified by thin-layer chromatography plate (dichloromethane: methanol = 10:1) to obtain 15 mg of a white solid with a yield of 15.25%.

LC-MS: m/z: (M+H)⁺ = 532.0, ¹H NMR (400 MHz, CD₃OD) δ 8.68 (dd, *J* = 26.6, 5.0 Hz, 1H), 8.63-8.61 (m, 1H), 8.32- 8.22 (m, 1H), 8.19 - 8.07 (m, 2H), 7.92 - 7.51 (m, 4H), 7.36 - 7.26 (m, 2H), 7.20 (dd, *J* = 14.8, 5.0 Hz,1H), 6.84-6.82 (m, 1H), 4.29 - 3.65 (m, 8H), 3.17 - 3.03 (m, 2H).

### Embodiment 27: (9-(6-(1H-benzo[d]imidazol-2-yl)picolinoyl)-3,9-diazaspiro[5.5]undecan-3-yl)(2-(phenylamino)pyrimidin-4-yl)methanone (L-27)

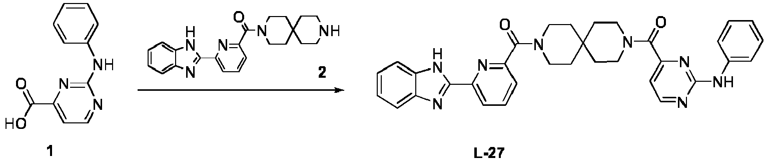

### (9-(6-(1-H-benzo[d]imidazol-2-yl)picolinoyl)-3,9-diazaspiro[5.5]undecan-3-yl)(2-(phenylamino)pyrimidin-4-yl)methanone

### Same operation as L-26

LC-MS: m/z: (M+H)⁺ = 573.0, ¹H NMR (400 MHz, DMSO-*d₆*) δ 12.96 (s, 1H), 9.82 (s, 1H), 8.59 (d, *J* = 4.9 Hz, 1H), 8.38 (dd, *J* = 7.9, 1.0Hz, 1H), 8.11 (t, *J* = 7.8 Hz, 1H), 7.75 - 7.72 (m, 3H), 7.62-7.56 (m, 2H), 7.31-7.21 (m, 4H), 6.96 (t, *J* = 7.3 Hz, 1H), 6.88 (d, *J* = 4.9 Hz, 1H), 3.78 - 3.54 (m, 4H), 3.35 (s, 4H),1.72 - 1.31 (m, 8H).

### Embodiment 28: 6-(1H-benzo[d]imidazol-2-yl)-N-(1-(2-(phenylamino)pyrimidine-4-carbonyl)pyrrolidin-3-yl)picolinamide (L-28)

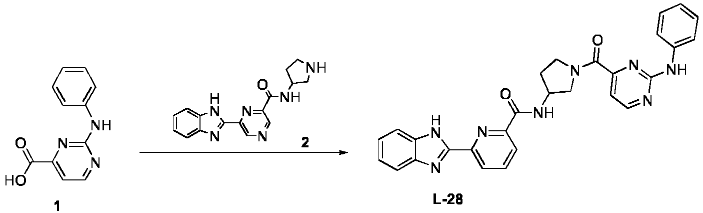

### 6-(1H-benzo[d]imidazol-2-yl)-N-(1-(2-(phenylatnino)pyrimidine-4-carbonyl)pyrrolidin-3 -yl)picolinamide

### Same operation as L-26

LC-MS: m/z: (M+H)⁺ = 505.0, ¹H NMR (400 MHz, CD₃OD) δ 8.62 (dd, *J* = 30.1, 4.9 Hz, 1H), 8.41 (t, *J* = 7.9 Hz, 1H), 8.12 (q, *J* = 7.8Hz, 1H), 7.86 (d, *J* = 7.7 Hz, 1H), 7.75 - 7.54 (m, 4H), 7.41 - 7.22 (m, 5H), 6.97 (dd, *J* = 12.5, 7.3 Hz,1H), 4.72 - 4.52 (m, 1H), 4.34-3.54 (m, 4H), 2.42 (dd, *J* = 12.9, 5.7 Hz, 1H),2.20 (d, *J* = 5.5 Hz, 1H).

### Embodiment 29: 6-(1H-benzo[d]imidazol-2-yl)-N-(1-(2-(pyridin-2-ylamino)pyrimidine-4-carbonyl)pyrrolidin-3-yl)picolinamide (L-29)

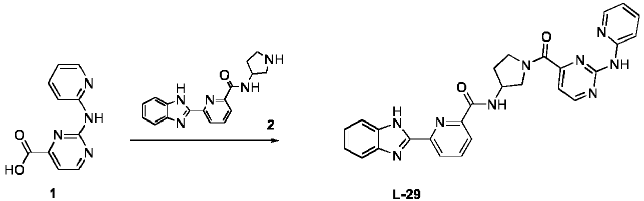

### 6-(1H-benzo[d]imidazol-2-yl)-N-(1-(2-(pyridin-2-ylamino)pyrimidine-4-carbonyl)pyrrolidin-3-yl)picolinamide

### Same operation as L-26

LC-MS: m/z: (M+H)⁺ = 506.0, ¹H NMR (400 MHz, CD₃OD) δ 8.73 (dd, *J* = 33.7, 4.9 Hz, 1H), 8.48 - 8.35 (m, 1H), 8.32 - 8.00 (m, 3H),7.87 (ddd, *J* = 7.8, 2.1, 1.0 Hz, 1H), 7.75-7.73 (m, 2H), 7.61 (s, 1H), 7.46 (dd, *J* = 43.3,4.9 Hz, 1H), 7.36 - 7.23 (m, 2H), 6.99 (dd, *J* = 7.3, 5.0 Hz, 1H), 4.78 - 4.30 (m, 2H), 4.09-4.01 (m, 1H), 4.02 - 3.77 (m, 2H), 2.49 - 2.12 (m, 2H).

### Embodiment 30: N-(1-(1-(6-(1H-benzo[d]imidazol-2-yl)picolinoyl)piperidin-4-yl]-6-(1H-benzo[d]imidazol-2-yl)picolinamide (L-30)

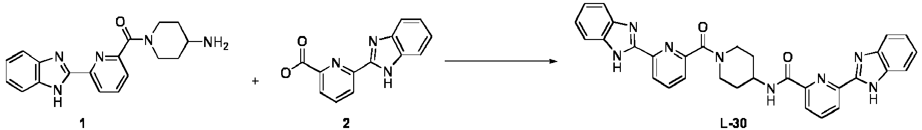

### N-(1-(1-(6-(1H-benzo[d]imidazol-2-yl)picolinoyl)piperidin-4-yl]-6-(1H-benzo[d]imidazol-2-yl)picolinamide

### Same operation as L-5

LC-MS: m/z: (M+H)⁺ = 543.22, ¹H NMR (400 MHz, CDCl₃) δ 8.49 (d, *J* = 7.2 Hz, 2H), 8.07 - 7.86 (m, 2H), 7.72 (s, 4H), 7.58 (dd, *J* = 7.7, 1.0 Hz, 2H), 7.38 - 7.31 (m, 4H), 4.69 (d, *J* = 13.1 Hz, 2H), 4.55 (d, *J* = 7.8 Hz, 2H), 3.83 (d, *J* = 13.8 Hz, 4H), 3.15 (dt, *J* = 22.4, 11.4 Hz, 4H), 2.06 (dd, *J* = 56.4, 13.3 Hz, 4H).

### Embodiment 31: N-(4-(9-methyl-3,9-diazaspiro[5.5]undecan-3-yl)phenyl)-1-(2-(phenylamino)pyrimidine-4-carbonyl)pyrrolidine-3-carboxamide (L-31)

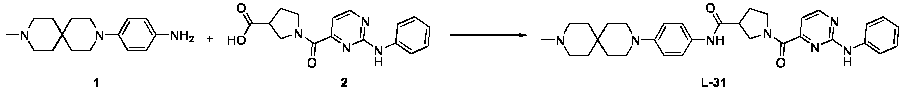

### N-(4-(9-methyl-3,9-diazaspiro[5.5]undecan-3-yl)phenyl)-1-(2-(phenylamino)pyrimidine-4-carbonyl)pyrrolidine-3-carboxamide

### Same operation as L-5

4-(9-Methyl-3,9-diazaspiro[5.5]undecan-3-yl)aniline was purchased from Bide Pharmatech.

LC-MS: m/z: (M+H)⁺ = 554.23, ¹H NMR (400 MHz, MeOD) δ 8.50 - 8.34 (m, 1H), 8.19 - 8.07 (m, 1H), 7.83 (t, *J* = 13.8 Hz, 1H), 7.69 (s, 2H), 7.49 (d, *J* = 9.0 Hz, 1H), 7.42 - 7.27 (m, 2H), 6.97 (dd, *J* = 28.4, 9.0 Hz, 2H), 4.21 - 3.87 (m, 4H), 3.77 (dt, *J* = 12.3, 7.7 Hz, 1H), 3.19 - 3.06 (m, 4H), 2.83 (s, 4H), 2.57 (d, *J* = 5.6 Hz, 3H), 2.44 - 2.26 (m, 2H).

### Embodiment 32: N-(1-(1-(6-(1H-benzo[d]imidazol-2-yl)picolinoyl)pyrrolidin-3-yl]-6-(1H-benzo[d]imidazol-2-yl)picolinamide (L-32)

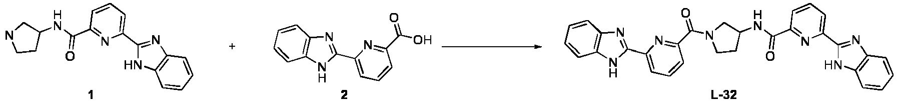

### N-(1-(1-(6-(1H-benzo[d]imidazol-2-yl)picolinoyl)pyrrolidin-3-yl]-6-(1H-benzo[d]imidazol-2-yl)picolinamide

### Same operation as L-6

LC-MS: m/z: (M+H)⁺ = 529.5, ¹H NMR (400 MHz, CDCl₃) δ 8.70 (s, 2H), 8.57 (d, *J* = 23.6 Hz, 1H), 8.46 (d, *J* = 7.9 Hz, 1H), 8.34 (d, *J* = 6.2 Hz, 2H), 8.16 (dd, *J* = 22.0, 7.1 Hz, 2H), 7.86 (dd, *J* = 17.5, 9.7 Hz, 2H), 7.80 -7.50 (m, 4H), 7.36 - 7.16 (m, 4H), 4.61 (d, *J* = 5.9 Hz, 2H), 4.41 (s, 1H), 4.03 (dd, *J* = 11.5, 6.2 Hz, 1H), 3.78 (dd, *J* = 58.9, 16.7 Hz, 4H), 3.53 (s, 3H), 2.18 (dd, *J* = 12.9, 6.3 Hz, 1H), 1.93 (dd, *J* = 39.9, 18.0 Hz, 2H), 1.74 - 1.50 (m, 2H), 1.32 (dt, *J* = 14.2, 10.8 Hz, 4H).

### Embodiment 33: cyclohexane-1,4-diylbis((5-(4-nitrophenyl)hexahydropyrrolo[3,4-c]pyrrol-2(1H)-yl)methanone) (L-33)

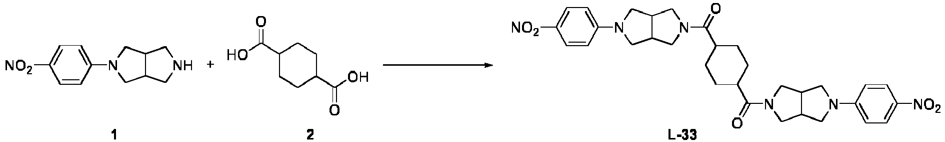

### Cyclohexane-1,4-diylbis((5-(4-nitrophenyl)hexahydropyrrolo[3,4-c]pyrrol-2(1H)-yl)methanone)

### Same operation as L-8

2-(4-Nitrophenyl)octahydropyrrolo[3,4-c]pyrrole was purchased from Bide Pharmatech.

LC-MS: m/z: (M+H)⁺ = 603.21, ¹H NMR (400 MHz, CDCl₃) δ 8.15 (d, *J* = 8.1 Hz, 4H), 6.50 (d, *J* = 8.6 Hz, 4H), 4.00 - 3.65 (m, 8H), 3.65 - 3.27 (m, 8H), 3.15 (d, *J* = 34.2 Hz, 4H), 2.39 (s, 2H), 2.07 - 1.72 (m, 4H), 1.59 (dd, *J* = 25.9, 13.6 Hz, 4H), 1.45 - 1.20 (m, 4H).

### Embodiment 34: 1,4-phenylenebis((5-(4-nitrophenyl)hexahydropyrrolo[3,4-c]pyrrol-2(1H)-yl)methanone) (L-34)

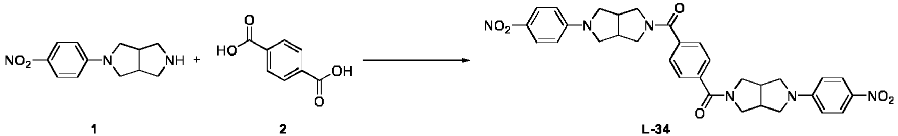

### 1,4-phenylenebis((5-(4-nitrophenyl)hexahydropyrrolo[3,4-c]pyrrol-2(1H)-yl)methanone)

### Same operation as L-8

LC-MS: m/z: (M+H)⁺ = 597.21, ¹H NMR (400 MHz, CDCl₃) δ 8.15 (t, *J* = 8.8 Hz, 4H), 8.01 - 7.86 (m, 4H), 6.61 - 6.43 (m, 4H), 4.28 - 3.88 (m, 4H), 3.84 - 3.51 (m, 8H), 3.51 - 3.38 (m, 2H), 3.38 - 3.21 (m, 2H), 3.21 - 2.81 (m, 4H).

### Embodiment 35: 1-(6-(1H-benzo[d]imidazol-2-yl)picolinoyl)-N-(4-(9-methyl-3,9-diazaspiro[5.5]undecan-3-yl)phenyl)pyrrolidine-3-carboxamide (L-35)

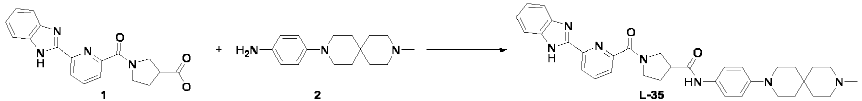

### 1-(6-(1H-benzo[d]imidazol-2-yl)picolinoyl)-N-(4-(9-methyl-3,9-diazaspiro[5.5]undecan-3-yl)phenyl)pyrrolidine-3-carboxamide

### Same operation as L-6

LC-MS: m/z: (M+H)⁺ = 578.31, ¹H NMR (400 MHz, MeOD) δ 8.59 (d, *J* = 4.7 Hz, 1H), 7.66 (dd, *J* = 15.3, 8.1 Hz, 2H), 7.44 (dd, *J* = 15.0, 8.9 Hz, 2H), 7.36 - 7.25 (m, 2H), 7.04 (ddd, *J* = 30.4, 15.7, 6.8 Hz, 4H), 4.16 - 3.76 (m, 5H), 3.70 (s, 4H), 3.27 - 3.05 (m, 4H), 2.80 (s, 3H), 2.54 (s, 2H), 2.27 (d, *J* = 5.2 Hz, 2H), 1.69 (s, 5H).

### Embodiment 36: Piperazine-1,4-diylbis((3-(imidazol[1,2-a]pyridin-2-yl)phenyl)methanone) (L-36)

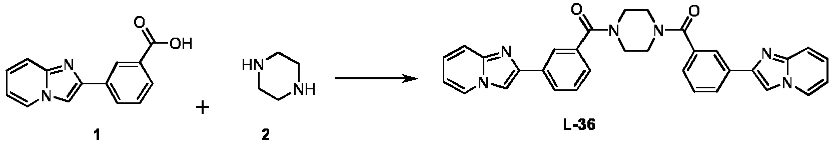

3-(Imidazo[1,2-a]pyridin-2-yl)benzoic acid (0.12g, 0.5 mmol), benzotriazol-1-yl-oxytripyrrolidinophosphonium hexafluorophosphate (0.28 g, 0.55 mmol) and N,N-diisopropylethylamine (0.14 g, 1.1 mmol) were dissolved in 6 mL of N,N-dimethylformamide under ice bath, the mixture was stirred for 0.5 hours, then added with piperazine (0.04 g, 0.5 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (12 mg, 22 µmol) with a yield of 4.5%. LC-MS: m/z: (M+H)⁺ = 527.

¹H NMR (400 MHz, Chloroform-*d*) δ 8.15 (d, 2H), 8.08 - 8.00 (m, 4H), 7.91 (s, 2H), 7.65 (d, *J* = 9.1 Hz, 2H), 7.56 - 7.46 (m, 2H), 7.39 (d, *J* = 7.6 Hz, 2H), 7.21 (m, 2H), 6.82 (m, 2H), 3.73 (d, *J* = 89.9 Hz, 8H).

### Embodiment 37: 6-(1H-benzo[d]imidazol-2-yl)-N-(1-(3-nitrobenzyl)pyrrolidin-3-yl)pyridineamide (L-37)

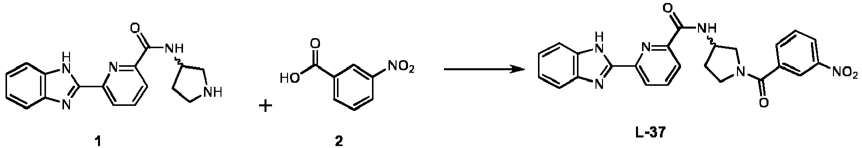

m-Nitrobenzoic acid (0.08g, 0.48 mmol), benzotriazol-1-yl-oxytripyrrolidinophosphonium hexafluorophosphate (0.30 g, 0.58 mmol) and N,N-diisopropylethylamine (0.16 g, 1.3 mmol) were dissolved in 6 mL of N,N-dimethylformamide under ice bath, the mixture was stirred for 0.5 hours, then added with 6-(1H-benzo[d]imidazol-2-yl)-N-(pyrrolidin-3-yl)pyridineamide (0.15 g, 0.48 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (15 mg, 32 µmol) with a yield of 6.8%. LC-MS: m/z: (M+H)⁺ = 457.

### Embodiment 38: N-(1-(6-(1H-benzo[d]imidazol-2-yl)pyridinyl)piperidin-4-yl)-3-nitrobenzamide (L-38)

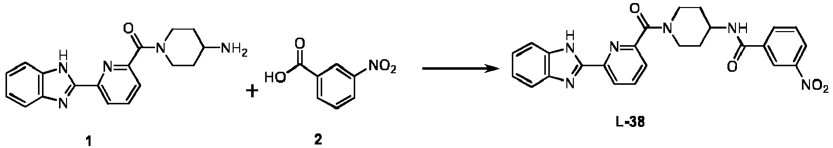

(6-1H-benzo[d]imidazol-2-yl)pyridin-2-yl)(4-aminopiperidin-1-yl)methanone (0.088g, 0.27 mmol), benzotriazol-1-yl-oxytripyrrolidinophosphonium hexafluorophosphate (0.15 g, 0.3 mmol) and N,N-diisopropylethylamine (0.12 g, 1.0 mmol) were dissolved in 6 mL of N,N-dimethylformamide under ice bath, the mixture was stirred for 0.5 hours, then added with m-nitrobenzoic acid (0.04 g, 0.27 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (11 mg, 32 µmol) with a yield of 8.6%. LC-MS: m/z: (M+H)⁺ = 471.

### Embodiment 39: Synthesis of (1-(3-(imidazol[1,2-a]pyridin-2-yl)benzoyl)pyrrolidin-2-yl)(5-(4-nitrophenyl)hexahydropyrrolo[3,4-c]pyrrol-2(1H)-yl)methanone (L-39)

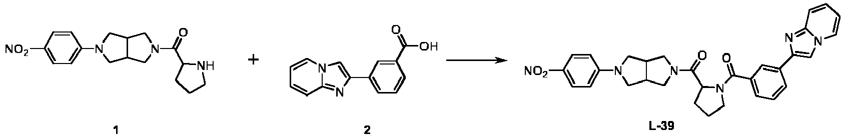

3-(Imidazo[1,2-a]pyridin-2-yl)benzoic acid (0.1g, 0.4 mmol), benzotriazol-1-yl-oxytripyrrolidinophosphonium hexafluorophosphate (0.24 g, 0.44 mmol) and N,N-diisopropylethylamine (0.12 g, 1.0 mmol) were dissolved in 6 mL of N,N-dimethylformamide under ice bath, the mixture was stirred for 0.5 hours, then added with 2-(4-nitrophenyl)-5-prolyloctahydropyrrole[3,4-c]pyrrole (0.13 g, 0.4 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (16 mg, µmol) with a yield of 7.2%. LC-MS: m/z: (M+H)⁺ = 551.

¹H NMR (400 MHz, Chloroform-*d*) δ 8.20 -7.99 (m, 5H), 7.90 (d, *J* = 9.8 Hz, 1H), 7.64 (t, *J* = 8.8 Hz, 1H), 7.53 - 7.45 (m, 2H), 7.21 (m, 1H), 6.82 (m, 1H), 6.57 - 6.38 (m, 2H), 3.81 (m, 7H), 3.62 - 3.06 (m, 8H), 2.25 (m, 2H).

### Embodiment 40: (9-(3-(1H-benzo[d]imidazol-2-yl)benzoyl)-3,9-diazaspiro[5.5]undecan-3-yl)(3-nitrophenyl)methanone (L-40)

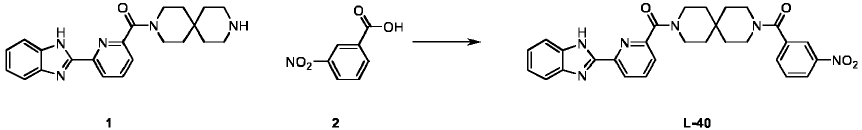

m-Nitrobenzoic acid (0.041g, 0.25 mmol), benzotriazol-1-yl-oxytripyrrolidinophosphonium hexafluorophosphate (0.14 g, 0.27 mmol) and N,N-diisopropylethylamine (0.12 g, 1.0 mmol) were dissolved in 6 mL of N,N-dimethylformamide under ice bath, the mixture was stirred for 0.5 hours, then added with (6-(1H-benzo[d]imidazol-2-yl)pyridin-2-yl)(3,9-diazaspiro[5.5]undecan-3-yl)methadone (0.093 g, 0.25 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (17 mg, µmol) with a yield of 12.9%. LC-MS: m/z: (M+H)⁺ = 525.

¹H NMR(400 MHz, Chloroform-*d*) δ 10.74 (s, 1H), 8.48 (dd, *J* = 8.0, 1.1 Hz, 1H), 8.34 - 8.27 (m, 2H), 7.97 (t, *J* = 7.8 Hz, 1H), 7.87 (d, *J* = 6.9 Hz, 1H), 7.77 (m, 1H), 7.64 (t, *J* = 7.9 Hz, 1H), 7.57 (m, 2H), 7.37 - 7.32 (m, 2H), 3.94 - 3.74 (m, 4H), 3.52 (s, 2H), 3.42 (s, 2H), 1.59 (m, 8H).

### Embodiment 41: Synthesis of (5-(6-(1H-benzo[d]imidazol-2-yl)pyridinyl)hexahydropyrrolo[3,4-c]pyrrol-2(1H)-yl)(3-nitrophenyl)methanone (L-41)

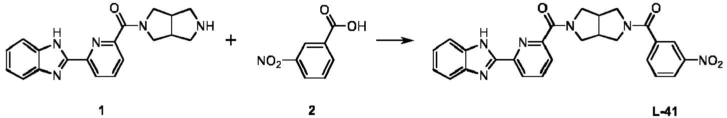

m-Nitrobenzoic acid (0.041g, 0.25 mmol), benzotriazol-1-yl-oxytripyrrolidinophosphonium hexafluorophosphate (0.14 g, 0.27 mmol) and N,N-diisopropylethylamine (0.12 g, 1.0 mmol) were dissolved in 6 mL of N,N-dimethylformamide under ice bath, the mixture was stirred for 0.5 hours, then added with (6-(1H-benzo[d]imidazol-2-yl)pyridin-2-yl)(hexahydropyrrolo[3,4-c]pyrrol-2(1H)-yl)methanone (0.083 g, 0.25 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (17 mg, µmol) with a yield of 12.9%. LC-MS: m/z: (M+H)⁺ = 483.

¹H NMR (400 MHz, Chloroform-*d*) δ 11.58 (s, 1H), 8.52 (dd, *J* = 28.6, 7.9 Hz, 1H), 8.38 (s, 1H), 8.30 (t, *J* = 9.1 Hz, 1H), 8.04 - 7.49 (m, 6H), 7.32 (m 2H), 4.22 (m, 1H), 4.09 - 4.00 (m, 1H), 3.91 - 3.32 (m, 6H), 3.05 (m, 2H).

### Embodiment 42: N-(1-(3-(imidazo[1,2-a]pyridin-2-yl)benzoyl)piperidin-4-yl)-2-(phenylamino)pyrimidine-4-formamide (L-42)

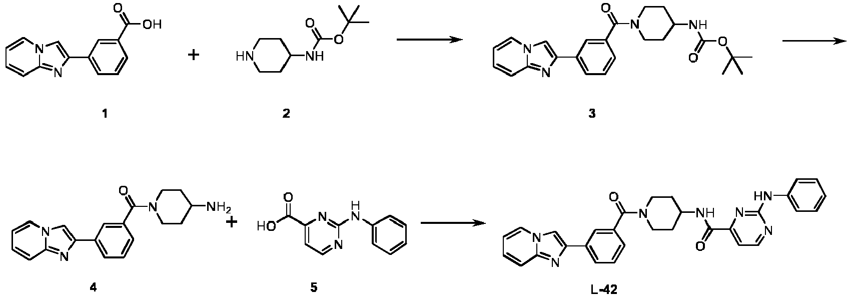

### I. Synthesis of tert-butyl (1-(3-(imidazol[1,2-a]pyridin-2-yl)benzoyl)piperidin-4-yl)carbamate

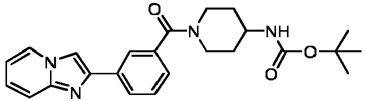

3-(Imidazo[1,2-a]pyridin-2-yl)benzoic acid (1 g, 4.2 mmol), 1-(3-dimethylaminopropyl)-3-ethylcarbodiimide hydrochloride (0.96 g, 5 mmol), 1-hydroxybenzotriazole (0.66 g, 5 mmol) and N,N-diisopropylethylamine (1.19 g, 9.2 mmol) were dissolved in 20 mL of N,N-dimethylformamide under ice bath, the mixture was stirred for 0.5 hours, then added with *tert*-butyl piperidin-4-yl carbamate (0.92 g, 4.6 mmol), stirred continuously for 0.5 hours, returned to room temperature and reacted overnight. The reaction mixture was poured into 80 mL of water, extracted with ethyl acetate (80 mL × 3), and washed with saturated brine. The organic phases were combined, dried over anhydrous sodium sulfate, concentrated under reduced pressure, and purified by silica gel column chromatography (PE:EA = 5:1) to obtain an off-white solid (1.3 g, 3 mmol) with a yield of 73%. LC-MS: m/z: (M+H)⁺ = 421.

### II. Synthesis of (4-atninopiperidin-1-yl)(3-(imidazo[1,2-a]pyridin-2-yl)phenyl)methanone

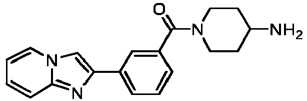

*tert*-Butyl (1-(3-(imidazol[1,2-a]pyridin-2-yl)benzoyl)piperidin-4-yl)carbatnate (1.3 g, 3 mmol) was dissolved in 20 mL of dichloromethane under ice bath, the mixture was added with a saturated solution of 1,4-dioxane in hydrochloric acid (2 mL, 8 mmol), stirred continuously for 0.5 hours, returned to room temperature and reacted overnight. The reaction mixture was concentrated under reduced pressure, and the resulting solid was directly used in the next reaction step. Yield > 90%. LC-MS: m/z: (M+H)⁺ = 321.

### III. Synthesis of N-(1-(3-(imidazo[1,2-a]pyridin-2-yl)benzoyl)piperidin-4-yl)-2-(phenylamino)pyrimidine-4-form amide

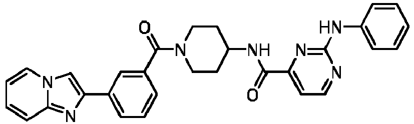

2-(Phenylamino)pyrimidine-4-carboxylic acid (0.060g, 0.27 mmol), benzotriazol-1-yl-oxytripyrrolidinophosphonium hexafluorophosphate (0.14 g, 0.27 mmol) and N,N-diisopropylethylamine (0.12 g, 1.0 mmol) were dissolved in 6 mL of N,N-dimethylformamide under ice bath, the mixture was stirred for 0.5 hours, then added with (4-aminopiperidin-1-yl)(3-(imidazo[1,2-a]pyridin-2-yl)phenyl)methanone (0.089 g, 0.27 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (20 mg, 38 µmol) with a yield of 14.3%. LC-MS: m/z: (M+H)⁺ = 518.

¹H NMR (400 MHz, Chloroform-*d*) δ 8.65 (d, *J* = 4.8 Hz, 1H), 8.22 (d, *J* = 6.7 Hz, 1H), 7.97 (m, 4H), 7.65 (m, 3H), 7.52 - 7.44 (m, 2H), 7.43 - 7.30 (m, 5H), 7.13 (t, *J* = 7.3 Hz, 1H), 6.92 (t, *J* = 6.7 Hz, 1H), 4.70 (s, 1H), 4.27 (s, 1H), 3.84 (s, 1H), 3.20 (m, 2H), 1.67 (s, 2H), 1.31 (m, 2H).

### Embodiment 43: N-(1-(3-(imidazo[1,2-a]pyridin-2-yl)benzoyl)piperidin-4-yl)-2-(phenylamino)pyrimidine-4-formamide (L-43)

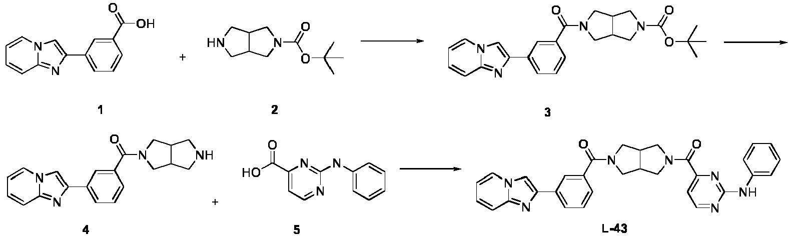

### I. Synthesis of tert-butyl 5-(3-(imidazo[1,2-a]pyridin-2-yl)benzoyl)hexahydropyrrolopyrrole-2(1H)-carboxylate

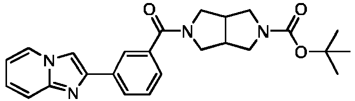

3-(Imidazo[1,2-a]pyridin-2-yl)benzoic acid (1 g, 4.2 mmol), 1-(3-dimethylaminopropyl)-3-ethylcarbodiimide hydrochloride (0.96 g, 5 mmol), 1-hydroxybenzotriazole (0.66 g, 5 mmol) and N,N-diisopropylethylamine (1.19 g, 9.2 mmol) were dissolved in 20 mL of N,N-dimethylformamide under ice bath, the mixture was stirred for 0.5 hours, then added with *tert*-butyl piperidin-4-yl carbamate (0.98 g, 4.6 mmol), stirred continuously for 0.5 hours, returned to room temperature and reacted overnight. The reaction mixture was poured into 80 mL of water, extracted with ethyl acetate (80 mL × 3), and washed with saturated brine. The organic phases were combined, dried over anhydrous sodium sulfate, concentrated under reduced pressure, and purified by silica gel column chromatography (PE:EA = 5:1) to obtain an off-white solid (1.26 g, 2.9 mmol) with a yield of 72%. LC-MS: m/z: (M+H)⁺ = 433.

### II. Synthesis of (hexahydropyrrolo[3,4-c]pyrrol-2(1H)-yl)(3-(imidazo[1,2-a]pyridin-2-yl)phenyl)methanone

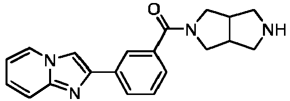

*tert*-Butyl 5-(3-(imidazo[1,2-a]pyridin-2-yl)benzoyl)hexahydropyrrolopyrrole-2(1H)-carboxylate (1.3 g, 3 mmol) was dissolved in 20 mL of dichloromethane under ice bath, the mixture was added with a saturated solution of 1,4-dioxane in hydrochloric acid (2 mL, 8 mmol), stirred continuously for 0.5 hours, returned to room temperature and reacted overnight. The reaction mixture was concentrated under reduced pressure, and the resulting solid was directly used in the next reaction step. Yield > 90%. LC-MS: m/z: (M+H)⁺ = 333.

### III. Synthesis of N-(1-(3-(imidazo[1,2-a]pyridin-2-yl)benzoyl)piperidin-4-yl)-2-(phenylamino)pyrimidine-4-form amide

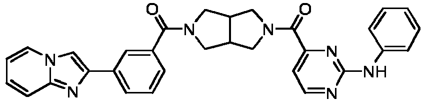

2-(Phenylamino)pyrimidine-4-carboxylic acid (0.060g, 0.27 mmol), benzotriazol-1-yl-oxytripyrrolidinophosphonium hexafluorophosphate (0.14 g, 0.27 mmol) and N,N-diisopropylethylamine (0.12 g, 1.0 mmol) were dissolved in 6 mL of N,N-dimethylformamide under ice bath, the mixture was stirred for 0.5 hours, then added with (hexahydropyrrolo[3,4-c]pyrrol-2(1H)-yl)(3-(imidazo[1,2-a]pyridin-2-yl)phenyl)methanone (0.092 g, 0.27 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (12 mg, 22 µmol) with a yield of 8.4%. LC-MS: m/z: (M+H)⁺ = 530.

¹H NMR (400 MHz, Chloroform-*d*) δ 8.57 (d, *J* = 13.0 Hz, 1H), 8.12 (d, 2H), 8.03 (d, *J* = 7.0 Hz, 1H), 7.90 (d, *J* = 8.8 Hz, 1H), 7.66 - 7.36 (m, 7H), 7.27 - 6.95 (m, 4H), 6.81 (t, *J* = 6.8 Hz, 1H), 4.04 - 3.40 (m, 8H), 3.01 (d, 2H).

### Embodiment 44: N-(3-(6-(1H-benzo[d]imidazol-2-yl)picolinoyl)-3-azabicyclo[3.1.0]hexan-6-yl)-6-(phenylamino)picolinamide (L-44)

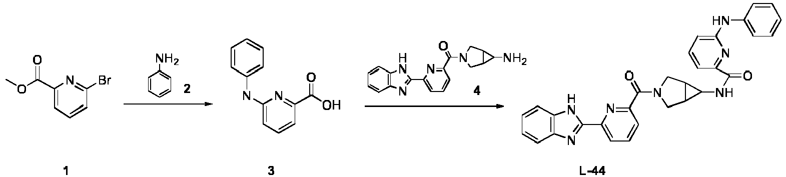

### I. 6-(phenylamino)picolinic acid

Methyl 6-bromopyridine-2-carboxylate (2 g, 9.26 mmol) was dissolved in 50 mL of dioxane, then aniline (826 mg, 9.26 mmol), tris(dibenzylideneacetone)dipalladium (424 mg, 0.46 mmol), 4,5-bisdiphenylphosphino-9,9-dimethylxanthene (536mg, 0.92 mmol) and cesium carbonate (7.54 g, 23.1 mmol) were added, and the reaction mixture was stirred at 95°C for 15 hours under the protection of nitrogen. The reaction mixture was cooled to room temperature, filtered and concentrated to obtain a yellow solid. The solid was dissolved in 15 mL of tetrahydrofuran, 10 mL of methanol and 15 mL of water, added with sodium hydroxide (1.2 g, 29 mmol), and the reaction mixture was stirred at 20°C for 15 hours. The reaction mixture was extracted three times with ethyl acetate (20 mL × 3). The aqueous phase was acidified with 2 N hydrochloric acid to adjust the pH to 1, extracted three times with ethyl acetate (20 mL × 3), then neutralized with saturated sodium bicarbonate aqueous solution to adjust the pH to 1, and the solid was filtered and dried to obtain 500 mg of a white solid with a yield of 25.2%. LC-MS: m/z: (M+H)⁺ = 215.0.

### II. N-(3-(6-(1H-benzo[d]imidazol-2-yl)picolinoyl)-3-azabicyclo[3.1.0]hexan-6-yl)-6-(phenylamino)picolinamide

### Same operation as L-14

LC-MS: m/z: (M+H)⁺ = 516.0, ¹H NMR (400 MHz, CD₃OD) δ 8.47 (dd, *J* = 7.4, 1.5 Hz, 1H), 8.26 - 8.13 (m, 2H), 7.85 - 7.54 (m, 5H),7.45 - 7.23 (m, 4H), 7.08 (dd, *J* = 7.3, 0.8 Hz, 1H), 6.99-6.92 (m, 2H), 4.29 (d, *J* = 12.4Hz, 1H), 4.20 (d, *J* = 11.7 Hz, 1H), 3.97 (dd, *J* = 11.7, 4.2 Hz, 1H), 3.75 (dd, *J* = 12.4, 4.4 Hz, 1H), 2.74 (t, *J* = 2.4 Hz, 1H), 2.15 - 2.05 (m, 2H).

### Embodiment 45: (9-(6-(1-H-benzo[d]imidazol-2-yl)picolinoyl)-3,9-diazaspiro[5.5]undecan-3-yl)(6-(phenylamino)pyridin-2-yl)methanone (L-45)

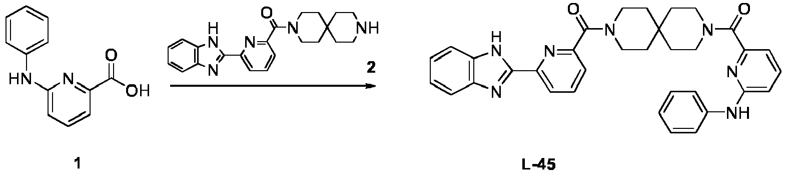

### (9-(6-(1-H-benzo[d]imidazol-2-yl)picolinoyl)-3,9-diazaspiro[5.5]undecan-3-yl)(6-(phenylamino)pyridin-2-yl)methanone

### Same operation as L-14

LC-MS: m/z: (M+H)⁺ = 572.0, ¹H NMR(400 MHz, CD₃OD) δ 8.40 (dd, *J* = 8.0, 1.0 Hz, 1H), 8.13 (t, *J* = 7.9 Hz, 1H), 7.86 - 7.53 (m,6H), 7.33 (d, *J* = 5.1 Hz, 2H), 7.29 - 7.20 (m, 2H), 6.94 (t, *J* = 7.4 Hz, 1H), 6.91 - 6.82 (m, 2H), 3.77 (dd, *J* = 28.2, 21.3 Hz, 4H), 3.62 - 3.47 (m, 4H), 1.70 (d, *J* = 37.8 Hz, 8H).

### Embodiment 46: (5-(6-(1H-benzo[d]imidazol-2-yl)picolinoyl)hexahydropyrrolo[3,4-c]pyrrol-2(1H)-yl)(6-(phenylamino)pyridin-2-yl)methanone (L-46)

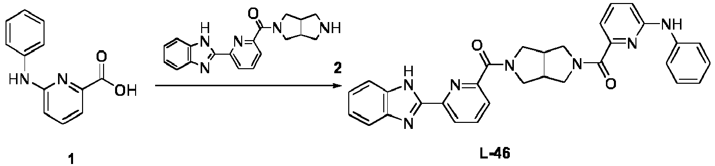

### (5-(6-(1H-benzo[d]imidazol-2-yl)picolinoyl)hexahydropyrrolo[3,4-c]pyrrol-2(1H)-yl)(6-(phenylamino)pyridin-2-yl)methanone

### Same operation as L-14

LC-MS: m/z: (M+H)⁺ = 530.0, ¹H NMR (400 MHz, CD₃OD) δ 8.43 (ddd, *J* = 7.9, 5.4, 0.9 Hz, 1H), 8.15 (dd, *J* = 15.4, 7.7 Hz, 1H), 7.92- 7.81 (m, 1H), 7.79 7.27 (m, 7H), 7.23 - 6.52 (m, 5H), 4.25 - 3.62 (m, 8H), 3.20 - 3.03 (m, 2H).

### Embodiment 47: N-(1-(1-(6-(1H-benzo[d]imidazol-2-yl]picolinoyl)piperidin-4-yl]-6-(phenylamino)picolinamide (L-47)

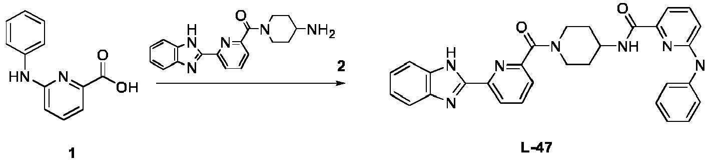

### N-(1-(1-(6-(1H-benzo[d]imidazol-2-yl]picolinoyl)piperidin-4-yl]-6-(phenylamino)picolinamide

### Same operation as L-14

LC-MS: m/z: (M+H)⁺ = 518.0, ¹H NMR (400 MHz, CD₃OD) δ 8.41 (dd, *J* = 8.0, 0.9 Hz, 1H), 8.15 (t, *J* = 7.9 Hz, 1H), 7.84 - 7.57 (m, 4H), 7.56 - 7.42 (m, 3H), 7.41 - 7.16 (m, 4H), 7.04 - 6.91 (m, 2H), 4.54 (d, *J* = 13.7 Hz, 1H), 4.24 - 4.17(m, 1H), 3.82 (d, *J* = 13.8 Hz, 1H), 3.50-3.21 (m, 2H), 2.23 (d, *J* = 10.1 Hz, 1H), 2.05(d, *J* = 10.1 Hz, 1H), 1.75 (dt, *J* = 19.7, 10.2 Hz, 2H).

### Embodiment 48: N-(6-(5-(6-(1H-benzo[d]imidazol-2-yl]picolinoyl)octahydropyrrolo[3,4-c]pyrrole-2-carbonyl)pyridin-2-yl)acetamide (L-48)

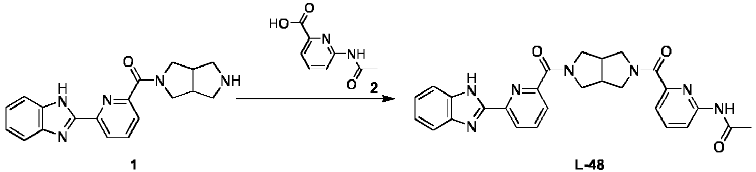

### Same operation as L-14

### 6-Acetamidopicolinic acid was purchased from Bide Pharmatech.

LC-MS: m/z: (M+H)⁺ = 496.2 , ¹H NMR (400 MHz, MeOD) δ 8.43 (dd, *J* = 13.6, 7.8 Hz, 1H), 8.15 (dd, *J* = 17.8, 8.1 Hz, 1H), 7.96 - 7.82 (m, 2H), 7.67 (dd, *J* = 41.3, 19.9 Hz, 2H), 7.53 - 7.41 (m, 1H), 7.31 (dt, *J* = 21.6, 7.7 Hz, 3H), 4.24-3.83 (m, 5H), 3.80 - 3.57 (m, 3H), 3.50 (s, 1H), 3.13 (d, *J* = 20.5 Hz, 2H), 2.20 (dd, *J* = 15.6, 8.0 Hz, 2H), 2.05 (d, *J* = 15.3 Hz, 2H), 1.63 (s, 1H).

### Embodiment 50: N-(1-(3-(imidazo[1,2-a]pyridin-2-yl)benzoyl)piperidin-4-yl)-2-(phenylamino)pyridine-4-formamide (L-50)

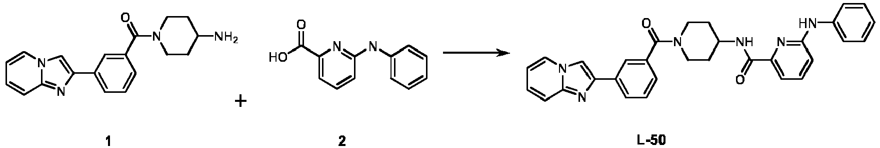

### Synthesis of N-(1-(3-(imidazo[1,2-a]pyridin-2-yl)benzoyl)piperidin-4-yl)-2-(phenylamino)pyridine-4-formamide

2-(Phenylamino)pyridine-4-carboxylic acid (0.060g, 0.27 mmol), benzotriazol-1-yl-oxytripyrrolidinophosphonium hexafluorophosphate (0.14 g, 0.27 mmol) and N,N-diisopropylethylamine (0.12 g, 1.0 mmol) were dissolved in 6 mL of N,N-dimethylformamide under ice bath, the mixture was stirred for 0.5 hours, then added with (4-aminopiperidin-1-yl)(3-(imidazo[1,2-a]pyridin-2-yl)phenyl)methanone (0.089 g, 0.27 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (18 mg, 34 µmol) with a yield of 12.8%. LC-MS: m/z: (M+H)⁺ = 517.

¹H NMR (400 MHz, Chloroform-*d*) δ 8.14 (m, 1H), 8.05 - 7.99 (m, 2H), 7.96 (d, *J* = 8.2 Hz, 1H), 7.89 (d, *J* = 0.7 Hz, 1H), 7.65 - 7.60 (m, 3H), 7.48 (m, 1H), 7.40 - 7.34 (m, 5H), 7.19 (m, 1H), 7.14 - 7.08 (m, 1H), 7.04 (s, 1H), 7.00 - 6.94 (m, 1H), 6.80 (m, 1H), 4.62 (s, 1H), 4.29 - 4.14 (m, 1H), 3.82 (s, 1H), 3.31 - 3.08 (m, 2H), 2.02 (m, 2H), 1.57 (d, 2H).

### Embodiment 51: N-(1-(3-(imidazo[1,2-a]pyridin-2-yl)benzoyl)piperidin-4-yl)-2-(phenylamino)pyridine-4-formamide (L-51)

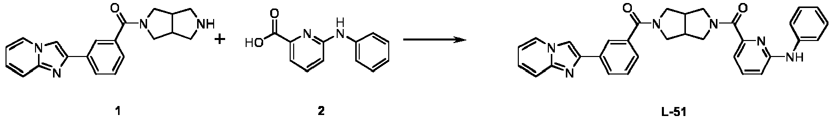

### Synthesis of N-(1-(3-(imidazo[1,2-a]pyridin-2-yl)benzoyl)piperidin-4-yl)-2-(phenylamino)pyridine-4-formamide

2-(Phenylamino)pyridine-4-carboxylic acid (0.060g, 0.27 mmol), benzotriazol-1-yl-oxytripyrrolidinophosphonium hexafluorophosphate (0.14 g, 0.27 mmol) and N,N-diisopropylethylamine (0.12 g, 1.0 mmol) were dissolved in 6 mL of N,N-dimethylformamide under ice bath, the mixture was stirred for 0.5 hours, then added with (hexahydropyrrolo[3,4-c]pyrrol-2(1H)-yl)(3-(imidazo[1,2-a]pyridin-2-yl)phenyl)methanone (0.092 g, 0.27 mmol), stirred continuously for 0.5 hours, returned to room temperature and reacted overnight. The reaction mixture was poured into 20 mL of water, extracted with ethyl acetate (20 mL × 3), and washed with saturated brine. The organic phases were combined, dried over anhydrous sodium sulfate, concentrated under reduced pressure, and purified by silica gel column chromatography (DCM:MeOH = 20:1) to obtain an off-white solid (16 mg, 30 µmol) with a yield of 11.2%. LC-MS: m/z: (M+H)⁺ = 529.

¹H NMR (400 MHz, Chloroform-*d*) δ 8.09 (td, *J* = 20.1, 18.0, 6.8 Hz, 3H), 7.89 (d, *J* = 14.3 Hz, 1H), 7.54 (dt, *J* = 53.5, 7.3 Hz, 4H), 7.36 (d, *J* = 4.4 Hz, 2H), 7.32 - 7.05 (m, 5H), 6.99 - 6.85 (m, 2H), 6.83 - 6.75 (m, 1H), 4.09 - 3.50 (m, 8H), 3.06 - 2.85 (m, 2H).

### Embodiment 52: N-(3-(6-(1H-benzo[d]imidazol-2-yl)picolinoyl)-3-azabicyclo[3.1.0]hexan-6-yl)-6-(pyridin-2-ylamino)picolinamide (L-52)

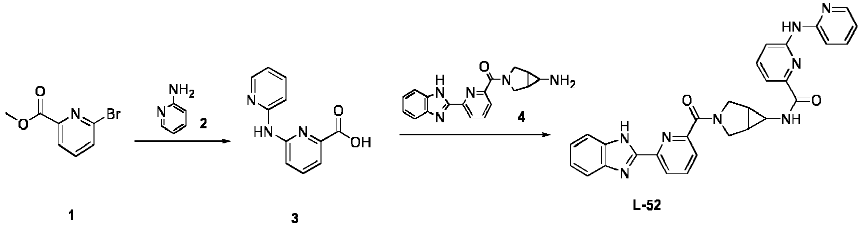

### I. Methyl 6-(pyridin-2-ylamino)picolinate

Methyl 6-bromopyridine-2-carboxylate (2 g, 9.26 mmol) was dissolved in 50 mL of dioxane, then 2-aminopyridine (871 mg, 9.26 mmol), tris(dibenzylideneacetone)dipalladium (424 mg, 0.46 mmol), 4,5-bisdiphenylphosphino-9,9-dimethylxanthene (536mg, 0.92 mmol) and cesium carbonate (7.54 g, 23.1 mmol) were added, and the reaction mixture was stirred at 95°C for 15 hours under the protection of nitrogen. The reaction mixture was cooled to room temperature, filtered and concentrated to obtain a yellow solid. The solid was dissolved in 15 mL of tetrahydrofuran, 10 mL of methanol and 15 mL of water, added with sodium hydroxide (0.94 g, 24 mmol), and the reaction mixture was stirred at 20°C for 15 hours. The reaction mixture was extracted three times with ethyl acetate (20 mL × 3). The aqueous phase was acidified with 2 N hydrochloric acid to adjust the pH to 1, extracted three times with ethyl acetate (20 mL × 3), then neutralized with saturated sodium bicarbonate aqueous solution to adjust the pH to 1, and the aqueous phase was concentrated to obtain a solid. The solid was added to 50 mL of N,N-dimethylformamide, the mixture was stirred at 20°C for 2 hours, filtered and concentrated to obtain 460 mg of a white solid with a yield of 23.0%. LC-MS: m/z: (M+H)⁺ = 216.0.

### II. (N-(3 -(6-(1H-benzo [d]imidazol-2-yl)picolinoyl)-3 -azabicyclo [3.1.0]hexan-6-yl)-2-(pyridin-2-ylamino)isonicotinamide

### Same operation as L-51

LC-MS: m/z: (M+H)⁺ = 517.0, ¹H NMR (400 MHz, CD₃OD) δ 8.45 (dd, *J* = 7.5, 1.4 Hz, 1H), 8.27 - 8.11 (m, 3H), 7.92 - 7.56 (m, 6H), 7.36 (dd, *J* = 6.1, 3.1 Hz, 2H), 7.24 (d, *J* = 7.0 Hz, 1H), 7.02 - 6.88 (m, 1H), 4.25 (dd, *J* = 21.8, 12.0 Hz, 2H), 3.99 (dd, *J* = 11.6, 4.4 Hz, 1H), 3.76 (dd, *J* = 12.4, 4.5 Hz, 1H), 2.74 (t, *J* = 2.2 Hz, 1H), 2.17 - 2.06 (m, 2H).

### Embodiment 53: (5-(6-(1-H-benzo[d]imidazol-2-yl)picolinoyl)hexahydropyrrolo[3,4-c]pyrrol-2(1H)-yl)(2-(pyridin-2-ylamino)pyridin-4-yl)methanone (L-53)

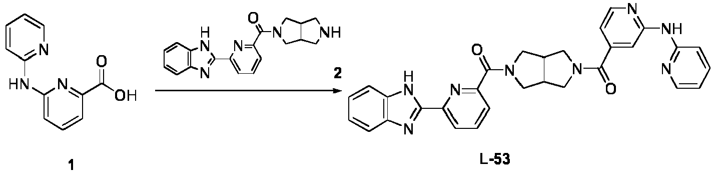

### (5-(6-(1-H-benzo[d]imidazol-2-yl)picolinoyl)hexahydropyrrolo[3,4-c]pyrrol-2(1H)-yl)(2-(pyridin-2-ylamino)pyridin-4-yl)methanone

### Same operation as L-14

LC-MS: m/z: (M+H)⁺ = 531.0, ¹H NMR (400 MHz, CD₃OD) δ 8.46 - 8.35 (m, 1H), 8.27 - 8.08 (m, 2H), 7.99 - 6.66 (m, 11H), 4.31 - 3.53 (m, 8H), 3.13 (dt, *J* = 13.3, 7.1 Hz, 2H).

### Embodiment 54: (5-(6-(imidazo[1,2-a]pyridin-2-yl]picolinoyl)hexahydropyrrolo[3,4-c]pyrrol-2(1H)-yl)(2-(pyridin-2-ylamino)pyridin-4-yl)methanone (L-54)

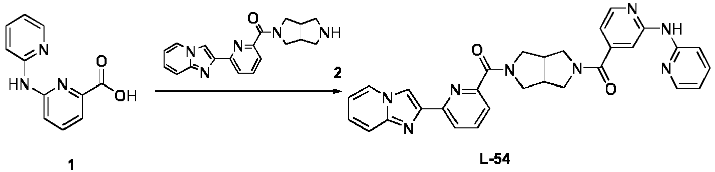

### (5-(6-(imidazo[1,2-a]pyridin-2-yl]picolinoyl)hexahydropyrrolo[3,4-c]pyrrol-2(1H)-yl)(2-(pyridin-2-ylamino)pyridin-4-yl)methanone

### Same operation as L-14

LC-MS: m/z: (M+H)⁺ = 531.0, ¹H NMR (400 MHz, CD₃OD) δ 8.43 (dd, *J* = 13.1, 6.8 Hz, 1H), 8.25 (d, *J* = 32.8 Hz, 1H), 8.09 (d, *J* =21.0 Hz, 2H), 7.67 - 7.47 (m, 5H), 7.40 - 7.28 (m, 2H), 7.13 (dt, *J* = 8.0, 5.8 Hz, 2H), 6.88 (ddt, *J* = 35.3,14.7, 7.3 Hz, 3H), 4.11-3.41 (m, 8H), 3.17 - 2.96 (m, 2H).

### Embodiment 55: (5-(6-(1-(1H-benzo[d]imidazol-2-yl]picolinoyl)hexahydropyrrolo[3,4-c]pyrrol-2(1H)-yl)(3-(imidazo[1,2-a]pyridin-2-yl)phenyl)methanone (L-55)

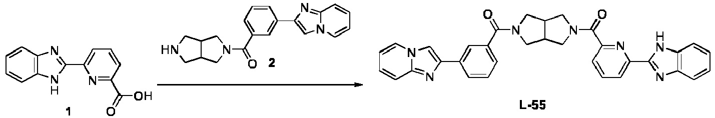

### Same operation as L-53

### (5-(6-(1-(1H-benzo[d]imidazol-2-yl]picolinoyl)hexahydropyrrolo[3,4-c]pyrrol-2(1H)-yl)(3-(imidazo[1,2-a]pyridin-2-yl)phenyl)methanone

LC-MS: m/z: (M+H)⁺ = 554.63, ¹H NMR (400 MHz, CDCl₃) δ 12.67 (s, 1H), 8.13 - 7.82 (m, 4H), 7.54 - 7.33 (m, 4H), 7.26 (d, *J* = 7.6 Hz, 2H), 4.54 (d, *J* = 11.9 Hz, 3H), 4.04 (t, *J* = 15.9 Hz, 3H), 1.85 (s, 1H), 1.77 (s, 1H).

### Embodiment 56: 6-(1H-benzo[d]imidazol-2-yl)-N-(1-(3-(imidazol[1,2-a]pyridin-2-yl)benzoyl)piperidin-4-yl)picolinamide (L-56)

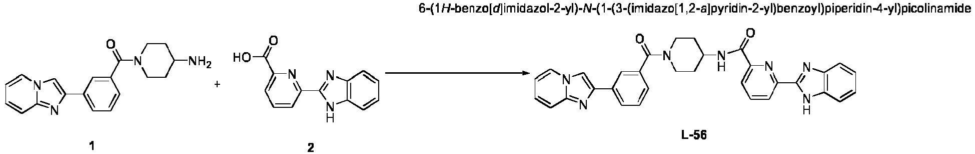

6-(1H-benzo[d]imidazol-2-yl)picofinic acid 2 (15 mg, 0.063 mmol) was dissolved in N,N-dimethylformamide (5 mL), added with 1H-benzotriazol-1-yl-oxytripyrrolidinophosphonium hexafluorophosphate (65 mg, 0.13 mmol) and N,N-diisopropylethylamine (0.02 mL), and stirred together at room temperature for 15 minutes. The reaction mixture was then added with (4-aminopiperidin-1-yl)(3-(imidazo[1,2-a]pyridin-2-yl)phenyl)methanone 1 (20 mg, 0.063 mmol), and stirred at room temperature for 16 hours. The reaction mixture was concentrated, and the obtained solid was purified twice by thin-layer chromatography plate (dichloromethane:methanol = 10:1) to obtain 8 mg of a white solid with a yield of 24%.

LC-MS m/z: (M+H)⁺ = 542.0, ¹H NMR (400 MHz, Chloroform-*d*) δ 8.68 (dd, *J* = 7.9, 1.1 Hz, 1H), 8.40 (d, *J* = 6.8 Hz, 1H), 8.31 (dd, *J* = 7.7, 1.1 Hz, 1H), 8.24 (s, 2H), 8.01 (t, *J* = 7.8 Hz, 1H), 7.93 (d, *J* = 7.8 Hz, 1H), 7.82 (d, *J* = 9.0 Hz, 1H), 7.45 (t, *J* = 7.7 Hz, 1H), 7.39 -7.31 (m, 2H), 7.21 (s, 2H), 7.03 (t, *J* = 6.7 Hz, 1H), 4.59 (s, 1H), 4.18 (s, 1H), 3.55 (s, 1H), 2.93 - 2.55 (m, 3H).

### Embodiment 57: (4-(1H-benzo[d]imidazol-2-yl)piperidin-1-yl)(6-(1H-benzo[d]imidazol-2-yl)pyridin-2-yl)methanone (L-57)

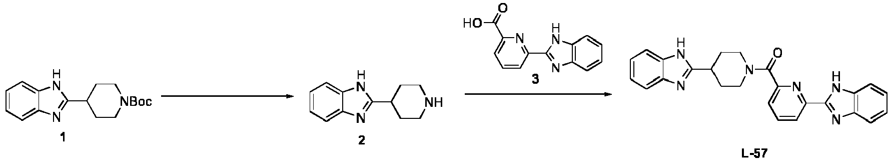

The operation of compounds 1 to 2 was the same as Boc deprotection in Embodiment 42, and the operation of compounds 2 to L-57 was the same as that of L-56

### 4-(1H-benzo[d]imidazol-2-yl)piperidin-1-yl)(6-(1H-benzo[d]imidazol-2-yl)pyridin-2-yl)methanone

LC-MS m/z: (M+H)⁺ = 422.9, ¹H NMR (400 MHz, Methanol-*d₄*) δ 8.43 (dd, *J* = 8.0, 1.0 Hz, 1H), 8.16 (t, *J* = 7.9 Hz, 1H), 7.72 (d, *J* = 1.1 Hz, 1H), 7.71 - 7.67 (m, 2H), 7.60 -7.53 (m, 2H), 7.34 (dd, *J* = 6.1, 3.1 Hz, 2H), 7.27 (dd, *J* = 6.1, 3.1 Hz, 2H), 3.96 (d, *J* = 13.7 Hz, 1H), 3.53 - 3.38 (m, 2H), 3.21 (dd, *J* = 12.8, 2.8 Hz, 1H), 2.33 (d, *J* = 12.9 Hz, 1H), 2.26 - 2.02 (m, 4H).

### Embodiment 58: (2,9-diazaspiro[5.5]undecane-2,9-diyl)bis(6-(1H-benzo[d]imidazol-2-yl)pyridin-2-yl)methanone (L-58)

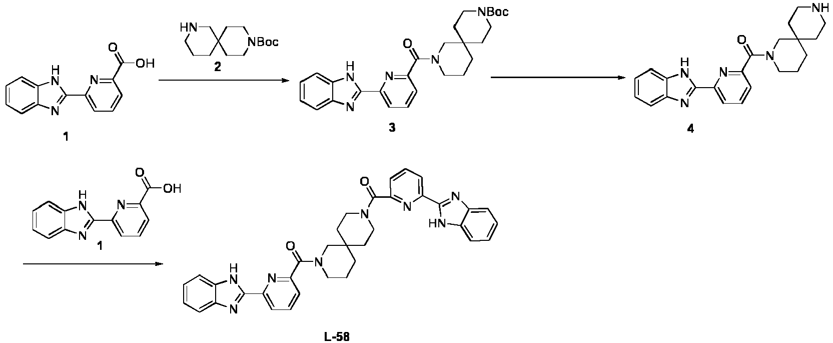

### Same operation as L-43

### tert-Butyl 2,9-diazaspiro[5.5]undecane-2-carboxylate 2 was purchased from Bide Pharmatech.

### (2,9-diazaspiro[5.5]undecane-2,9-diyl)bis(6-(1H-benzo[d]imidazol-2-yl)pyridin-2-yl)methanone

LC-MS m/z: (M+H)⁺ = 597.1, ¹H NMR (400 MHz, Methanol-*d₄*) δ 8.44 - 8.36 (m, 2H), 8.21 - 8.14 (m, 1H), 8.12 - 8.06 (m, 1H), 7.78 (q, *J* = 7.7 Hz, 1H), 7.71 - 7.68 (m, 2H), 7.64 - 7.60 (m, 2H), 7.32 (dt, *J* = 6.6, 3.1 Hz, 4H), 7.29 - 7.25 (m, 1H), 4.23 - 4.11 (m, 1H), 3.95 (dd, *J* = 12.2, 6.4 Hz, 1H), 3.83 (d, *J* = 21.5 Hz, 1H), 3.74 (dt, *J* = 13.3, 6.7 Hz, 3H), 3.68 - 3.58 (m, 2H), 3.47 (s, 2H), 1.92 - 1.83 (m, 1H), 1.80 (d, *J* = 11.9 Hz, 2H), 1.66 - 1.57 (m, 2H), 1.54 (d, *J* = 4.5 Hz, 1H).

### Embodiment 59: (2,8-diaza[4.5]decane-2,8-diyl)bis(6-(1H-benzo[d]imidazol-2-yl)pyridin-2-yl)methanone (L-59)

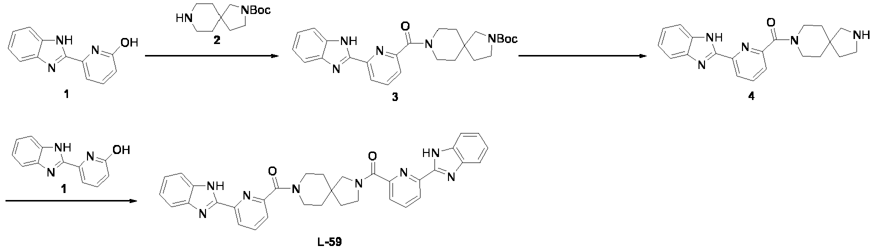

### Same operation as L-43

### tert-Butyl 2,9-diazaspiro[5.5]undecane-2-carboxylate 2 was purchased from Bide Pharmatech.

### (2,8-diaza[4.5]decane-2,8-diyl)bis(6-(1H-benzo[d]imidazol-2-yl)pyridin-2-yl)methanone

LC-MS m/z: (M+H)⁺ = 583.1, ¹H NMR (400 MHz, Methanol-*d₄*) δ 8.45 - 8.39 (m, 2H), 8.35 (dd, *J* = 7.9, 1.1 Hz, 1H), 8.17 - 8.09 (m, 2H), 8.06 (td, *J* = 8.0, 2.6 Hz, 1H), 7.82 (ddd, *J* = 12.1, 7.8, 1.0 Hz, 2H), 7.70 (d, *J* = 1.1 Hz, 1H), 7.58 (dd, *J* = 7.7, 1.0 Hz, 1H), 7.35 - 7.31 (m, 3H), 7.28 (dd, *J* = 6.1, 3.1 Hz, 1H), 3.97 (dd, *J* = 15.2, 7.7 Hz, 2H), 3.80 (dd, *J* = 12.5, 5.2 Hz, 3H), 3.66 - 3.58 (m, 2H), 2.08 - 1.97 (m, 3H), 1.91 (dd, *J* = 7.6, 4.9 Hz, 1H), 1.79 (t, *J* = 6.1 Hz, 2H), 1.69 (d, *J* = 5.1 Hz, 1H).

### Embodiment 60: (7-(6-(1H-benzo[d]imidazol-2-yl)nicotinoyl)-2,7-diazaspiro[4.4]nonan-2-yl)(6-(1H-benzo[d]imidazol-2-yl)pyridin-2-yl)methanone (L-60)

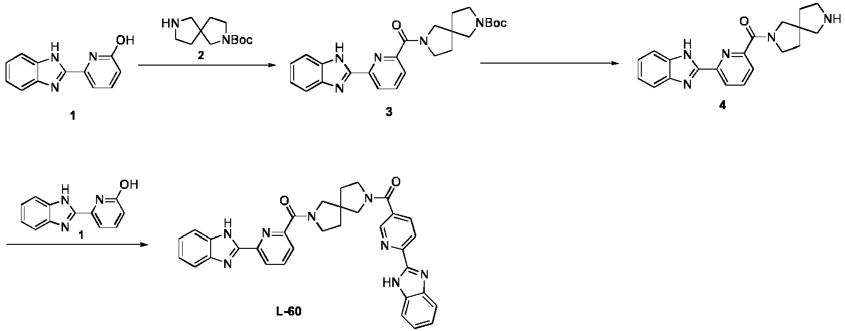

### Same operation as L-43

### tert-Butyl 2,9-diazaspiro[5.5]undecane-2-carboxylate 2 was purchased from Bide Pharmatech.

### (7-(6-(1H-benzo[d]imidazol-2-yl)nicotinoyl)-2,7-diazaspiro[4.4]nonan-2-yl)(6-(1H-benzo[d]imidazol-2-yl)pyridin-2-yl)methanone

LC-MS m/z: (M+H)⁺ = 569.1, ¹H NMR (400 MHz, Methanol-*d₄*) δ 8.44 (dt, *J* = 8.0, 1.1 Hz, 1H), 8.37 (ddd, *J* = 17.3, 7.9, 1.0 Hz, 1H), 8.16 (t, *J* = 7.8 Hz, 1H), 8.06 (dt, *J* = 10.2, 7.9 Hz, 1H), 7.88 (ddd, *J* = 10.2, 7.8, 1.0 Hz, 1H), 7.80 - 7.72 (m, 2H), 7.66 (s, 2H), 7.50 (s, 1H), 7.34 (d, *J* = 6.0 Hz, 1H), 7.30 (dq, *J* = 5.6, 3.0, 2.3 Hz, 3H), 4.20 - 3.97 (m, 2H), 3.89 (dd, *J* = 13.2, 6.2 Hz, 2H), 3.85 - 3.76 (m, 2H), 3.78 - 3.64 (m, 2H), 2.30 - 2.15 (m, 2H), 2.07 (dt, *J* = 13.7, 7.1 Hz, 2H).

### Embodiment 49: BT-11

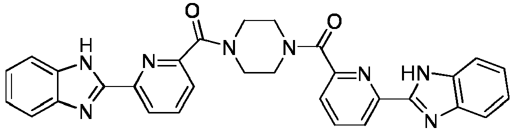

BT-11 was prepared with reference to Embodiment 2 in CN107108573A.

### Embodiment 61: (8-(6-(1H-benzo[d]imidazol-2-yl)pyridinyl)-2,8-diazaspiro[4.5]dec-2-yl)(6-(phenylamino)pyridin-2-yl)methanone (compound L-61)

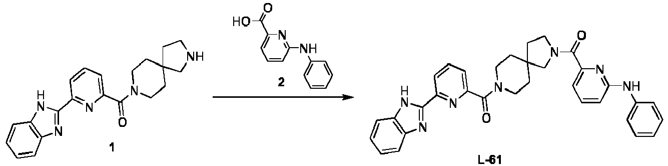

The operation was the same as the preparation of compound L-82 in Embodiment 82.

LC-MS: m/z: (M+H)⁺ = 558.1.

¹H NMR(400 MHz, Methanol-*d₄*) δ 8.40 (ddd, *J* = 11.2, 8.0, 1.0 Hz, 1H), 8.13 (dt, *J* = 13.7, 7.8 Hz,1H), 7.76 - 7.62 (m, 3H), 7.60 (dd, *J* = 7.7, 1.0 Hz, 1H), 7.58 - 7.50 (m, 2H), 7.39 - 7.28 (m, 2H), 7.22 (dtd, *J* = 16.2, 7.3, 1.9 Hz, 2H), 7.08 (ddd, *J* = 7.0, 6.1, 0.9 Hz, 1H), 6.97 - 6.81 (m, 2H), 4.10 - 3.91 (m, 1H),3.95 - 3.78 (m, 2H), 3.73 (dt, *J* = 13.3, 8.0 Hz, 2H), 3.64 - 3.54 (m, 2H), 3.44 (dt, *J* = 11.3, 5.5 Hz, 1H), 2.03 - 1.91 (m, 2H), 1.75 (td, *J* = 12.5, 12.1, 5.9 Hz, 2H), 1.62 (t, *J* = 5.9 Hz, 2H).

### Embodiment 62: (8-(6-(1H-benzo[d]imidazol-2-yl)picolinoyl)-2,8-diazaspiro[4.5]dec-2-yl)(2-(phenylamino)pyrimidin-4-yl)methanone (compound L-62)

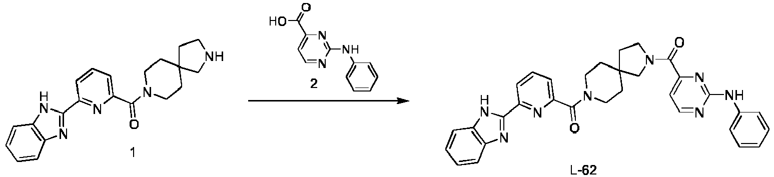

The operation was the same as the preparation of compound L-82 in Embodiment 82.

LC-MS: m/z: (M+H)⁺ = 559.1.

¹H NMR (400 MHz, Methanol-*d₄*) δ 8.61 - 8.56 (m, 1H), 8.40 (ddd, *J* = 9.1, 7.9, 1.0 Hz, 1H), 8.14 (dt, *J* = 10.8, 7.9 Hz, 1H), 7.74 - 7.67 (m, 2H), 7.66 (d, *J* = 1.1 Hz, 1H), 7.65 -7.61 (m, 2H), 7.34 (tt, *J* = 7.0,2.8 Hz, 2H), 7.31 - 7.23 (m, 2H), 7.05 - 7.02 (m, 1H), 7.01 - 6.94 (m, 1H), 3.85 (dq, *J* = 10.6, 6.2, 5.1Hz, 2H), 3.76 - 3.70 (m, 2H), 3.61 - 3.55 (m, 1H), 3.48 (d, *J* = 5.9 Hz, 1H), 2.04 - 1.96 (m, 2H), 1.76 (s,2H), 1.67 - 1.60 (m, 2H).

### Embodiment 63: (9-(6-(1H-benzo[d]imidazol-2-yl)picolinoyl)-2,9-diazaspiro[5.5]undecan-2-yl)(6-(phenylamino)pyridin-2-yl)methanone (compound L-63)

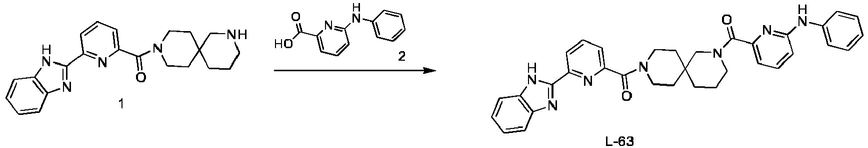

The operation was the same as the preparation of compound L-82 in Embodiment 82.

LC-MS m/z: (M+H)⁺ = 587.7. ¹H NMR (400 MHz, Chloroform-*d*) δ 8.68 (s, 1H), 8.56 (d, *J* = 8.0 Hz, 1H), 7.98 (t, *J* = 7.8 Hz, 1H), 7.80 - 7.74 (m, 2H), 7.48 7.40 (m, 1H), 7.31 (dd, *J* = 6.1, 3.2 Hz, 2H), 7.28 - 7.20 (m, 1H), 7.13 (dt, *J* =7.4, 4.5 Hz, 2H), 4.04 - 3.81 (m, 2H), 3.86 - 3.72 (m, 2H), 3.74 - 3.48 (m, 4H), 3.01 (s, 2H), 2.36 (s, 3H).

### Embodiment 64: (9-(6-(1H-benzo[d]imidazol-2-yl)picolinoyl)-2,9-diazaspiro[5.5]undecan-2-yl)(2-(phenylamino)pyrimidin-4-yl)methanone (compound L-64)

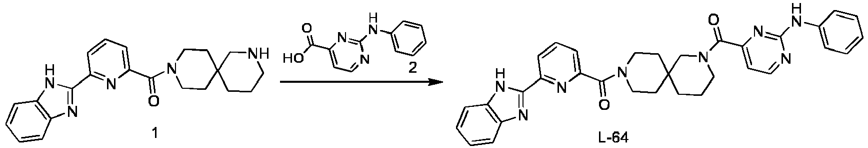

The operation was the same as the preparation of compound L-82 in Embodiment 82.

LC-MS m/z: (M+H)⁺ = 572.9. ¹H NMR (400 MHz, Methanol-*d₄*) δ 8.58 (d, *J* = 4.9 Hz, 1H), 8.43 - 8.36 (m, 1.5H), 8.26 (d, *J* = 4.9 Hz, 0.5H), 8.12 (td, *J* = 7.9, 1.4 Hz, 1.5H), 7.72 - 7.68 (m, 2H), 7.64 (dd, *J* = 7.8, 1.0 Hz, 2H), 7.58 - 7.54 (m, 1.5H), 7.33(tq, *J* = 7.5, 2.6 Hz, 5H), 7.19 (dd, *J* = 8.6, 7.3 Hz, 1H), 7.04 (tt, *J* = 7.4, 1.2 Hz, 1H), 6.91 (d, *J* = 4.9 Hz, 1H), 6.55 (d, *J* = 4.9 Hz, 0.5H), 4.10 (dt, *J* = 13.8, 4.9 Hz, 1H), 3.88 - 3.74 (m, 3H), 3.64 (tdd, *J* = 13.8, 9.6, 3.8 Hz, 4H), 3.47 (d, *J* = 5.3 Hz, 2H), 3.42 (d, *J* = 1.7 Hz, 1H), 1.80 (d, *J* = 16.3 Hz, 2H), 1.64 (dd, *J* = 9.7, 4.8 Hz, 3H).

### Embodiment 65: (7-(6-(1H-benzo[d]imidazol-2-yl)picolinoyl)-2,7-diazaspiro[4.4]non-2-yl)(6-(phenylamino)pyridin-2-yl)methanone (compound L-65)

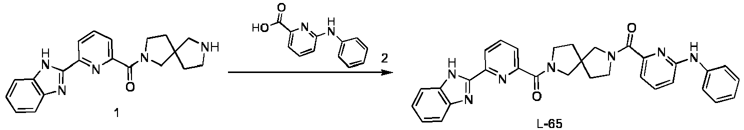

The operation was the same as the preparation of compound L-82 in Embodiment 82.

LC-MS m/z: (M+H)⁺ = 554.1. ¹H NMR (400 MHz, Methanol-*d₄*) δ 8.49 - 8.42 (m, 1H), 8.38 (d, *J* = 7.5 Hz, 1H), 8.32 (s, 1H), 8.21 - 8.18 (m, 1H), 8.17 - 8.05 (m, 2H), 7.86 (ddd, *J* = 7.8, 5.4, 1.1 Hz, 1H), 7.71 (dt, *J* = 7.1, 3.5 Hz, 1H), 7.61- 7.55 (m, 2H), 7.52 - 7.48 (m, 1H), 7.40 - 7.27 (m, 2H), 7.22 (dt, *J* = 6.2, 3.6 Hz, 1H), 6.95 (dtd, *J* =11.7, 6.8, 1.2 Hz, 1H), 4.18 - 3.59 (m, 8H), 3.24 - 3.04 (m, 2H).

### Embodiment 66: (7-(6-(1H-benzo[d]imidazol-2-yl)picolinoyl)-2,7-diazaspiro[4.4]non-2-yl)(4-(phenylamino)pyrimidin-2-yl)methanone (compound L-66)

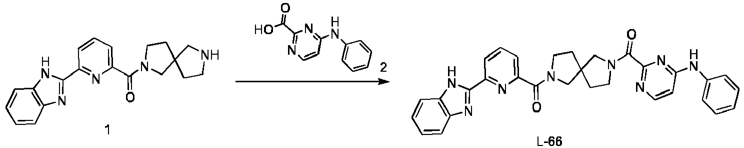

The operation was the same as the preparation of compound L-82 in Embodiment 82.

LC-MS: m/z: (M+H)⁺ = 544.9.

¹H NMR (400 MHz, Chloroform-*d*) δ 8.62 - 8.57 (m, 1H), 8.57 - 8.53 (m, 1 H), 8.01 - 7.91 (m, 1H), 7.82 - 7.77 (m, 1H), 7.75 - 7.68 (s, 1 H), 7.71 (s, 1H), 7.68 - 7.61 (m, 2H), 7.47 (ddd, *J* = 8.5, 3.6, 1.2 Hz, 1H), 7.36 - 7.30 (m, 2H), 7.27 - 7.22 (m, 1H), 7.12 - 7.08 (m, 1H), 7.05 - 6.96 (m, 1H), 3.96 - 3.68 (m, 6H), 3.69 - 3.53 (m, 2H), 2.02 - 1.88 (m, 4H).

### Embodiment 67: N-(3-(6-(1H-benzo[d]imidazol-2-yl)picolinoyl)-3-azabicyclo[3.1.0]hexan-6-yl)-6-methyl-1H-indole-2-formamide (compound L-67)

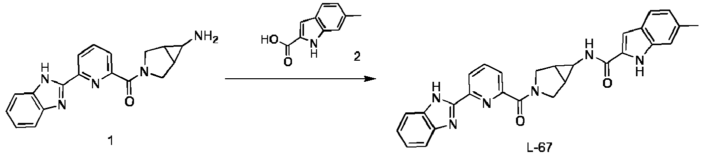

The operation was the same as the preparation of compound L-82 in Embodiment 82.

LC-MS: m/z: (M+H)⁺ = 476.9.

¹H NMR(400 MHz, DMSO-*d₆*) δ 13.20 (s, 1H), 11.43 (d, *J* = 2.1 Hz, 1H), 9.33 (d, *J* = 3.1 Hz, 1H), 8.46 (dd, *J* = 7.7, 1.2 Hz, 1H), 8.18 (t, *J* = 7.7 Hz, 1H), 8.11 (dd, *J* = 7.7, 1.2 Hz, 1H), 7.77 (dd, *J* = 7.9, 1.1Hz, 1H), 7.73 - 7.64 (m, 1H), 7.55 (d, *J* = 8.2 Hz, 1H), 7.35 (ddd, *J* = 8.2, 7.2, 1.2 Hz, 1H), 7.28 (ddd, *J* = 8.3, 7.2, 1.3 Hz, 1H), 7.25 (dd, *J* = 1.6, 0.8 Hz, 1H), 6.96 (dd, *J* = 2.3, 0.9 Hz, 1H), 6.90 (dd, *J* = 8.2, 1.5 Hz, 1H), 4.17 (s, 3H), 3.74 (s, 1H), 2.62 (q, *J* = 2.7 Hz, 1H), 2.41 (s, 3H), 2.21 (s, 1H), 2.09 (s, 1H).

### Embodiment 68: N-(3-(6-(1H-benzo[d]imidazol-2-yl)picolinoyl)-3-azabicyclo[3.1.0]hexan-6-yl)quinoline-2-formamide (compound L-68)

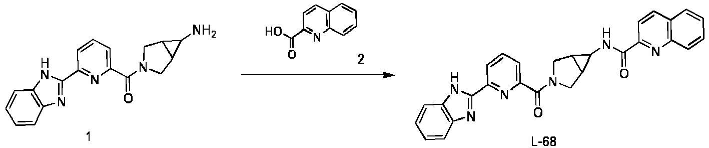

The operation was the same as the preparation of compound L-82 in Embodiment 82.

LC-MS: m/z: (M+H)⁺ = 476.1.

¹H NMR(400 MHz, DMSO) δ 13.18 (s, 1H), 9.31 (s, 1H), 8.50 (dd, *J* = 28.7, 8.2 Hz, 1H), 8.32 - 8.01 (m, 2H), 7.98 - 7.81 (m, 1H), 7.82 - 7.66 (m, 2H), 7.40 - 7.22 (m, 2H), 4.16 (d, *J* = 12.5 Hz, 1H), 3.77 (d, *J* = 12.2 Hz, 1H), 3.62 (s, 1H), 3.18 (d, *J* = 5.2 Hz, 1H), 2.10 (s, 1H).

### Embodiment 69: (5-(6-(1H-benzo[d]imidazol-2-yl)picolinoyl)hexahydropyrrolo[3,4-c]pyrrol-2(1H)-yl)(quinolin-2-yl)methanone (compound L-69)

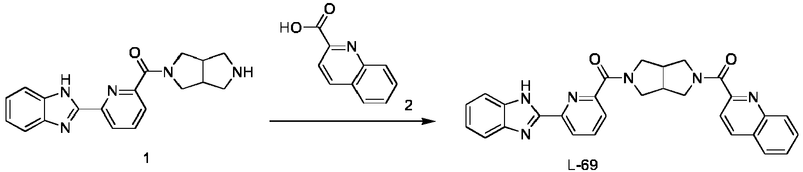

(6-(1H-benzo[d]imidazol-2-yl)pyridin-2-yl)(hexahydropyrrolo[3,4-c]pyrrol-2(1H)-yl)methanone (33 mg, 0.1 mmol) and quinoline-2-carboxylic acid (17 mg, 0.1 mmol) were suspended in N,N-dimethylformamide (3 mL), and N,N-diisopropylethylamine (26 mg, 0.2 mmol) and 1-propylphosphonic anhydride (41 mg, 0.13 mmol) were added thereto. The reaction mixture was stirred at 25°C for 16 hours. The reaction mixture was concentrated to obtain a crude product, and the resulting crude product was purified by thin-layer chromatography plate (dichloromethane:methanol = 15:1) to obtain a light yellow solid (15 mg, 31%). LC-MS: m/z: (M+H)⁺ = 488.9; ¹H NMR (400 MHz, DMSO-*d₆*) δ 12.93 (d, *J* = 6.3 Hz, 1H), 8.61 - 8.34 (m, 2H), 8.14 - 8.03 (m, 3H), 7.96 - 7.53 (m, 6H), 7.38 - 7.17 (m, 2H), 4.21 - 3.56 (m, 8H), 3.06 (s, 2H).

### Embodiment 70: (5-(6-(1H-benzo[d]imidazol-2-yl)picolinoyl)hexahydropyrrolo[3,4-c]pyrrol-2(1H)-yl)(6-methyl-1H-indol-2-yl)methanone (compound L-70)

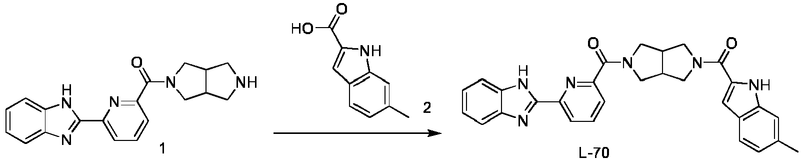

The operation was the same as the preparation of compound L-82 in Embodiment 82.

LC-MS: m/z: (M+H)⁺ = 491.1; ¹H NMR (400 MHz, DMSO-*d₆*) δ 12.91 (s, 1H), 11.42 (s, 1H), 8.42 (d, *J* = 7.9 Hz, 1H), 8.13 (t, *J* = 7.8 Hz, 1H), 7.77 (dd, *J* = 27.7, 7.7 Hz, 2H), 7.59 (d, *J* = 7.4 Hz, 1H), 7.54 - 7.38 (m, 1H), 7.35 - 7.15 (m, 3H), 6.92 (t, *J* = 20.7 Hz, 2H), 4.20 - 3.56 (m, 8H), 3.09 (d, *J* = 40.8 Hz, 2H), 2.40 (s, 3H).

### Embodiment 71: (5-(6-(1H-benzo[d]imidazol-2-yl)picolinoyl)hexahydropyrrolo[3,4-c]pyrrol-2(1H)-yl)(2-methyl-1H-indol-3-yl)methanone (compound L-71)

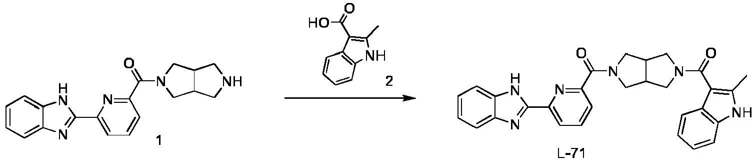

The operation was the same as the preparation of compound L-82 in Embodiment 82.

LC-MS: m/z: (M+H)⁺ = 491.1.

¹H NMR (400 MHz, CDCl₃) δ 8.68 (s, 1H), 8.56 (d, *J* = 7.7 Hz, 1H), 7.98 (t, *J* = 7.8 Hz, 1H), 7.77 (d, *J* = 7.4 Hz, 3H), 7.51 - 7.40 (m, 1H), 7.38 - 7.27 (m, 4H), 7.18 - 7.06 (m, 2H), 3.96 - 3.54 (m, 6H), 3.01 (s, 2H), 2.37 (s, 3H), 2.03 (d, *J* = 5.9 Hz, 1H), 1.66 (s, 1H).

### Embodiment 72: (9-(3-(imidazo[1,2-a]pyridin-2-yl)benzoyl)-3,9-diazaspiro[5.5]undecan-3-yl)(6-(phenylamino)pyridin-2-yl)methanone (compound L-72)

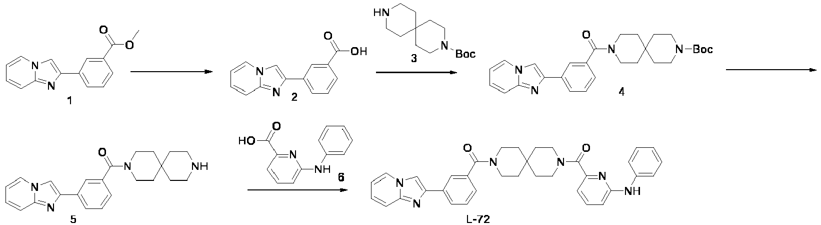

The operation was the same as the preparation of compound L-82 in Embodiment 82.

LC-MS: m/z: (M+H)⁺ = 433.2; ¹H NMR (400 MHz, Methanol-*d₄*) δ 8.46 (d, *J* = 6.8 Hz, 1H), 8.31 (s, 1H), 8.06 (d, *J* = 7.8 Hz, 1H), 8.00 (s, 1H), 7.66 - 7.54 (m, 5H), 7.45 - 7.33 (m, 2H), 7.27 (t, *J* = 7.9 Hz, 2H), 6.96 (td, *J* = 6.8, 4.4 Hz, 2H), 6.88 (t, *J* = 7.2 Hz, 2H), 3.79 (d, *J* = 16.7 Hz, 4H), 3.54 (s, 5H), 1.73 (s, 3H), 1.63 (s, 5H).

### Embodiment 73: (3,9-diazaspiro[5.5]undecane-3,9-diyl)bis((3-(imidazo[1,2-a]pyridin-2-yl)phenyl)methanone) (compound L-73)

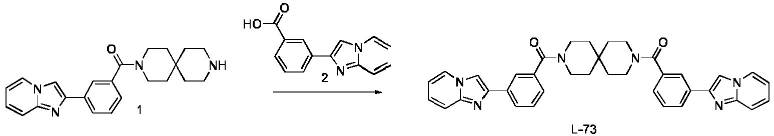

The operation was the same as the preparation of compound L-82 in Embodiment 82.

LC-MS: m/z: (M+H)⁺/2 = 298.2; ¹H NMR (400 MHz, Methanol-*d₄*) δ 8.45 (d, *J*= 6.8 Hz, 2H), 8.30 (s, 2H), 8.05 (d, *J* = 7.9 Hz, 2H), 8.00 (d, *J* = 1.8 Hz, 2H), 7.66 - 7.53 (m, 4H), 7.46 - 7.31 (m, 4H), 6.95 (t, *J* = 6.8 Hz, 2H), 3.83 (s, 4H), 3.52 (s, 4H), 1.69 (d, *J* = 46.5 Hz, 9H).

### Embodiment 74: (tetrahydropyrrolo[3,4-c]pyrrole-2,5(1H,3H)-diyl)bis((3-(imidazo[1,2-a]pyridin-2-yl)phenyl)methanone) (compound L-74)

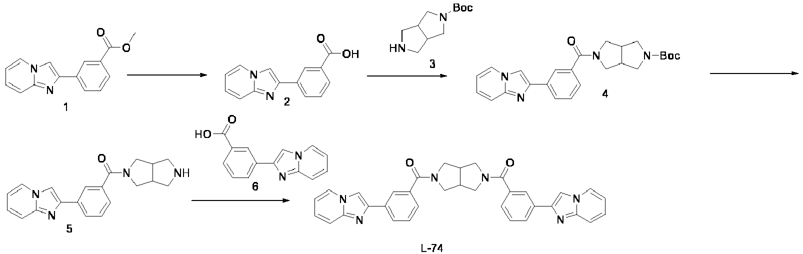

The operation was the same as the preparation of compound L-82 in Embodiment 82.

LC-MS: m/z: (M+H)⁺ = 553.2; ¹H NMR (400 MHz, Methanol-*d₄*) δ 8.43 (dd, *J* = 24.7, 6.9 Hz, 2H), 8.36 - 8.22 (m, 2H), 8.10 (dd, *J* = 29.2, 20.1 Hz, 4H), 7.57 (dd, *J* = 25.5, 8.6 Hz, 6H), 7.34 (dt, *J* = 17.0, 8.2 Hz, 2H), 6.94 (dt, *J* = 13.9, 6.8 Hz, 2H), 4.05 - 3.50 (m, 9H), 3.24 - 3.01 (m, 2H).

### Embodiment 75: (4-(2-hydroxyethyl)piperazin-1-yl)(3-(imidazo[1,2-a]pyridin-2-yl)phenyl)methanone) (compound L-75)

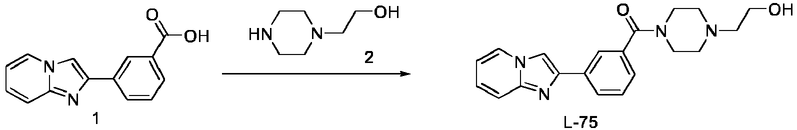

The operation was the same as the preparation of compound L-82 in Embodiment 82.

LC-MS: m/z: (M+H)⁺ = 351.2; ¹H NMR (400 MHz, Methanol-*d₄*) δ 8.46 (dt, *J* = 6.9, 1.2 Hz, 1H), 8.30 (s, 1H), 8.06 (dt, *J* = 7.9, 1.4 Hz, 1H), 8.00 (d, *J* = 1.7 Hz, 1H), 7.64 - 7.54 (m, 2H), 7.45 - 7.33 (m, 2H), 6.96 (td, *J* = 6.8, 1.2 Hz, 1H), 3.85 (s, 2H), 3.72 (t, *J* = 5.8 Hz, 2H), 3.56 (s, 2H), 2.75 - 2.51 (m, 6H).

### Embodiment 76: (6-(1H-benzo[d]imidazol-2-yl)pyridin-2-yl)(4-(2-hydroxyethyl)piperazin-1-yl)methanone (compound L-76)

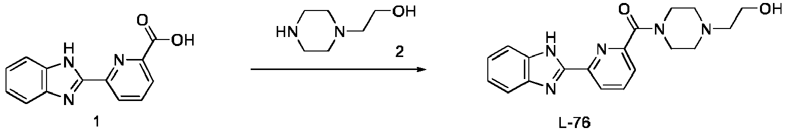

The operation was the same as the preparation of compound L-82 in Embodiment 82.

LC-MS: m/z: (M+H)⁺ = 352.2; ¹H NMR (400 MHz, Methanol-*d₄*) δ 8.40 (dd, *J* = 8.0, 0.9 Hz, 1H), 8.14 (t, *J* = 7.9 Hz, 1H), 7.75 (s, 1H), 7.68 (dd, *J* = 7.8, 1.0 Hz, 1H), 7.63 (d, *J* = 7.2 Hz, 1H), 7.33 (d, *J* = 6.0 Hz, 2H), 3.89 (t, *J* = 5.2 Hz, 2H), 3.72 (t, *J* = 5.8 Hz, 2H), 3.62 (t, *J* = 5.1 Hz, 2H), 2.71 (t, *J* = 5.2 Hz, 2H), 2.60 (dt, *J* = 12.0, 5.5 Hz, 4H).

### Embodiment 77: 6-(1H-benzo[d]imidazol-2-yl)-N-(3-(3-(benzo[d]thiazol-2-yl)benzoyl)-3-azabicyclo[3.1.0]hexane-6-yl)picolinamide (compound L-77)

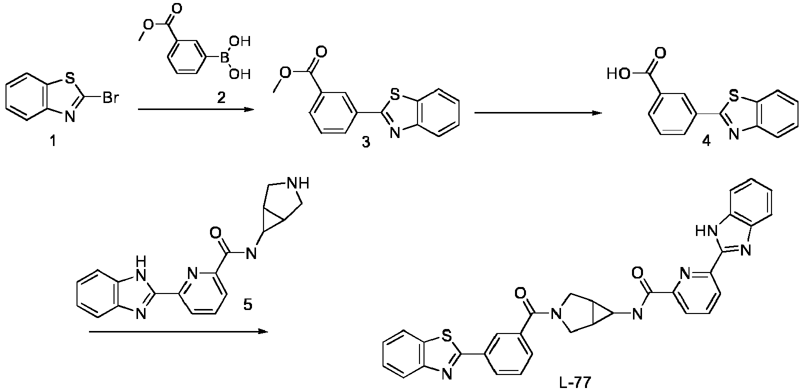

### I. Methyl 3-(benzo[d]thiazol-2-yl)benzoate

A solution of 2-bromo-1,3-benzothiazole (1.07 g, 5.0 mmol), (3-methoxycarbonylphenyl)boronic acid (0.9 g, 5.0 mmol), K₂CO₃ (1.38 g, 10.0 mmol) and [1,1'-bis(diphenylphosphino)ferrocene]palladium dichloride (0.1 g, 0.5 mmol) in dioxane (50 mL) and water (10 mL) was stirred at 80°C for 4 hours. The reaction mixture was concentrated to dryness and purified by flash chromatography (silica) (petroleum ether:ethyl acetate = 3:1) to obtain methyl 3-(1,3-benzothiazol-2-yl)benzoate as a white solid (1.0 g, 74.3%).

### II. 3-(Benzo[d]thiazol-2-yl)benzoic acid

Methyl 3-(1,3-benzothiazol-2-yl)benzoate (54 mg, 0.2 mmol) was dissolved in MeOH (5 mL) and water (1 mL), then LiOH (24 mg, 1.0 mmol) was added, and the reaction mixture was stirred at 80°C for 1 hour. The reaction mixture was concentrated to dryness to obtain a crude product, which was directly used in the next reaction.

### III. 6-(1H-benzo[d]imidazol-2-yl)-N-(3-(3-(benzo[d]thiazol-2-yl)benzoyl)-3-azabicyclo[3.1.0]hexane-6-yl)picolinamide

The operation was the same as the preparation of compound L-82 in Embodiment 82.

LC-MS: m/z: (M+H)⁺ = 557.2; ¹H NMR (400 MHz, DMSO-*d₆*) δ 13.18 (s, 1H), 9.29 (d, *J* = 3.1 Hz, 1H), 8.46 (dd, *J* = 7.8, 1.2 Hz, 1H), 8.24 - 8.15 (m, 4H), 8.13 - 8.08 (m, 2H), 7.83 - 7.65 (m, 4H), 7.59 (td, *J* = 8.2, 7.7, 1.3 Hz, 1H), 7.55 - 7.47 (m, 1H), 7.31 (s, 2H), 4.17 (d, *J* = 12.1 Hz, 1H), 4.00 - 3.85 (m, 1H), 3.71 - 3.61 (m, 2H), 2.07 (s, 2H).

### Embodiment 78: (3,9-diazaspiro[5.5]undecane-3,9-diyl)bis((2-(phenylamino)pyrimidin-4-yl)methanone) (compound L-78)

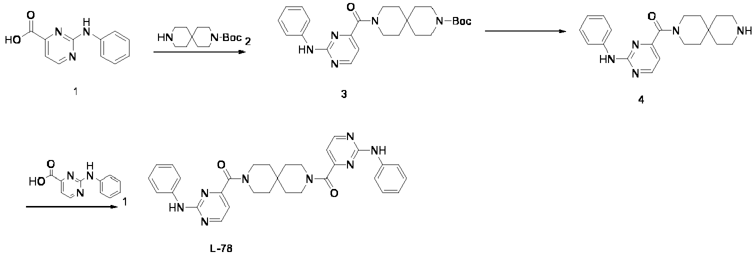

### I. tert-Butyl 9-(2-(2-(phenylamino)pyrimidine-4-carbonyl)-3,9-diazaspiro[5.5]undecane-3-carboxylate

*tert-*Butyl 3,9-diazaspiro[5.5]undecane-3-carboxylate (100 mg, 0.39 mmol) (compound represented by formula 2) and 2-phenylaminopyrimidine-4-carboxylic acid (85 mg, 0.39 mmol) (compound represented by formula 1) were suspended in 3 mL of N,N-dimethylformamide, then N,N-diisopropylethylamine (101 mg, 0.78 mmol) and 2-(7-azobenzotriazole)-N,N,N',N'-tetramethyluronium hexafluorophosphate (179 mg, 0.47 mmol) were added. The reaction mixture was stirred at 30°C for 16 hours. The reaction mixture was added with 10 mL of ice water, stirred for 5 minutes, filtered, and the solid was dried to obtain 175 mg of a crude product as a brown solid with a yield of 98.58%. LC-MS: m/z: (M+H)⁺ = 452.0.

### II. (2-(phenylamino)pyrimidin-4-yl)(3,9-diazaspiro[5.5]undecan-3-yl)methanone

*tert*-Butyl 9-(2-(2-(phenylamino)pyrimidine-4-carbonyl)-3,9-diazaspiro[5.5]undecane-3-carboxylate (175 mg, 0.39 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated and dried to obtain 136 mg of a crude product as a brown solid with a yield of 99.87%. LC-MS: m/z: (M+H)⁺ = 352.0.

### III. (3,9-diazaspiro[5.5]undecane-3,9-diyl)bis((2-(phenylamino)pyrimidin-4-yl)methanone)

The operation was the same as the preparation of compound L-82 in Embodiment 82.

LC-MS: m/z: (M+H)⁺ = 549.0, ¹H NMR (400 MHz, CD₃OD) δ 8.55 (d, *J* = 4.9 Hz, 2H), 7.67 (dd, *J* = 8.6, 1.0 Hz, 4H), 7.36 - 7.26 (m,4H), 7.03 (t, *J* = 7.4 Hz, 2H), 6.87 (d, *J* = 4.9 Hz, 2H), 3.77 (dd, *J* = 11.7, 5.6 Hz, 4H), 3.50 (dd, *J* = 11.1, 5.3 Hz, 4H), 1.77 - 1.57 (m, 8H).

### Embodiment 79: 2-(phenylamino)-N-(1-(2-(2-(phenylamino)pyrimidine-4-carbonyl)piperidin-4-yl)pyrimidine-4-carboxamide (compound L-79)

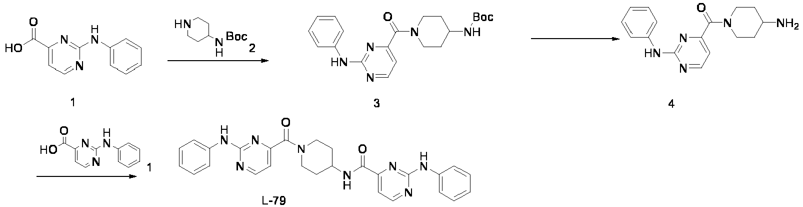

### I. tert-Butyl (1-(2-(phenylamino)pyrimidine-4-carbonyl)piperidin-4-yl)carbamate

*tert*-Butylpiperidin-4-ylcarbamate (100 mg, 0.50 mmol) (compound represented by formula 2) and 2-phenylaminopyrimidine-4-carboxylic acid (107 mg, 0.50 mmol) (compound represented by formula 1) were suspended in 3 mL of N,N-dimethylformamide, then N,N-diisopropylethylamine (129 mg, 1.0 mmol) and 2-(7-azobenzotriazole)-N,N,N',N'-tetramethyluronium hexafluorophosphate (228 mg, 0.60 mmol) were added. The reaction mixture was stirred at 30°C for 16 hours. The reaction mixture was added with 10 mL of ice water, stirred for 5 minutes, filtered, and the solid was dried to obtain 185 mg of a crude product as a brown solid with a yield of 93.21%. LC-MS: m/z: (M+H)⁺ = 498.0.

### II. (4-aminopiperidin-1-yl)(2-(phenylamino)pyrimidin-4-yl)methanone

*tert*-Butyl (1-(2-(phenylamino)pyrimidine-4-carbonyl)piperidin-4-yl)carbatnate (185 mg, 0.47 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated and dried to obtain 138 mg of a crude product as a brown solid with a yield of 99.70%. LC-MS: m/z: (M+H)⁺ = 298.0.

### III. 2-(phenylatnino)-N-(1-(2-(2-(phenylatnino)pyrimidine-4-carbonyl)piperidin-4-yl)pyrimidine-4-carboxamide

The operation was the same as the preparation of compound L-82 in Embodiment 82.

LC-MS: m/z: (M+H)⁺ = 495.0, ¹H NMR (400 MHz, CD₃OD) δ 8.66 (d, *J* = 4.9 Hz, 1H), 8.57 (d, *J* = 4.9 Hz, 1H), 7.74 - 7.60 (m, 4H), 7.41 - 7.24 (m, 5H), 7.04 (dt, *J* = 20.0, 7.4 Hz, 2H), 6.91 (d, *J* = 4.9 Hz, 1H), 4.56 (d, *J* = 14.7 Hz, 1H), 4.26 - 4.15 (m, 1H), 3.98 - 3.89 (m, 1H), 3.41 - 3.34 (m, 1H), 3.23 - 3.13 (m, 1H), 2.19 - 2.08 (m, 1H), 2.02 (dd, *J* = 9.1, 4.0 Hz, 1H), 1.79 -1.63 (m, 2H).

### Embodiment 80: (9-(6-(phenylamino)picolinoyl)-3,9-diazaspiro[5.5]undecan-3-yl)(2-(phenylamino)pyrimidin-4-yl)methanone (compound L-80)

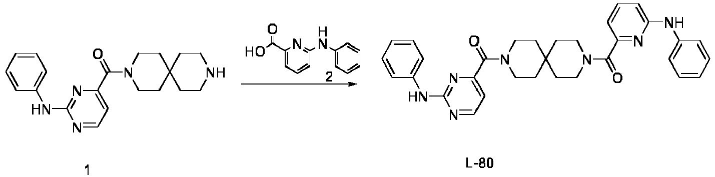

The operation was the same as the preparation of compound L-82 in Embodiment 82.

LC-MS: m/z: (M+H)⁺ = 548.0, ¹H NMR (400 MHz, CD₃OD) δ (d, *J* = 4.9 Hz, 1H), 7.64 (ddd, *J* = 11.8, 9.4, 6.1 Hz, 5H), 7.29 (dt, *J* = 12.4, 7.9 Hz, 4H), 6.99 (dt, *J* = 26.9, 7.4 Hz, 2H), 6.88 (dd, *J* = 9.5, 5.7 Hz, 3H), 3.77 (dt, *J* = 11.6, 5.7Hz, 4H), 3.61 - 3.44 (m, 4H), 1.83 - 1.50 (m, 8H).

### Embodiment 81: 6-(phenylamino)-N-(1-(2-(phenylamino)pyrimidine-4-carbonyl)piperidin-4-yl)picolinamide (compound L-81)

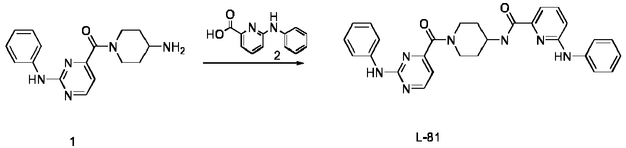

The operation was the same as the preparation of compound L-82 in Embodiment 82.

LC-MS: m/z: (M+H)⁺ = 494.0, ¹H NMR (400 MHz, CD₃OD) δ 8.57 (d, *J* = 4.9 Hz, 1H), 7.69 (dd, *J* = 16.7, 8.2 Hz, 3H), 7.49 (dd, *J* =18.9, 7.5 Hz, 3H), 7.31 (dt, *J* = 24.3, 7.9 Hz, 4H), 7.07 - 6.96 (m, 3H), 6.91 (d, *J* = 4.9 Hz, 1H), 4.46 (d, *J* = 13.3 Hz, 1H), 4.19 (ddd, *J* = 14.0, 9.9, 4.0 Hz, 1H), 3.86 (d, *J* = 13.4 Hz, 1H), 3.43 - 3.36 (m, 1H), 3.27(dd, *J* = 13.6, 3.0 Hz, 1H), 2.23 - 2.11 (m, 1H), 2.09 - 1.97 (m, 1H), 1.76 - 1.60 (m, 2H).

### Embodiment 82: (5-(6-(1H-benzo[d]imidazol-2-yl)picolinoyl)hexahydropyrrolo[3,4-c]pyrrol-2(1H)-yl)(2-(((4-fluorophenyl)amino)pyrimidin-4-yl)methanone (compound L-82)

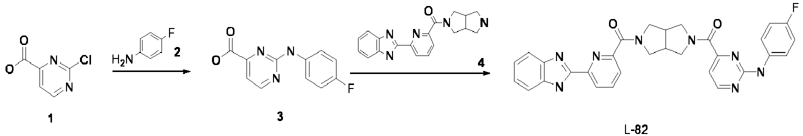

### I. 2-((4-fluorophenyl)amino)pyrimidine-4-carboxylic acid

2-Chloropyrimidine-4-carboxylic acid (500 mg, 3.15 mmol) was dissolved in 15 mL of dioxane, then p-fluoroaniline (881 mg, 9.46 mmol) was added, and the reaction mixture was stirred at 70°C for 18 hours. The reaction mixture was cooled to room temperature, then 20 mL of water and 10 mL of 1 N sodium hydroxide were added, and the reaction mixture was extracted twice with ethyl acetate (20 mL × 2). The aqueous phase was acidified with 1 N hydrochloric acid to adjust the pH to 3, and the solid was filtered and dried to obtain 500 mg of a white solid with a yield of 67.98%. LC-MS: m/z: (M+H)⁺ = 234.0.

### II. (5-(6-(1H-benzo[d]imidazol-2-yl)picolinoyl)hexahydropyrrolo[3,4-c]pyrrol-2(1H)-yl)(2-(((4-fluorophenyl)amino)pyrimidin-4-yl)methanone

2,3,3a,4,6,6a-Hexahydro-1H-pyrrolo[3,4-c]pyrrol-5-yl-[6-(1H-benzimidazol-2-yl)-2-pyridyl (40 mg, 0.12 mmol) (compound represented by formula 4) and 2-((4-fluorophenyl)amino)pyrimidine-4-carboxylic acid (28 mg, 0.12 mmol) (compound represented by formula 3) were suspended in 5 mL of N,N-dimethylformamide, then N,N-diisopropylethylamine (62 mg, 0.48 mmol) and 1-propylphosphonic anhydride (115 mg, 0.18 mmol) were added. The reaction mixture was stirred at 15°C for 16 hours. The reaction mixture was concentrated to obtain a crude product, and the resulting crude product was purified by thin-layer chromatography plate (dichloromethane:methanol = 10:1) to obtain 20 mg of the desired product as a white solid with a yield of 30.39%.

LC-MS: m/z: (M+H)⁺ = 549.0, ¹H NMR (400 MHz, CDCl₃) δ 8.48 (ddd, *J* = 39.8, 21.6, 6.4 Hz, 2H), 8.03 - 7.54 (m, 6H), 7.49 - 7.30 (m, 2H), 7.26 - 6.93 (m, 3H), 4.31 - 3.67 (m, 8H), 3.12 - 2.91 (m, 2H).

### Embodiment 83: (Tetrahydropyrrolo[3,4-c]pyrrole-2,5(1H,3H)-diyl)bis((2-phenylamino)pyrimidin-4-yl)methanone) (compound L-83)

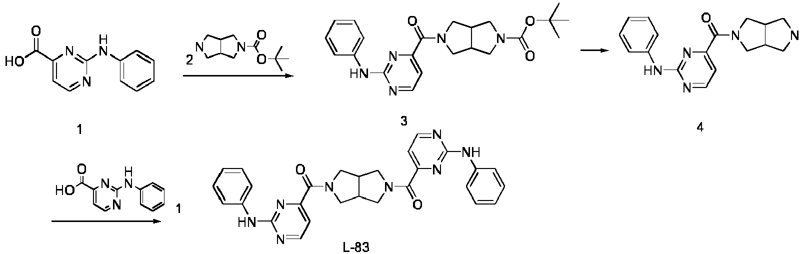

### I. tert-Butyl 5-(2-(phenylamino)pyrimidine-4-carbonyl)hexahydropyrrolo[3,4-c]pyrrole-2(1H)-carboxylate

*tert*-Butyl 2,3,3a,4,6,6a-hexahydro-1H-pyrrolo[3,4-c]pyrrole-5-carboxylate (100 mg, 0.47 mmol) (compound represented by formula 2) and 2-phenylaminopyrimidine-4-carboxylic acid (101 mg, 0.47 mmol) (compound represented by formula 1) were suspended in 3 mL of N,N-dimethylformamide, then N,N-diisopropylethylamine (122 mg, 0.94 mmol) and 1-propylphosphonic anhydride (449 mg, 0.71 mmol) were added. The reaction mixture was stirred at 15°C for 16 hours. The reaction mixture was added with 10 mL of ice water, stirred for 5 minutes, filtered, and the solid was dried to obtain 180 mg of a crude product as a brown solid with a yield of 93.31%. LC-MS: m/z: (M+H)⁺ = 410.0.

### II. (Hexahydropyrrolo[3,4-c]pyrrol-2(1H)-yl)(2-(phenylamino)pyrimidin-4-yl)methanone

*tert*-Butyl (5-(2-(phenylamino)pyrimidine-4-carbonyl)hexahydropyrrolo[3,4-c]pyrrole-2(1H)-carboxylate (180 mg, 0.44 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated, and the solid was washed with ethyl acetate (10 mL) and dried to obtain 120 mg of a crude product as a brown solid with a yield of 88.24%. LC-MS: m/z: (M+H)⁺ = 310.0.

### III. (Tetrahydropyrrolo[3,4-c]pyrrole-2,5(1H,3H)-diyl)bis((2-phenylamino)pyrimidin-4-yl)methanone)

The operation was the same as the preparation of compound L-82 in Embodiment 82.

LC-MS: m/z: (M+H)⁺ = 507.0, ¹H NMR (400 MHz, CDCl₃) δ 8.56 - 8.42 (m, 2H), 7.53 (dd, *J* = 19.5, 8.3 Hz, 4H), 7.18 (dt, *J* = 30.7, 7.3Hz, 4H), 7.03 - 6.79 (m, 4H), 3.93 - 3.46 (m, 8H), 2.94 (d, *J* = 7.4 Hz, 2H).

### Embodiment 84: (2-(phenylamino)-N-(3-(2-(2-(phenylamino)pyrimidine-4-carbonyl)-3-azabicyclo[3.1.0]hexan-6-yl)pyrimidine-4-carboxamide (compound L-84)

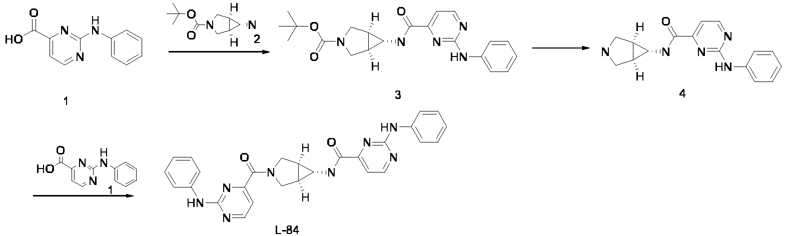

### I. tert-Butyl ((1R,5S,6s)-6-(2-(phenylamino)pyrimidine-4-carboxamido)-3-azabicyclo[3.1.0]hexane-3-carboxylate

*tert*-Butyl (1R,SS,6s)-6-amino-3-azabicyclo[3.1.0]hexane-3-carboxylate (100 mg, 0.50 mmol) (compound represented by formula 2) and 2-phenylaminopyrimidine-4-carboxylic acid (108 mg, 0.51 mmol) (compound represented by formula 1) were suspended in 3 mL of N,N-dimethylformamide, then N,N-diisopropylethylamine (130 mg, 1.0 mmol) and 1-propylphosphonic anhydride (480 mg, 0.75 mmol) were added. The reaction mixture was stirred at 15°C for 16 hours. The reaction mixture was added with 10 mL of ice water, stirred for 5 minutes, filtered, and the solid was dried to obtain 180 mg of a crude product as a brown solid with a yield of 90.28%. LC-MS: m/z: (M+H)⁺ = 396.0.

### II. N-((1R,5S,6s)-3-azabicyclo[3.1.0]hexan-6-yl)-2-(phenylamino)pyrimidine-4-carboxamide

*tert*-Butyl ((1R,5S,6s)-6-(2-(phenylamino)pyrimidine-4-carboxamido)-3-azabicyclo[3.1.0]hexane-3-carboxylate (180 mg, 0.45 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated, and the solid was washed with ethyl acetate (10 mL) and dried to obtain 120 mg of a crude product as a brown solid with a yield of 89.27%. LC-MS: m/z: (M+H)⁺ = 296.0.

### III. (2-(phenylamino)-N-(3-(2-(2-(phenylamino)pyrimidine-4-carbonyl)-3-azabicyclo[3.1.0]hexan-6-yl)pyrimidine-4-carboxamide

The operation was the same as the preparation of compound L-82 in Embodiment 82.

LC-MS: m/z: (M+H)⁺ = 493.0, ¹H NMR (400 MHz, DMSO-*d₆*) δ 9.80 (d, *J* = 28.2 Hz, 1H), 8.70 - 8.62 (m, 2H), 7.76 - 7.74 (m, 4H), 7.32 - 7.25 (m, 4H), 7.01 - 6.97 (3, 3H), 4.04 - 3.96 (m, 1H), 3.87 (d, *J* = 11.3 Hz, 1H), 3.79 (dd, *J* = 11.4, 3.9 Hz, 1H), 3.59 (dd, *J* = 12.3, 4.0Hz, 1H), 2.66 (d, *J* = 2.2 Hz, 1H), 1.98 (d, *J* = 5.9 Hz, 2H).

### Embodiment 85: (2-(phenylamino)pyrimidin-4-yl)(piperazin-1-yl)methanone piperazine-1,4-diylbis((2-(phenylamino)pyrimidin-4-yl)methanone (compound L-85)

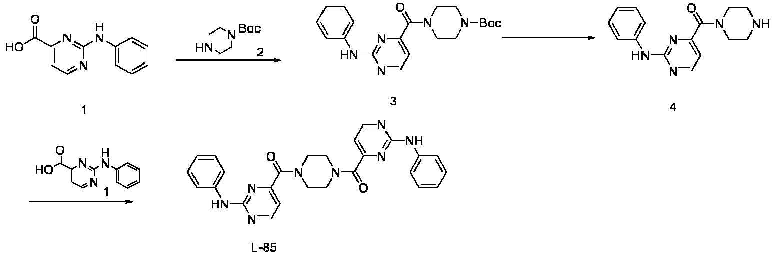

### I. tert-Butyl 4-(2-(phenylatnino)pyrimidine-4-carbonyl)piperazine-1-carboxylate

1-*tert*-Butoxycarbonyl-piperazine (100 mg, 0.54 mmol) (compound represented by formula 2) and 2-phenylaminopyrimidine-4-carboxylic acid (115 mg, 0.54 mmol) (compound represented by formula 1) were suspended in 3 mL of N,N-dimethylformamide, then N,N-diisopropylethylamine (139 mg, 1.07 mmol) and 2-(7-azobenzotriazole)-N,N,N',N'-tetramethyluronium hexafluorophosphate (245 mg, 0.64 mmol) were added. The reaction mixture was stirred at 30°C for 16 hours. The reaction mixture was added with 10 mL of ice water, stirred for 5 minutes, filtered, and the solid was dried to obtain 190 mg of a crude product as a brown solid with a yield of 92%. LC-MS: m/z: (M+H)⁺ = 384.0.

### II. ((2-(phenylamino)pyrimidin-4-yl)(piperazin-1-yl)methanone

*tert*-Butyl 4-(2-(phenylatnino)pyrimidine-4-carbonyl)piperazine-1-carboxylate (190 mg, 0.49 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated and dried to obtain 139 mg of a crude product as a brown solid with a yield of 99.01%. LC-MS: m/z: (M+H)⁺ = 284.0.

### III. (2-(phenylamino)pyrimidin-4-yl)(piperazin-1-yl)methanone piperazine-1,4-diylbis((2-(phenylamino)pyrimidin-4-yl)methanone

The operation was the same as the preparation of compound L-82 in Embodiment 82.

LC-MS: m/z: (M+H)⁺ = 481.0, ¹H NMR (400 MHz, DMSO-*d₆*) δ 9.84 (d, *J* = 21.0 Hz, 2H), 8.74 - 8.51 (m, 2H), 7.71 (dd, *J* = 20.8, 7.4 Hz, 4H), 7.50 - 7.21 (m, 4H), 7.13 - 6.78 (m, 4H), 3.62 (dd, *J* = 76.7, 37.7 Hz, 8H).

### Embodiment 86: Piperazine-1,4-diylbis((6-(phenylamino)pyridin-2-yl)methanone) (compound L-86)

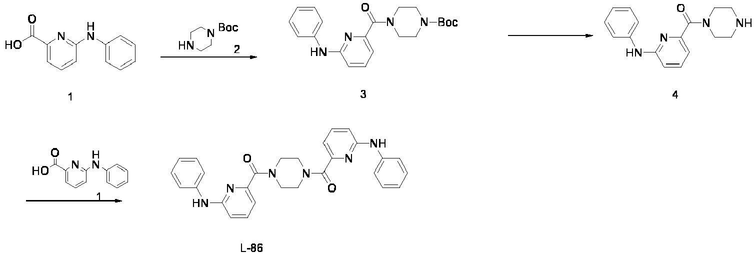

### I. tert-Butyl 4-(6-(phenylamino)picolinoyl)piperazine-1-carboxylate

1-*tert*-Butoxycarbonyl-piperazine (100 mg, 0.54 mmol) (compound represented by formula 2) and 6-(phenylamino)picolinic acid (115 mg, 0.54 mmol) (compound represented by formula 1) were suspended in 3 mL of N,N-dimethylformamide, then N,N-diisopropylethylamine (139 mg, 1.07 mmol) and 2-(7-azobenzotriazole)-N,N,N',N'-tetramethyluronium hexafluorophosphate (245 mg, 0.64 mmol) were added. The reaction mixture was stirred at 30°C for 16 hours. The reaction mixture was added with 10 mL of ice water, stirred for 5 minutes, filtered, and the solid was dried to obtain 190 mg of a crude product as a brown solid with a yield of 92%. LC-MS: m/z: (M+H)⁺ = 383.0.

### II. (6-(phenylamino)pyridin-2-yl)(piperazin-1-yl)methanone

*tert*-Butyl 4-(6-(phenylatnino)picolinoyl)piperazine-1-carboxylate (190 mg, 0.49 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated and dried to obtain 139 mg of a crude product as a brown solid with a yield of 99.12%. LC-MS: m/z: (M+H)⁺ = 283.0.

### III. Piperazine-1,4-diylbis((6-(phenylamino)pyridin-2-yl)methanone)

The operation was the same as the preparation of compound L-82 in Embodiment 82.

LC-MS: m/z: (M+H)⁺ = 479.0, ¹H NMR (400 MHz, DMSO-*d₆*) δ 9.21 (d, *J* = 26.7 Hz, 2H), 7.81 - 7.50 (m, 6H), 7.26 (dt, *J* = 44.9, 7.5 Hz, 4H), 7.03 - 6.70 (m, 6H), 3.83 - 3.44 (m, 8H).

### Embodiment 87: (2,6-diazaspiro[3.3]heptane-2,6-diyl)bis((6-(1H-benzo[d]imidazol-2-yl)pyridin-2-yl)methanone) (compound L-87)

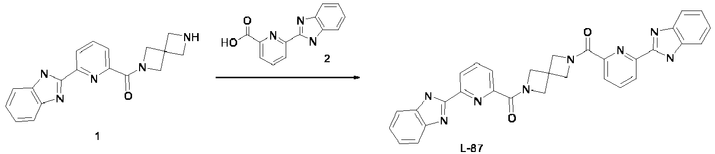

The operation was the same as the preparation of compound L-82 in Embodiment 82.

LC-MS: m/z: (M+H)⁺ = 542.2.

¹H NMR (400 MHz, DMSO-*d₆*) δ 8.45 (dd, *J* = 7.9, 1.1 Hz, 2H), 8.16 (t, *J* = 7.8 Hz, 2H), 8.00 (dd, *J* = 7.8, 1.1 Hz, 2H), 7.70 (s, 4H), 7.28 (d, *J* = 6.5 Hz, 4H), 5.10 (d, *J* = 10.7 Hz, 2H), 5.01 (d, *J* = 10.8 Hz, 2H), 4.46 - 4.39 (m, 4H).

### Embodiment 88: 3-(imidazo[1,2-a]pyridin-2-yl)-N-(1-(3-(imidazo[1,2-a]pyridin-2-yl)benzoyl)piperidin-4-yl)benzamide (compound L-88)

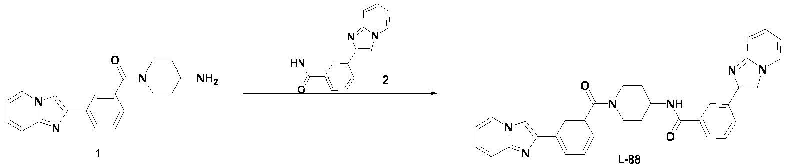

The operation was the same as the preparation of compound L-82 in Embodiment 82.

LC-MS: m/z: (M+H)⁺ = 542.2.

¹H NMR (400 MHz, Chloroform-*d*) δ 8.53 (s, 1H), 8.20 (t, *J* = 7.8 Hz, 2H), 8.03 (dd, *J* = 12.9, 7.2 Hz, 4H), 7.97 (s, 1H), 7.90 (d, *J* = 7.7 Hz, 1H), 7.74 (d, *J* = 9.0 Hz, 1H), 7.68 (d, *J* = 9.1 Hz, 1H), 7.51 (q, *J* =8.0 Hz, 2H), 7.39 (d, *J* = 7.6 Hz, 1H), 7.35 - 7.30 (m, 1H), 7.22 (dd, *J* = 9.1, 6.7 Hz, 1H), 6.99 (d, *J* = 7.8Hz, 1H), 6.92 (t, *J* = 6.8 Hz, 1H), 6.83 (t, *J* = 6.7 Hz, 1H), 4.74 (d, *J* = 28.3 Hz, 1H), 4.33 (d, *J* = 10.0 Hz, 1H), 3.89 (s, 1H), 3.23 (s, 1H), 3.05 (s, 1H), 2.66 (s, 3H), 2.23 (t, *J* = 7.7 Hz, 1H).

### Embodiment 89: 3-(imidazo[1,2-a]pyridin-2-yl)-N-((1R,SS,6s)-3-(imidazo[1,2-a]pyridin-2-yl)benzoyl)-3-azabicyclo[3.1.0]hexan-6-yl)benzamide (compound L-89)

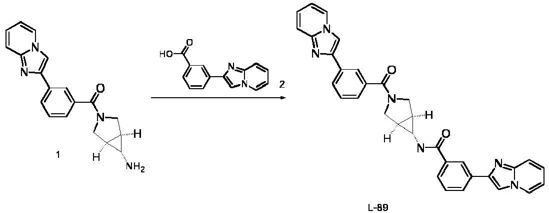

The operation was the same as the preparation of compound L-82 in Embodiment 82.

LC-MS: m/z: (M+H)⁺ = 539.2.

¹H NMR(400 MHz, Chloroform-*d*) δ 8.36 (d, *J*= 1.8 Hz, 1H), 8.14 (ddd, *J* = 7.8, 6.7, 3.3 Hz, 2H), 8.06 - 8.01 (m, 2H), 8.02 - 7.98 (m, 2H), 7.81 (dt, *J* = 7.8, 1.5 Hz, 1H), 7.63 (t, *J* = 9.2 Hz, 2H), 7.47 (t, *J* = 7.7 Hz, 2H), 7.39 (dt, *J* = 7.7, 1.5 Hz, 1H), 7.20 (tdd, J = 9.3, 7.4, 1.3 Hz, 2H), 7.06 (d, *J* = 2.7 Hz, 1H), 6.84 - 6.78 (m, 2H), 4.32 (d, *J* = 12.4 Hz, 1H), 3.83 - 3.75 (m, 2H), 3.64 (dd, *J* = 12.4, 4.3 Hz, 1H), 2.70 (q, *J* = 2.5 Hz, 1H), 1.91 (d, *J* = 29.8 Hz, 2H).

### Embodiment 90: 3-(imidazo[1,2-a]pyridin-2-yl)-N-((1R,5S,6s)-3-(6-(phenylamino)picolinoyl)-3-azabicyclo[3.1.0]hexan-6-yl)benzamide (compound L-90)

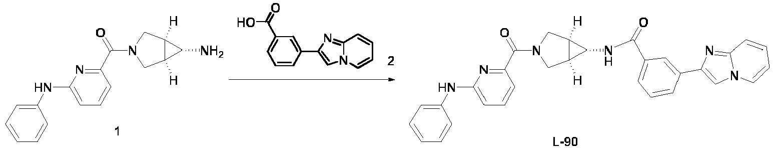

The operation was the same as the preparation of compound L-82 in Embodiment 82.

LC-MS: m/z: (M+H)⁺ = 515.2.

¹H NMR (400 MHz, Chloroform-*d*) δ 8.42 (s, 1H), 8.17 (d, *J* = 6.8 Hz, 1H), 8.01 (d, *J* = 7.7 Hz, 1H), 7.94 (s, 1H), 7.85 (d, *J* = 7.8 Hz, 1H), 7.69 (d, *J* = 9.1 Hz, 1H), 7.58 (t, *J* = 7.9 Hz, 1H), 7.51 (t, *J* = 7.8 Hz, 1H), 7.37 (s, 2H), 7.26 (s, 1H), 7.20 (d, *J* = 7.4 Hz, 1H), 7.10 (q, *J* = 5.1, 4.3 Hz, 1H), 6.90 (dt, *J* = 15.0, 7.7 Hz, 3H), 6.78 (s, 1H), 4.29 (d, *J* = 12.4 Hz, 1H), 4.20 (d, *J* = 11.8 Hz, 1H), 3.94 (dd, *J* = 11.8, 4.1 Hz, 1H), 3.77 - 3.65 (m, 2H), 2.71 (d, *J* = 2.7 Hz, 1H), 2.03 (s, 1H).

### Embodiment 91: 6-(1H-benzo[d]imidazol-2-yl)-N-(3-((1,4-dioxy-1,4-dihydronaphthalen-2-yl)amino)-4-methylbenzoyl)-3-azabicyclo[3.1.0]hexane-6-yl)picolinamide (compound L-91)

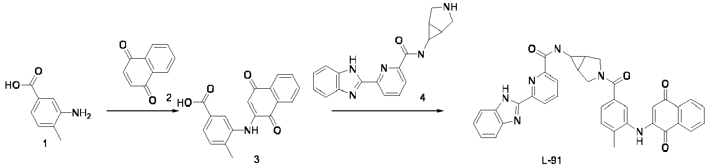

I. 3-Amino-4-methyl-benzoic acid (1.5 g, 9.9 mmol), naphthalene-1,4-dione (1.6 g, 9.9 mmol) and copper acetate (0.2 g, 0.99 mmol) were dissolved in AcOH (50 mL), and the mixture was stirred at 60°C for 16 hours. The reaction mixture was concentrated to dryness, recrystallized with EtOH (10 mL), and dried to obtain 3-[(1,4-dioxy-2-naphthyl)amino]-4-methyl-benzoic acid (2 g, 66%) as a red solid.
II. The operation was the same as the preparation of compound L-82 in Embodiment 82.

LC-MS: m/z: (M+H)⁺ = 609.3; ¹H NMR (400 MHz, Methanol-*d₄*) δ 8.45 (dd, *J* = 7.2, 1.7 Hz, 1H), 8.25 - 8.13 (m, 3H), 8.04 (dd, *J* = 7.6, 1.3 Hz, 1H), 7.79 (dtd, *J* = 25.1, 7.4, 1.4 Hz, 3H), 7.63 (d, *J* = 7.4 Hz, 1H), 7.54 - 7.44 (m, 3H), 7.36 (s, 2H), 5.60 (s, 1H), 4.31 (d, *J* = 12.3 Hz, 1H), 3.99 - 3.82 (m, 2H), 3.71 (dd, *J* = 12.3, 4.5 Hz, 1H), 2.72 (t, *J* = 2.5 Hz, 1H), 2.36 (s, 3H), 2.19 - 2.12 (m, 1H), 2.07 (d, *J* = 9.9 Hz, 1H).

### Embodiment 92: (9-(3-(imidazo[1,2-a]pyridin-2-yl)benzoyl)-3,9-diazaspiro[5.5]undecan-3-yl)(3-(4-methyl-1H-imidazol-1-yl)phenyl)methanone (compound L-92)

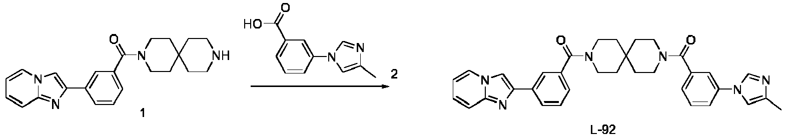

The operation was the same as the preparation of compound L-82 in Embodiment 82.

LC-MS: m/z: (M+H)⁺/2 = 280.2; ¹H NMR (400 MHz, Methanol-*d₄*) δ 8.46 (dt, *J* = 6.8, 1.2 Hz, 1H), 8.30 (d, *J* = 0.7 Hz, 1H), 8.11 (d, *J* = 1.4 Hz, 1H), 8.05 (dt, *J* = 7.9, 1.3 Hz, 1H), 8.00 (t, *J* = 1.6 Hz, 1H), 7.72 - 7.54 (m, 5H), 7.46 - 7.30 (m, 4H), 6.96 (td, *J* = 6.8, 1.2 Hz, 1H), 3.82 (s, 4H), 3.50 (d, *J* = 1.6 Hz, 4H), 2.27 (d, *J* = 1.1 Hz, 3H), 1.68 (d, *J* = 48.3 Hz, 8H).

### Embodiment 93: 6-(1H-benzo[d]imidazol-2-yl)-N-(3-(3-(4-methyl-1H-imidazol-1-yl)benzoyl)-3-azabicyclo[3.1.0]hexane-6-yl)picolinamide (compound L-93)

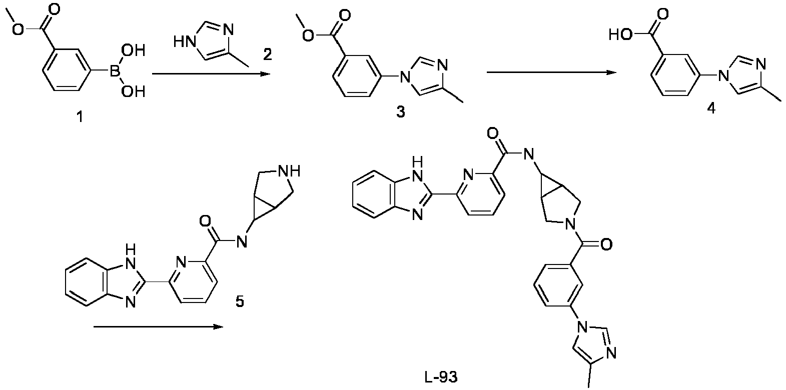

I. (3-Methoxycarbonylphenyl)boronic acid (1.8 g, 10 mmol), 4-methyl-1H-imidazole (0.82 g, 10 mmol), pyridine (0.81 mL, 10 mmol) and copper acetate (0.4 g, 2.0 mmol) were dissolved in DCM (50 mL), and the reaction mixture was stirred at room temperature for 16 hours. The reaction mixture was concentrated to dryness and purified by flash chromatography (silica) (petroleum ether:ethyl acetate = 2:1) to obtain methyl 3-(4-methylimidazol-1-yl)benzoate as a white solid (1 g, 46%).
II. Methyl 3-(4-methylimidazol-1-yl)benzoate (43 mg, 0.2 mmol) was dissolved in THF (5 mL) and water (1 mL), the reaction mixture was added with LiOH (24 mg, 1.0 mmol), and stirred at 80°C for 2 hours. The reaction mixture was concentrated to dryness and directly used in the next reaction.
III. The operation was the same as the preparation of compound L-82 in Embodiment 82.

LC-MS: m/z: (M+H)⁺ = 504.2; ¹H NMR (400 MHz, Methanol-*d₄*) δ 8.46 (dd, *J* = 7.3, 1.6 Hz, 1H), 8.26 - 8.12 (m, 3H), 7.81 - 7.62 (m, 5H), 7.53 (dt, *J* = 7.5, 1.4 Hz, 1H), 7.45 - 7.32 (m, 3H), 4.32 (d, *J* = 12.3 Hz, 1H), 3.90 (dd, *J* = 11.0, 4.5 Hz, 1H), 3.82 (d, *J* = 10.9 Hz, 1H), 3.75 (dd, *J* = 12.4, 4.7 Hz, 1H), 2.76 (t, J= 2.4 Hz, 1H), 2.29 (d, *J*= 1.0 Hz, 3H), 2.18 (d, *J* = 4.6 Hz, 1H), 2.08 (d, *J* = 12.6 Hz, 1H).

### Embodiment 94: (9-(3-(Benzo[d]thiazol-2-yl)benzoyl)-3,9-diazaspiro[5.5]undecan-3-yl)(3-(imidazo[1,2-a]pyridin-2-yl)phenyl)methanone (compound L-94)

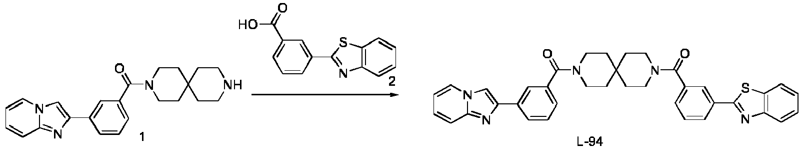

The operation was the same as the preparation of compound L-82 in Embodiment 82.

LC-MS: m/z: (M+H)⁺ = 612.3; ¹H NMR (400 MHz, Methanol-*d₄*) δ 8.46 (dt, *J* = 6.9, 1.2 Hz, 1H), 8.30 (d, *J* = 0.7 Hz, 1H), 8.21 (dt, *J* = 7.8, 1.4 Hz, 1H), 8.19 - 8.16 (m, 1H), 8.06 (ddt, *J* = 6.8, 5.2, 1.0 Hz, 3H), 8.02 - 7.99 (m, 1H), 7.73 - 7.64 (m, 1H), 7.64 - 7.54 (m, 4H), 7.48 (ddd, *J* = 8.3, 7.2, 1.2 Hz, 1H), 7.44 - 7.33 (m, 2H), 6.96 (td, *J* = 6.8, 1.2 Hz, 1H), 3.84 (s, 4H), 3.52 (s, 4H), 1.70 (d, *J* = 48.3 Hz, 8H).

### Embodiment 95: 2-((5-(9-(3-(imidazo[1,2-a]pyridin-2-yl)benzoyl)-3,9-diazaspiro[5.5]undecane-3-carbonyl)-2-methylphenyl)amino)naphthalene-1,4-dione (compound L-95)

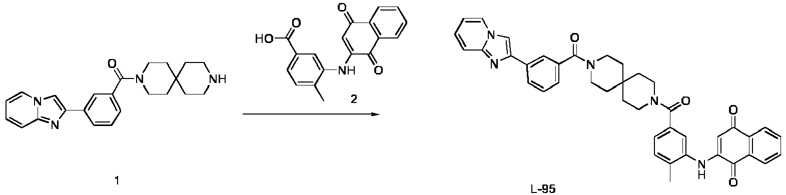

The operation was the same as the preparation of compound L-82 in Embodiment 82.

LC-MS: m/z: (M+H)⁺ = 664.3; ¹H NMR (400 MHz, Chloroform-*d*) δ 8.14 (ddd, *J* = 15.6, 7.3, 2.1 Hz, 3H), 8.04 (d, *J* = 7.9 Hz, 1H), 7.99 (d, *J* = 1.8 Hz, 1H), 7.91 (s, 1H), 7.79 (td, *J* = 7.6, 1.4 Hz, 1H), 7.74 - 7.62 (m, 2H), 7.50 (t, *J* = 7.7 Hz, 1H), 7.43 - 7.33 (m, 4H), 7.28 - 7.18 (m, 2H), 6.83 (t, *J* = 6.7 Hz, 1H), 5.98 (s, 1H), 3.79 (s, 4H), 3.50 (s, 4H), 2.34 (s, 3H), 1.61 (d, *J* = 57.2 Hz, 8H).

### Embodiment 96: 3-(4-(9-(3-(imidazo[1,2-a]pyridin-2-yl)benzoyl)-3,9-diazaspiro[5.5]undecane-3-carbonyl)benzamido)thiophene-2-carboxamide (compound L-96)

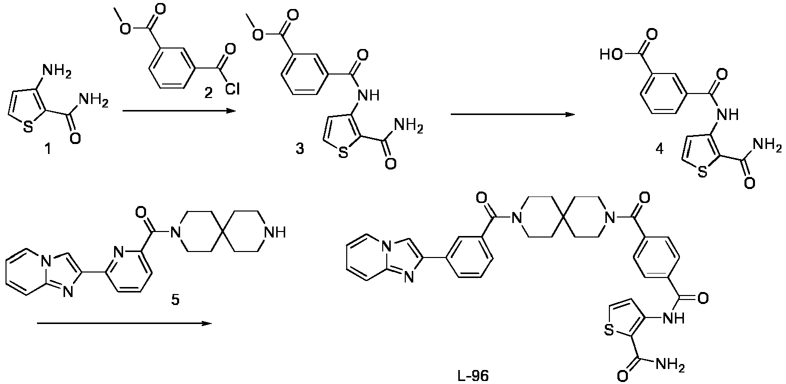

I. 3-Aminothiophene-2-carboxamide (1.4 g, 9.8 mmol) and N,N-diisopropylethylamine (2.5 g, 20 mmol) were dissolved in tetrahydrofuran (200 mL), then methyl 3-chlorocarbonylbenzoate (2.0 g, 9.8 mmol) was added, and the mixture was stirred at 25°C for 16 hours. The reaction mixture was concentrated to dryness and purified by flash chromatography (silica) (petroleum ether: ethyl acetate = 50:50) to obtain methyl 3-[(2-carbamoyl-3-thienyl)carbamoyl]benzoate as a yellow solid (1 g, 33%).
II. Methyl 3-[(2-carbamoyl-3-thienyl)carbamoyl]benzoate (180 mg, 0.6 mmol) was dissolved in MeOH (5 mL) and water (1 mL), then NaOH (71 mg, 3.0 mmol) was added, and the mixture was stirred at 100°C for 5 days. The reaction mixture was concentrated to dryness, and the crude product was added with water (5 mL). The mixture was added with 1 N dilute hydrochloric acid to adjust the pH to 5, and filtered to obtain methyl 3-[(2-carbamoyl-3-thienyl)carbamoyl]benzoate as a yellow solid (90 mg, 52%).
III. The operation was the same as the preparation of compound L-82 in Embodiment 82.

LC-MS: m/z: (M+H)⁺ = 647.3; ¹H NMR (400 MHz, Methanol-*d₄*) δ 8.47 (dd, *J* = 6.8, 1.3 Hz, 1H), 8.31 (d, *J* = 1.4 Hz, 1H), 8.12 - 8.08 (m, 2H), 8.07 - 8.03 (m, 1H), 8.00 (d, *J* = 1.7 Hz, 1H), 7.66 - 7.56 (m, 4H), 7.44 - 7.34 (m, 3H), 7.03 - 6.93 (m, 2H), 3.82 (s, 4H), 3.48 (d, *J* = 30.4 Hz, 4H), 1.68 (d, *J* = 50.0 Hz, 8H).

### Embodiment 97: ((2-(phenylamino)pyrimidin-4-yl)(9-(2-(pyridin-2-ylamino)pyrimidine-4-carbonyl)-3,9-diazaspiro[5.5]undecan-3-yl)methanone (compound L-97)

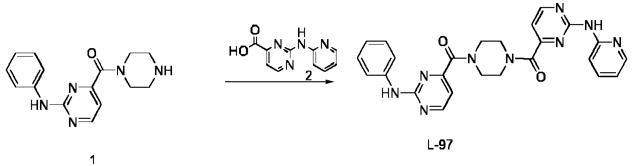

The operation was the same as the preparation of compound L-82 in Embodiment 82.

LC-MS: m/z: (M+H)⁺ = 482.0, ¹H NMR (400 MHz, DMSO-*d₆*) δ 10.08 (d, *J* = 26.4 Hz, 1H), 9.86 (d, *J* = 20.0 Hz, 1H), 8.66 (ddd, *J* = 20.0,14.7, 4.9 Hz, 2H), 8.23 (ddd, *J* = 29.4, 21.0, 6.0 Hz, 2H), 7.86 - 7.63 (m, 3H), 7.29 (dt, *J* = 21.7, 8.0 Hz,2H), 7.18 - 6.88 (m, 4H), 3.79 - 3.46 (m, 8H).

### Embodiment 98: ((2-(phenylamino)pyrimidin-4-yl)(9-(2-(pyridin-2-ylamino)pyrimidine-4-carbonyl)-3,9-diazaspiro[5.5]undecan-3-yl)methanone (compound L-98)

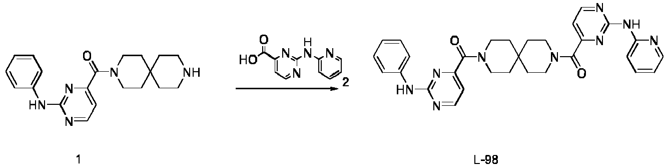

The operation was the same as the preparation of compound L-82 in Embodiment 82.

LC-MS: m/z: (M+H)⁺ = 550.0, ¹H NMR (400 MHz, CDCl₃) δ 8.66 (d, *J* = 4.9 Hz, 1H), 8.57 (d, J= 4.9 Hz, 1H), 8.39 (d, *J* =8.5 Hz, 2H), 7.72 (t, *J* = 8.5 Hz, 1H), 7.62 (d, *J* = 8.1 Hz, 2H), 7.56 (s, 1H), 7.35 (t, *J* = 7.8 Hz, 2H), 7.08(t, *J* = 7.3 Hz, 1H), 7.00 (dd, *J* = 13.2, 5.9 Hz, 2H), 6.92 (d, *J* = 4.9 Hz, 1H), 3.86 - 3.67 (m, 4H), 3.50 (d, *J* = 4.0 Hz, 4H), 1.73 - 1.52 (m, 8H).

### Embodiment 99: (N-(1-(2-(phenylamino)pyrimidine-4-carbonyl)piperidin-4-yl)-2-(pyridin-2-ylamino)pyrimidine-4-carboxamide (E100124-081) (compound L-99)

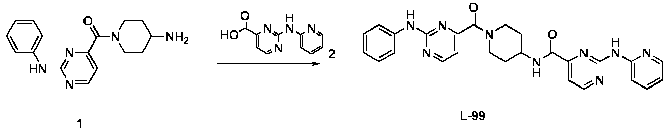

The operation was the same as the preparation of compound L-82 in Embodiment 82.

LC-MS: m/z: (M+H)⁺ = 495.2, ¹H NMR (400 MHz, CDCl₃) δ 9.31 (s, 1H), 8.76 (d, *J* = 4.9 Hz, 1H), 8.59 (d, *J* = 4.9 Hz, 1H), 8.41 (d, *J* = 4.8 Hz, 1H), 8.33 (d, *J* = 8.4 Hz, 1H), 8.09 (s, 1H), 7.86 (d, *J* = 8.2 Hz, 1H), 7.79 - 7.72 (m, 1H), 7.62 (dd, *J* = 14.3, 6.4 Hz, 3H), 7.34 (t, *J* = 7.8 Hz, 2H), 7.10 - 7.00 (m, 2H), 6.95 (d, *J* = 4.9 Hz, 1H), 4.63 (d, *J* = 13.5 Hz, 1H), 4.32 - 4.18 (m, 1H), 3.98 (d, *J* = 13.8 Hz, 1H), 3.27 (t, *J* = 11.4 Hz, 1H), 3.11 - 3.03 (m, 1H), 2.09 (dd, *J* = 38.8, 10.9 Hz, 2H), 1.69 - 1.56 (m, 2H).

### Embodiment 100: (Tetrahydropyrrolo[3,4-c]pyrrole-2,5(1H,3H)-diyl)bis((6-phenylamino)pyridin-2-yl)methanone) (compound L-100)

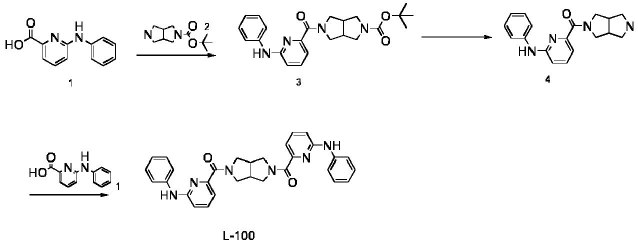

### I. tert-Butyl 5-(6-(phenylatnino)picolinoyl)hexahydropyrrolo[3,4-c]pyrrole-2(1H)-carboxylate

*tert*-Butyl hexahydropyrrolo[3,4-c]pyrrole-2(1H)-carboxylate (100 mg, 0.47 mmol) (compound represented by formula 2) and 6-(phenylamino)picolinic acid (101 mg, 0.47 mmol) (compound represented by formula 1) were suspended in 3 mL of N,N-dimethylformamide, then N,N-diisopropylethylamine (122 mg, 0.94 mmol) and 1-propylphosphonic anhydride (449 mg, 0.71 mmol) were added. The reaction mixture was stirred at 30°C for 16 hours. The reaction mixture was added with 10 mL of ice water, stirred for 5 minutes, filtered, and the solid was dried to obtain 190 mg of a crude product as a brown solid with a yield of 98.96%. LC-MS: m/z: (M+H)⁺ = 409.0.

### II. (Hexahydropyrrolo[3,4-c]pyrrol-2(1H)-yl)(6-(phenylatnino)pyridin-2-yl)methanone

*tert*-Butyl 5-(6-(phenylatnino)picolinoyl)hexahydropyrrolo[3,4-c]pyrrole-2(1H)-carboxylate (190 mg, 0.47 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated and dried to obtain 150 mg of a crude product as a brown solid with a yield of 94.38%. LC-MS: m/z: (M+H)⁺ = 309.0.

### III. (Tetrahydropyrrolo[3,4-c]pyrrole-2,5(1H,3H)-diyl)bis((6-phenylamino)pyridin-2-yl)methanone)

The operation was the same as the preparation of compound L-82 in Embodiment 82.

LC-MS: m/z: (M+H)⁺ = 505.0, ¹H NMR (400 MHz, CDCl₃) δ 7.62 (td, *J* = 8.1, 3.4 Hz, 2H), 7.41 - 7.30 (m, 8H), 7.08 (ddd, *J* = 12.8, 9.9, 5.5 Hz, 2H), 6.97 - 6.73 (m, 4H), 4.05 (ddd, *J* = 13.8, 9.7, 4.8 Hz, 3H), 3.91 - 3.65 (m, 5H), 2.98 (s,2H).

### Embodiment 101: 6-(phenylamino)-N-(((1R,SS,6s)-3-(6-(phenylamino)picolinoyl)-3-azabicyclo[3.1.0]hexan-6-yl)picolinamide (compound L-101)

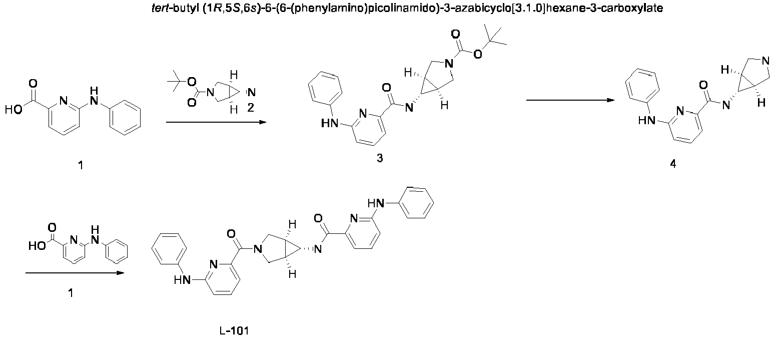

### I. tert-Butyl (1R,5S,6s)-6-(6-(phenylamino)picolinamido)-3-azabicyclo[3.1.0]hexane-3-carboxylate (E100124-067)

*tert*-Butyl (1R,5S,6s)-6-amino-3-azabicyclo[3.1.0]hexane-3-carboxylate (100 mg, 0.50 mmol) (compound represented by formula 2) and 6-(phenylamino)picolinic acid (108 mg, 0.50 mmol) (compound represented by formula 1) were suspended in 3 mL of N,N-dimethylformamide, then N,N-diisopropylethylamine (130 mg, 1.01 mmol) and 1-propylphosphonic anhydride (481 mg, 0.76 mmol) were added. The reaction mixture was stirred at 30°C for 16 hours. The reaction mixture was added with 10 mL of ice water, stirred for 5 minutes, filtered, and the solid was dried to obtain 198 mg of a crude product as a brown solid with a yield of 99.6%. LC-MS: m/z: (M+H)⁺ = 395.0.

### II. N-((1R,5S,6s)-3-azabicyclo[3.1.0]hexan-6-yl)-6-(phenylamino)picolinamide

*tert*-Butyl (1R,5S,6s)-6-(6-(phenylamino)picolinamido)-3 -azabicyclo [3.1.0]hexane-3 - carboxylate (198 mg, 0.50 mmol) (compound represented by formula 3) was dissolved in 3 mL of methanol, and a solution of hydrochloric acid in dioxane (3 mL, 4 M, 12 mmol) was added. The reaction mixture was stirred at 15°C for 2 hours. The reaction mixture was concentrated and dried to obtain 148 mg of a crude product as a brown solid with a yield of 99.1%. LC-MS: m/z: (M+H)⁺ = 295.0.

### III. 6-(phenylamino)-N-(((1R,5S,6s)-3-(6-(phenylamino)picolinoyl)-3-azabicyclo[3.1.0]hexan-6-yl)picolinamide

The operation was the same as the preparation of compound L-82 in Embodiment 82.

LC-MS: m/z: (M+H)⁺ = 491.0, ¹H NMR (400 MHz, CDCl₃) δ 7.98 (s, 1H), 7.68 - 7.57 (m, 3H), 7.45 - 7.30 (m, 8H), 7.21 - 7.08 (m, 3H), 7.03 - 6.89 (m, 3H), 6.70 (s, 1H), 4.30 (d, *J* = 12.5 Hz, 1H), 4.18 (d, *J* = 11.6 Hz, 1H), 3.98 (d, *J* = 11.6 Hz, 1H), 3.76 - 3.68 (m, 1H), 2.69 (d, *J* = 2.0 Hz, 1H), 1.91 (s, 2H).

### Embodiment 102: (5-(6-(phenylamino)pyridyl)hexahydropyrrolo[3,4-c]pyrrol-2(1H)-yl)(2-(phenylamino)pyrimidin-4-yl)methanone (compound L-102)

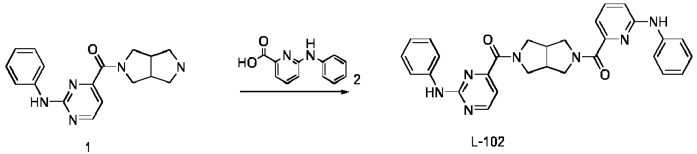

The operation was the same as the preparation of compound L-82 in Embodiment 82.

LC-MS: m/z: (M+H)⁺ = 506.0, ¹H NMR (400 MHz, CD₃OD) δ 8.57 (dd, J= 12.9, 4.9 Hz, 1H), 7.70 - 7.61 (m, 2H), 7.59 - 7.42 (m, 3H), 7.36 - 6.71 (m, 9H), 4.01 - 3.46 (m, 8H), 3.11 - 2.92 (m, 2H).

### Embodiment 103: (2-(phenylamino)-N-(((1R,5S,6s)-3-(6-(phenylamino)pyridyl)-3-azabicyclo[3.1.0]hexan-6-yl)pyrimidine-4-carboxamide (compound L-103)

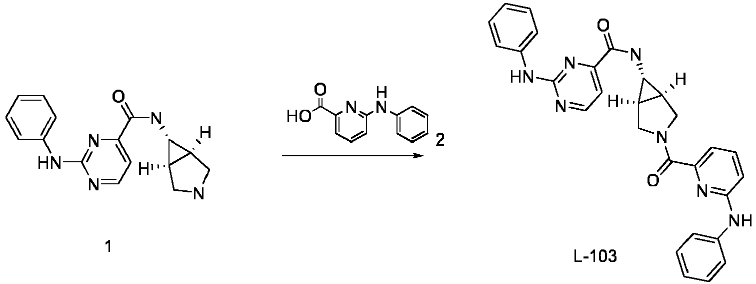

The operation was the same as the preparation of compound L-82 in Embodiment 82.

LC-MS: m/z: (M+H)⁺ = 492.0, ¹H NMR (400 MHz, CD₃OD) δ 8.61 (d, *J* = 4.9 Hz, 1H), 7.69 - 7.54 (m, 5H), 7.32 (dt, *J* = 16.2, 4.8 Hz, 5H), 7.08 - 6.86 (m, 4H), 420 (d, *J* = 12.5 Hz, 1H), 4.12 (d, *J* = 11.7 Hz, 1H), 3.88 (dd, *J* = 11.8, 4.3 Hz, 1H), 3.72 - 3.63 (m, 1H), 2.60 (t, *J* = 2.3 Hz, 1H), 2.01-1.89 (m, 2H).

### Embodiment 104: N-((1R,SS,6s)-3-(3-(1H-pyrrol-1-yl)thiophene-2-carbonyl)-3-azabicyclo[3.1.0]hexan-6-yl)-3-(imidazo[1,2-a]pyridin-2-yl)benzamide (compound L-104)

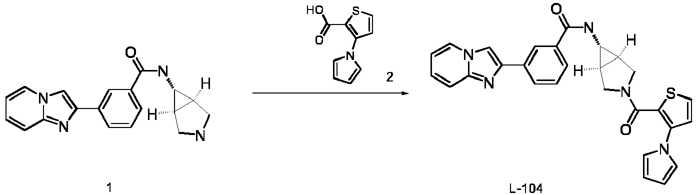

The operation was the same as the preparation of compound L-82 in Embodiment 82.

LC-MS: m/z: (M+H)⁺ = 494.2.

¹H NMR (400 MHz, Chloroform-*d*) δ 8.38 (s, 1H), 8.18 (d, *J* = 6.7 Hz, 1H), 7.99 (d, *J* = 7.8 Hz, 1H), 7.94 (s, 1H), 7.82 (d, *J* = 7.8 Hz, 1H), 7.68 (d, *J* = 9.1 Hz, 1H), 7.50 (t, *J* = 7.7 Hz, 1H), 7.44 (d, *J* = 5.3 Hz, 1H), 7.27 (s, 1H), 7.06 (d, *J* = 5.3 Hz, 1H), 6.90 - 6.85 (m, 1H), 6.74 (s, 1H), 6.33 (t, *J* = 2.2 Hz, 2H), 4.25 (d, *J* = 12.5 Hz, 1H), 3.53 (d, *J* = 13.3 Hz, 1H), 3.32 (d, *J* = 11.0 Hz, 1H), 2.70 - 2.61 (m, 1H), 2.45 (t, *J* = 2.5 Hz, 1H), 1.86 (s, 1H).

### Embodiment 105: 3-(imidazo[1,2-a]pyridin-2-yl)-N-((1R,5S,6s)-3-(2-phenoxynicotinamido)-3-azabicyclo[3.1.0]hexan-6-yl)benzamide (compound L-105)

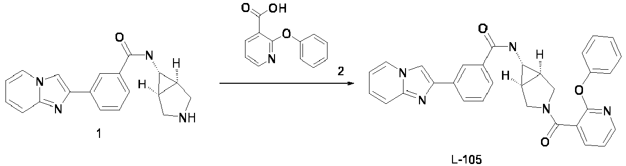

The operation was the same as the preparation of compound L-82 in Embodiment 82.

LC-MS: m/z: (M+H)⁺ = 516.2.

¹H NMR (400 MHz, Chloroform-*d*) δ 8.47 (s, 1H), 8.21 (dt, *J* = 5.1, 2.4 Hz, 2H), 8.00 (d, *J* = 7.7 Hz, 1H), 7.87 (d, *J* = 7.7 Hz, 1H), 7.77 - 7.68 (m, 2H), 7.53 (t, *J* = 7.7 Hz, 1H), 7.44 (t, *J* = 7.8 Hz, 2H), 7.37 - 7.30 (m, 1H), 7.26 - 7.18 (m, 3H), 7.08 (dd, *J* = 7.4, 4.9 Hz, 1H), 6.93 (t, *J* = 7.2 Hz, 2H), 4.31 (d, *J* = 12.4Hz, 1H), 3.88 - 3.80 (m, 1H), 3.76 (d, *J* = 10.8 Hz, 1H), 3.69 (dd, *J* = 12.4, 4.5 Hz, 1H), 2.76 (d, *J* = 2.6Hz, 1H), 2.03 (d, *J* = 7.6 Hz, 1H), 1.97 (d, *J* = 4.1 Hz, 1H).

### Embodiment 106: N-((1R,SS,6s)-3-(1H-indazole-3-carbonyl)-3-azabicyclo[3.1.0]hexan-6-yl)-3-(imidazo[1,2-a]pyridin-2-yl)benzamide (compound L-106)

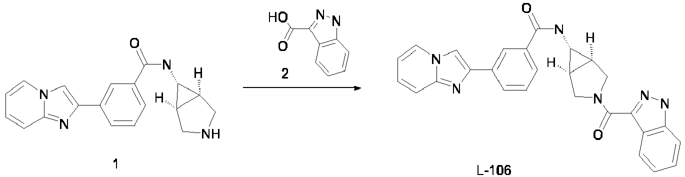

The operation was the same as the preparation of compound L-82 in Embodiment 82.

LC-MS: m/z: (M+H)⁺ = 463.1.

¹H NMR (400 MHz, DMSO-*d₆*) δ 13.60 (s, 1H), 8.73 (d, *J* = 3.9 Hz, 1H), 8.57 (d, *J* = 6.7 Hz, 1H), 8.47(s, 1H), 8.42 (d, *J* = 1.9 Hz, 1H), 8.16 (d, *J* = 8.2 Hz, 1H), 8.11 (dd, *J* = 7.7, 1.7 Hz, 1H), 7.80 - 7.75 (m, 1H), 7.65 - 7.58 (m, 2H), 7.54 (t, *J* = 7.8 Hz, 1H), 7.45 - 7.41 (m, 1H), 7.29 (s, 1H), 7.24 (t, *J* = 7.5 Hz, 1H), 6.94 (s, 1H), 4.49 (d, *J* = 11.7 Hz, 1H), 4.10 (d, *J* = 12.3 Hz, 1H), 4.04 - 4.00 (m, 1H), 3.70 - 3.62 (m, 2H), 2.04 (s, 1H), 1.97 (s, 1H).

### Embodiment 107: 4-Butoxy-N-(1-(3-(imidazo[1,2-a]pyridin-2-yl)benzoyl)piperidin-4-yl)benzamide (compound L-107)

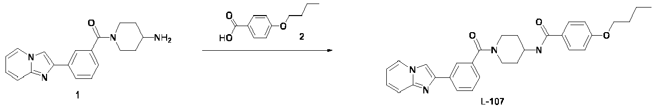

The operation was the same as the preparation of compound L-82 in Embodiment 82.

LC-MS: m/z: (M+H)⁺ = 497.3.

¹H NMR (400 MHz, Chloroform-*d*) δ 8.17 (d, *J* = 6.8 Hz, 1H), 8.04 - 7.98 (m, 2H), 7.91 (s, 1H), 7.79 -7.73 (m, 2H), 7.70 (d, *J* = 9.1 Hz, 1H), 7.49 (t, *J* = 7.6 Hz, 1H), 7.37 (d, *J* = 7.6 Hz, 1H), 7.25 (t, *J* = 8.0Hz, 1H), 6.94 - 6.89 (m, 2H), 6.85 (t, *J* = 6.7 Hz, 1H), 6.30 (d, *J* = 7.9 Hz, 1H), 4.75 (s, 1H), 4.28 (d, *J* = 6.9 Hz, 1H), 3.86 (s, 1H), 3.22 (s, 1H), 3.02 (s, 1H), 2.29 (s, 2H), 2.03 (s, 1H), 1.79 (p, *J* = 6.8 Hz, 2H), 1.50 (hept, *J* = 6.8, 6.2 Hz, 4H), 1.02 - 0.95 (m, 3H).

### Embodiment 108: (4-(6-(1H-benzo[d]imidazol-2-yl)picolinoyl)piperazin-1-yl)(quinolin-3-yl)methanone (compound L-108)

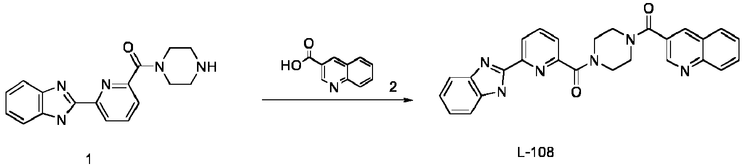

The operation was the same as the preparation of compound L-82 in Embodiment 82.

LC-MS: m/z: (M+H)⁺ = 463.0, ¹H NMR (400 MHz, CDCl₃) δ 9.01 (s, 1H), 8.63 (s, 1H), 8.32 (s, 1H), 8.17 (d, *J* = 8.1 Hz, 1H), 8.02 (t, *J* = 7.6 Hz, 1H), 7.95 - 7.80 (m, 2H), 7.79 - 7.63 (m, 3H), 7.38 (dd, *J* = 6.0, 3.0 Hz, 2H), 3.85 (d, *J* = 72.4Hz, 8H).

### Embodiment 109: (4-(6-(1H-benzo[d]imidazol-2-yl)picolinoyl)piperazin-1-yl)(4-methylpyridin-3-yl)methanone (compound L-109)

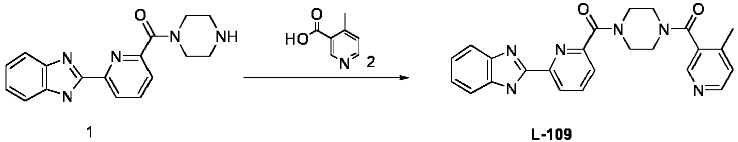

The operation was the same as the preparation of compound L-82 in Embodiment 82.

LC-MS: m/z: (M+H)⁺ = 427.0, ¹H NMR (400 MHz, CDCl₃) δ 8.78 - 8.43 (m, 3H), 8.04 (d, *J* = 7.4 Hz, 1H), 7.74 (d, *J* = 22.5 Hz, 3H), 7.39 (s, 2H), 7.21 (s, 1H), 4.01 - 3.29 (m, 8H), 2.40 (s, 3H).

### Embodiment 110: (4-(6-(1H-benzo[d]imidazol-2-yl)picolinoyl)piperazin-1-yl)(1H-indazol-3-yl)methanone (compound L-110)

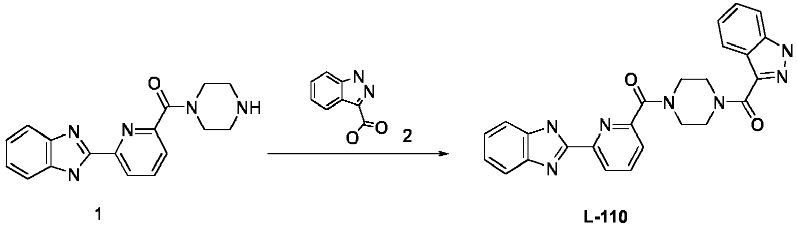

The operation was the same as the preparation of compound L-82 in Embodiment 82.

LC-MS: m/z: (M+H)⁺ = 452.0, ¹H NMR (400 MHz, CDCl₃) δ 11.00 (d, *J* = 117.6 Hz, 2H), 8.52 (s, 1H), 8.11 (s, 1H), 7.97 (t, *J* = 7.8 Hz, 1H), 7.69 (d, *J* = 6.8 Hz, 3H), 7.49 (d, *J* = 8.4 Hz, 1H), 7.45 - 7.30 (m, 3H), 7.22 (s, 1H), 4.44 - 3.58 (m, 8H).

### Embodiment 111: 6-(4-(6-(1H-benzo[d]imidazol-2-yl)picolinoyl)piperazine-1-carbonyl)-4,5-dihydropyridazin-3(2H)-one (compound L-111)

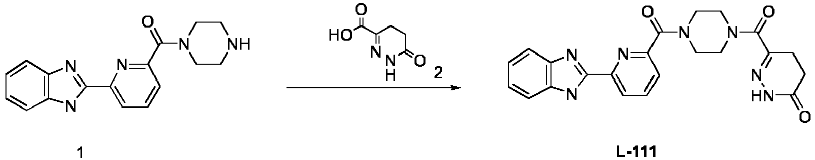

The operation was the same as the preparation of compound L-82 in Embodiment 82.

LC-MS: m/z: (M+H)⁺ = 432.0, ¹H NMR (400 MHz, CDCl₃) δ 8.91 (s, 1H), 8.57 (s, 1H), 7.99 (t, *J* = 7.8 Hz, 1H), 7.86 - 7.60 (m, 3H), 7.41 - 7.32 (m, 2H), 4.00 - 3.61 (m, 8H), 2.91 (s, 2H), 2.62 (s, 2H).

### Embodiment 112: (4-(6-(1H-benzo[d]imidazol-2-yl)picolinoyl)piperazin-1-yl)(thiophen-2-yl)methanone (compound L-112)

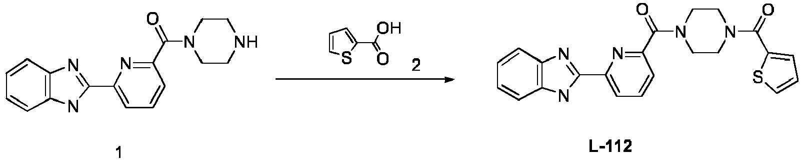

The operation was the same as the preparation of compound L-82 in Embodiment 82.

LC-MS: m/z: (M+H)⁺ = 418.0, ¹H NMR (400 MHz, CDCl₃) δ 8.57 (d, *J* = 7.8 Hz, 1H), 8.01 (dd, *J* = 15.8, 8.0 Hz, 1H), 7.71 (t, *J* = 8.5Hz, 3H), 7.50 (d, *J* = 5.0 Hz, 1H), 7.41 - 7.32 (m, 3H), 7.11 - 7.05 (m, 1H), 4.09 - 3.67 (m, 8H).

### Embodiment 113: (3,9-diazaspiro[5.5]undecane-3,9-diyl)bis((6-(phenylamino)pyridin-2-yl)methanone) (compound L-113)

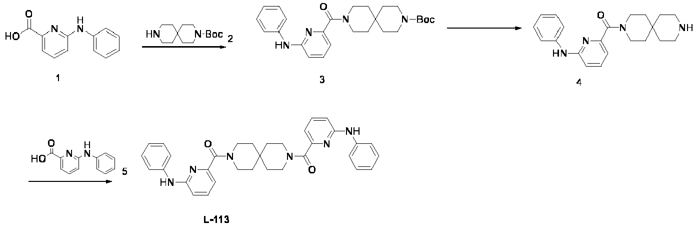

### I. tert-Butyl 9-(6-(6-(phenylamino)picolinoyl)-3,9-diazaspiro[5.5]undecane-3-carboxylate

*tert*-Butyl 3.9-diazaspiro[5.5]undecane-3-carboxylate (187 mg, 0.74 mmol) (compound represented by formula 2) and 6-(phenylamino)picolinic acid (150 mg, 0.70 mmol) (compound represented by formula 1) were suspended in 3 mL of N,N-dimethylformamide, then N,N-diisopropylethylamine (181 mg, 1.40 mmol) and 1-propylphosphonic anhydride (668 mg, 1.05 mmol) were added. The reaction mixture was stirred at 15°C for 16 hours. The reaction mixture was added with 10 mL of ice water, stirred for 5 minutes, filtered, and the solid was dried to obtain 220 mg of a crude product as a brown solid with a yield of 69.73%. LC-MS: m/z: (M+H)⁺ = 451.0.

### II. (6-(phenylamino)pyridin-2-yl)(3,9-diazaspiro[5.5]undecan-3-yl)methanone

*tert*-Butyl 9-(6-(6-(phenylamino)picolinoyl)-3,9-diazaspiro[5.5]undecane-3-carboxylate (175 mg, 0.33 mmol) (compound represented by formula 3) was dissolved in 5 mL of methanol, and a solution of hydrochloric acid in dioxane (5 mL, 4 M, 20 mmol) was added. The reaction mixture was stirred at 15°C for 2 hours. The reaction mixture was concentrated and dried to obtain 115 mg of a crude product as a brown solid with a yield of 98.56%. LC-MS: m/z: (M+H)⁺ = 351.0.

### III. (3,9-diazaspiro[5.5]undecane-3,9-diyl)bis((6-(phenylamino)pyridin-2-yl)methanone)

The operation was the same as the preparation of compound L-82 in Embodiment 82.

LC-MS: m/z: (M+H)⁺ = 547.0, ¹H NMR (400 MHz, CDCl₃) δ 8.04 (s, 1H), 7.64 - 7.56 (m, 2H), 7.39 - 7.33 (m, 7H), 7.11 (dt, *J* = 8.5, 3.5Hz, 2H), 6.99 (d, *J* = 7.2 Hz, 2H), 6.91 (d, *J* = 8.4 Hz, 3H), 3.67 (d*, J* = 96.1 Hz, 8H), 1.75 - 1.51 (m, 8H).

### Embodiment 114: 6-(phenylamino)-N-(1-(6-(6-(phenylamino)picolinoyl)piperidin-4-yl)picolinamide (compound L-114)

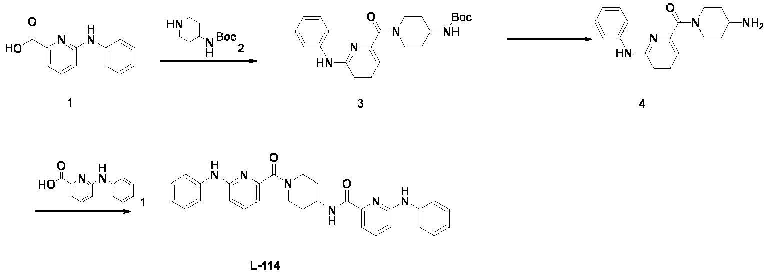

### I. tert-Butyl (1-(6-(phenylamino)picolinoyl)piperidin-4-yl)carbamate

*tert*-Butylpiperidin-4-ylcarbamate (147 mg, 0.74 mmol) (compound represented by formula 2) and 6-(phenylamino)picolinic acid (150 mg, 0.70 mmol) (compound represented by formula 1) were suspended in 3 mL of N,N-dimethylformamide, then N,N-diisopropylethylamine (181 mg, 1.4 mmol) and 1-propylphosphonic anhydride (668 mg, 1.05 mmol) were added. The reaction mixture was stirred at 30°C for 16 hours. The reaction mixture was added with 10 mL of ice water, stirred for 5 minutes, filtered, and the solid was dried to obtain 230 mg of a crude product as a brown solid with a yield of 82.34%. LC-MS: m/z: (M+H)⁺ = 397.0.

### II. (4-aminopiperidin-1-yl)(6-(phenylamino)pyridin-2-yl)methanone

*tert*-Butyl (1-(2-(phenylamino)pyrimidine-4-carbonyl)piperidin-4-yl)carbamate (150 mg, 0.38 mmol) (compound represented by formula 3) was dissolved in 5 mL of methanol, and a solution of hydrochloric acid in dioxane (5 mL, 4 M, 20 mmol) was added. The reaction mixture was stirred at 15°C for 2 hours. The reaction mixture was concentrated and dried to obtain 110 mg of a crude product as a brown solid with a yield of 98.10%. LC-MS: m/z: (M+H)⁺ = 297.0.

### III. 6-(phenylamino)-N-(1-(6-(6-(phenylamino)picolinoyl)piperidin-4-yl)picolinamide

The operation was the same as the preparation of compound L-82 in Embodiment 82.

LC-MS: m/z: (M+H)⁺ = 493.0, ¹H NMR (400 MHz, CDCl₃) δ 8.06 - 7.99 (m, 1H), 7.72 - 7.61 (m, 3H), 7.38 (dt, *J* = 13.4, 8.0 Hz, 8H),7.15 (t, *J* = 7.1 Hz, 2H), 6.98 (dd, *J* = 21.4, 7.9 Hz, 3H), 4.61 (s, 1H), 4.28 (d, *J* = 8.2 Hz, 1H), 3.96 (s,1H), 3.37 - 3.14 (m, 2H), 2.12 (d, *J* = 28.1 Hz, 2H), 1.73 *(d, J=* 10.2 Hz, 2H).

### Embodiment 115: 3-(4-(4-(6-(1H-benzo[d]imidazol-2-yl)picolinoyl)piperazine-1-carbonyl)benzamido)thiophene-2-carboxamide (compound L-115)

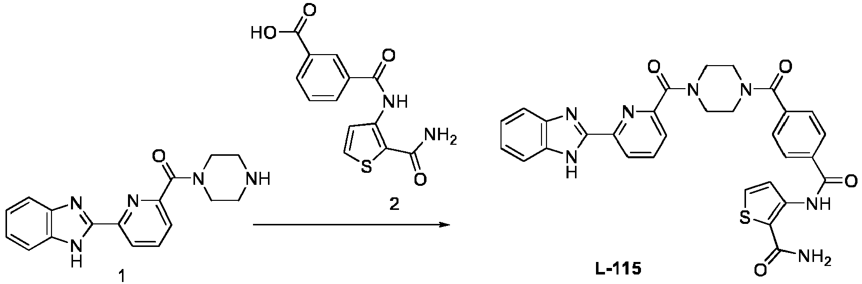

The operation was the same as the preparation of compound L-82 in Embodiment 82.

LC-MS: m/z: (M+H)⁺ = 579.8; ¹H NMR (400 MHz, Methanol-*d₄*) δ 8.41 (s, 1H), 8.14 (s, 3H), 7.81 - 7.61 (m, 5H), 7.36 (d, *J* = 16.9 Hz, 3H), 6.98 (s, 1H), 3.95 (d, *J* = 36.7 Hz, 4H), 3.67 (t, *J* = 29.0 Hz, 4H).

### Embodiment 116: 2-((5-(4-(6-(1H-benzo[d]imidazol-2-yl)picolinoyl)piperazine-1-carbonyl)-2-methylphenyl)amino)naphthalene-1,4-dione (compound L-116)

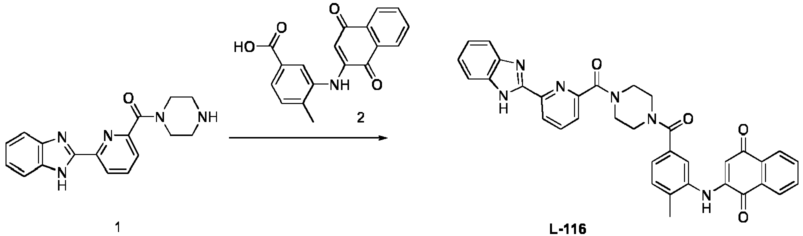

The operation was the same as the preparation of compound L-82 in Embodiment 82.

LC-MS: m/z: (M+H)⁺ = 596.8; ¹H NMR (400 MHz, Chloroform-*d*) δ 8.58 (s, 1H), 8.40 - 7.92 (m, 3H), 7.72 (s, 5H), 7.55 - 7.19 (m, 7H), 6.03 (s, 1H), 3.85 (d, *J* = 36.6 Hz, 8H), 2.34 (s, 3H).

### Embodiment 117: (4-(6-(1H-benzo[d]imidazol-2-yl)picolinoyl)piperazin-1-yl)(3-(4-methyl-1H-imidazol-1-yl)phenyl)methanone (compound L-117)

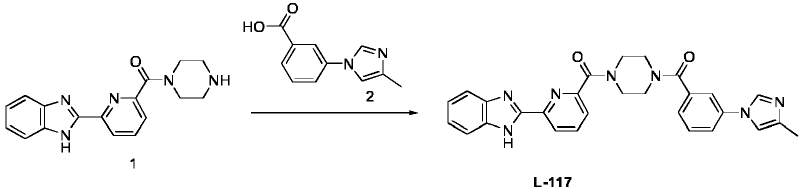

The operation was the same as the preparation of compound L-82 in Embodiment 82.

LC-MS: m/z: (M+H)⁺ = 419.9; ¹H NMR (400 MHz, Methanol-*d₄*) δ 8.41 (s, 1H), 8.14 (d, *J* = 8.1 Hz, 2H), 7.89 - 7.54 (m, 6H), 7.48 (s, 1H), 7.34 (s, 3H), 4.19 - 3.48 (m, 8H), 2.27 (s, 3H).

### Embodiment 118: (4-(6-(1H-benzo[d]imidazol-2-yl)picolinoyl)-N-(3-(imidazol[1,2-a]pyridin-2-yl)phenyl)piperazine-1-carboxamide (L-118)

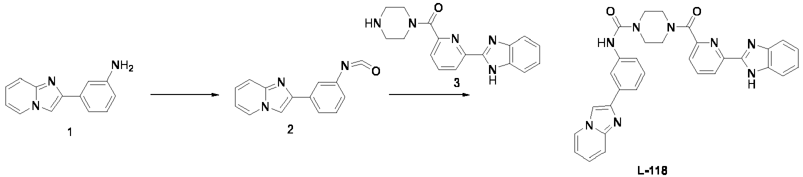

3-Imidazo[1,2-a]pyridin-2-ylaniline (42 mg, 0.2 mmol) was dissolved in anhydrous dichloromethane (5 mL), then triphosgene (20 mg, 0.067 mmol) was added, and the mixture was stirred at 25°C for 16 hours. The reaction mixture was concentrated to dryness and directly used in the next reaction.

[6-(1H-benzimidazol-2-yl)-2-pyridyl]-piperazin-1-yl-methanone (63 mg, 0.2 mmol) and N,N-diisopropylethylamine (79 mg, 0.6 mmol) were dissolved in N,N-dimethylformamide (1 mL), then 2-(3-isocyanatophenyl)imidazo[1,2-a]pyridine (48 mg, 0.2 mmol) was added, and the reaction mixture was stirred at 25°C for 2 hours. The reaction mixture was concentrated to dryness and purified by flash chromatography (silica) (dichloromethane:methanol = 20:1) to obtain 4-[6-(1H-benzimidazol-2-yl)pyridine-2-carbonyl]-N-(3-imidazo[1,2-a]pyridin-2-ylphenyl)piperazine-1-carboxamide as a yellow solid (35 mg, 32%).

LC-MS: m/z: (M+H)⁺/2 = 272.0; ¹H NMR (400 MHz, Methanol-*d₄*) δ 8.47 - 8.40 (m, 2H), 8.20 - 8.12 (m, 2H), 7.91 (t, *J* = 1.8 Hz, 1H), 7.75 (dd, *J* = 7.8, 1.0 Hz, 2H), 7.62 (dt, *J* = 7.4, 1.5 Hz, 2H), 7.56 (d, *J* = 9.1 Hz, 1H), 7.46 7.29 (m, 5H), 6.93 (td, *J* = 6.8, 1.2 Hz, 1H), 3.95 (t, *J* = 5.2 Hz, 2H), 3.81 (d, *J* = 5.5 Hz, 2H), 3.71 (s, 4H).

### Embodiment 119: 1,3-Bis(3-(imidazo[1,2-a]pyridin-2-yl)phenyl)urea (compound L-119)

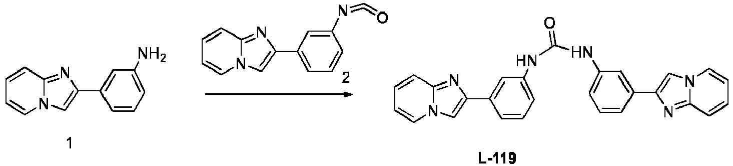

The operation was the same as the preparation of compound L-118 in Embodiment 118.

LC-MS: m/z: (M+H)⁺/2 = 223.0; ¹H NMR (400 MHz, Methanol-*d₄*) δ 8.27 (dt, *J* = 6.8, 1.2 Hz, 1H), 8.04 (s, 1H), 7.92 (t, J= 1.9 Hz, 1H), 7.64 - 7.51 (m, 3H), 7.38 (t, *J* = 7.9 Hz, 1H), 7.30 (ddd,J= 9.1, 6.8, 1.2 Hz, 1H), 6.90 (td, *J* = 6.8, 1.1 Hz, 1H).

### Embodiment 120: Synthesis of (5-(6-(1Hbenzo[d]imidazol-2-yl)picolinoyl)hexahydropyrrolo[3,4-c]pyrrol-2(1H)-yl)(1H-indazol-3-yl)methanone (compound L-120)

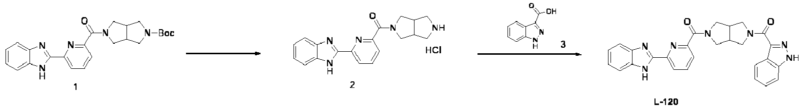

### I. Synthesis of (6-(1Hbenzo[d]imidazol-2-yl)pyridin-2-yl)(hexahydropyrrolo[3,4-c]pyrrol-2(1H)-yl)methanone hydrochloride

Operation: 540 mg of raw material tert-butyl 5-(6-(1Hbenzo[d]imidazol-2-yl)picolinoyl)hexahydropyrrolo[3,4-c]pyrrole-2(1H)-carboxylate was put into a reaction flask, then 40 mL of methanol was added, the mixture was stirred, added with 20 mL of 4 M/L hydrochloric acid/1,4-dioxane solution, and stirred at room temperature overnight. The next day, the reaction was completed as shown by LC-MS. The mixture was directly concentrated under reduced pressure to remove the solvent. 500 mg of a product was obtained with a yield of 100%.

LC-MS: m/z: (M+H)⁺ = 369.9.

### II. Synthesis of (5-(6-(1Hbenzo[d]imidazol-2-yl)picolinoyl)hexahydropyrrolo[3,4-c]pyrrol-2(1H)-yl)(1H-indazol-3-yl)methanone

Operation: 60 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 26.25 mg (1 eq) of raw material 1H-indazole-3-carboxyfic acid was added. The mixture was stirred, added with 103.2 mg (50% EA solution, 1.5 eq) of T₃P and 83.7 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 32.5 mg of a pure product with a yield of 41.9%.

LC-MS: m/z: (M+H)⁺ = 478.2.

¹H NMR (400 MHz, Methanol-*d₄*) δ 8.46 - 8.37 (m, 1H), 8.20 (d, *J* = 8.2 Hz, 1H), 8.12 (dt, *J* = 10.1, 7.9 Hz, 1H), 7.88 - 7.81 (m, 1H), 7.77 - 7.52 (m, 3H), 7.48 - 7.39 (m, 1H), 7.37 - 7.19 (m, 3H), 4.49 - 3.67 (m, 8H), 3.17 (ttd, *J* = 15.6, 7.3, 3.4 Hz, 2H).

### Embodiment 121: Synthesis of 6-(5-(6-(1Hbenzo[d]imidazol-2-yl)picolinoyl)octahydropyrrolo[3,4-c]pyrrole-2-carbonyl)-4,5-dihydropyridazin-3(2H)-one (compound L-121)

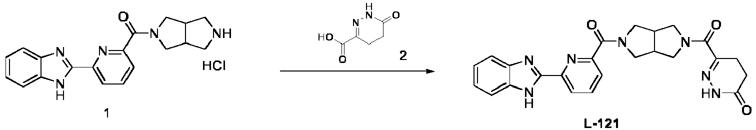

Operation: 60 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 23.04 mg (1 eq) of raw material 6-oxo-1,4,5,6-tetrahydropyridazine-3-carboxylic acid was added. The mixture was stirred, added with 103.2 mg (50% EA solution, 1.5 eq) of T₃P and 83.7 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 19.6 mg of a pure product with a yield of 26.4%.

LC-MS: m/z: (M+H)⁺ = 458.2.

¹H NMR (400 MHz, Methanol-*d₄*) δ 8.42 (ddd, *J* = 7.9, 4.6, 0.9 Hz, 1H), 8.14 (td, *J* = 7.9, 1.0 Hz, 1H), 7.88 - 7.82 (m, 1H), 7.69 (d, *J* = 40.9 Hz, 2H), 7.34 (d, *J* = 4.9 Hz, 2H), 4.24 - 3.52 (m, 8H), 3.12 (dddd, *J* = 14.9, 12.4, 4.0, 2.1 Hz, 2H), 2.91 - 2.79 (m, 2H), 2.61 - 2.45 (m, 2H).

### Embodiment 122: Synthesis of (5-(6-(1Hbenzo[d]imidazol-2-yl)picolinoyl)hexahydropyrrolo[3,4-c]pyrrol-2(1H)-yl)(quinolin-3-yl)methanone (compound L-122)

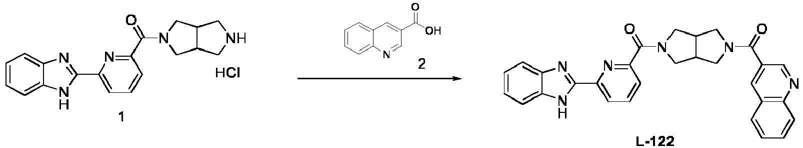

Operation: 60 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 28.08 mg (1 eq) of raw material quinoline-3-carboxylic acid was added. The mixture was stirred, added with 103.2 mg (50% EA solution, 1.5 eq) of T₃P and 83.7 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 41.8 mg of a pure product with a yield of 52.8%.

LC-MS: m/z: (M+H)⁺ = 489.2.

¹H NMR (400 MHz, Methanol-*d₄*) δ 9.04 (d, *J* = 17.9 Hz, 1H), 8.59 (d, *J* = 27.0 Hz, 1H), 8.41 (dd, *J* = 22.3, 7.9 Hz, 1H), 8.18 - 7.82 (m, 5H), 7.69 (dt, *J* = 32.4, 7.4 Hz, 3H), 7.33 (s, 2H), 4.28 - 4.04 (m, 2H), 3.96 (tt, *J* = 13.6, 8.3 Hz, 2H), 3.88 - 3.56 (m, 4H), 3.29 - 3.05 (m, 2H).

### Embodiment 123: Synthesis of (5-(6-(1Hbenzo[d]imidazol-2-yl)picolinoyl)hexahydropyrrolo[3,4-c]pyrrol-2(1H)-yl)(4-methylpyridin-3-yl)methanone (compound L-123)

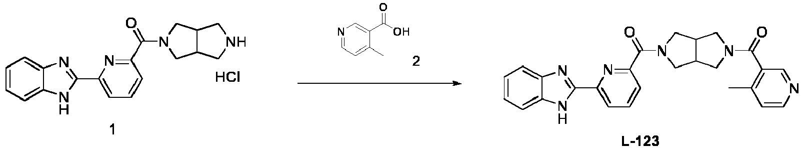

Operation: 60 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 22.2 mg (1 eq) of raw material 4-methylnicotinic acid was added. The mixture was stirred, added with 103.2 mg (50% EA solution, 1.5 eq) of T₃P and 83.7 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 49.1 mg of a pure product with a yield of 67%.

LC-MS: m/z: (M+H)⁺ = 453.2.

¹H NMR (400 MHz, Methanol-*d₄*) δ 8.50 (d, *J* = 5.5 Hz, 1H), 8.47 - 8.37 (m, 2H), 8.14 (dt, *J* = 10.7, 7.8 Hz, 1H), 7.90 - 7.81 (m, 1H), 7.72 (s, 2H), 7.46 - 7.29 (m, 3H), 4.25 - 3.85 (m, 4H), 3.81 - 3.49 (m, 4H), 3.20 - 3.06 (m, 2H), 2.38 (d, *J* = 29.1 Hz, 3H).

### Embodiment 124: Synthesis of (5-(6-(1Hbenzo[d]imidazol-2-yl)picolinoyl)hexahydropyrrolo[3,4-c]pyrrol-2(1H)-yl)(3-chloro-4-fluorophenyl)methanone (compound L-124)

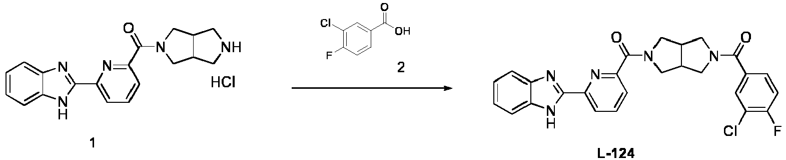

Operation: 80 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 37.76 mg (1 eq) of raw material 3-chloro-4-fluorobenzoic acid was added. The mixture was stirred, added with 137.6 mg (50% EA solution, 1.5 eq) of T₃P and 111.6 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 55 mg of a pure product with a yield of 51.88%.

LC-MS: m/z: (M+H)⁺ = 489.8.

¹H NMR (400 MHz, DMSO-*d₆*) δ 12.92 (s, 1H), 8.41 (t, *J* = 7.6 Hz, 1H), 8.13 (q, *J* = 7.5 Hz, 1H), 7.83 - 7.77 (m, 2H), 7.73 (t, *J* = 6.9 Hz, 1H), 7.59 (q, *J* = 7.5, 6.9 Hz, 2H), 7.48 (dt, *J* = 32.4, 8.9 Hz, 1H), 7.26 (dq, *J* = 14.6, 7.7, 7.2 Hz, 2H), 4.18 - 3.35 (m, 8H), 3.11 - 2.90 (m, 2H).

### Embodiment 125: Synthesis of (5-(6-(1Hbenzo[d]imidazol-2-yl)picolinoyl)hexahydropyrrolo[3,4-c]pyrrol-2(1H)-yl)(2-methylquinolin-6-yl)methanone (compound L-125)

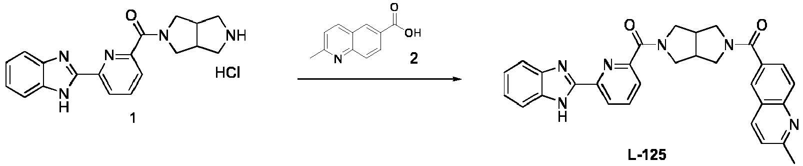

Operation: 80 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 40.48 mg (1 eq) of raw material 2-methylquinoline-6-carboxylic acid was added. The mixture was stirred, added with 137.6 mg (50% EA solution, 1.5 eq) of T₃P and 111.6 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 70 mg of a pure product with a yield of 64.5%.

LC-MS: m/z: (M+H)⁺ = 502.9.

¹H NMR (400 MHz, DMSO-*d₆*) δ 12.93 (s, 1H), 8.46 - 8.24 (m, 2H), 8.20 - 8.07 (m, 2H), 7.94 (dd, *J* = 29.9, 8.9 Hz, 1H), 7.86 (d, *J* = 8.6 Hz, 1H), 7.80 (d, *J* = 7.6 Hz, 1H), 7.73 (dd, *J* = 11.9, 7.9 Hz, 1H), 7.61 (dd, *J* = 16.2, 7.9 Hz, 1H), 7.48 (dd, *J* = 26.5, 8.6 Hz, 1H), 7.26 (dq, *J* = 14.1, 7.1 Hz, 2H), 4.19 - 3.47 (m, 8H), 3.12 - 2.91 (m, 2H), 2.72 - 2.62 (m, 3H).

### Embodiment 126: Synthesis of (5-(6-(1Hbenzo[d]imidazol-2-yl)picolinoyl)hexahydropyrrolo[3,4-c]pyrrol-2(1H)-yl)(4-(4-chlorophenyl)cyclohexyl)methanone (compound L-126)

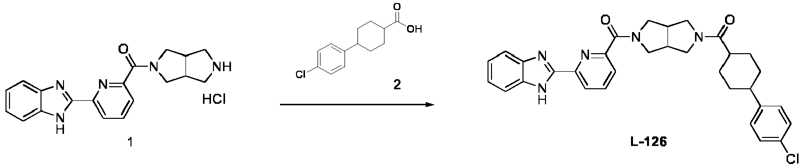

Operation: 80 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 51.6 mg (1 eq) of raw material 4-(4-chlorophenyl)cyclohexane-1-carboxyfic acid was added. The mixture was stirred, added with 137.6 mg (50% EA solution, 1.5 eq) of T₃P and 111.6 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 53 mg of a pure product with a yield of 44.2%.

LC-MS: m/z: (M+H)⁺ = 553.8.

¹H NMR (400 MHz, DMSO-*d₆*) δ 12.93 (d, *J* = 22.3 Hz, 1H), 8.41 (d, *J* = 7.9 Hz, 1H), 8.17 - 8.08 (m, 1H), 7.82 - 7.69 (m, 2H), 7.59 (t, *J* = 6.7 Hz, 1H), 7.28 (ddd, *J* = 30.5, 16.0, 7.3 Hz, 6H), 4.16 - 3.33 (m, 8H), 3.11 - 2.87 (m, 2H), 2.43 (t, *J=* 10.0 Hz, 2H), 1.94 - 1.70 (m, 4H), 1.61 - 1.39 (m, 4H).

### Embodiment 127: Synthesis of (5-(6-(1Hbenzo[d]imidazol-2-yl)picolinoyl)hexahydropyrrolo[3,4-c]pyrrol-2(1H)-yl)([1,1'-biphenyl]-4-yl)methanone (compound L-127)

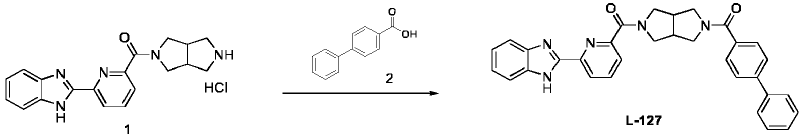

Operation: 80 mg (1 eq) of raw material (6-(1Hbenzo[d]imidazol-2-yl)pyridin-2-yl)(hexahydropyrrolo[3,4-c]pyrrol-2(1H)-yl)methanone hydrochloride was put into a reaction flask, then 2 mL of anhydrous DMF was added, 43 mg (1 eq) of raw material [1,1'-biphenyl]-4-carboxylic acid was added. The mixture was stirred, added with 137.6 mg (50% EA solution, 1.5 eq) of T₃P and 111.6 mg (3 eq) of DIPEA, and stirred at 30°C overnight. The next day, the reaction was completed as shown by LC-MS. Post-processing: the reaction mixture was poured into 20 mL of ice water and stirred, and extracted with dichloromethane (50 mL × 3). The organic layers were combined, extracted with saturated brine, and dried over anhydrous sodium sulfate. After filtration and concentration to dryness, the resulting residue was purified by column chromatography (DCM:MEOH = 0-5%) to obtain a product, and then the product was purified by thick-layer chromatography plate (DCM:MEOH = 95:5) to obtain 32 mg of a pure product with a yield of 28.8%.

LC-MS: m/z: (M+H)⁺ = 513.9.

¹H NMR (400 MHz, DMSO-*d₆*) δ 12.94 (s, 1H), 8.41 (dd, *J* = 13.3, 8.0 Hz, 1H), 8.13 (q, *J* = 7.9 Hz, 1H), 7.83 - 7.70 (m, 4H), 7.70 - 7.57 (m, 5H), 7.46 (ddt, *J* = 25.7, 13.0, 6.6 Hz, 3H), 7.27 (dt, *J* = 13.4, 6.5 Hz, 2H), 4.14 - 3.36 (m, 8H), 3.13 - 2.91 (m, 2H).

### Examples of receptor binding: examples of LANCL2 binding

**1.** Experimental materials
   **1.1** Reagent materials
      Ligand: LANCL2
      Running buffer: 20 mM MES, 150 mM NaCl, 0.05% P20, pH 6.5, 1% DMSO
   **1.2** Equipments
      Equipment name: Biacore S200
      Chip type: CM5 (29-1496-03)
**2.** Experimental methods
   **2.1** Ligand coupling
      LANCL2 protein was immobilized, and diluted to 50 µg/mL using sodium acetate solution at pH 4.0.
      Injection conditions: the CM5 chip surface was activated with a mixture of EDC/NHS with a flow rate of 10 µL/min and an injection time of 420 s; then LANCL2 injection was performed with a flow rate of 5 µL/min and an injection time of 2000 s, and the ligand coupling volume each time was about 1800 RU; finally, the chip surface was blocked with ethanolamine with a flow rate of 10 µL/min and an injection time of 420 s.
      Coupling buffer: 20 mM MES, 150 mM NaCl, 0.05% P20, pH 6.5.
   **2.2** Experimental conditions
      Analytes: All sample analytes of small molecule compounds were diluted 2-fold from a concentration of 50 µM to a concentration of 0.78 µM, so as to obtain a compound solution containing 1% DMSO.
      Injection conditions for small molecule compounds: a flow rate of 30 µL/min, a binding time of 60 s, a dissociation time of 300 s.
      Running buffer: 20 mM MES, 150 mM NaCl, 0.05% P20, pH 6.5, 1% DMSO.
      Sample chamber temperature: 25°C; analysis temperature: 25°C.

### Methods

Kinetic determination of LANCL2-small molecule interactions was performed. The kinetic parameters of small molecules BT-11 and L-1 to 60 (analytes) binding to LANCL2 (ligand) were determined using BIACORE S200. Data were generated in triplicate in a dose-dependent manner (5-8 titration points) and analyzed to determine binding models (Langmuir, conformational shifts, etc.), real-time association and dissociation constants, and equilibrium dissociation constants. SPR technology allows the validation of specific LANCL2-phytochemical interactions as well as an increased insight into the gold standard of binding mechanisms and rates. Experiments were performed on carboxymethyl polydextrose (CM5) sensor chips by amine-coupled covalent attachment of LANCL2. Data were analyzed with BIACORE S200T200 evaluation software (version 1), so as to determine affinity binding constants (KD) using a 1:1 binding model.

### Results

The compounds of the present disclosure could strongly bind to LANCL2. In order to confirm the binding of compounds to LANCL2 protein, SPR analysis was performed in a BIACORE S200 equipment. An optical technique for detecting molecular interactions, SPR, was used to measure the binding affinity between LANCL2 and a ligand thereof (compound to be tested). Purified recombinant LANCL2 protein was immobilized on a BIACORE sensor chip and small molecules were injected onto the protein surface using an equipment of microfluidic system. The change in total mass on the chip surface, corresponding to a small binding to the protein, was measured. The binding signal of the compound to be tested bound to LANCL2, the dissociation signal, the steady-state binding affinity of the compound and the binding signal curve of the compound at different concentrations could be calculated by injecting small molecule in a series of concentrations (for some of the compounds in the present disclosure whose affinity was hardly calculated, their binding signal curves at different concentrations were placed in the present disclosure as evidence that the compound has binding activity). Binding sensorgrams showed typical small molecule-protein interactions with extremely fast association and dissociation rates. These rapid interactions were beyond the technical capabilities of the equipment. Therefore, reliable association rate constants (ka) and dissociation rate constants (kd) were not determined. The equilibrium dissociation constant (Kd) was often used to describe the affinity between a ligand and a protein, such as affinity degree of a ligand binding to a specific protein. Ligand-protein affinity was affected by non-covalent intermolecular interactions between two molecules, such as hydrogen bonding, electrostatic interactions, hydrophobic bonds and van der Waals forces. The steady-state affinity (Kd) for each interaction could be measured by plotting equilibrium binding levels against compound concentrations. The compounds of the present disclosure had good binding properties to LANCL2 protein, and even some compounds of the present disclosure, such as L-2, L-9, L-11, L-15, L-25, L-40, L-52, L-77, L-89, L-95, L-96, L-106, had better binding activities than the positive reference compound BT-ll.

The specific data were shown in the table below.

| No. | Compound No. | KD (M) | Rmax (RU) | No. | Compound No. | KD (M) | Rmax (RU) |
|---|---|---|---|---|---|---|---|
| | BT-11 | 4.46E-06 | 3.6 | | BT-11 | 4.46E-06 | 3.6 |
| 1 | L-1 | 4.46E-06 | 3.6 | 65 | L-65 | 2.60E-05 | 6.5 |
| 2 | L-2 | 7.19E-07 | 9.6 | 66 | L-66 | 3.18E-05 | 7.2 |
| 3 | L-3 | 6.92E-06 | 3 | 67 | L-67 | N/A | N/A |
| 4 | L-4 | 4.00E-05 | 8 | 68 | L-68 | N/A | N/A |
| 5 | L-5 | 1.70E-05 | 7.3 | 69 | L-69 | 2.17E-05 | 6.1 |
| 6 | L-6 | / | / | 70 | L-70 | N/A | N/A |
| 7 | L-7 | / | / | 71 | L-71 | 4.06E-05 | 5.9 |
| 8 | L-8 | / | / | 72 | L-72 | 6.93E-06 | 5.3 |
| 9 | L-9 | 3.44E-06 | 3.9 | 73 | L-73 | 8.73E-06 | 4.5 |
| 10 | L-10 | 1.84E-05 | 5.3 | 74 | L-74 | 2.58E-05 | 0.102 |
| 11 | L-11 | 3.67E-06 | 4.2 | 75 | L-77 | 1.90E-06 | 7.3 |
| 12 | L-12 | / | / | 76 | L-78 | 8.15E-05 | 14.4 |
| 13 | L-13 | 3.83E-05 | 1.5 | 77 | L-79 | 2.48E-05 | 13 |
| 14 | L-14 | 2.11E-05 | 7.1 | 78 | L-80 | 2.84E-05 | 8.5 |
| 15 | L-15 | 2.30E-06 | 3.2 | 79 | L-81 | 2.30E-05 | 10.5 |
| 16 | L-16 | 8.49E-05 | 6.9 | 80 | L-82 | 4.23E-05 | 9.4 |
| 17 | L-17 | / | / | 81 | L-83 | 5.37E-05 | 9.6 |
| 18 | L-18 | 1.27E-04 | 5.2 | 82 | L-84 | / | / |
| 19 | L-19 | 2.53E-05 | 9.7 | 83 | L-85 | / | / |
| 20 | L-20 | 4.33E-05 | 5.3 | 84 | L-86 | / | / |
| 21 | L-21 | 9.88E-06 | 7.3 | 85 | L-87 | / | / |
| 22 | L-22 | / | / | 86 | L-88 | 1.64E-05 | 9.7 |
| 23 | L-23 | 1.03E-05 | 7.5 | 87 | L-89 | 2.53E-06 | 3.9 |
| 24 | L-24 | 5.73E-05 | 12.7 | 88 | L-90 | 9.50E-06 | 6.5 |
| 25 | L-25 | 2.01E-06 | 8.1 | 89 | L-91 | 1.71E-06 | 5.2 |
| 26 | L-26 | 6.58E-05 | 10.7 | 90 | L-92 | 5.88E-05 | 11 |
| 27 | L-27 | 1.33E-05 | 6.5 | 91 | L-93 | 5.84E-06 | 8 |
| 28 | L-28 | / | / | 92 | L-95 | 2.09E-06 | 5.2 |
| 29 | L-29 | / | / | 93 | L-96 | 1.91E-06 | 2.3 |
| 30 | L-30 | / | / | 94 | L-97 | / | / |
| 31 | L-32 | / | / | 95 | L-98 | 1.22E-05 | 3.2 |
| 32 | L-35 | 6.61E-05 | 14.4 | 96 | L-99 | 5.41E-06 | 5.1 |
| 33 | L-37 | / | / | 97 | L-100 | 3.85E-05 | 7.8 |
| 34 | L-38 | 9.09E-06 | 10.9 | 98 | L-101 | 2.33E-05 | 5.5 |
| 35 | L-39 | 6.33E-06 | 8.5 | 99 | L-102 | 2.95E-05 | 7.5 |
| 36 | L-40 | 3.21E-06 | 3.1 | 100 | L-103 | 2.41E-05 | 6 |
| 37 | L-41 | 2.76E-05 | 6.3 | 101 | L-104 | 1.25E-05 | 9.7 |
| 38 | L-42 | 1.32E-05 | 12 | 102 | L-105 | 1.75E-05 | 8.6 |
| 39 | L-43 | 4.92E-05 | 15.1 | 103 | L-106 | 4.32E-06 | 6.4 |
| 40 | L-44 | / | / | 104 | L-107 | 6.19E-06 | 4.5 |
| 41 | L-45 | 8.31E-06 | 6.3 | 105 | L-108 | 4.84E-05 | 13.7 |
| 42 | L-46 | 3.04E-05 | 10.6 | 106 | L-109 | / | / |
| 43 | L-47 | 1.33E-05 | 11.1 | 107 | L-110 | 2.62E-05 | 15 |
| 45 | L-50 | 7.04E-06 | 7.5 | 108 | L-111 | / | / |
| 46 | L-51 | 1.64E-05 | 7.5 | 109 | L-112 | 2.48E-05 | 12.7 |
| 47 | L-52 | 2.83E-06 | 3.9 | 110 | L-113 | / | / |
| 48 | L-53 | 3.60E-05 | 6.2 | 111 | L-114 | 1.08E-05 | 24 |
| 49 | L-54 | 2.78E-05 | 8.4 | 112 | L-115 | 1.77E-05 | 20.8 |
| 50 | L-55 | 1.68E-05 | 6.8 | 113 | L-116 | 1.48E-05 | 32.5 |
| 51 | L-56 | / | / | 114 | L-117 | 2.70E-05 | 3.2 |
| 52 | L-57 | 8.02E-06 | 8 | 115 | L-118 | 8.73E-06 | 74.9 |
| 53 | L-58 | 2.15E-05 | 3.3 | 116 | L-119 | 6.78E-06 | 10.8 |
| 54 | L-59 | 1.49E-05 | 3.6 | 117 | L-120 | 2.21E-05 | 8.9 |
| 55 | L-60 | 2.74E-05 | 3.6 | 118 | L-121 | 2.08E-05 | 2.3 |
| 56 | L-31 | N/A | N/A | 119 | L-122 | 1.03E-05 | 7 |
| 57 | L-33 | N/A | N/A | 120 | L-123 | 1.83E-05 | 3.9 |
| 58 | L-34 | N/A | N/A | 121 | L-124 | 8.79E-06 | 4.8 |
| 59 | L-36 | N/A | N/A^{a} | 122 | L-125 | 2.35E-05 | 5.2 |
| 60 | L-48 | N/A | N/A | 123 | L-126 | 4.05E-05 | 3.3 |
| 61 | L-61 | 2.32E-05 | 5.8 | 124 | L-127 | 9.38E-06 | 5.1 |
| 62 | L-62 | 2.93E-05 | 5.7 | 125 | L-75 | N/A | N/A |
| 63 | L-63 | 5.35E-05 | 7.6 | 126 | L-76 | N/A | N/A |
| 64 | L-64 | 4.88E-05 | 6.1 | 127 | L-94 | N/A | N/A |

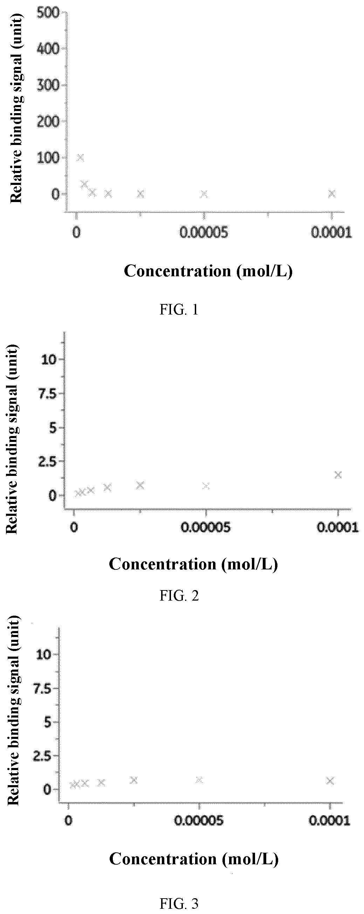

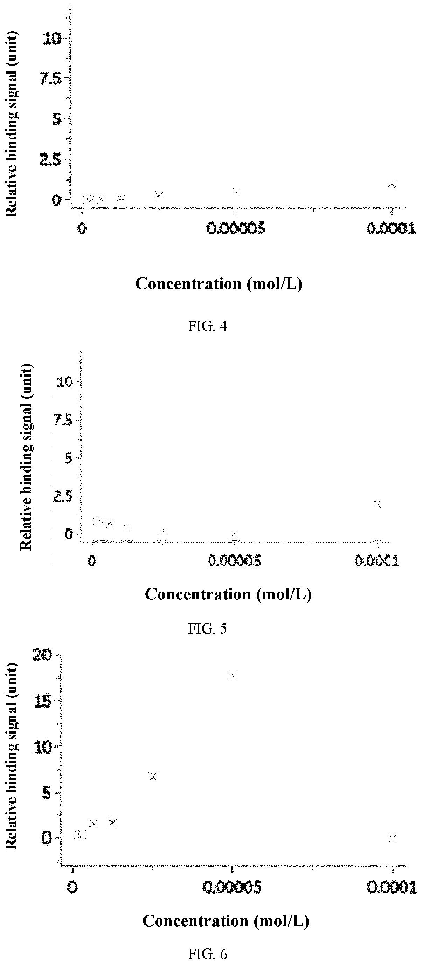

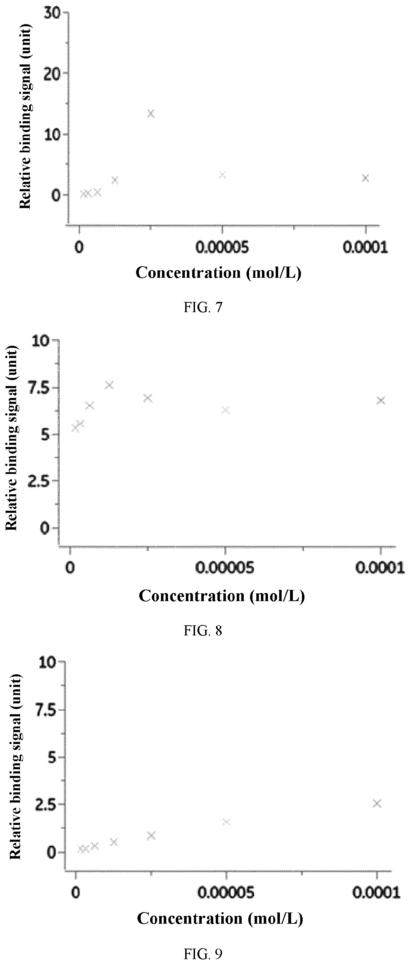

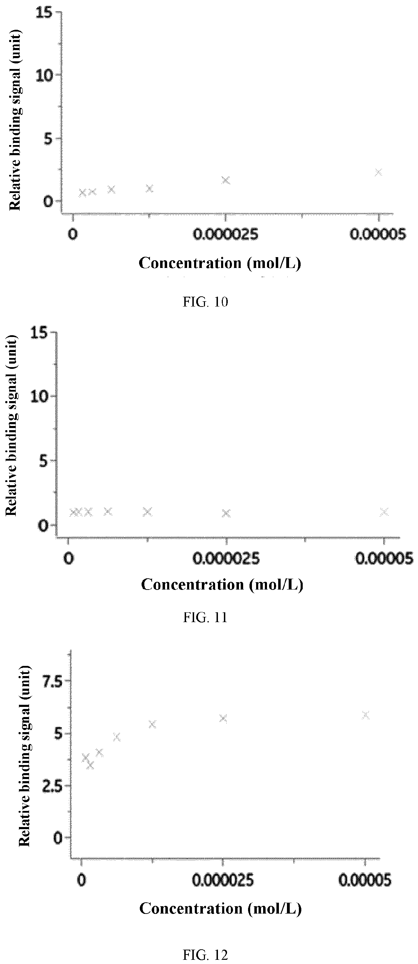

KD (M) indicated the binding force (mol); Rmax (RU) indicated the maximum binding force; N/A indicated no binding activity; "/" indicated that it was inconvenient to calculate KD according to test results, its binding activity was showed in the form of a binding curve, and the figure showed the binding signal intensity of the compound at different concentrations; "a" indicated poor solubility. The binding curves of some compounds were shown in FIG. 1 to FIG. 12.

### Study on the alleviating effect of the compounds of the present disclosure on TNBS-induced enteritis in mice (I)

### 1. Background of the study

IBD was a group of autoimmune diseases that could be divided into Crohn's disease and ulcerative colitis. This project used a TNBS-induced colitis model in mice to simulate Crohn's disease and evaluated the efficacy of the corresponding compounds with a view to developing a medicament for the treatment of Crohn's disease.

### 2. Purpose of the study

This project aimed to test the alleviation effect of representative compounds on TNBS-induced colitis in mice.

### 3. Reagents

| Reagents | Vendor | Cat |
|---|---|---|
| DPBS | Corning | 21-031-CVR |
| TNBS | Beijing Ouhe Technology Co., Ltd. | 2508-19-2 |
| Anhydrous ethanol | Aladdin | 64-17-5 |
| Mesalamine (finished drug product) | Losan Pharma GmbH | NA |
| Sodium chloride injection | Shandong Kelun Pharmaceutical Co., Ltd. | NA |
| 1.25% Avertin | Nanjing Aibei Biotechnology Co., Ltd. | M2910 |

### 4. Equipments

| **Equipment** | **Vendor** | **Model** |
|---|---|---|
| Electronic balance | Changzhou Zhitianping Instruments Co., Ltd. | YH-2000 |
| Electronic analytical balance | Mettle Toledo | 585310 |

### 5. Experimental methods

### 5.1 Dissolution and preservation of compounds

**Preparation method of L56:** an appropriate amount of L56 compound was weighed into a brown sample vial, added with a certain volume of solvent 95% (20% HP-β-CD) + 5% (10% sodium benzoate), the mixture was vortexed for 1 minute, and dissolved ultrasonically. The compound was prepared once a day.

**Preparation method of L30:** an appropriate amount of L30 compound was weighed into a brown sample vial, added with a certain volume of solvent 95% (20% HP-β-CD) + 5% (10% sodium benzoate), the mixture was vortexed for 1 minute, and dissolved ultrasonically. The compound was prepared once a day.

**Preparation method of Mesa (Mesalamine):** an appropriate amount of Mesalamine (Selleck) was weighed into a brown sample vial, added with a certain volume of solvent 0.5% CMC-Na, the mixture was vortexed for 1 minute, and dissolved ultrasonically. The compound was prepared once a day.

### 5.2 Route and frequency of compound administration

Fifty-six female Balb/c mice, weighing about 18 g and 8-10 weeks old, were randomly divided into seven groups, including six model groups and one Sham control group. The administration was started on Day 1 and ended on Day 7, with the compounds prepared once a day, as detailed in Table 1 for route and frequency of administration, and Table 2 for compound preparation and volume of administration.

**Table 1: Animal grouping and administration method**

| Group | Number of animals | Dose (mg/kg) | Route of administration | Frequency of administration | Administration cycle |
|---|---|---|---|---|---|
| Sham group | 5 | - | - | - | - |
| Solvent control group | 5 | - | PO | BID | 9 days |
| L56 group | 5 | 50 mpk | PO | BID | 9 days |
| L30 group | 6 | 50 mpk | PO | BID | 9 days |
| Mesa | 5 | 100 mpk | PO | QD | 9 days |

| | | | | | |
|---|---|---|---|---|---|
| Note: The positive drug is Mesalamine (Mesa) | | | | | |

**Table 2: Volume of administration and final concentration of drug**

| Group | Dose | Volume of administration (mL) | Final concentration of drug (mg/mL) | Frequency of administration |
|---|---|---|---|---|
| Solvent control group | --- | 0.2 | 0 | BID |
| L56 group | 50 mpk | 0.2 | 5 | BID |
| L30 group | 50 mpk | 0.2 | 5 | BID |
| Mesa | 100 mpk | 0.2 | 10 | QD |

| | | | | |
|---|---|---|---|---|
| Note: The mice were weighed for administration, and the above table showed the calculated volume of administration based on 20 g mice. | | | | |

### 5.3 Construction of TNBS-induced colitis model in mice

Balb/c mice weighing about 18-20 g were anesthetized with 0.25 mL of 1.25% Avertin on Day 0. The mice in the model group were perfused rectally with 150 µL of 1% TNBS solution (ethanol with a final concentration of 50%). The mice in the Sham control group were perfused rectally with 50% ethanol on Day 0.

### 5.4 Fixation of mouse colon tissue

The mouse colon was photographed, measured for the length, and weighed after the contents were removed. The colon of each mouse was then cut longitudinally at 1/2, rolled in a uniform direction by Swiss-roll method and fixed in neutral paraformaldehyde.

### 5.5 DAI scoring criteria

The DAI scoring consisted of 3 parts, taking the method of mixed scoring of weight change, stool and blood in stool, and the specific DAI scoring criteria were shown in Table 3. Throughout the experiment, the DAI scoring of all mice was done by the same person to ensure the scale consistency of the scoring.

**Table 3: DAI scoring criteria**

| **Score** | **Weight loss %** | **Stool properties** | **Occult blood or blood in stool** |
|---|---|---|---|
| 0 | 0 | Normal | Negative for occult blood |
| 1 | 1-5 | Soft stool | Weak positive for occult blood |
| 2 | 6-10 | Incompact stool | Positive for occult blood |
| 3 | 11-20 | Loose stool | Blood in stool |
| 4 | >20 | Extremely loose stool | A large amount of blood in stool |

### 5.6 Colon histopathology scoring criteria

The mouse colon histopathological scoring consisted of 5 parts, and the specific scoring criteria were shown in Table 4. The histopathological scoring was performed by a professional pathologist on the clinical pathology platform in a double-blind manner.

**Table 4: Colon histopathology scoring criteria**

| Score | Crypt structure | Immune cell infiltration | Sclerosis of muscle layers | Goblet cells | Crypt abscess |
|---|---|---|---|---|---|
| 0 | Normal | Normal | Normal | Existence | Normal |
| 1 | Slightly distorted | Little infiltration | Mild fibrosis | Missing | Abscess |
| 2 | Distorted | Infiltration | Fibrosis | | |
| 3 | Seriously distorted | Mass infiltration | High degree of fibrosis | | |

### 5.7 Statistical analysis

The experimental data were statistically analyzed by ANOVA method, positive drug Mesalamine (PO, 100 mpk, QD) group, L56 (PO, 50 mpk, BID) group, L30 (PO, 50 mpk, BID) group, ^{∗}*p*<0.05, ^{∗∗}*p*<0.01, ^{∗∗∗}*p*<0.005, ^{∗∗∗∗}*p*<0.0001

### 6. Results and analysis

### 6.1 Body weight changes and DAI scores of mice

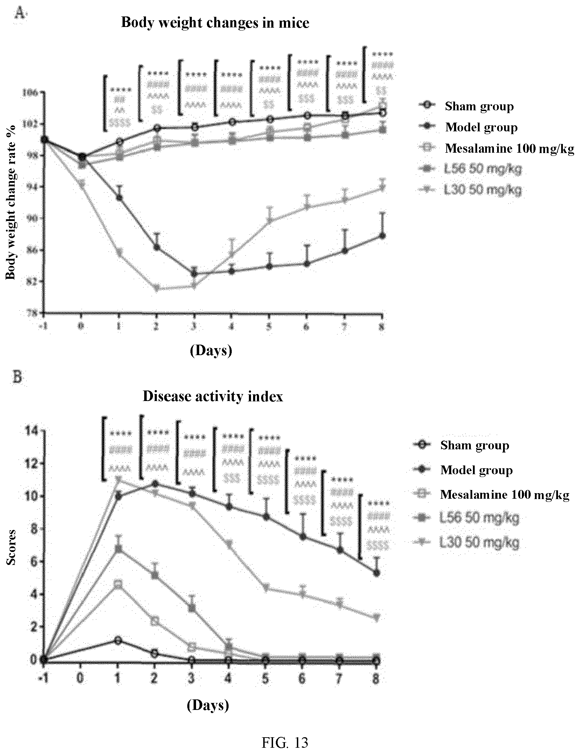

The weight data of mice in each group for 10 days (Day 1 to Day 8) were collected and the corresponding DAI scores were performed. The data were analyzed by the Two way ANOVA method. The data in FIG. 13A showed that, the weight loss of the mice in the L56 (PO, 50 mpk, BID) group and the Mesalamine (PO, 100 mpk, QD) group was significantly slowed down compared to the Vehicle group, while the weight loss of the mice in the L30 (PO, 50 mpk, BID) group was significantly slowed down from Day 5 to Day 8 compared to the Vehicle group. The DAI score data in FIG. 13B also showed that, the DAI scores of the mice in the L30 (PO, 50 mpk, BID) group, the L56 (PO, 50 mpk, BID) group, and the Mesalamine (PO, 100 mpk, QD) group were significantly lower compared to the Vehicle group. Combining body weight changes and DAI scores, it was found that L30 (PO, 50 mpk, BID) and L56 (PO, 50 mpk, BID) could effectively alleviate the weight loss in mice caused by TNBS-induced colitis.

Note: In FIG. 13, the statistical analysis of the body weight change and disease activity index (DAI) of the mice in the model group and other groups was performed by the Two way ANOVA method. Compared with the model group, the sham group: **p<0.05* **p<0.01 ^{∗∗∗}*p*<0.005 ****p<0.0001; the Mesalamine group: *#*p<0.05 *##p<0.01 ###p*<0.005 ####p<0.0001; the L56 50 mg/kg group: ^*p*<0.05 ^^*p*<0.01 ^^^*p*<0.005 ^^^^*p*><0.0001; the L30 50 mg/kg group: $p<0.05 $$*p*<0.01 $$$*p*<0.005 $$$$*p*<0.0001. Multiple comparison was performed by Dunnett's test in two way ANOVA.

### 6.2 Changes in the weight-to-length ratio of mouse colon

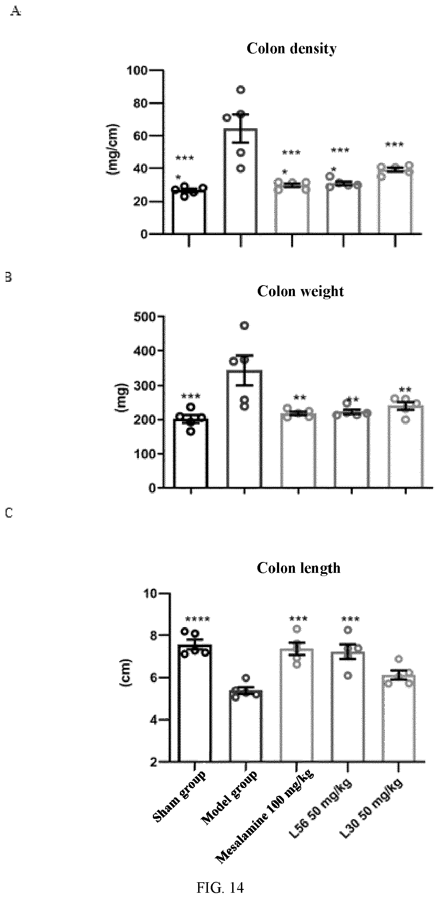

In general, inflammation resulted in a decline in colon length and an increase in colon weight. The data in FIG. 14A showed that, compared to the mice in the Vehicle group, the mice in the L30 (PO, 50 mpk, BID) group, the L56 (PO, 50 mpk, BID) group and the Mesalamine (PO, 100 mpk, QD) group had significantly lower weight-to-length ratios of colon with a statistically significant difference. The data in FIG. 14B showed that, compared to the mice in the Vehicle group, the mice in the L56 (PO, 50 mpk, BID) group and the Mesalamine (PO, 100 mpk, QD) group had significantly more colon length. The data in FIG. 14C showed that, compared to the mice in the Vehicle group, the mice in the L30 (PO, 50 mpk, BID) group, the L56 (PO, 50 mpk, BID) group and the Mesalamine (PO, 100 mpk, QD) group had less colon weight, which was close to that of the mice in the Sham group. These data further demonstrated that L30 (PO, 50 mpk, BID) and L56 (PO, 50 mpk, BID) could significantly alleviate TNBS-induced colitis in mice.

Note: In FIG. 14, the statistical analysis of the weight-to-length ratio of mouse colon in the model group and other groups was performed by the one way ANOVA method. Compared with the model group, ^{∗}*p*<0.05 **p<0.01 ***p<0.005 ^{∗∗∗∗}*p*><0.0001. Multiple comparison was performed by Dunnett's test in one way ANOVA.

### 6.3 HE staining and pathological scoring of mouse colon tissue

HE staining was perfomred on the colon tissues of mice in the seven groups and then scored by pathologists. Compared to the mice in the Vehicle group, the mice in the L56 (PO, 50 mpk, BID) group and the Mesalamine (PO, 100 mpk, QD) group had lower colon tissue pathological scores with a statistically significant difference. It further illustrated that L56 (PO, 50 mpk, BID) could effectively alleviate the occurrence of colitis in TNBS model mice.

### Conclusion

Combined with the data from In-life experiment and pathological analysis, L56 (PO, 50 mpk, BID) could effectively alleviate TNBS-induced colitis in mice.

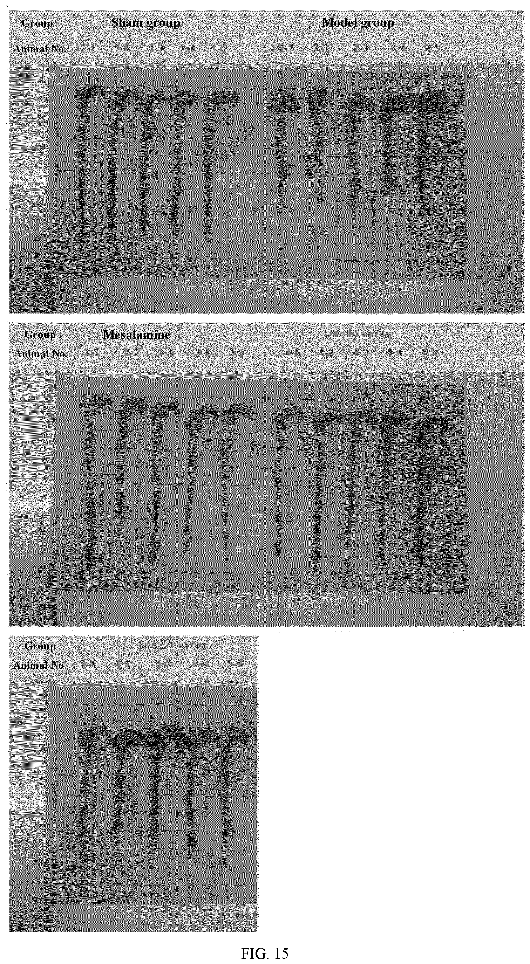

The intestinal morphology was shown in FIG. 15 (Sham group & Vehicle group, Mesalamine (PO, 100 mpk, QD) group & L56 (PO, 50 mpk, BID) group and L30 (PO, 50 mpk, BID) group).

### Study on the alleviating effect of the compounds of the present disclosure on TNBS-induced colitis in mice (II)

### 1. Background of the study

IBD was a group of autoimmune diseases that could be divided into Crohn's disease and ulcerative colitis. This project used a TNBS-induced colitis model in mice to simulate Crohn's disease and evaluated the efficacy of the corresponding compounds with a view to developing a medicament for the treatment of Crohn's disease.

### 2. Purpose of the study

This project aimed to test the alleviation effect of representative compounds on TNBS-induced colitis in mice.

### 3. Reagents

| Reagents | Vendor | Cat |
|---|---|---|
| DPBS | Corning | 21-031-CVR |
| TNBS | Beijing Ouhe Technology Co., Ltd. | 2508-19-2 |
| Anhydrous ethanol | Aladdin | 64-17-5 |
| Mesalamine (finished drug product) | Losan Pharma GmbH | NA |
| Sodium chloride injection | Shandong Kelun Pharmaceutical Co., Ltd. | NA |
| 1.25% Avertin | Nanjing Aibei Biotechnology Co., Ltd. | M2910 |

### 4. Equipments

| **Equipment** | **Vendor** | **Model** |
|---|---|---|
| Electronic balance | Changzhou Zhitianping Instruments Co., Ltd. | YH-2000 |
| Electronic analytical balance | Mettle Toledo | 585310 |

### 5. Experimental methods

### 5.1 Dissolution and preservation of compounds

**Preparation method of BT-11:** an appropriate amount of BT-11 compound was weighed into a brown sample vial, added with a certain volume of solvent 0.5% CMC-Na, the mixture was vortexed for 1 minute, and dissolved ultrasonically. The compound was prepared once a day.

**Preparation method of L11:** an appropriate amount of L11 compound was weighed into a brown sample vial, added with a certain volume of solvent 0.5% CMC-Na, the mixture was vortexed for 1 minute, and dissolved ultrasonically. The compound was prepared once a day.

**Preparation method of L25:** an appropriate amount of L25 compound was weighed into a brown sample vial, added with a certain volume of solvent 0.5% CMC-Na, the mixture was vortexed for 1 minute, and dissolved ultrasonically. The compound was prepared once a day.

**Preparation method of L84:** an appropriate amount of L84 compound was weighed into a brown sample vial, added with a certain volume of solvent 0.5% CMC-Na, the mixture was vortexed for 1 minute, and dissolved ultrasonically. The compound was prepared once a day.

**Preparation method of L77:** an appropriate amount of L77 compound was weighed into a brown sample vial, added with a certain volume of solvent 0.5% CMC-Na, the mixture was vortexed for 1 minute, and dissolved ultrasonically. The compound was prepared once a day.

**Preparation method of L101:** an appropriate amount of L101 compound was weighed into a brown sample vial, added with a certain volume of sodium chloride injection (saline), the mixture was vortexed for 1 minute, and dissolved ultrasonically. The compound was prepared once a day.

**Preparation method of L10:** an appropriate amount of L10 compound was weighed into a brown sample vial, added with a certain volume of solvent 0.5% CMC-Na, the mixture was vortexed for 1 minute, and dissolved ultrasonically. The compound was prepared once a day.

**Preparation method of L23:** an appropriate amount of L23 compound was weighed into a brown sample vial, added with a certain volume of solvent 0.5% CMC-Na, the mixture was vortexed for 1 minute, and dissolved ultrasonically. The compound was prepared once a day.

**Preparation method of Mesa (Mesalamine):** an appropriate amount of Mesalamine (Selleck) was weighed into a brown sample vial, added with a certain volume of solvent 0.5% CMC-Na, the mixture was vortexed for 1 minute, and dissolved ultrasonically. The compound was prepared once a day.

### 5.2 Route and frequency of compound administration

Sixty female Balb/c mice, weighing about 18 g and 8-10 weeks old, were randomly dividedinto twelve groups, including eleven model groups and one Sham control group. The administration was started on Day 1 and ended on Day 7, with the compounds prepared once a day, as detailed in Table 1 for route and frequency of administration, and Table 2 for compound preparation and volume of administration.

**Table 1: Animal grouping and administration method**

| Group | Number of animals | Dose (mg/kg) | Route of administration | Frequency of administration | Administration cycle |
|---|---|---|---|---|---|
| Sham group | 5 | - | - | - | - |
| Solvent control group | 5 | - | PO | BID | 9 days |
| Mesalazine group | 5 | 100 mpk | PO | QD | 9 days |
| L11 group | 5 | 50 mpk | PO | BID | 9 days |
| L25 group | 5 | 50 mpk | PO | BID | 9 days |
| L84 group | 5 | 50 mpk | PO | BID | 9 days |
| L77 group | 5 | 50 mpk | PO | BID | 9 days |
| L101 group | 5 | 50 mpk | PO | BID | 9 days |
| L10 group | 5 | 50 mpk | PO | BID | 9 days |
| L23 group | 5 | 50 mpk | PO | BID | 9 days |
| BT-11 group | 5 | 50 mpk | PO | BID | 9 days |

**Table 2: Volume of administration and final concentration of drug**

| Group | Dose | Volume of administration (mL) | Final drug concentration (mg/mL) | Frequency of administration |
|---|---|---|---|---|
| Model control group | --- | 0.2 | 0 | BID |
| Mesalazine group | 100 mpk | 0.2 | 10 | QD |
| L11 group | 50 mpk | 0.2 | 5 | BID |
| L25 group | 50 mpk | 0.2 | 5 | BID |
| L84 group | 50 mpk | 0.2 | 5 | BID |
| L77 group | 50 mpk | 0.2 | 5 | BID |
| L101 group | 50 mpk | 0.2 | 5 | BID |
| L10 group | 50 mpk | 0.2 | 5 | BID |
| L23 group | 50 mpk | 0.2 | 5 | BID |
| BT-11 group | 50 mpk | 0.2 | 5 | BID |

| | | | | |
|---|---|---|---|---|
| Note: The mice were weighed for administration, and the above table showed the calculated volume of administration based on 20 g mice. The solvent group is the model control group. | | | | |

### 5.3 Construction of TNBS-induced colitis model in mice

Balb/c mice weighing about 18-20 g were anesthetized with 0.25 mL of 1.25% Avertin on Day 0. The mice in the model group were perfused rectally with 150 µL of 1% TNBS solution (ethanol with a final concentration of 50%). The mice in the Sham control group were perfused rectally with 50% ethanol on Day 0.

### 5.4 Fixation of mouse colon tissue

The mouse colon was photographed, measured for the length, and weighed after the contents were removed. The colon of each mouse was then cut longitudinally at 1/2, rolled in a uniform direction by Swiss-roll method and fixed in neutral paraformaldehyde.

### 5.5 Collection of fresh mouse colon tissue

The remaining 1/2 of the colon tissue was then cut longitudinally along the 1/2, divided into two tubes, quick-frozen in liquid nitrogen, stored at -80°C, and transported on dry ice for subsequent experiments.

### 5.6 Collection of mouse mesenteric lymph nodes

The mouse mesenteric lymph nodes were collected and stored at 4°C. After all samples were collected, they were immediately transferred to the client for flow analysis.

### 5.7 DAI scoring criteria

The DAI scoring consisted of 3 parts, taking the method of mixed scoring of weight change, stool and blood in stool, and the specific DAI scoring criteria were shown in Table 3. Throughout the experiment, the DAI scoring of all mice was done by the same person to ensure the scale consistency of the scoring.

**Table 3: DAI scoring criteria**

| **Score** | **Weight loss %** | **Stool properties** | **Occult blood or blood in stool** |
|---|---|---|---|
| 0 | 0 | Normal | Negative for occult blood |
| 1 | 1-5 | Soft stool | Weak positive for occult blood |
| 2 | 6-10 | Incompact stool | Positive for occult blood |
| 3 | 11-20 | Loose stool | Blood in stool |
| 4 | >20 | Extremely loose stool | A large amount of blood in stool |

### 5.8 Statistical analysis

The experimental data were statistically analyzed by ANOVA method, and data of other groups and the Vehicle (PO, QD) group were analyzed by Dunnett's test to compare data of multiple groups, ^{∗}*p*<0.05, ^{∗∗}*p*<0.01, ^{∗∗∗}*p*<0.005, ^{∗∗∗∗}*p*<0.0001

### 6. Results and analysis

### 6.1 Body weight changes and DAI scores of mice

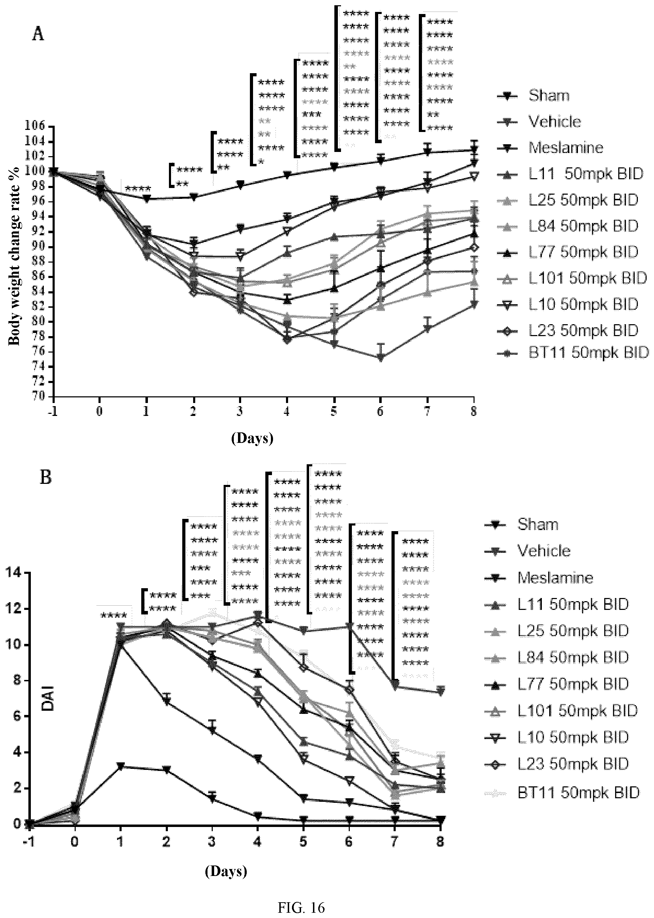

The weight data of mice in each group for 10 days (Day 1 to Day 8) were collected and the corresponding DAI scores were performed. The data were analyzed by the Two way ANOVA method. The data in FIG. 16A showed that, the weight loss of the mice in the L11 (PO, 50 mpk, BID) group and the L10 (PO, 50 mpk, BID) group in the nine test compound groups was significantly slowed down compared to the Vehicle group. Only the weight loss of the mice in the L84 (PO, 50 mpk, BID) group and the BT11 (PO, 50 mpk, BID) group was not significantly slowed down compared to the Vehicle group. The other five test compounds all had significant effects in alleviating the weight loss of the mice, but not as significantly as L11 and L10. The DAI score data in FIG. 16B also showed that, the DAI scores of the mice in the L11 (PO, 50 mpk, BID) group and the L10 (PO, 50 mpk, BID) group in the nine test compound groups were significantly lower compared to the Vehicle group. The other seven test compounds all had significant effects in improving DAI scores, but not as significantly as L11 and L10. Combining body weight changes and DAI scores, it was found that L11 (PO, 50 mpk, BID) and L10 (PO, 50 mpk, BID) could effectively alleviate the weight loss in mice caused by TNBS-induced colitis. More interestingly, L10 (PO, 50 mpk, BID) was comparable in efficacy to the positive drug Mesalamine (PO, 100 mpk, QD).

### 6.2 Scoring of diarrhea and blood in stool in mice

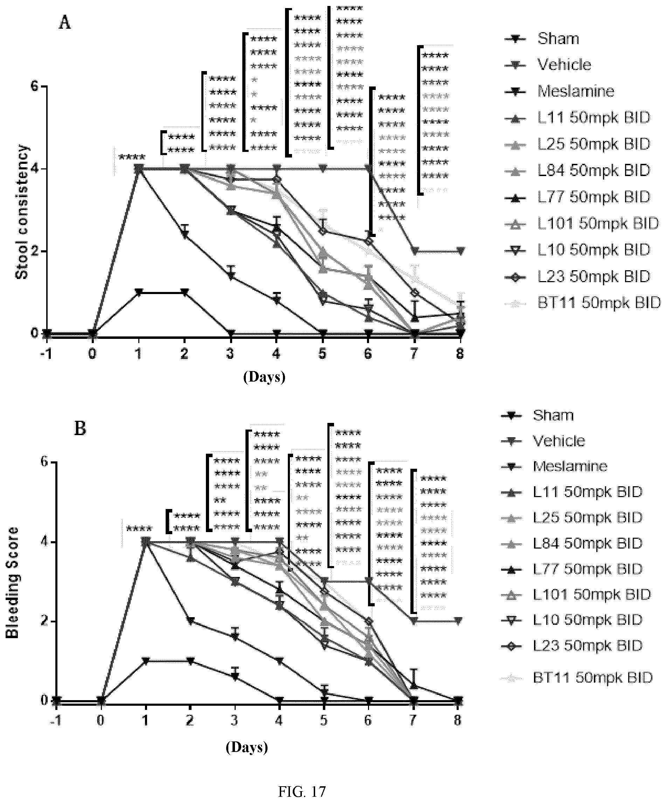

Similarly, scores for diarrhea and blood in stool were analyzed separately using the Two way ANOVA method. As shown in FIG. 17A, the diarrhea of the mice in the L11 (PO, 50 mpk, BID) group and the L10 (PO, 50 mpk, BID) group in the nine test compound groups was significantly slowed down compared to the Vehicle group. The other seven test compounds all had significant effects in ameliorating diarrhea in mice with colitis, but not as significantly as L11 and L10. As shown in FIG. 17B, the blood in stool of the mice in the L11 (PO, 50 mpk, BID) group and the L10 (PO, 50 mpk, BID) group in the nine test compound groups was significantly slowed down compared to the Vehicle group. The other seven test compounds all had significant effects in ameliorating blood in stool in mice with colitis, but not as significantly as L11 and L10. Therefore, it was found that L11 (PO, 50 mpk, BID) and L10 (PO, 50 mpk, BID) could effectively alleviate the symptoms of diarrhea and blood in stool in TNBS model mice.

### 6.3 Changes in the weight-to-length ratio of mouse colon

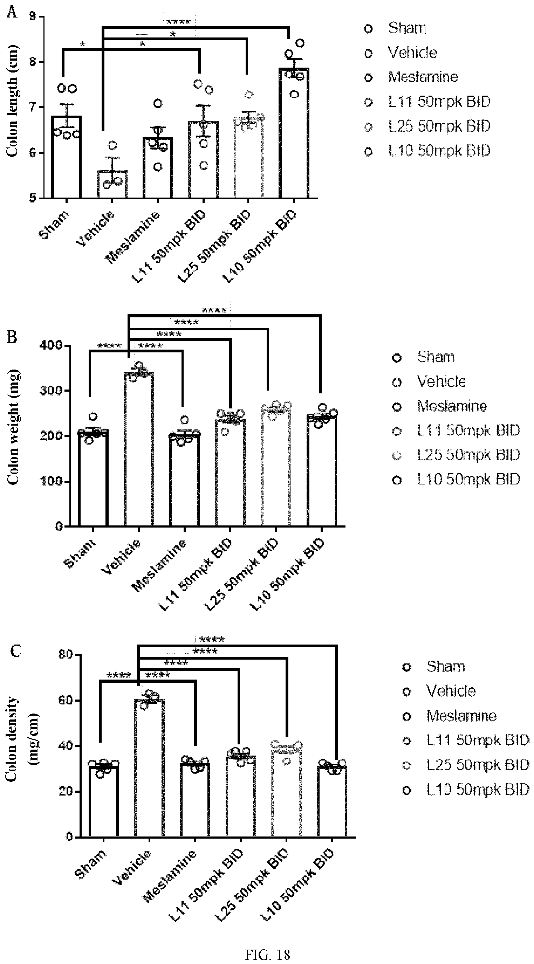

In general, inflammation resulted in a decline in colon length and an increase in colon weight. At the end of sample collection, the mice in the L10 (PO, 50 mpk, BID) group, L25 (PO, 50 mpk, BID) group and L11 (PO, 50 mpk, BID) group with significant efficacy were selected, whose colons were photographed, measured for the length, and weighed. The data in FIG. 18C showed that, compared to the mice in the Vehicle group, the mice in the L10 (PO, 50 mpk, BID) group, the L25 (PO, 50 mpk, BID) group, the L11 (PO, 50 mpk, BID) group and the Mesalazine (PO, 100 mpk, QD) group had significantly lower weight-to-length ratios of colon with a statistically significant difference. The data in FIG. 18A showed that, compared to the mice in the Vehicle group, the mice in the L10 (PO, 50 mpk, BID) group, the L25 (PO, 50 mpk, BID) group and the L11(PO, 50 mpk, BID) group had significantly more colon length. The data in FIG. 18B showed that, compared to the mice in the Vehicle group, the mice in the L10 (PO, 50 mpk, BID) group, the L25 (PO, 50 mpk, BID), the L11(PO, 50 mpk, BID) group and the Mesalazine (PO, 100 mpk, QD) group had less colon weight. These data further demonstrated that L11 (PO, 50 mpk, BID) and L10 (PO, 50 mpk, BID) could significantly alleviate the occurrence of inflammation in mice with TNBS-induced colitis.

### 7. Conclusion

Combined with the data from In-life experiments, L11 (PO, 50 mpk, BID) and L10 (PO, 50 mpk, BID) could effectively alleviate TNBS-induced colitis in mice.

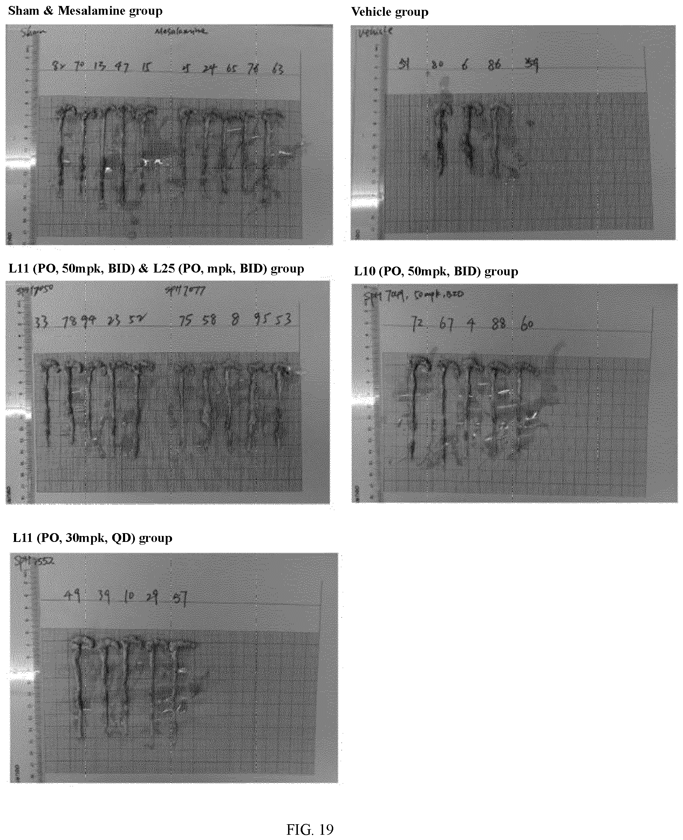

The intestinal morphology was shown in FIG. 19.

Although the specific embodiments of the present disclosure have been described above, it should be understood by those skilled in the art that these are merely illustrative examples and that a variety of changes or modifications can be made to these embodiments without departing from the principles and substance of the present disclosure. Therefore, the scope of protection of the present disclosure is limited by the appended claims.

## Claims

1. A carbonyl heterocyclic compound represented by formula I or a pharmaceutically acceptable salt thereof; 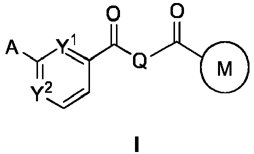
wherein, A is 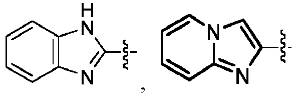 or -NR¹R²;
R¹ and R² are independently H or C₆₋₁₈ aryl;
Y¹ and Y² are independently CH or N;
Q is 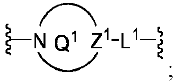
Z¹-L¹- is 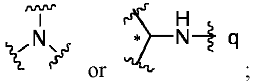
the carbon atom with "*" indicates that when the carbon atom is a chiral carbon atom, it is S-configuration, R-configuration or a mixture of both;
ring Q' is 6- to 10-membered fused heterocycloalkyl, 6- to 12-membered spiro heterocycloalkyl or 6- to 10-membered bridged heterocycloalkyl; Q' contains 1 to 3 N atoms;
M is 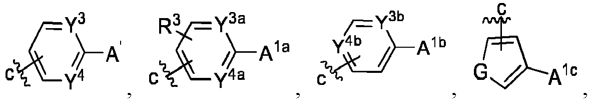 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 5- to 10-membered cycloalkyl substituted by R⁵; the heteroatoms in the 6- to 10-membered fused heterocycloaryl, the heteroatoms in the 6- to 10-membered fused heterocycloaryl substituted by R⁴, the heteroatoms in the 5- to 10-membered heterocycloalkenyl and the heteroatoms in the 5- to 10-membered heterocycloalkenyl substituted by oxo are independently one or more than one of N, S and O, and the number of heteroatoms is independently 1, 2 or 3; the number of R³ is 1, 2 or 3; c-terminus indicates a connection to the C=O as shown;
G is S or O;
A', A^{1a}, A^{1b} and A^{1c} are independently 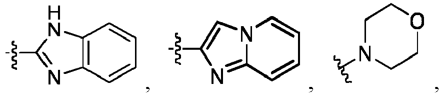 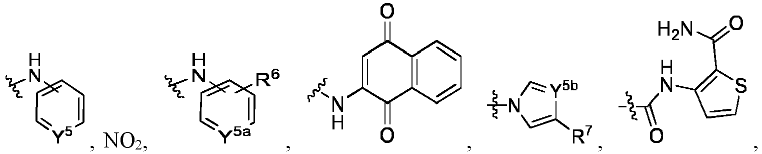 -OR⁸, C₆₋₁₄ aryl or H;
Y³, Y^{3a}, Y^{3b}, Y⁴, Y^{4a}, Y^{4b}, Y⁵, Y^{5a}, Y^{5b} and Y^{5c} are independently CH or N;
R⁶ is halogen;
R⁷ is H or C₁₋₆ alkyl;
R⁸ is C₆₋₁₄ aryl;
R³ is C₁₋₆ alkyl or halogen;
R⁴ is C₁₋₆ alkyl;
R⁵ is C₆₋₁₄ aryl or C₆₋₁₄ aryl substituted by halogen.

2. The carbonyl heterocyclic compound represented by formula I or the pharmaceutically acceptable salt thereof as claimed in claim 1, wherein, 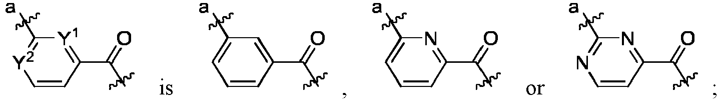 a-terminus indicates the position connected to A;
and/or, when Q' is 6- to 10-membered fused heterocycloalkyl, the 6- to 10-membered fused heterocycloalkyl is 6- to 8-membered fused heterocycloalkyl, containing 1 to 2 N atoms, and can also be heterocycloalkyl of 5-membered N-heterocycloalkyl fused with 3-membered cycloalkyl or heterocycloalkyl of 5-membered N-heterocycloalkyl fused with 5-membered N-heterocycloalkyl, for example, 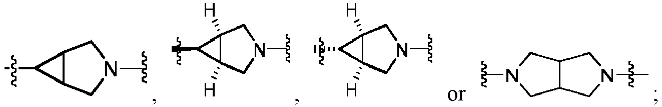
and/or, when Q' is 6- to 12-membered spiro heterocycloalkyl, the 6- to 12-membered spiro heterocycloalkyl is 7- to 11-membered spiro heterocycloalkyl, containing 1 or 2 N atoms, and can also be 9- to 11-membered spiro heterocycloalkyl, containing 1 or 2 N atoms, and can also be azaspiro[5,5]undecane heterocycloalkyl, azaspiro[5,4]decane heterocycloalkyl, or azaspiro[4,4]nonane heterocycloalkyl, for example, 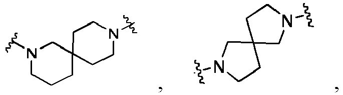 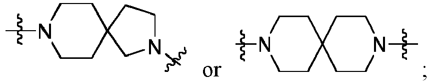
and/or, when Q' is 6- to 10-membered bridged heterocycloalkyl, the 6- to 10-membered bridged heterocycloalkyl is 7-membered bridged heterocycloalkyl, for example, 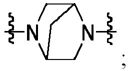
and/or, when M is 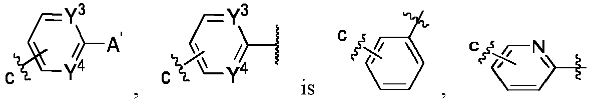 or 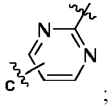
and/or, when M is 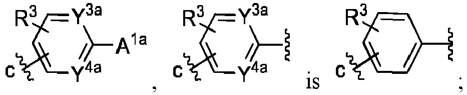
and/or, when M is 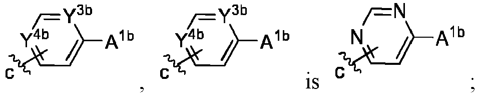
and/or, when M is 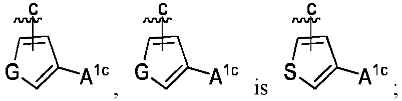
and/or, when M is 6- to 10-membered fused heterocycloaryl or 6- to 10-membered fused heterocycloaryl substituted by R4, the 6- to 10-membered fused heterocycloaryl is independently 9-to 10-membered fused heterocycloaryl, the heteroatom is N and/or S, and the number is 1 or 2, for example, indolyl, quinolinyl, isoindolyl or imidazopyridyl;
and/or, when M is 5- to 10-membered heterocycloalkenyl or 5- to 10-membered heterocycloalkenyl substituted by oxo, the 5- to 10-membered heterocycloalkenyl is 6-membered heterocycloalkenyl, the heteroatom is N, the number is 2, for example, 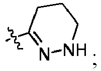
and/or, when M is 4- to 10-membered cycloalkyl substituted by R⁵, the 4- to 10-membered cycloalkyl is cyclopentyl, cyclohexyl or cycloheptyl, for example, cyclohexyl;
and/or, when A', A^{1a}, A^{1b} and A^{1c} are independently 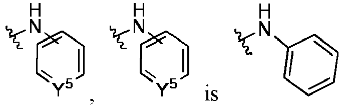 or 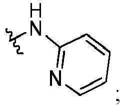
and/or, when A', A^{1a}, A^{1b} and A^{1c} are independently 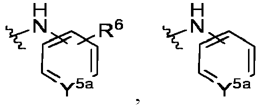 is 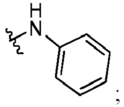
and/or, when A', A^{1a}, A^{1b} and A^{1c} are independently 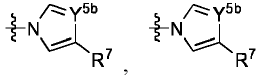 is 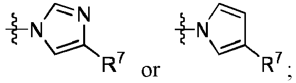
and/or, when A', A^{1a}, A^{1b} and A^{1c} are independently C₆₋₁₄ aryl, the C₆₋₁₄ aryl is independently phenyl, naphthyl, anthracenyl or phenanthryl;
and/or, when R⁶ is halogen, the halogen is F, Cl, Br or I, for example, F;
and/or, when R⁷ is C₁₋₆ alkyl, the C₁₋₆ alkyl is methyl, ethyl, n-propyl, isopropyl, n-butyl, isobutyl, *sec*-butyl or *tert*-butyl; for example, methyl;
and/or, when R⁸ is C₆₋₁₄ aryl, the C₆₋₁₄ aryl is independently phenyl, naphthyl, anthracenyl or phenanthryl, for example, phenyl;
and/or, when R³ is C₁₋₆ alkyl, the C₁₋₆ alkyl is methyl, ethyl, n-propyl, isopropyl, n-butyl, isobutyl, *sec*-butyl or *tert*-butyl; for example, methyl;
and/or, when R³ is halogen, the halogen is F, Cl, Br or I, for example, F or Cl;
and/or, when R⁴ is C₁₋₆ alkyl, the C₁₋₆ alkyl is methyl, ethyl, n-propyl, isopropyl, n-butyl, isobutyl, *sec*-butyl or *tert*-butyl; for example, methyl;
and/or, when R⁵ is C₆₋₁₄ aryl or C₆₋₁₄ aryl substituted by halogen, the C₆₋₁₄ aryl is independently phenyl, naphthyl, anthracenyl or phenanthryl, for example, phenyl;
and/or, when R⁵ is C₆₋₁₄ aryl substituted by halogen, the halogen is F, Cl, Br or I, for example, Cl.

3. The carbonyl heterocyclic compound represented by formula I or the pharmaceutically acceptable salt thereof as claimed in claim 2, wherein, when M is 6- to 10-membered fused heterocycloaryl substituted by R⁴, the 6- to 10-membered fused heterocycloaryl substituted by R⁴ is 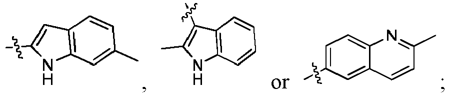
and/or, when M is 5- to 10-membered heterocycloalkenyl substituted by oxo, the 5- to 10-membered heterocycloalkenyl substituted by oxo is 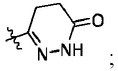
and/or, when M is 4- to 10-membered cycloalkyl substituted by R⁵, the 4- to 10-membered cycloalkyl substituted by R⁵ is 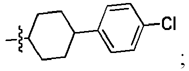
and/or, when A', A^{1a}, A^{1b} and A^{1c} are independently 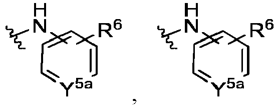 is 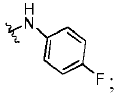
and/or, when A', A^{1a}, A^{1b} and A^{1c} are independently 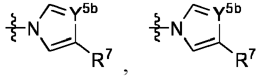 is

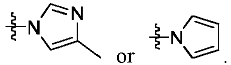

4. The carbonyl heterocyclic compound represented by formula I or the pharmaceutically acceptable salt thereof as claimed in claim 1, wherein, one of R¹ and R² is H, and the other is C₆₋₁₄ aryl;
and/or, Y² is CH;
and/or, when Q' is 6- to 10-membered fused heterocycloalkyl, Z¹-L¹- is 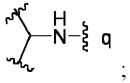 alternatively, when Q' is 6- to 10-membered fused heterocycloalkyl, Z¹-L¹- is 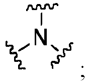
and/or, when Q' is 6- to 12-membered spiro heterocycloalkyl; Z¹-L¹- is 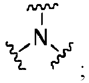
and/or, when Q' is 6- to 10-membered bridged heterocycloalkyl; Z¹-L¹- is 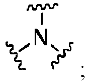
and/or, A' is 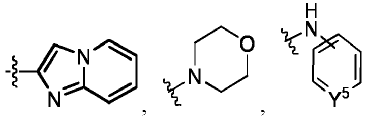 or NO₂;
and/or, A is the same as A';
and/or, 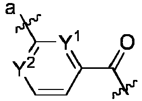 is the same as

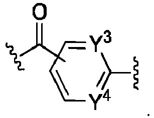

5. The carbonyl heterocyclic compound represented by formula I or the pharmaceutically acceptable salt thereof as claimed in claim 1, wherein, 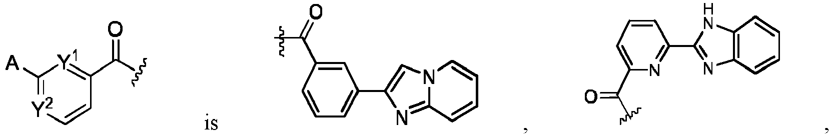 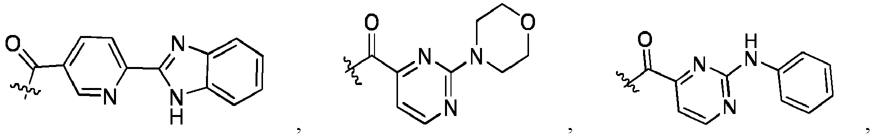 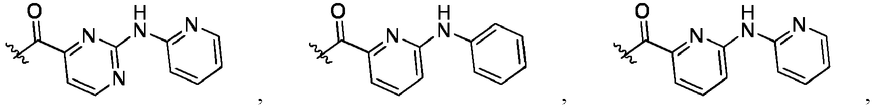 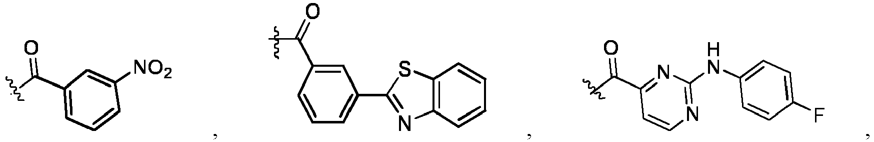 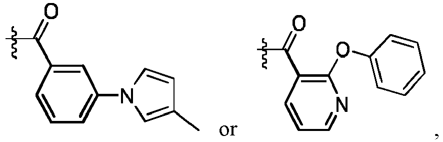 and can also be 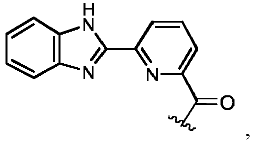 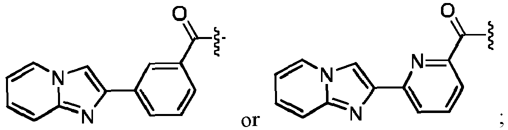 and/or, Q is 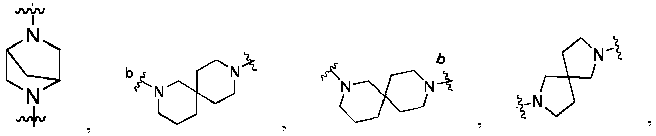 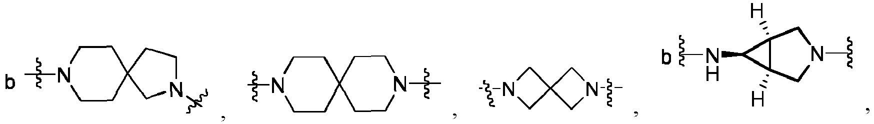 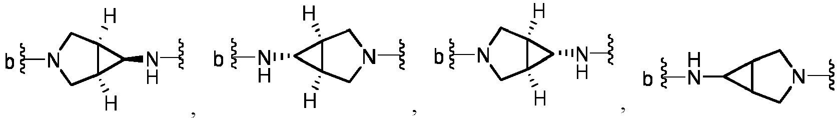 or 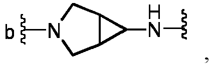 and can also be 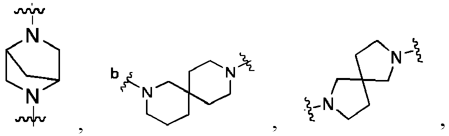 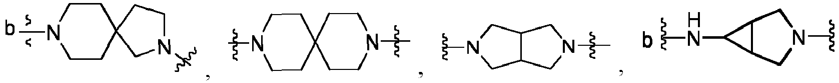 or 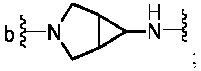 and/or, 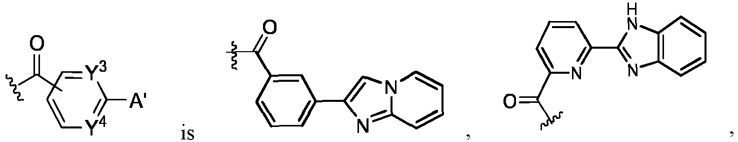 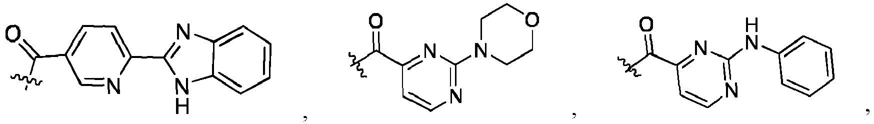 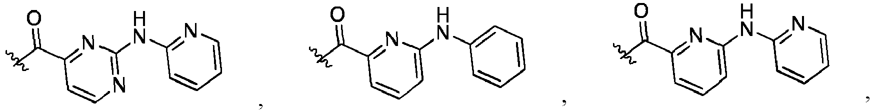 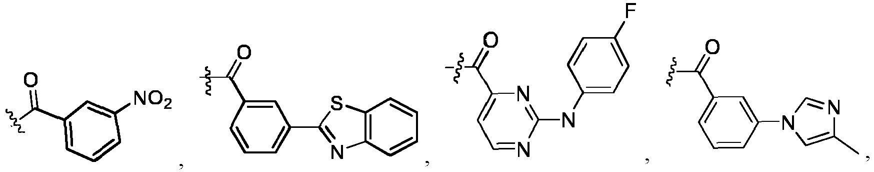 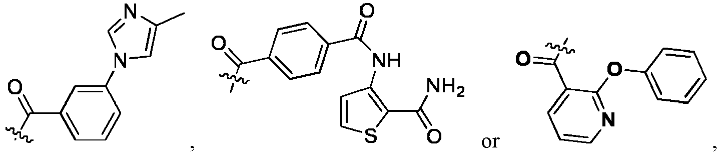 and can also be 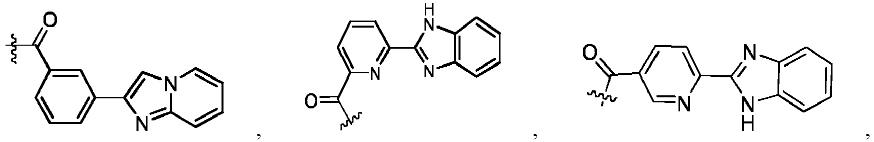 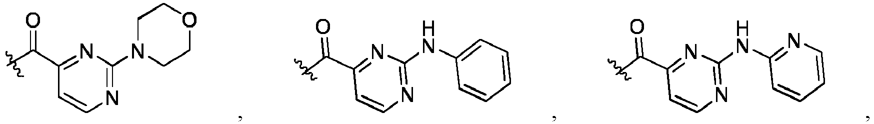 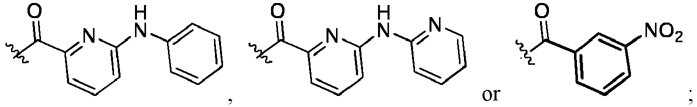 and/or, 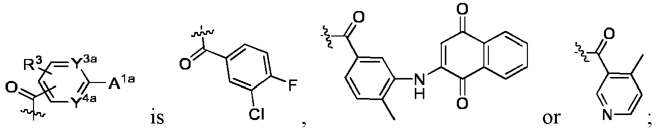 and/or, 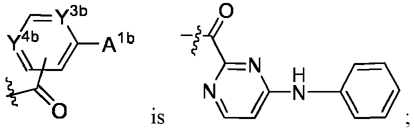 and/or,

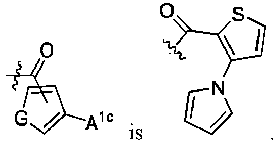

6. The carbonyl heterocyclic compound represented by formula I or the pharmaceutically acceptable salt thereof as claimed in claim 1, wherein, the carbonyl heterocyclic compound represented by formula I is selected from any one of the following schemes:
scheme **1:**
A is 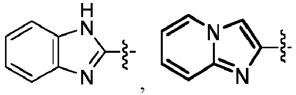 or -NR¹R²;
R' and R² are independently H or C₆₋₁₈ aryl;
Y' and Y² are independently CH or N;
Q is 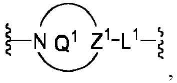 Z¹-L¹- is 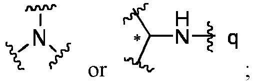
the carbon atom with "*" indicates that when the carbon atom is a chiral carbon atom, it is S-configuration, R-configuration or a mixture of both;
ring Q' is 6- to 10-membered fused heterocycloalkyl or 6- to 12-membered spiro heterocycloalkyl; Q' contains 1 to 3 N atoms;
M is 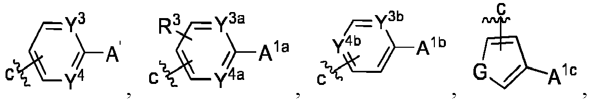 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 5- to 10-membered cycloalkyl substituted by R⁵;
G is S and O;
A', A^{1a}, A^{1b} and A^{1c} are independently 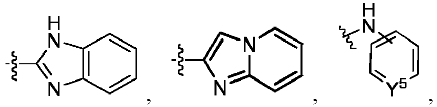 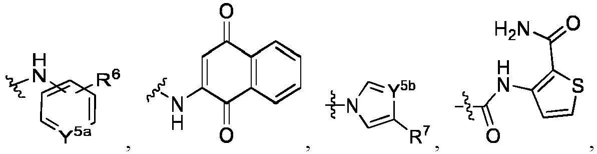 -OR⁸, C₆₋₁₄ aryl or H;
Y⁵, Y^{5a} and Y^{5b} are independently CH or N;
R⁶ is halogen;
R⁷ is C₁₋₆ alkyl;
R⁸ is C₆₋₁₄ aryl;
R³ is C₁₋₆ alkyl or halogen;
R⁴ is C₁₋₆ alkyl;
R⁵ is C₆₋₁₄ aryl or C₆₋₁₄ aryl substituted by halogen;
scheme 2:
A is 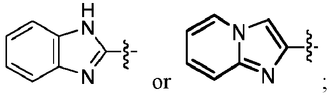
Y² is CH;
Q is 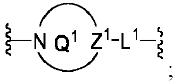
Z¹-L¹- is 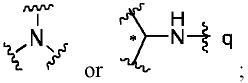
ring Q' is heterocycloalkyl of 5-membered N-heterocycloalkyl fused with 3-membered cycloalkyl or spiro[5,5]undecane heterocycloalkyl (e.g., 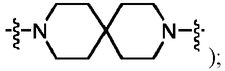
M is 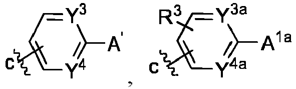 or 6- to 10-membered fused heterocycloaryl;
A' and A^{1b} are independently 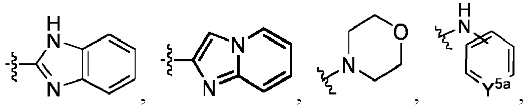 NO₂, 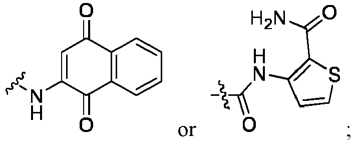
scheme 3:
A is 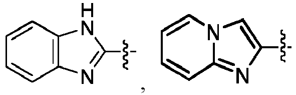 or -NR¹R²;
R' and R² are independently H or C₆₋₁₄ aryl;
Y' and Y² are independently CH or N;
Q is 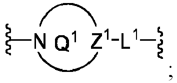
Z¹-L¹- is 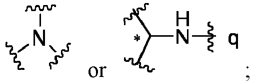
ring Q' is 6- to 10-membered fused heterocycloalkyl; Q' contains 1 or 2 N atoms;
M is 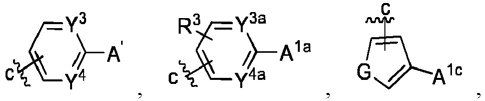 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 4- to 10-membered cycloalkyl substituted by R⁵;
G is S;
A' and A^{1c} are independently 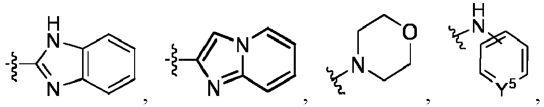 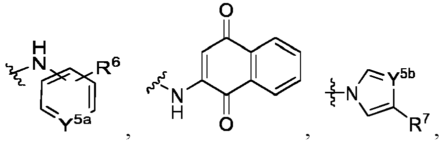 -OR⁸, C₆₋₁₄ aryl or H;
Y⁵, Y^{5a} and Y^{5b} are independently CH or N;
R⁶ is halogen;
R⁷ is C₁₋₆ alkyl;
R⁸ is C₆₋₁₄ aryl;
R³ is C₁₋₆ alkyl or halogen;
R⁴ is C₁₋₆ alkyl;
R⁵ is C₆₋₁₄ aryl or C₆₋₁₄ aryl substituted by halogen;
scheme 4:
A is 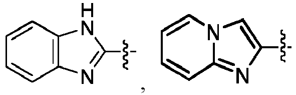 or -NR¹R²;
R' and R² are independently H or C₆₋₁₄ aryl;
Y' and Y² are independently CH or N;
Q is 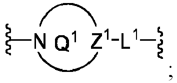
Z¹-L¹- is 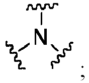
ring Q' is 6- to 12-membered spiro heterocycloalkyl; Q' contains 2 N atoms;
M is 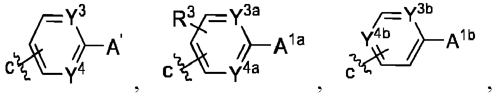 6- to 10-membered fused heterocycloaryl, 6- to 10-membered fused heterocycloaryl substituted by R⁴, 5- to 10-membered heterocycloalkenyl, 5- to 10-membered heterocycloalkenyl substituted by oxo or 4- to 10-membered cycloalkyl substituted by R⁵;
A', A^{1a}, A^{1b} and A^{1c} are independently 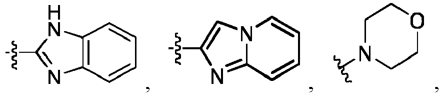 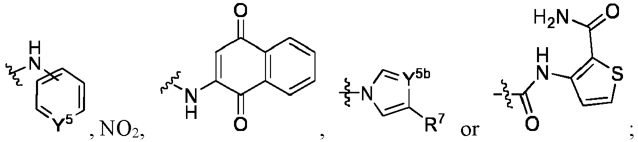
Y⁵ and Y ^{5b} are independently CH or N;
scheme 5:
the carbonyl heterocyclic compound represented by formula I is as shown in formula I-1: 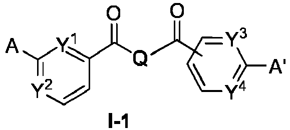
wherein, A is 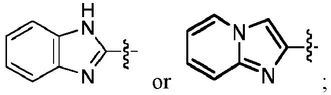
Y¹ and Y² are independently CH or N;
Q is 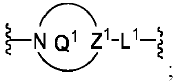
Z¹-L¹- is 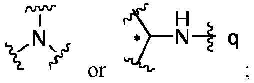
ring Q' is 6- to 10-membered fused heterocycloalkyl, 6- to 12-membered spiro heterocycloalkyl or 6- to 10-membered bridged heterocycloalkyl; Q' contains 1 to 3 N atoms;
Y³ and Y⁴ are independently CH or N;
A' is 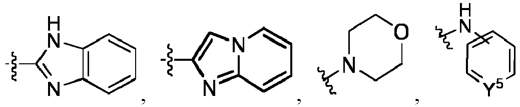 or NO₂;
Y⁵ is CH or N;
the carbon atom with "*" indicates that when the carbon atom is a chiral carbon atom, it is S-configuration, R-configuration or a mixture of both;
scheme 6:
A is 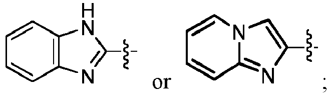
Y¹ and Y² are independently CH or N;
Q is 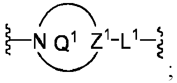 Q¹ is 6- to 10-membered fused heterocycloalkyl, 6- to 12-membered spiro heterocycloalkyl or 6- to 10-membered bridged heterocycloalkyl, and Z¹-L¹- is 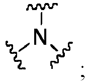 alternatively, Q' is 6- to 10-membered fused heterocycloalkyl, and Z¹-L¹- is 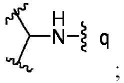
Y³ and Y⁴ are independently CH or N;
A' is 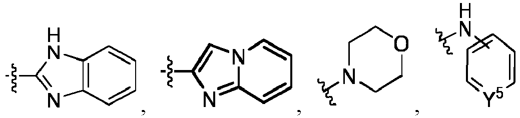 or NO₂;
Y⁵ is CH or N;
scheme 7:
A is 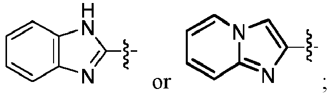
Y¹ and Y² are independently CH or N;
Q is 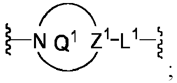 Q¹ is independently 6- to 10-membered fused heterocycloalkyl or 6-to 12-membered spiro heterocycloalkyl, and Z¹-L¹- is 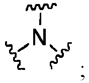 alternatively, Q' is 6- to 10-membered fused heterocycloalkyl, and Z¹-L¹- is 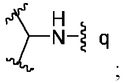
Y³ and Y⁴ are independently CH or N;
A' is 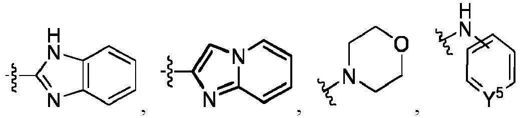 or NO₂;
Y⁵ is CH or N;
scheme **8:**
A is 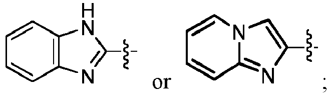 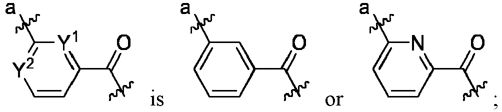
Q is 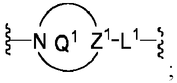 Q¹ is independently 6- to 10-membered fused heterocycloalkyl; Z¹-L¹- is 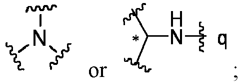 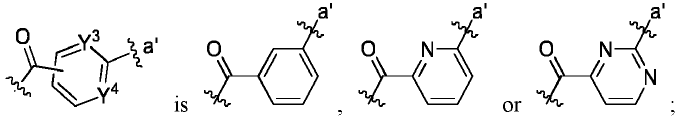
A' is 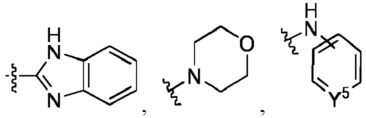 or NO₂;
Y⁵ is CH or N;
scheme 9:
A is 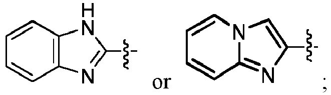 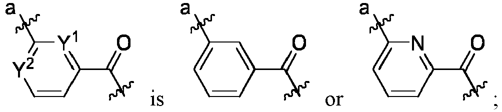
Q is 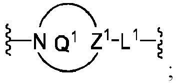 Q¹ is independently 6- to 12-membered spiro heterocycloalkyl; Z¹-L¹- is 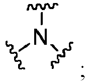 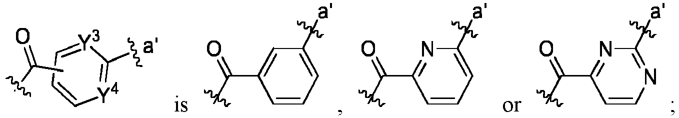
A' is 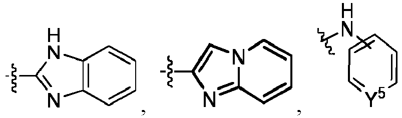 or NO₂;
scheme **10:**
A is 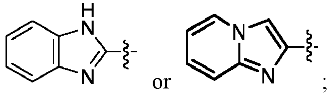 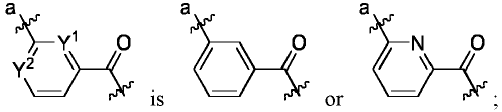
Q is 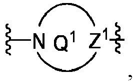 Q¹ is 6- to 10-membered bridged heterocycloalkyl; Z¹-L¹- is 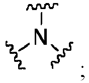 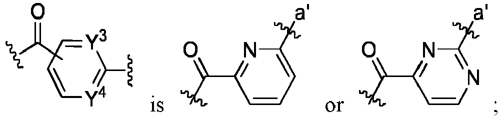
A' is

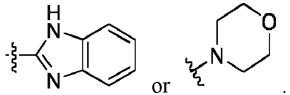

7. The carbonyl heterocyclic compound represented by formula I or the pharmaceutically acceptable salt thereof as claimed in claim 1, wherein, the carbonyl heterocyclic compound represented by formula I is selected from the following group consisting of:

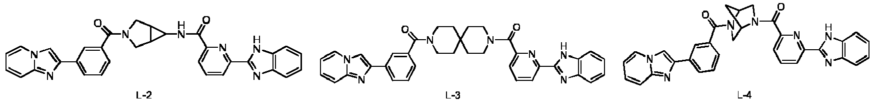

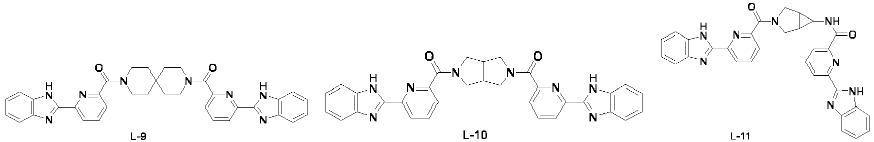

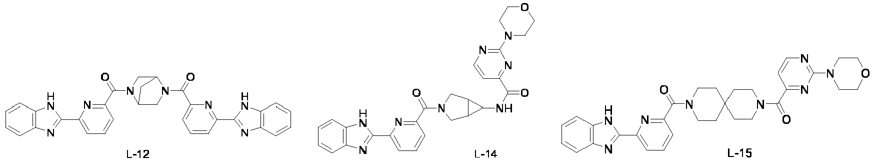

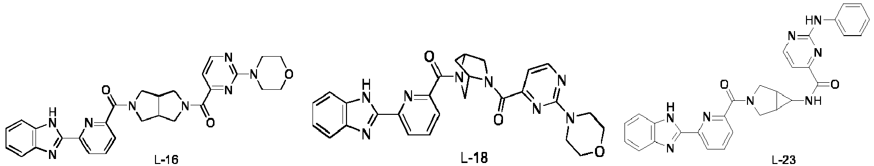

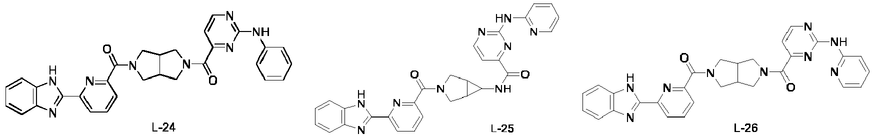

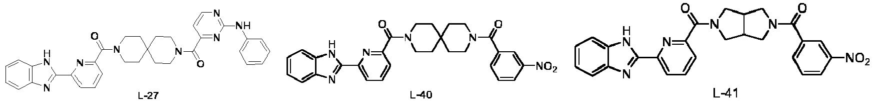

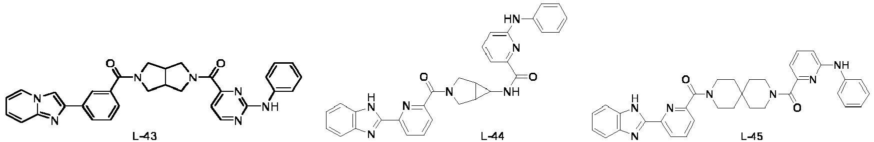

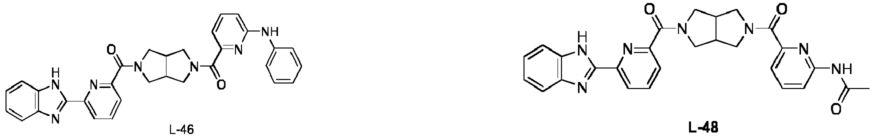

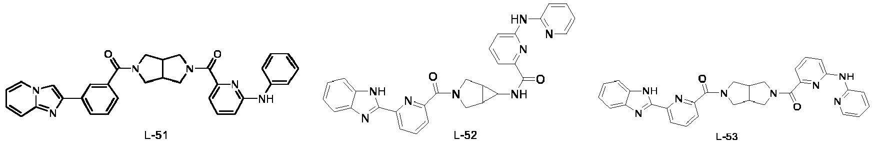

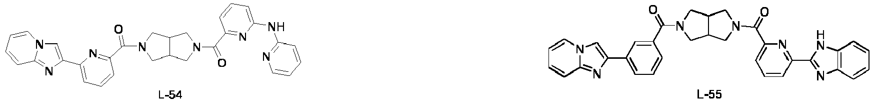

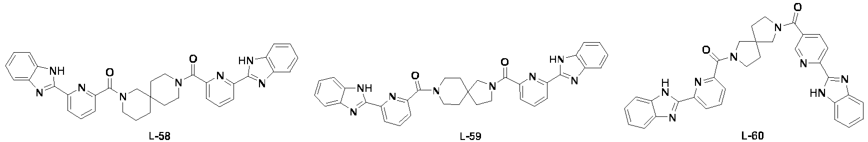

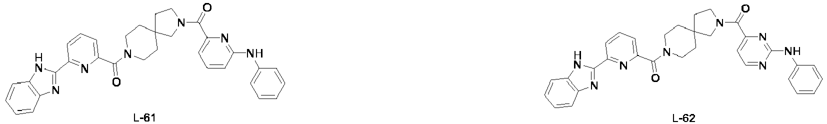

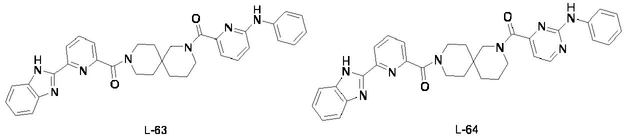

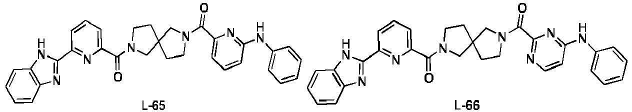

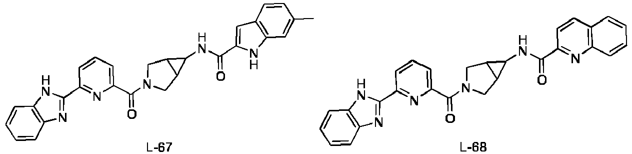

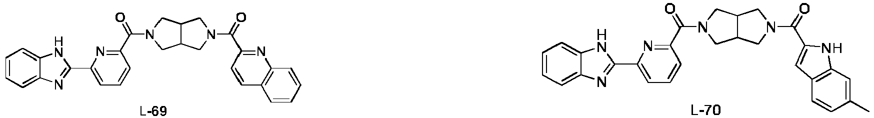

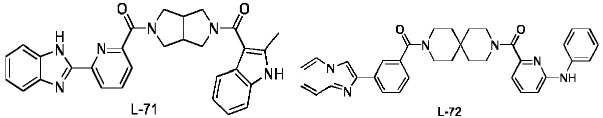

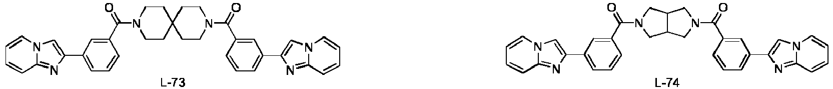

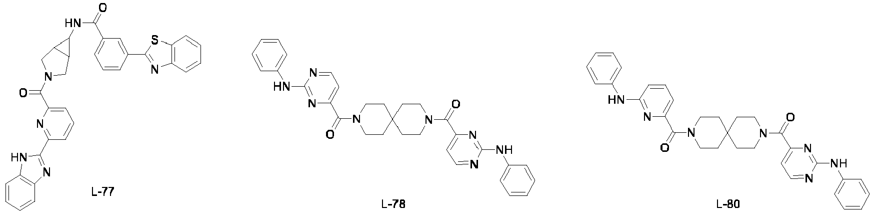

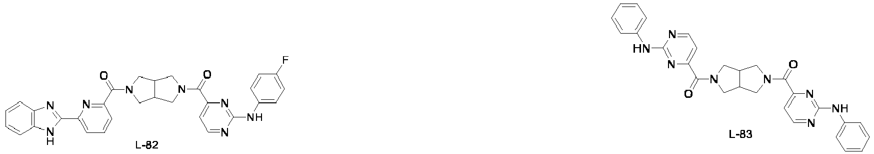

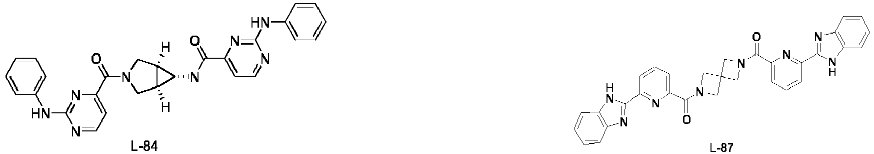

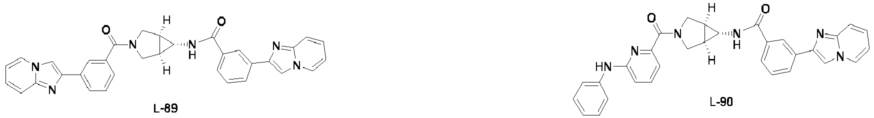

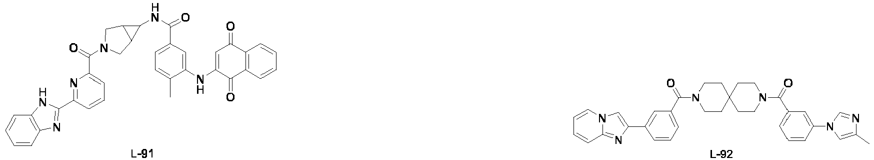

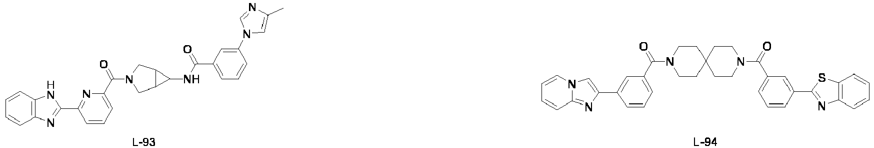

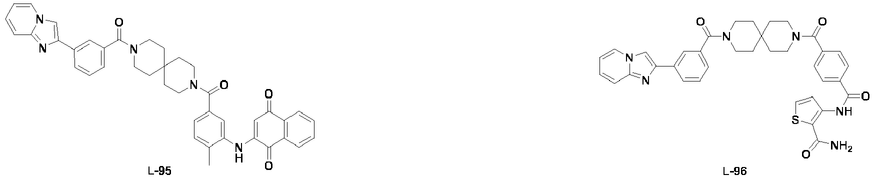

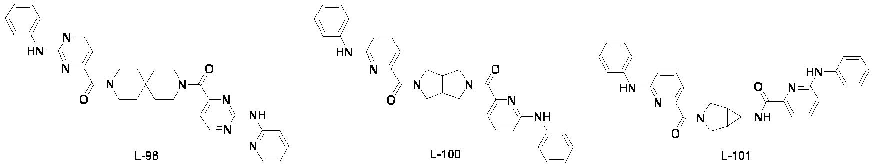

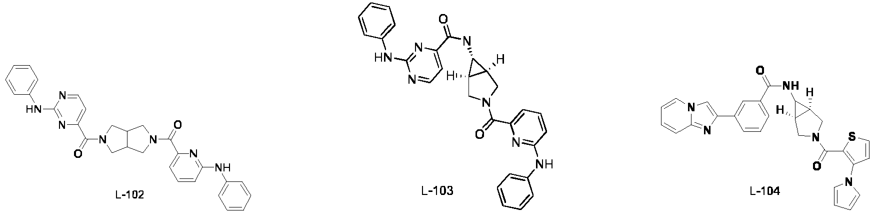

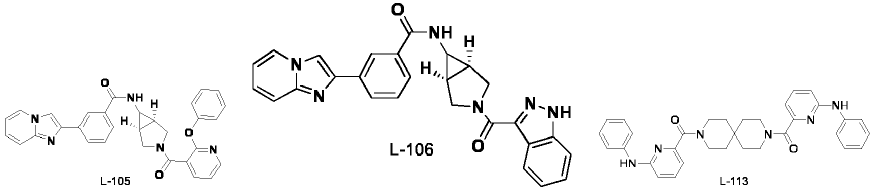

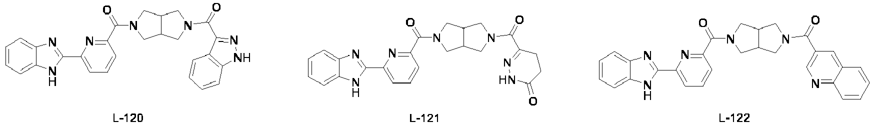

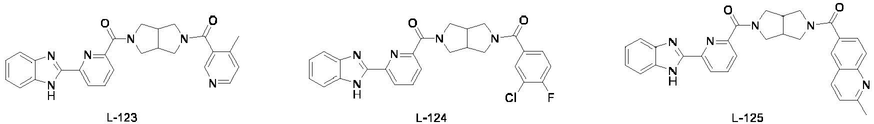

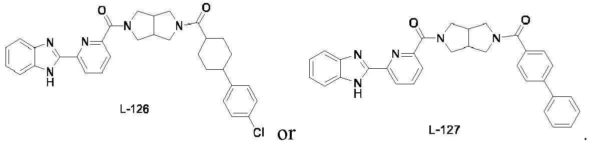

8. A pharmaceutical composition, comprising the carbonyl heterocyclic compound represented by formula I or the pharmaceutically acceptable salt thereof as claimed in any one of claims 1 to 7, and one or more than one of pharmaceutically acceptable carriers.

9. A use of the carbonyl heterocyclic compound represented by formula I, the pharmaceutically acceptable salt thereof as claimed in any one of claims 1 to 7, or the pharmaceutical composition as claimed in claim 8 in the manufacture of an agonist of lanthionine C-like protein 2.

10. A use of the carbonyl heterocyclic compound represented by formula I, the pharmaceutically acceptable salt thereof as claimed in any one of claims 1 to 7, or the pharmaceutical composition as claimed in claim 8 in the manufacture of a medicament; the medicament can be the medicament used for the prevention and/or treatment of diseases related to lanthionine C-like protein 2, and/or, the medicament can be the medicament used for the prevention and/or treatment of autoimmune, chronic inflammatory, chronic metabolic or infectious diseases.

11. The use as claimed in claim 10, wherein,
the diseases related to lanthionine C-like protein 2 are one or more than one of autoimmune, chronic inflammatory, chronic metabolic and infectious diseases;
and/or, the autoimmune diseases are inflammatory bowel disease, systemic lupus, rheumatoid arthritis, type 1 diabetes, psoriasis, multiple sclerosis;
and/or, the chronic metabolic diseases are metabolic syndrome, obesity, prediabetes, cardiovascular disease, and type 2 diabetes;
and/or, the infectious diseases are viral diseases.
